(19) Europäisches Patentamt
European Patent Office
Office européen des brevets

(11) EP 1 145 714 A1

(12) **EUROPEAN PATENT APPLICATION**
published in accordance with Art. 158(3) EPC

(43) Date of publication:
**17.10.2001 Bulletin 2001/42**

(21) Application number: **99972920.5**

(22) Date of filing: **25.11.1999**

(51) Int Cl.[7]: **A61K 31/4375**, A61K 31/4985, A61K 45/00, A61P 1/08

(86) International application number:
**PCT/JP99/06569**

(87) International publication number:
**WO 00/32192 (08.06.2000 Gazette 2000/23)**

(84) Designated Contracting States:
**AT BE CH CY DE DK ES FI FR GB GR IE IT LI LU MC NL PT SE**
Designated Extension States:
**AL LT LV MK RO SI**

(30) Priority: **27.11.1998 JP 33743898**
**19.01.1999 JP 1090799**

(71) Applicant: **Takeda Chemical Industries, Ltd.**
**Osaka-shi, Osaka 541-0045 (JP)**

(72) Inventors:
- **DOI, Takayuki**
  **Izumi-shi, Osaka 594-0013 (JP)**
- **YAMAMOTO, Masaki**
  **Nishinomiya-shi, Hyogo 662-0811 (JP)**
- **FUKUI, Hideo**
  **Fort Lee, NJ 07024 (US)**

(74) Representative:
**von Kreisler, Alek, Dipl.-Chem. et al**
**Patentanwälte**
**von Kreisler-Selting-Werner**
**Postfach 10 22 41**
**50462 Köln (DE)**

(54) **DRUGS**

(57) The present invention relates to a medicine which comprises a compound (I) of the formula:

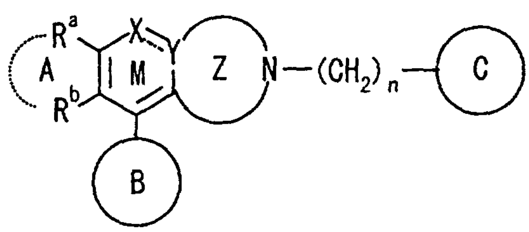

[wherein the ring M is a heterocylic ring having -N=C<, -CO-N< or -CS-N< as a partial structure —X=Y<,
$R^a$ and $R^b$ are bound to each other to form the ring A, or they are the same or different each representing a hydrogen atom or a substituent on the ring M;
the rings A and B each is an optionally substituted homocycle or heterocycle, and at least one of them is an optionally substituted heterocycle;
the ring C is an optionally substituted homocycle or heterocycle;
the ring Z is an optionally substituted nitrogen-containing heterocycle; and
n is an integer of 1 to 6]

or a salt thereof in combination with a drug having an emetic action.

The compounds (I) or their salts are useful as anti-emetic drugs. Particularly, vomiting caused by drugs having an emetic action can be inhibited by these compounds rapidly and safely at a low dose.

EP 1 145 714 A1

**Description**

Technical Field

The present invention relates to medicines which comprise the following compounds (I) or salts thereof having a potent tachykinin receptor antagonizing effect in combination with a drug having an emetic action.

Background Art

Nausea and vomiting caused by administration of drugs having an emetic action such as anti-cancer drugs, opioid analgesics, etc., are the most serious adverse reaction for patients.

Since 5-$HT_3$ antagonists were found to have a potent effect on vomiting caused by an anti-cancer drug, they have been used widely. In recent years, however, it was found that the 5-$HT_3$ antagonists are poorly effective on delayed vomiting generated over several days from two days after administration of anti-cancer drugs. This produced a new problem.

Although an analgesic morphine has widely been used for patients suffering from pains caused by cancer, the effectiveness of any anti-emetic drugs including 5-$HT_3$ antagonists on vomiting caused by morphine is not clear. This is a primary factor of decrease of the QOL (Quality of Life) of patients.

Since apomorphine was reported to have an effect in improving impotence or erectile dysfunction, it has been expected to develop such drugs though they are accompanied with an undesired side effect such as vomiting in many cases. Combination with known anti-emetic drugs (e.g., nicotine, domperidone, etc.) was unsuccessful in sufficiently suppressing the side effect such as vomiting though it was attempted in various ways.

As described above, drugs having an emetic action as side effect decrease the QOL of patients, and the range of their application is limited as well.

In such a situation, recently, a tachykinin antagonist was reported effective in treatment of various kinds of vomiting (Japanese Unexamined Patent Publication (hereinafter referred to as JP-A) 6-107563/1994). In this publication, however, there is no description on condensed heterocyclic compounds having as a basic skeleton a partial chemical structure represented by the formula:

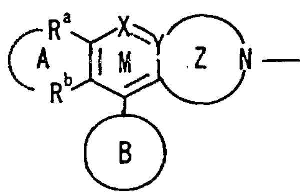

[wherein the ring M is a heterocylic ring having -N=C<, -CO-N< or -CS-N< as a partial structure —X=Y<;
$R^a$ and $R^b$ are bound to each other to form the ring A, or they are the same or different each representing a hydrogen atom or a substituent on the ring M;
the rings A and B each is an optionally substituted homocycle or heterocycle, and at least one of them is an optionally substituted heterocycle; and
the ring Z is an optionally substituted nitrogen-containing heterocycle]

The purpose of the invention is to provide medicines which can widely be applied to vomiting caused by drugs having an emetic action.

Disclosure of Invention

In view of the above situation, the present inventors worked assiduously to investigate and unexpectedly found that condensed heterocyclic compounds having as a basic skeleton a partial chemical structure represented by the formula (JP-A 9-263585):

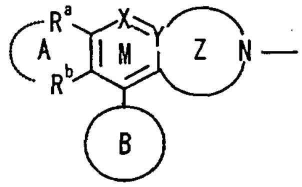

[wherein each symbol has the same meanings as described above] show a potent inhibitory effect on vomiting caused by drugs having an emetic action, which effect is highly satisfactory for use as medicines. The invention was completed on the basis of these findings.

That is, the invention relates to:

[1] A medicine which comprises a compound (I) of the formula:

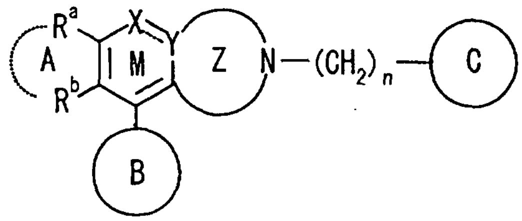

[wherein the ring M is a heterocylic ring having -N=C<, -CO-N< or -CS-N< as a partial structure —X=Y<;
$R^a$ and $R^b$ are bound to each other to form the ring A, or they are the same or different each representing a hydrogen atom or a substituent on the ring M;
the rings A and B each is an optionally substituted homocycle or heterocycle, and at least one of them is an optionally substituted heterocycle;
the ring C is an optionally substituted homocycle or heterocycle;
the ring Z is an optionally substituted nitrogen-containing heterocycle; and
n is an integer of 1 to 6]
or a salt thereof in combination with a drug having an emetic action;

[2]A medicine as described in the above item [1], wherein $R^a$ and $R^b$ each is a hydrogen atom or substituent selected from

(1) halogen atom;
(2) $C_{1-6}$ alkyl group which may have 1 - 5 substituents selected from (i) hydroxyl group, (ii) $C_{1-6}$ alkoxy group, (iii) $C_{1-6}$ alkylthio group, (iv) amino group, (v) $C_{1-7}$ acylamino group, (vi) carboxyl group, (vii) nitro group, (viii) mono- or di-$C_{1-6}$ alkylamino group, (ix) mono- or di-$C_{3-8}$ cycloalkylamino group, (x) $C_{6-10}$ arylamino group, (xi) 5- to 9-membered cyclic amino group which may be substituted by $C_{1-6}$ alkyl and may contain 1 - 3 heteroatoms selected from oxygen atom and sulfur atom in addition to the nitrogen atom, (xii) 5- or 6-membered aromatic heterocyclic group which contains 1 - 3 heteroatoms selected from nitrogen atom, oxygen atom and sulfur atom in addition to the carbon atom, (xiii) 5- to 9-membered non-aromatic heterocyclic group which contains 1 - 3 heteroatoms selected from nitrogen atom, oxygen atom and sulfur atom in addition to the carbon atom, (xiv) $C_{1-4}$ alkylsulfonylamino group, (xv) $C_{1-6}$ alkyl-carbonyloxy group, and (xvi) halogen atom;
(3) optionally halogenated $C_{1-6}$ alkoxy group;
(4) optionally halogenated $C_{1-6}$ alkylthio group;
(5) $C_{3-10}$ cycloalkyl group;
(6) $C_{6-10}$ aryl group;
(7) $C_{1-7}$ acylamino group;
(8) $C_{1-3}$ acyloxy group;
(9) hydroxyl group;
(10) nitro group;
(11) cyano group;
(12) amino group;
(13) mono- or di-$C_{1-6}$ alkylamino group;

(14) 5- to 9-membered cyclic amino group which may be substituted by $C_{1-6}$ alkyl and may contain 1 - 3 heteroatoms selected from oxygen atom and sulfur atom in addition to the nitrogen atom;
(15) $C_{1-6}$ alkyl-carbonylamino group;
(16) $C_{1-6}$ alkyl-sulfonylamino group;
(17) $C_{1-6}$ alkoxy-carbonyl group;
(18) carboxyl group;
(19) $C_{1-6}$ alkyl-carbonyl group;
(20) carbamoyl group;
(21) mono- or di-$C_{1-6}$ alkylcarbamoyl group; and
(22) $C_{1-6}$ alkylsulfonyl group;

or $R^a$ and $R^b$ are bound to each other to form the ring A, wherein the ring A represents (i) 5- to 9-membered aromatic heterocycle which contains 1 to 3 of 1 or 2 kinds of heteroatoms selected from nitrogen atom, sulfur atom and oxygen atom in addition to the carbon atom; (ii) 5- to 9-membered non-aromatic heterocycle which contains 1 to 3 of 1 or 2 kinds of heteroatoms selected from nitrogen atom, sulfur atom and oxygen atom in addition to the carbon atom; or (iii) 3- to 10-membered cyclic hydrocarbon, which may be substituted by 1 to 4 substituents selected from:

(1) halogen atom;
(2) $C_{1-6}$ alkyl group which may have 1 - 5 substituents selected from (i) hydroxyl group, (ii) amino group, (iii) carboxyl group, (iv) nitro group, (v) mono- or di-$C_{1-6}$ alkylamino group, (vi) $C_{1-6}$ alkyl-carbonyloxy group, and (vii) halogen atom;
(3) optionally halogenated $C_{1-6}$ alkoxy group;
(4) optionally halogenated $C_{1-6}$ alkylthio group;
(5) $C_{6-10}$ aryl group;
(6) $C_{1-7}$ acylamino group;
(7) $C_{1-3}$ acyloxy group;
(8) hydroxyl group;
(9) nitro group;
(10) cyano group;
(11) amino group;
(12) mono- or di-$C_{1-6}$ alkylamino group;
(13) 5- to 9-membered cyclic amino group which may contain 1 - 3 heteroatoms selected from oxygen atom and sulfur atom in addition to the nitrogen atom;
(14) $C_{1-6}$ alkyl-carbonylamino group;
(15) $C_{1-6}$ alkyl-sulfonylamino group;
(16) $C_{1-6}$ alkoxy-carbonyl group;
(17) carboxyl group;
(18) $C_{1-6}$ alkylcarbonyl group;
(19) carbamoyl group;
(20) mono- or di-$C_{1-6}$ alkylcarbamoyl group;
(21) $C_{1-6}$ alkylsulfonyl group; and
(22) oxo group;

the ring B represents (i) 5- to 9-membered aromatic heterocycle which contains 1 to 3 of 1 or 2 kinds of heteroatoms selected from nitrogen atom, sulfur atom and oxygen atom in addition to the carbon atom; (ii) 5- to 9-membered non-aromatic heterocycle which contains 1 to 3 of 1 or 2 kinds of heteroatoms selected from nitrogen atom, sulfur atom and oxygen atom in addition to the carbon atom; or (iii) 3- to 10-membered cyclic hydrocarbon, which may be substituted by 1 to 4 substituents selected from:

(1) halogen atom;
(2) $C_{1-6}$ alkyl group which may have 1 - 5 substituents selected from (i) hydroxyl group, (ii) amino group, (iii) carboxyl group, (iv) nitro group, (v) mono- or di-$C_{1-6}$ alkylamino group, (vi) $C_{1-6}$ alkyl-carbonyloxy group, and (vii) halogen atom;
(3) optionally halogenated $C_{1-6}$ alkoxy group;
(4) optionally halogenated $C_{1-6}$ alkylthio group;
(5) $C_{6-10}$ aryl group;
(6) $C_{1-7}$ acylamino group;

(7) $C_{1-3}$ acyloxy group;
(8) hydroxyl group;
(9) nitro group;
(10) cyano group;
(11) amino group;
(12) mono- or di-$C_{1-6}$ alkylamino group;
(13) 5- to 9-membered cyclic amino group which may contain 1 - 3 heteroatoms selected from oxygen atom and sulfur atom in addition to the nitrogen atom;
(14) $C_{1-6}$ alkyl-carbonylamino group;
(15) $C_{1-6}$ alkyl-sulfonylamino group;
(16) $C_{1-6}$ alkoxy-carbonyl group;
(17) carboxyl group;
(18) $C_{1-6}$ alkylcarbonyl group;
(19) carbamoyl group;
(20) mono- or di-$C_{1-6}$ alkylcarbamoyl group;
(21) $C_{1-6}$ alkylsulfonyl group; and
(22) oxo group;

the ring C represents a 5- to 9-membered heterocycle which contains 1 to 3 of 1 or 2 kinds of heteroatoms selected from nitrogen atom, sulfur atom and oxygen atom in addition to the carbon atoms, and which may be substituted by 1 to 5 substituents selected from:

(1) halogen atom;
(2) optionally halogenated $C_{1-10}$ alkyl group;
(3) $C_{1-4}$ alkyl group substituted by amino group;
(4) $C_{1-4}$ alkyl group substituted by mono- or di-$C_{1-4}$ alkylamino group;
(5) $C_{1-4}$ alkyl group substituted by carboxyl group;
(6) $C_{1-4}$ alkyl group substituted by $C_{1-4}$ alkoxy-carbonyl group;
(7) $C_{1-4}$ alkyl group substituted by hydroxyl group;
(8) $C_{3-10}$ cycloalkyl group;
(9) nitro group;
(10) cyano group;
(11) hydroxyl gorup;
(12) optionally halogenated $C_{1-10}$ alkoxy group;
(13) optionally halogenated $C_{1-4}$ alkylthio group;
(14) amino group;
(15) mono- or di-$C_{1-4}$ alkylamino group;
(16) 5- to 9-membered cyclic amino group which may contain 1 - 3 heteroatoms selected from oxygen atom and sulfur atom in addition to the nitrogen atom;
(17) $C_{1-4}$ alkyl-carbonylamino group;
(18) aminocarbonyloxy group;
(19) mono- or di-$C_{1-4}$ alkylaminocarbonyloxy group;
(20) $C_{1-4}$ alkylsulfonylamino group;
(21) $C_{1-4}$ alkoxy-carbonyl group;
(22) aralkyloxycarbonyl group;
(23) carboxyl group;
(24) $C_{1-6}$ alkyl-carbonyl group;
(25) $C_{3-6}$ cycloalkyl-carbonyl group;
(26) carbamoyl group;
(27) mono- or di-$C_{1-4}$ alkylcarbamoyl group;
(28) $C_{1-6}$ alkylsulfonyl group; and
(29) 5- or 6-membered aromatic monocyclic heterocycle which contains 1 to 4 of 1 or 2 kinds of heteroatoms selected from nitrogen atom, sulfur atom and oxygen atom in addition to the carbon atom and which may be substituted by 1 to 3 of optionally halogenated $C_{1-4}$ alkyl groups;

or a 3- to 10-membered homocycle which may be substituted by 1 to 5 substituents selected from:

(1) halogen atom;

(2) optionally halogenated $C_{1-10}$ alkyl group;
(3) $C_{1-4}$ alkyl group substituted by amino group;
(4) $C_{1-4}$ alkyl group substituted by mono- or di-$C_{1-4}$ alkylamino group;
(5) $C_{1-4}$ alkyl group substituted by carboxyl group;
(6) $C_{1-4}$ alkyl group substituted by $C_{1-4}$ alkoxy-carbonyl group;
(7) $C_{1-4}$ alkyl group substituted by hydroxyl group;
(8) $C_{3-10}$ cycloalkyl group;
(9) nitro group;
(10) cyano group;
(11) hydroxyl group;
(12) optionally halogenated $C_{1-10}$ alkoxy group;
(13) optionally halogenated $C_{1-4}$ alkylthio group;
(14) amino group;
(15) mono- or di-$C_{1-4}$ alkylamino group;
(16) 5- to 9-membered cyclic amino group which may contain 1 - 3 heteroatoms selected from oxygen atom and sulfur atom in addition to the nitrogen atom;
(17) $C_{1-4}$ alkyl-carbonylamino group;
(18) aminocarbonyloxy group;
(19) mono- or di-$C_{1-4}$ alkylaminocarbonyloxy group;
(20) $C_{1-4}$ alkylsulfonylamino group;
(21) $C_{1-4}$ alkoxy-carbonyl group;
(22) aralkyloxycarbonyl group;
(23) carboxyl group;
(24) $C_{1-6}$ alkyl-carbonyl group;
(25) $C_{3-6}$ cycloalkyl-carbonyl group;
(26) carbamoyl group;
(27) mono- or di-$C_{1-4}$ alkylcarbamoyl group;
(28) $C_{1-6}$ alkylsulfonyl group; and
(29) 5- or 6-membered aromatic monocyclic heterocycle which contains 1 to 4 of 1 or 2 kinds of heteroatoms selected from nitrogen atom, sulfur atom and oxygen atom in addition to the carbon atom and which may be substituted by 1 to 3 of optionally halogenated $C_{1-4}$ alkyl groups; and

the ring Z represents a 5- to 12-membered heterocycle which may contain at least one heteroatom selected from nitrogen atom, oxygen atom and sulfur atom in addition to Y and the carbon atom and the nitrogen atom and which may be substituted by 1 to 5 substituents selected from:

(1) $C_{1-6}$ alkyl group;
(2) $C_{2-6}$ alkenyl group;
(3) $C_{2-6}$ alkynyl group;
(4) $C_{3-8}$ cycloalkyl group;
(5) $C_{3-8}$ cycloalkyl-$C_{1-4}$ alkyl group;
(6) $C_{6-14}$ aryl group;
(7) nitro group;
(8) cyano group;
(9) hydroxyl group;
(10) $C_{1-4}$ alkoxy group;
(11) $C_{1-4}$ alkylthio group;
(12) amino group;
(13) mono- or di-$C_{1-4}$ alkylamino group;
(14) 5- to 9-membered cyclic amino group which may contain 1 - 3 heteroatoms selected from oxygen atom and sulfur atom in addition to the nitrogen atom;
(15) $C_{1-4}$ alkyl-carbonylamino group;
(16) $C_{1-4}$ alkylsulfonylamino group;
(17) $C_{1-4}$ alkoxy-carbonyl group;
(18) carboxyl group;
(19) $C_{1-6}$ alkyl-carbonyl group;
(20) carbamoyl group;
(21) mono- or di-$C_{1-4}$ alkylcarbamoyl group;

(22) $C_{1-6}$ alkylsulfonyl group;
(23) oxo group; and
(24) thioxo group;

[3] A medicine as described in the above item [1], wherein $R^a$ and $R^b$ are bound to each other to form the ring A, the ring C is an optionally substituted benzene ring or optionally substituted heterocycle, the ring Z is an optionally oxo-substituted nitrogen-containing heterocycle, and n is 1 or 2;
[4] A medicine as described in the above item [1], wherein the ring Z is an optionally oxo-substituted nitrogen-containing heterocycle;
[5] A medicine as described in the above item [1], wherein one of the rings A and B is an optionally substituted aromatic ring, and the other is an optionally substituted aromatic heterocycle;
[6] A medicine as described in the above item [1], wherein the ring A is an optionally substituted aromatic heterocycle, and the ring B is an optionally substituted benzene ring;
[7] A medicine as described in the above item [5], wherein the aromatic heterocycle is a 5- or 6-membered aromatic heterocycle which contains 1 or 2 kinds of heteroatoms selected from nitrogen atom, sulfur atom and oxygen atom in addition to the carbon atom;
[8] A medicine as described in the above item [1], wherein the ring C is an optionally substituted benzene ring;
[9] A medicine as described in the above item [1], wherein the ring C is a benzene ring which contains 1 to 3 substituents selected from halogen atom; optionally halogenated $C_{1-6}$ alkyl group and optionally halogenated $C_{1-6}$ alkoxy group;
[10] A medicine as described in the above item [1], wherein the ring Z is a 5- to 10-membered ring which may be substituted by 1 or 2 oxo groups;
[11] A medicine as described in the above item [1], wherein —X=Y⟨ is -CO-N< or -N=C<;
[12] A medicine as described in the above item [1], wherein n is 1;
[13]A medicine as described in the above item [1], wherein the ring A is an optionally substituted pyridine ring, the ring B is an optionally substituted benzene ring, the ring C is an optionally substituted benzene ring, the ring Z is an optionally oxo-substituted 5- to 10-membered ring, —X=Y⟨ is -CO-N< or -N=C<, and n is 1;
[14]A medicine as described in the above item [1], wherein $R^a$ and $R^b$ are the same or different each representing hydrogen atom, halogen atom, optionally substituted alkyl group, optionally halogenated alkoxy group, optionally halogenated alkylthio group, cycloalkyl group, aryl group, acylamino group, acyloxy group, hydroxyl group, nitro group, cyano group, amino group, mono- or di-alkylamino group, cyclic amino group, alkyl-carbonylamino group, alkylsulfonylamino group, alkoxycarbonyl group, carboxyl group, alkylcarbonyl group, carbamoyl group, mono- or di-alkylcarbamoyl group, alkylsulfonyl group or oxo group;
[15] A medicine as described in the above item [1], wherein $R^a$ and $R^b$ are the same or different each representing hydrogen atom or $C_{1-6}$ alkyl group which may be substituted by (i) $C_{1-6}$ alkoxy group, (ii) $C_{1-6}$ alkylthio group, (iii) amino group, (iv) $C_{1-7}$ acylamino group, (v) mono- or di-$C_{1-6}$ alkylamino group, (vi) $C_{3-10}$ cyclic amino group, (vii) 5- or 6-membered cyclic amino group which may be substituted by $C_{1-6}$ alkyl group, (viii) $C_{1-6}$ alkylsulfonylamino group or (ix) $C_{1-6}$ alkylcarbonyloxy group; or $R^a$ and $R^b$ are bound to each other to form a pyridine ring which may have 1 to 3 substituents selected from halogen atom and $C_{1-4}$ alkyl group;
the ring B is a benzene ring which may have 1 to 3 substituents selected from (i) halogen atom, (ii) optionally halogenated $C_{1-4}$ alkyl group, and (iii) optionally halogenated $C_{1-4}$ alkoxy group; the ring C is a benzene ring which may have 1 to 3 substituents selected from (i) halogen atom, (ii) optionally halogenated $C_{1-4}$ alkyl group, (iii) optionally halogenated $C_{1-4}$ alkoxy group, (iv) amino group which may be substituted by $C_{1-4}$ alkyl group, (v) $C_{1-3}$ acyloxy group, and (vi) hydroxyl group;
the ring Z is a 5- to 10-membered nitrogen-containing heterocycle which may be substituted by $C_{1-4}$ alkyl group or hydroxyl group and may be oxo-substituted;
—X=Y⟨ is -CO-N< or -N=C<; and
n is 1;
[16] A medicine as described in the above item [15], wherein $R^a$ and $R^b$ are bound to each other to form a pyridine ring which may contain 1 to 3 substituents selected from halogen atom and $C_{1-4}$ alkyl group; and
-X=Y⟨ is -CO-N<
[17] A medicine as described in the above item [16], wherein the pyridine ring is an unsubstituted pyridine ring;
[18]A medicine as described in the above item [15], wherein the ring B is a benzene ring which may have 1 to 3 of optionally halogentated $C_{1-4}$ alkyl groups;
[19] A medicine as described in the above item [15], wherein the ring C is a benzene ring which may have 1 to 3 substituents selected from (i) halogen atom, (ii) optionally halogentated $C_{1-4}$ alkyl group, and (iii) optionally halogentated $C_{1-4}$ alkoxy group;
[20]A medicine as described in the above item [15], wherein the ring Z is represented by the formula:

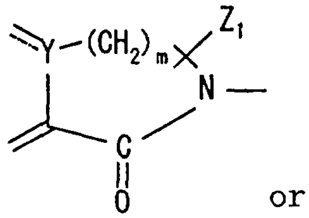

or

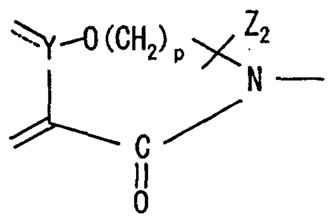

[wherein m and p are the same or different each indicating an integer of 1 to 5; $Z_1$ and $Z_2$ are the same or different each representing a hydrogen atom, $C_{1-4}$ alkyl group or hydroxyl group; and Y has the same meanings as described in the above item [15]] ;

[21] A medicine as described in the above item [1], wherein the compound (I) is (9S)-7-[3,5-bis(trifluoromethyl) benzyl]-6,7,8,9,10,12-hexahydro-9-methyl-6,12-dioxo-5-phenyl [1,4]-diazepino[2,1-g] [1,7]naphthyridine;

[22] A medicine as described in the above item [1], wherein the compound (I) is (9S)-7-[3,5-bis(trifluoromethyl) benzyl]-6,7,8,9,10,12-hexahydro-9-methyl-5-(4-methylphenyl)-6,12-dioxo-[1,4]diazepino[2,1-g] [1,7]naphthyridine;

[23] A medicine as described in the above item [1], wherein the compound (I) is (9R)-7-[3,5-bis(trifluoromethyl) benzyl]-6,7,8,9,10,11-hexahydro-9-methyl-6,13-dioxo-5-phenyl-13H-[1,4]diazocino[2,1-g][1,7]naphthyridine;

[24]A medicine as described in the above item [1], wherein the compound (I) is (9R)-7-[3,5-bis(trifluoromethyl) benzyl]-6,7,8,9,10,11-hexahydro-9-methyl-5-(4-methylphenyl)-6,13-dioxo-13H-[1,4]diazocino[2,1-g][1,7]naphthyridine;

[25] A medicine as described in the above item [1], wherein the compound (I) is (9R)-7-(3,5-dimethoxybenzyl)-5-(4-fluorophenyl)-6,7,8,9,10,11-hexahydro-9-methyl-6,13-dioxo-13H-[1,4]diazocino[2,1-g][1,7]naphthyridine;

[26]A medicine as described in the above item [1], wherein the compound (I) is (9R)-7-(3,5-dimethoxybenzyl)-6,7,8,9,10,11-hexahydro-9-methyl-5-(4-methylphenyl)-6,13-dioxo-13H-[1,4]diazocino[2,1-g][1,7]naphthyridine;

[27]A medicine as described in the above item [1], wherein the drug having an emetic action is an anti-cancer drug;

[28]A medicine as described in the above item [1], wherein the drug having an emetic action is morphine or a derivative thereof or a salt thereof;

[29] A medicine as described in the above item [1], wherein the drug having an emetic action is apomorphine or a salt thereof;

[30] A medicine as described in the above item [28], of which an orally applicable preparation comprises the compound (I) or a salt thereof;

[31] A medicine as described in the above item [28], of which a sustained release preparation comprises morphine or a derivative thereof or a salt thereof;

[32] A medicine as described in the above item [28], of which an orally applicable preparation comprises morphine or a derivative thereof or a salt thereof;

[33] A medicine as described in the above item [32], of which the orally applicable preparation is sublingual tablets or buccals;

[34]A medicine as described in the above item [32], of which the orally applicable preparation is orally rapidly disintegrating preparation;

[35] A medicine as described in the above item [28], which comprises an orally applicable preparation comprising the compound (I) or a salt thereof, in combination with an injection or orally applicable preparation comprising morphine or a derivative thereof or a salt thereof;

[36] A medicine as described in the above item [1], which comprises further combining with a serotonin antagonist and/or glucocorticoid;

[37]A medicine which comprises the compound (I) represented by the formula:

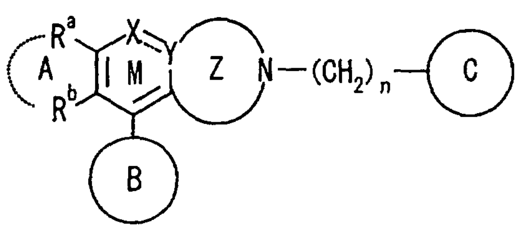

[wherein the ring M is a heterocylic ring having -N=C<, -CO-N< or -CS-N< as a partial structure —X=Y<;

$R^a$ and $R^b$ are bound to each other to form the ring A, or they are the same or different each representing a hydrogen atom or substituent on the ring M;
the rings A and B each is an optionally substituted homocycle or heterocycle, and at least one of them is an optionally substituted heterocycle;
the ring C is an optionally substituted homocycle or heterocycle;
the ring Z is an optionally substituted nitrogen-containing heterocycle; and
n is an integer of 1 to 6]
or a salt thereof and a drug having an emetic action;
[38] Use of the compound (I) represented by the formula:

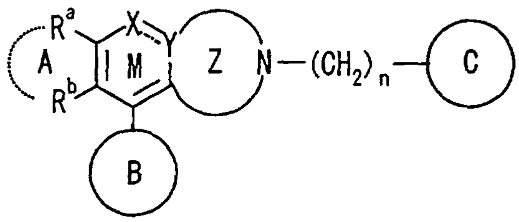

[wherein the ring M is a heterocylic ring having -N=C<, -CO-N< or -CS-N< as a partial structure —X=Y<;
$R^a$ and $R^b$ are bound to each other to form the ring A, or they are the same or different each representing a hydrogen atom or substituent on the ring M;
the rings A and B each is an optionally substituted homocycle or heterocycle, and at least one of them is an optionally substituted heterocycle;
the ring C is an optionally substituted homocycle or heterocycle;
the ring Z is an optionally substituted nitrogen-containing heterocycle; and
n is an integer of 1 to 6]
or a salt thereof for preparing a drug for inhibiting vomiting caused by a drug having an emetic action; and
[39] A method for inhibiting vomiting caused by a drug having an emetic action, which comprises administering the compound (I) represented by the formula:

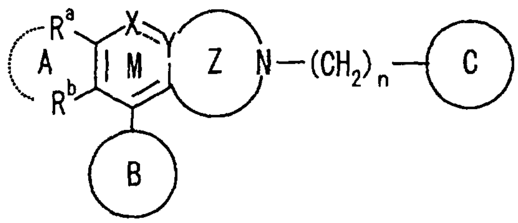

[wherein the ring M is a heterocylic ring having -N=C<, -CO-N< or -CS-N< as a partial structure —X=Y<;
$R^a$ and $R^b$ are bound to each other to form the ring A, or they are the same or different each representing a hydrogen atom or substituent on the ring M;
the rings A and B each is an optionally substituted homocycle or heterocycle, and at least one of them is an optionally substituted heterocycle;
the ring C is an optionally substituted homocycle or heterocycle;
the ring Z is an optionally substituted nitrogen-containing heterocycle; and
n is an integer of 1 to 6]
or a salt thereof to a mammal to whom said drug having an emetic action will be administered or has been administered.

In addition, the compounds (I) or their salts include compounds represented by the formula (Ia):

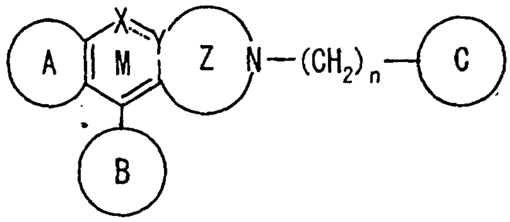

(Ia)

[wherein each symbol has the same meanings as described above] or their salts, in which $R^a$ and $R^b$ are bound to each other to form the ring A.

Brief Description of Drawings

Figure 1 shows patterns of acute retching and vomiting when 10 mg/kg of cisplatin was administered intraperitoneally to ferrets. The result shows the frequency of the retching and vomiting every 20 minutes throughout the time of observation as mean ± standard error.

Figure 2 shows the effect of oral administration of Compoud No. 3 ((9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g] [1,7]naphthyridine) on vomiting induced by cisplatin in ferrets. The result shows the frequency of the retching and vomiting throughout the time of observation as mean ± standard error.

Figure 3 shows a pattern of vomiting when 3 mg/kg of cisplatin was intravenously administered to dogs. The result shows the frequency of the vomiting every 10 minutes throughout the time of observation as mean ± standard error.

Figure 4 shows the effect of oral administration of Compound No. 3 on vomiting induced by cisplatin in dogs. The result shows the frequency of the vomiting throughout the time of observation as mean ± standard error.

Figure 5 shows the effect of intravenous administration of Compound No. 3 on vomiting induced by cisplatin in dogs. The result shows the frequency of the vomiting throughout the time of observation as mean ± standard error.

Figure 6 shows patterns of retching and vomiting when 5 mg/kg of cisplatin was administered intraperitoneally to ferrets. In addition, Figure 6 also shows the effect of Compound No. 3 administered twice a day on the retching and vomiting.

Figure 7 shows the effects of Compound No. 3 and ondansetron orally administered twice a day on cisplatin-induced acute and delayed vomiting in ferrets.

Figure 8 shows patterns of retching and vomiting when 5 mg/kg of cisplatin was administered intraperitoneally to ferrets. In addition, Figure 8 also shows the effect of Compound No. 3 administered once a day on the retching and vomiting.

Figure 9 shows the effects of Compound No. 3 orally administered once a day on cisplatin-induced acute and delayed vomitings in ferrets.

Figure 10 shows the effects of oral administration of Compound-No. 3 and ondansetron on MTX-induced delayed vomitings in dogs. The result shows the frequency of the vomiting every 4 hours throughout the time of observation (72 hours) as mean ± standard error.

Figure 11 shows the effects of oral administration of Compound No. 3 and ondansetron on MTX-induced delayed vomiting in dogs. The result shows the frequency of the vomiting throughout the time of observation (72 hours) as mean ± standard error.

Figure 12 shows patterns of retching and vomiting when morphine was administered to ferrets. The result shows the frequency of the vomiting every 10 minutes throughout the time of observation as mean ± standard error.

Figure 13 shows the effects of oral administration of Compound No. 3 and ondansetron on morphine-induced (0.3 mg/kg, subcutaneous) vomiting in ferrets. The result shows the frequency of the vomiting throughout the time of observation as mean ± standard error.

Figure 14 shows the effects of oral administration of Compound No. 3 and ondansetron on loperamide-induced vomiting in dogs. The result shows the frequency of the vomiting every 10 minutes throughout the time of observation as mean ± standard error.

Figure 15 shows the effects of oral administration of Compound No. 3 and ondansetron and the effect of intravenous administration of naloxone on loperamide-induced vomiting in dogs. The result shows the frequency of the vomiting throughout the time of observation as mean ± standard error.

Figure 16 shows the effects of oral administration of Compound No. 3 and ondansetron-on apomorphine-induced vomiting in dogs. The result shows the frequency of the vomiting every 10 minutes throughout the time of observation as mean ± standard error.

Figure 17 shows the effects of oral administration of Compound No. 3 and ondansetron on apomorphine-induced vomiting in dogs. The result shows the frequency of the vomiting throughout the time of observation-as mean ± standard error.

Figure 18 shows the effect of combined administration of Compound No. 3 and ondansetron (Cpd. No. 3 + Ond) on cisplatin-induced acute vomiting in ferrets. The result shows the frequency of retching and vomiting throughout the time of observation (180 minutes after administration of cisplatin) in 6 animals as mean ± standard error (*p<0.05).

Figure 19 shows the effect of combined administration of Compound No. 3/dexamethasone (Cpd. No. 3 + Dex) on cisplatin-induced delayed vomiting in ferrets. The result shows the frequency of retching and vomiting throughout the time of observation (31 - 44 hours after administration of cisplatin) in 6 animals as mean ± standard error (*p<0.05).

Figure 20 shows the effect of combined administration of Compound No. 3/ondansetron/dexamethasone (Drugs) on cisplatin-induced acute vomiting in ferrets. The result shows the frequency of retching and vomiting throughout the time of observation (180 minutes after administration of cisplatin) in 4 animals as mean ± standard error (*p<0.05).

Figure 21 shows the effect of combined administration of Compound No. 3/ondansetron/dexamethasone (Drugs) on cisplatin-induced delayed vomiting in ferrets. The result shows the frequency of retching and vomiting throughout the time of observation (31 - 44 hours after administration of cisplatin) in 4 animals as mean ± standard error (*p<0.05).

## Best Mode for Carrying Out the Invention

The present invention will be explained in detail as follows.

### As to "Ring M, X and Y"

In the above formulae (I) and (Ia), the ring M is a heterocycle having -N=C<, -CO-N< or -CS-N< as a partial structure —X=Y<. Preferably, the ring M has -CO-N< or -N=C< as a partial structure —X=Y<.

### As to "$R^a$ and $R^b$"

In the above formula (I), $R^a$ and $R^b$ are bound to each other to form the ring A, or they are the same or different each representing a hydrogen atom or a substituent on the ring M.

In the ring M, the substituents $R^a$ and $R^b$ include, for example, halogen atom, optionally substituted alkyl group, optionally halogenated alkoxy group, optionally halogenated alkylthio group, cycloalkyl group, aryl group, acylamino group, acyloxy group, hydroxyl group, nitro group, cyano group, amino group, mono- or di-alkylamino group, cyclic amino group (for example, cyclic amino group which may contain (a) heteroatom(s) such as oxygen atom and sulfur atom in addition to the nitrogen atom), alkylcarbonylamino group, alkylsulfonylamino group, alkoxycarbonyl group, carboxyl group, alkylcarbonyl group, carbamoyl group, mono- or di-alkylcarbamoyl group, alkylsulfonyl group, oxo group, etc.

The above-mentioned "halogen atom" includes, for example, fluorine, chlorine, bromine, and iodine atoms. Preferred halogen atom includes, for example, fluorine, chlorine and bromine atoms.

As "optionally substituted alkyl group", for example, $C_{1-6}$ alkyl group (for example, methyl, ethyl, propyl, isopropyl, butyl, isobutyl, sec-butyl, tert-butyl, etc.) which may be substituted by 1 - 5 substituents selected from hydroxyl group, $C_{1-6}$ alkoxy group ($C_{1-4}$ alkoxy group such as methoxy, ethoxy, propoxy, butoxy, isobutoxy, s-butoxy, t-butoxy, etc.), $C_{1-6}$ alkylthio group ($C_{1-4}$ alkylthio group such as methylthio, ethylthio, propylthio, butylthio, isobutylthio, s-butylthio, t-butylthio, etc.), amino group, $C_{1-7}$ acylamino group (formylamino, acetylamino, propionylamino, butyrylamino, benzoylamino, etc.), N-alkylamino group, carboxyl group, nitro group, mono- or di- $C_{1-6}$ alkylamino group (for example, mono- or di-$C_{1-4}$ alkylamino group such as methylamino, ethylamino, propylamino, butylamino, dimethylamino, diethylamino, etc.), N-substituted amino group to which 1 or 2 homocycles are substitued (for example, mono- or di-$C_{3-8}$ cycloalkylamino group such as cyclopropylamino, cyclobutylamino, cyclohexylamino, etc.; $C_{6-10}$ arylamino group such as phenylamino, etc.), optionally substituted heterocyclic group [for example, 5- to 9-membered cyclic amino group which may contain 1 - 3 heteroatoms such as oxygen atom, sulfur atom, etc. in addition to the nitrogen atom (for example, 5- or 6-membered non-aromatic cyclic amino group such as piperidino, 4-methylpiperidino, morpholino, thiomorpholino, piperazinyl, 4-methylpiperazinyl, 4-ethylpiperazinyl, pyrrolidinyl, imidazolidinyl, pyrazolidinyl, etc.; 5- or 6-membered aromatic cyclic amino group such as pyridyl, pyrazyl, pyrimidinyl, pyridazinyl, pyrrolyl, imidazolyl, pyrazolyl, etc.), aromatic heterocyclic group such as thiophenyl, furanyl, thiazole, isothiazole, oxazole, isoxazole, etc.; non-aromatic heterocyclic group such as tetrahydropyridyl, dihydropyridyl, tetrahydropyrazyl, tetrahydropyrimidinyl, tetrahydropyridazinyl, dihydropyranyl, dihydropyrrolyl, dihydroimidazolyl, dihydropyrazolyl, dihydrothiphenyl, dihydrofuranyl,

dihydrothiazolyl, dihydroisothiazolyl, dihydrooxazolyl, dihydroisoxazolyl, hexahydropyrimidinyl, hexahydropyridazinyl, tetrahydropyranyl, pyrazolidinyl, tetrahydrothiophenyl, tetrahydrofuranyl, tetrahydrothiazolyl, tetrahydroisothiazolyl, tetrahydrooxazolyl, tetrahydroisoxazolyl, etc.], alkylsulfonylamino group (for example, $C_{1-4}$ alkylsulfonylamino group such as methylsulfonylamino, ethylsulfonylamino, etc.), $C_{1-6}$ alkylcarbonyloxy group (for example, $C_{1-4}$ alkyl-carbonyloxy group such as acetoxy, ethylcarbonyloxy, propylcarbonyloxy, butylcarbonyloxy, etc.), and halogen atoms (for example, fluorine, chlorine, bromine atoms, etc.), etc. are exemplified.

Preferred "optionally substituted alkyl group" includes, for example, $C_{1-6}$ alkyl group which may be substituted by about 1 - 4 halogen atoms [particularly, optionally halogentated $C_{1-4}$ alkyl group (for example, $C_{1-4}$ alkyl group and $C_{1-4}$ alkyl group which may be substituted by about 1 - 5 (particularly, 1 - 3) halogen atoms, such as methyl, chloromethyl, fluoromethyl, difluoromethyl, trichloromethyl, trifluoromethyl, ethyl, 2-bromoethyl, 2,2,2-trichloroethyl, 2,2,2-trifluoroethyl, pentafluoroethyl, propyl, 3,3,3-trifluoropropyl, isopropyl, 1-(trifluoromethyl)ethyl, butyl, 4,4,4-trifluorobutyl, isobutyl, sec-butyl, tert-butyl, etc.)],

$C_{1-6}$ alkoxy-$C_{1-6}$ alkyl group (for example, $C_{1-4}$ alkoxy-$C_{1-4}$ alkyl group such as methoxymethyl, ethoxymethyl, isopropoxymethyl, butoxymethyl, methoxyethyl, ethoxyethyl, etc.),
$C_{1-6}$ alkylthio-$C_{1-6}$ alkyl group (for example, $C_{1-4}$ alkylthio-$C_{1-4}$ alkyl group such as methylthiomethyl, ethylthiomethyl, butylthiomethyl, methylthioethyl, ethylthioethyl, etc.),
Amino-$C_{1-6}$ alkyl group (for example, amino-$C_{1-4}$ alkyl group such as aminomethyl, 2-aminoethyl, 2-aminopropyl, 3-aminopropyl, 2-aminobutyl, 3-aminobutyl, 4-aminobutyl, etc.),
$C_{1-7}$ acylamino-$C_{1-6}$ alkyl group (for example, $C_{1-7}$ acylamino-$C_{1-4}$ alkyl group such as formylaminomethyl, acetylaminomethyl, propionylaminomethyl, formylaminoethyl, acetylaminoethyl, propionylaminoethyl, butyrylaminoethyl, benzoylaminomethyl, etc.),
mono- or di-$C_{1-6}$ alkylamino-$C_{1-6}$ alkyl group (for example, mono- or di-$C_{1-4}$ alkylamino-$C_{1-4}$ alkyl group such as methylaminomethyl, ethylaminomethyl, butylaminomethyl, dimethylaminomethyl, diethylaminomethyl, 2-(N-methylamino)ethyl, 2-(N-ethylamino)ethyl, 2-(N-methylamino)propyl, 3-(N-methylamino)propyl, 3-(N-methylamino) butyl, 4-(N-methylamino)butyl, 2-(N-dimethylamino)ethyl, 2-(N-diethylamino)ethyl, etc.),
$C_{3-10}$ cycloalkylamino-$C_{1-6}$ alkyl group (for example, $C_{3-10}$ cycloalkylamino-$C_{1-4}$ alkyl group such as cyclopropylaminomethyl, cyclobutylaminomethyl, cyclohexylaminomethyl, cyclopropylaminoethyl, cyclobutylaminoethyl, cyclohexylaminoethyl, phenylaminomethyl, etc.),
5- or 6-membered cyclic amino-$C_{1-6}$ alkyl group which may contain 1 - 3 heteroatoms such as oxygen atom, sulfur atom, etc. in addition to the nitrogen atom (for example, non-aromatic cyclic amino-$C_{1-4}$ alkyl group such as piperidinomethyl, 4-methylpiperidinomethyl, morpholinomethyl, thiomorpholinomethyl, piperazinylmethyl, 4-methylpiperazinylmethyl, piperidinoethyl, morpholinoethyl, piperazinylethyl, etc.; 5- or 6-membered aromatic cyclic amino-$C_{1-4}$ alkyl group such as pyridylmethyl, pyrimidinylmethyl, imidazolylmethyl, pyridylethyl, etc.),
$C_{1-6}$ alkylsulfonylamino-$C_{1-6}$ alkyl group (for example, $C_{1-6}$ alkylsulfonylamino-$C_{1-6}$ alkyl group such as methylsulfonylaminomethyl, ethylsulfonylaminomethyl, methylsulfonylaminoethyl, ethylsulfonylaminoethyl, etc.),
$C_{1-6}$ alkyl-carbonyloxy-$C_{1-6}$ alkyl group (for example, $C_{1-4}$ alkyl-carbonyloxy-$C_{1-4}$ alkyl group such as methylcarbonyloxymethyl, ethylcarbonyloxymethyl, butylcarbonyloxymethyl, methylcarbonyloxyethyl, ethylcarbonyloxyethyl, etc.), and the like.

As "optionally halogenated alkoxy group", for example, $C_{1-6}$ alkoxy group, $C_{1-6}$ alkoxy group substituted by about 1 - 5 halogen atoms, etc. are exemplified. Such an alkoxy group or halogenated alkoxy group includes, for example, methoxy, fluoromethoxy, difluoromethoxy, trifluoromethoxy, trichloromethoxy, ethoxy, 2,2,2-trifluoroethoxy, 2,2,2-trichloroethoxy, pentafluoroethoxy, propoxy, isopropoxy, butoxy, 4,4,4-trifluorobutoxy, isobutoxy, sec-butoxy, pentoxy, hexyloxy, etc. The preferred "optionally halogenated alkoxy group" includes $C_{1-4}$ alkoxy group or $C_{1-4}$ alkoxy group substituted by about 1 - 3 halogen atoms, for example, methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, etc.

The "optionally halogenated alkylthio group" includes, for example, $C_{1-6}$ alkylthio group, $C_{1-6}$ alkylthio group substituted by about 1 - 5 halogen atoms, etc. As such an alkylthio group or halogenated alkylthio group, for example, methylthio, difluoromethylthio, trifluoromethylthio, ethylthio, propylthio, isopropylthio, butylthio, 4,4,4-trifluorobutylthio, pentylthio, hexylthio, etc. are exemplified. The preferred "optionally halogenated alkylthio group" includes $C_{1-4}$ alkylthio group, or $C_{1-4}$ alkylthio group substituted by about 1 - 3 halogen atoms, for example, methylthio, difluoromethylthio, trifluoromethylthio, ethylthio, propylthio, isopropylthio, butylthio, 4,4,4-trifluorobutylthio, etc.

In addition, "cycloalkyl group" includes $C_{3-10}$ cycloalkyl group (for example, cyclopropyl, cyclobutyl, cyclopentyl, cyclohexyl, cyclooctyl, etc.); "aryl group" includes $C_{6-10}$ aryl group (for example, phenyl, etc.); "acylamino group", for example, $C_{1-7}$ acylamino group (for example, formylamino, acetylamino, propionylamino, butyrylamino, benzoylamino, etc.). The "acyloxy group" includes, for example, $C_{1-3}$ acyloxy (for example, formyloxy, acetoxy, propionyloxy, etc.). As "mono- or di-alkylamino group", for example, mono- or di-$C_{1-4}$ alkylamino groups (for example, methylamino, ethylamino, propylamino, dimethylamino, diethylamino, etc.) are exemplified. The "cyclic amino group" includes, for example, 5- to 9-membered cyclic amino group which may contain 1 - 3 heteroatoms such as oxygen atom, sulfur atom, etc. in addition . to the nitrogen atom (for example, pyrrolidino, piperidino, morpholino, thiomorpholino, etc.). The "alkylcarbonylamino group" includes, for example, $C_{1-4}$ alkyl-carbonylamino group (for example, acetylamino, propionylamino, butyrylamino, etc.); "alkylsulfonylamino group", for example, $C_{1-4}$ alkylsulfonylamino group (for example, methylsulfonylamino, ethylsulfonylamino, etc.); "alkoxycarbonyl group", for example, $C_{1-4}$ alkoxy-carbonyl group (for example, methoxycarbonyl, ethoxycarbonyl, propoxycarbonyl, butoxycarbonyl, etc.); "alkylcarbonyl group", for example, $C_{1-6}$ alkyl-carbonyl group (for example, formyl, methylcarbonyl, ethylcarbonyl, propylcarbonyl, etc.); "mono- or di-alkylcarbamoyl group", for example, mono- or di-$C_{1-4}$ alkylcarbamoyl group (for example, methylcarbamoyl, ethylcarbamoyl, dimethylcarbamoyl, diethylcarbamoyl, etc.); and "alkylsulfonyl group", for example, $C_{1-6}$ alkylsulfonyl group (for example, methylsulfonyl, ethylsulfonyl, propylsulfonyl, etc.).

As to "Ring A and Ring B"

In the above formulae (I) and (Ia), the rings A and B each is an optionally substituted homocycle or heterocycle, and at least one of them is an optionally substituted heterocycle.

The above-mentioned" homocycle or heterocycle" includes, for example, (i) aromatic heterocycle or non-aromatic heterocycle which contains preferably 1 to 3 of 1 or 2 kinds of heteroatoms selected from nitrogen atom, sulfur atom and oxygen atom in addition to the carbon atom, or (ii) cyclic hydrocarbon (homocycle) consisting of carbon atoms.

As "aromatic heterocycle", for example, a 5- or 6-membered aromatic heterocycle which contains 1 to 3 heteroatoms selected from nitrogen atom, oxygen atom and sulfur atom in addition to the carbon atom, for example, pyridine, pyrazine, pyrimidine, pyridazine, pyrrole, imidazole, pyrazole, triazole, thiophene, furan, thiazole, isothiazole, oxazole, isoxazole, etc. are exemplified. The preferred aromatic heterocycle includes, for example, pyridine, pyrazine, and thiophene, as well as, for example, pyrrole, thiazole, etc. Particularly, (i) a nitrogen-containing 6-membered heterocycle which contains 1 or 2 nitrogen atoms in addition to the carbon atom (for example, pyridine, pyrazine, etc.) or (ii) a 5-membered aromatic heterocycle containing 1 sulfur atom in addition to the carbon atom (for example, thiophene, etc.) is preferred.

The above-mentioned "non-aromatic heterocycle" includes, for example, a 5- to 9-membered non-aromatic heterocycle, preferably a 5- or 6-membered non-aromatic heterocycle, which contains 1 to 3 heteroatoms selected from nitrogen atom, oxygen atom and sulfur atom in addition to the carbon atom.

As to the ring A, for example, tetrahydropyridine, dihydropyridine, tetrahydropyrazine, tetrahydropyrimidine, tetrahydropyridazine, dihydropyran, dihydropyrrole, dihydroimidazole, dihydropyrazole, dihydrothiophene, dihydrofuran, dihydrothiazole, dihydroisothiazole, dihydrooxazole, dihydroisoxazole, etc. are exemplified. As to the ring B, in addition to the above-mentioned ring, piperidine, piperazine, hexahydropyrimidine, hexahydropyridazine, tetrahydropyran, morpholine, pyrrolidine, imidazolidine, pyrazolidine, tetrahydrothiophene, tetrahydrofuran, tetrahydrothiazole, tetrahydroisothiazole, tetrahydrooxazole, tetrahydroisoxaozle, etc. are exemplified. As to the ring A, for example, a 6-membered non-aromatic heterocycle which contains 1 or 2 nitrogen atoms in addition to the carbon atom, for example, tetrahydropyridine, tetrahydropyrimidine, tetrahydropyridazine, etc. are preferred, and particularly tetrahydropyridine ring, etc. are widely used. As to the ring B, for example, a 6-membered non-aromatic heterocycle which contains 1 or 2 nitrogen atoms in addition to the carbon atom, for example, piperidine, piperazine, etc. are preferred, and particularly piperazine ring, etc. are widely used.

The above-mentioned "cyclic hydrocarbon (homocycle)" includes, for example, a 3- to 10-membered (for example, 5- to 9-membered) cyclic hydrocarbon, preferably 5- or 6-membered cyclic hydrocarbon, etc. For example, as to the ring A, benzene, $C_{3-10}$ cycloalkene (for example, cyclobutene, cyclopentene, cyclohexene, cycloheptene, cyclooctene, etc.), etc., and as the cycloalkene, $C_{5-6}$ cycloalkene (for example, cyclopentene, cyclohexene, etc.), etc. are preferred. As to the ring B, in addition to the above-mentioned ring, $C_{3-10}$ cycloalkane (for example, cyclobutane, cyclopentane, cyclohexane, cycloheptane, cyclooctane, etc.), etc. are included, and as the cycloalkane, $C_{5-6}$ cycloalkane (for example, cyclohexane, cyclopentane, etc.), etc. are preferred. As to the ring A, for example, a 6-membered homocycle such as benzene, cyclohexene ring, etc. is preferred, and a benzene ring is particularly preferred. As to the ring B, for example, a 6-membered homocycle such as benzene, cyclohexane ring, etc. is preferred, and a benzene ring is particularly preferred.

At least one of the rings A and B is composed of optionally substituted heterocycle, and both of the rings A and B may be composed of optionally substituted heterocycle. Preferably, one of the rings A and B is composed of (i) optionally substituted aromatic ring, and the other is composed of (ii) optionally substituted heterocycle (particularly, aromatic heterocycle).

In the above (i) "aromatic ring", (i-i) the above-mentioned "aromatic heterocycle", that is, 5- or 6-membered aromatic heterocycles which may contain preferably 1 to 3 of 1 or 2 kinds of heteroatoms selected from nitrogen atom, sulfur atom and oxygen atom in addition to the carbon atom, for example, pyridine, pyrazine, pyrimidine, pyridazine,

pyrrole, imidazole, pyrazole, triazole, thiophene, furan, thiazole, isothiazole, oxazole, isoxazole, etc., or (i-ii) optionally substituted benzene rings are included.

As the substituent that the above (i) "aromatic ring" may have, for example, the same substituents as those in the rings A and B as described below are exemplified, and as "aromatic heterocycle" of the above (ii) "optionally substituted aromatic heterocycle", for example, the same aromatic heterocycles as those in the above "5- or 6-membered aromatic heterocycle" are exemplified. As the substituent that (ii) "optionally substituted aromatic heterocycle" may have, for example, the same substituents as those in the rings A and B as described below are exemplified. As the above "5- or 6-membered aromatic heterocycle", the same heterocycles as those in the above "aromatic heterocycle", and the like are preferred.

More preferably, one of the rings A and B is an optionally substituted aromatic heterocycle (for example, 5- or 6-membered aromatic heterocycle), and the other is an optionally substituted benzene ring.

As the substituent that "homocycle or heterocycle", "aromatic heterocycle", "non-aromatic heterocycle", "cyclic hydrocarbon", "aromatic ring", and "benzene ring" represented by the rings A and B may have, for example, halogen atom, optionally substituted alkyl group, optionally halogenated alkoxy group, optionally halogenated alkylthio group, aryl group, acylamino group, acyloxy group, hydroxyl group, nitro group, cyano group, amino group, mono- or di-alkylamino group, cyclic amino group (for example, cyclic amino group which may contain (a) heteroatom(s) such as oxygen atom, sulfuratom, etc. in addition to the nitrogen atom), alkylcarbonylamino group, alkylsulfonylamino group, alkoxycarbonyl group, carboxyl group, alkylcarbonyl group, carbamoyl group, mono- or di-alkylcarbamoyl group, alkylsulfonyl group, oxo group, etc. are exemplified.

As "halogen atom" that the rings A and B may have, for example, fluorine, chlorine, bromine, and iodine atoms are included. As the preferred halogen atom, for example, fluorine, chlorine, and bromine atoms (particularly, fluorine, chlorine atoms, etc.) are exemplified.

As "optionally substituted alkyl group" that the rings A and B may have, for example, $C_{1-6}$ alkyl group (for example, methyl, ethyl, propyl, isopropyl, butyl, isobutyl, sec-butyl, tert-butyl, etc.) which may have 1 - 5 substituents selected -from hydroxyl group, amino group, carboxyl group, nitro group, mono- or di-$C_{1-6}$ alkylamino group (for example, methylamino, ethylamino, dimethylamino, diethylamino, etc.), $C_{1-6}$ alkyl-carbonyloxy group (for example, acetoxy, ethylcarbonyloxy group, etc.) and halogen atom (for example, fluorine, chlorine, bromine atoms, etc.) are exemplified. Particularly, the optionally halogentated alkyl group, for example, $C_{1-6}$ alkyl group, and $C_{1-6}$ alkyl group substituted by about 1 - 4 halogen atoms, are preferred. Such an alkyl group or halogenated alkyl group includes, for example, methyl, chloromethyl, fluoromethyl, difluoromethyl, trichloromethyl, trifluoromethyl, ethyl, 2-bromoethyl, 2,2,2-trichloroethyl, 2,2,2-trifluoroethyl, pentafluoroethyl, propyl, 3,3,3-trifluoropropyl, isopropyl, 1-(trifluoromethyl)ethyl, butyl, 4,4,4-trifluorobutyl, isobutyl, sec-butyl, tert-butyl, pentyl, isopentyl, neopentyl, 5,5,5-trifluoropentyl, 4-trifluoromethylbutyl, hexyl, 6,6,6-trifluorohexyl, 5-trifluoromethylpentyl, etc.

More preferred "optionally substituted alkyl group" includes an optionally halogenated $C_{1-4}$ alkyl group, for example, $C_{1-4}$ alkyl group and $C_{1-4}$ alkyl group substituted by about 1 - 3 halogen atoms such as methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, 2-trifluoromethylethyl, butyl, 4,4,4-trifluorobutyl, isobutyl, sec-butyl, tert-butyl, etc.

As "optionally halogenated alkoxy group" that the rings A and B may have, for example, $C_{1-6}$ alkoxy groups or $C_{1-6}$ alkoxy groups substituted by about 1 - 5 halogen atoms as described above are exemplified. Such an alkoxy group or halogenated alkoxy group includes, for example, methoxy, difluoromethoxy, trifluoromethoxy, trichloromethoxy, ethoxy, 2,2,2-trifluoroethoxy, 2,2,2-trichloroethoxy, pentafluoroethoxy, propoxy, isopropoxy, butoxy, 4,4,4-trifluorobutoxy, isobutoxy, sec-butoxy, pentoxy, hexyloxy, etc. The preferred "optionally halogenated alkoxy group" involves $C_{1-4}$ alkoxy groups or $C_{1-4}$ alkoxy groups substituted by about 1 - 3 halogen atoms, for example, methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, etc.

As "optionally halogenated alkylthio group" that the rings A and B may have, for example, $C_{1-6}$ alkylthio groups or $C_{1-6}$ alkylthio groups substituted by about 1 - 5 halogen atoms as described above are included. As such an alkylthio group or halogenated alkylthio group, for example, methylthio, difluoromethylthio, trifluoromethylthio, ethylthio, propylthio, isopropylthio, butylthio, 4,4,4-trifluorobutylthio, pentylthio, hexylthio, etc. are exemplified. The preferred "optionally halogenated alkylthio group" includes $C_{1-4}$ alkylthio groups or $C_{1-4}$ alkylthio groups substituted by about 1 - 3 halogen atoms, for example, methylthio, difluoromethylthio, trifluoromethylthio, ethylthio, propylthio, isopropylthio, butylthio, 4,4,4-trifluorobutylthio, etc.

Moreover, the aryl group as substituent includes $C_{6-10}$ aryl group (for example, phenyl, etc.), and the acylamino group includes, for example, $C_{1-7}$ acylamino group (for example, formylamino, acetylamino, propionylamino, butyrylamino, benzoylamino, etc.). The acyloxy group includes, for example, $C_{1-3}$ acyloxy group (for example, formyloxy, acetoxy, propionyloxy, etc.). The mono- or di-alkylamino group is exemplified by, for example, mono- or di-$C_{1-4}$ alkylamino group (for example, methylamino, ethylamino, propylamino, dimethylamino, diethylamino, etc.). The cyclic amino group includes, for example, 5- to 9-membered cyclic amino group which may contain 1 - 3 heteroatoms selected from

oxygen atom and sulfur atom in addition to the nitrogen atom (for example, pyrrolidino, piperidino, morpholino, etc.). The alkylcarbonylamino group includes, for example, $C_{1-4}$ alkyl-carbonylamino group (for example, acetylamino, propionylamino, butyrylamino, etc.); the alkylsulfonylamino group, for example, $C_{1-4}$ alkyl-sulfonylamino group (for example, methylsulfonylamino, ethylsulfonylamino, etc.); the alkoxycarbonyl group, for example, $C_{1-4}$ alkoxy-carbonyl group (for example, methoxycarbonyl, ethoxycarbonyl, propoxycarbonyl, butoxycarbonyl, etc.); the alkylcarbonyl group, for example, $C_{1-6}$ alkyl-carbonyl group (for example, formyl, methylcarbonyl, ethylcarbonyl, propylcarbonyl, etc.); mono- or di-alkylcarbamoyl group, for example, mono- or di-$C_{1-4}$ alkyl-carbamoyl group (for example, methylcarbamoyl, ethylcarbamoyl, dimethylcarbamoyl, diethylcarbamoyl, etc.); and the alkylsulfonyl group, for example, $C_{1-6}$ alkylsulfonyl group (for example, methylsulfonyl, ethylsulfonyl, propylsulfonyl, etc.).

Hereinafter, when the term "optionally halogenated" is used in this specification, it indicates that the number of the halogen atom is 1 - 5, preferably 1 - 3.

The preferred substituents that the rings A and B may have, are exemplified by halogen atom, optionally halogenated $C_{1-4}$ alkyl group, optionally halogenated $C_{1-4}$ alkoxy group, optionally halogenated $C_{1-4}$ alkylthio group, $C_{1-3}$ acyloxy group, hydroxyl group, amino group, mono- or di-$C_{1-4}$ alkylamino group, carboxyl group, $C_{1-4}$ alkoxy-carbonyl group, oxo group, etc.

The more preferred substituents that the rings A and B may have, includes halogen atom, optionally halogenated $C_{1-4}$ alkyl group, optionally halogenated $C_{1-4}$ alkoxy group, hydroxyl group, amino group, mono- or di-$C_{1-4}$ alkylamino group, $C_{1-3}$ acyloxy group, oxo group, etc. Particularly, halogen atom, optionally halogenated $C_{1-4}$ alkyl group, optionally halogenated $C_{1-4}$ alkoxy group, etc. are preferred.

The substituent on the rings A and B may be substituted at any position on which it can be substituted. When the substituent is 2 or more, it may be the same or different to each other, and its number may be approximately 1 - 4. The number of the substituent is preferably about 1 - 3.

When the ring A and/or the ring B have a nitrogen atom, they may form a quaternary ammonium salt, for example, salt with an anion such as halogen ion (e.g., $Cl^-$, $Br^-$, $I^-$, etc.), sulfuric acid ion, hydroxy ion, etc.

"As to Ring A"

A preferable homocyclic ring as Ring A is a homocyclic ring consisting of carbon atoms which may have substituents, such as, for example, one represented by Formula (A-1):

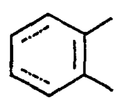 , 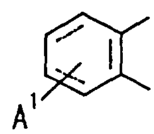 (A-1)

wherein ---- is a single or double bond, and hereinafter the same applies analogously, and wherein $A^1$ is a halogen atom (for example, fluorine and chlorine atoms, etc.), an optionally halogenated $C_{1-4}$ alkyl group (for example, methyl, isopropyl, trifluoromethyl, trichloromethyl, ethyl, 2,2,2-trifluoroethyl and pentafluoroethyl groups, etc.), or an optionally halogenated $C_{1-4}$ alkoxy group (for example, methoxy, trifluoromethoxy, trichloromethoxy, ethoxy, 2,2,2-trifluoroethoxy and pentafluoroethoxy groups, etc.), or by Formula (A-2):

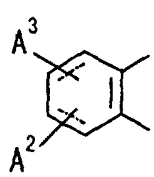 (A-2)

wherein $A^2$ and $A^3$ are same or different and each represents a halogen atom (for example, fluorine and chlorine atoms, etc.), an optionally halogenated $C_{1-4}$ alkyl group (for example, methyl, isopropyl, trifluoromethyl, trichloromethyl, ethyl, 2,2,2-trifluoroethyl and pentafluoroethyl groups, etc.), or an optionally halogenated $C_{1-4}$ alkoxy group (for example, methoxy, trifluoromethoxy, trichloromethoxy, ethoxy, 2,2,2-trifluoroethoxy and pentafluoroethoxy groups, etc.), etc.

A more preferably homocyclic ring may for example be one represented by Formula (A-3):

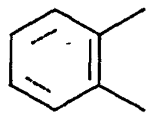 or 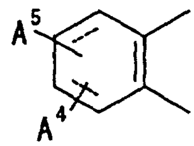 (A-3)

wherein $A^4$ and $A^5$ are same or different and each represents a halogen atom (for example, fluorine and chlorine atoms, etc.), an optionally halogenated $C_{1-4}$ alkyl group (for example, methyl, trifluoromethyl, trichloromethyl, ethyl, 2,2,2-trifluoroethyl, pentafluoroethyl, and isopropyl groups, etc.), especially a benzene ring, etc.

Another preferable homocyclic ring is an optionally substituted benzene ring represented by Formula (A-4):

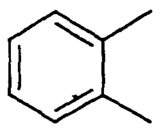 , 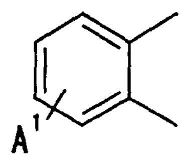 or 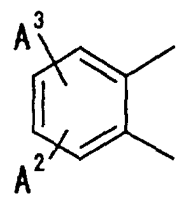

especially 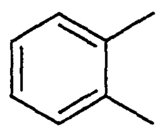 or 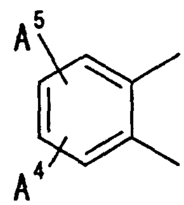 (A-4)

wherein each symbol is defined as described above.

Among the homocyclic rings represented by Formulae shown above, those preferred especially include the following substituted homocyclic rings.

(1) a homocyclic ring wherein $A^1$ is a halogen atom (for example, fluorine and chlorine atoms, etc.), or an optionally halogenated $C_{1-4}$ alkyl group (for example, methyl, trifluoromethyl, ethyl and isopropyl groups, etc.);
(2) a homocyclic ring wherein $A^2$ and $A^3$ are same or different and each represents an optionally halogenated $C_{1-4}$ alkyl group (for example, methyl, trifluoromethyl, ethyl and isopropyl groups, etc.) or an optionally halogenated $C_{1-4}$ alkoxy group (for example, methoxy, trifluoromethoxy and ethoxy groups, etc.);
(3) a homocyclic ring wherein $A^4$ and $A^5$ are same or different and each represents an optionally halogenated $C_{1-4}$ alkyl group (for example, methyl, ethyl and isopropyl groups);
(4) $A^1$ represents a halogen atom (for example, fluorine and chlorine atoms); and,
(5) a homocyclic ring wherein $A^2$ and $A^3$ are same or different and each represents a $C_{1-4}$ alkyl group (for example, methoxy and ethoxy groups, etc.).

A preferred aromatic heterocyclic ring or a non-aromatic heterocyclic ring as Ring A is a 5- or 6-membered aromatic or non-aromatic heterocyclic ring such as pyridine, pyrazine, thiophene, tetrahydropyridine, pyrrole and thiazole rings, etc. One preferred typically is a heterocyclic ring represented by Formula (A-5):

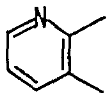 , 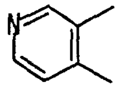 , 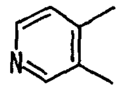 , 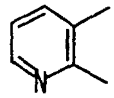 , 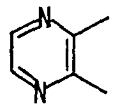 , 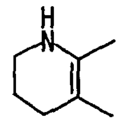 ,

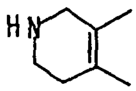 , 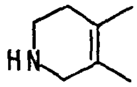 , 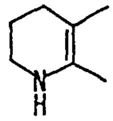 , 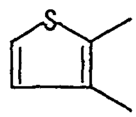 , 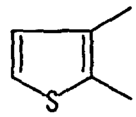 , 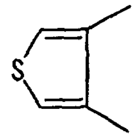

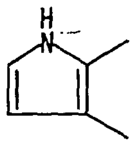 , 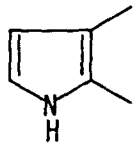 , 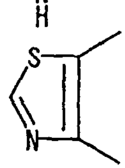 , 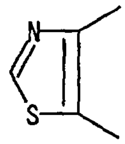 (A-5)

A preferred aromatic or non-aromatic heterocyclic ring which may have a substituent may for example be pyridine, pyrazine, thiophene, tetrahydropyridine, pyrrole and thiazole rings, etc. each of which may have one or two substituents selected from an oxo group, an optionally substituted alkyl group (similar to one defined as a substituent which may be present on Ring A and Ring B), a $C_{6-10}$ aryl group (for example, a phenyl group, etc.) and a halogen atom (for example, fluorine, chlorine and bromine atoms, etc.), one preferred typically being an aromatic or non-aromatic heterocyclic ring represented by Formula (A-6):

(i) 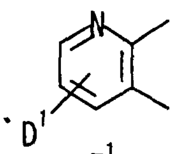 , 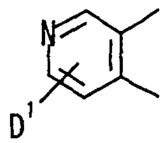 , 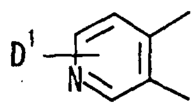 , 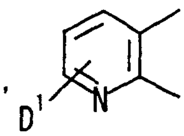 , 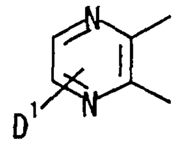

(ii) 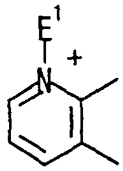 , 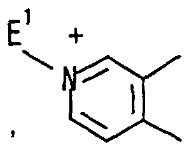 , 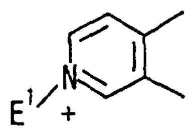 , 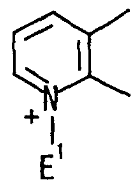 ;

(iii) 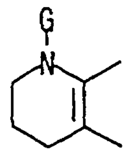 , 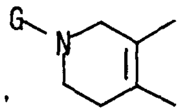 , 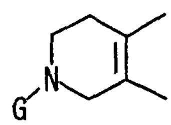 , 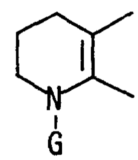 ;

(iv) 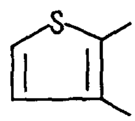 , 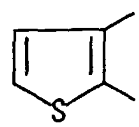 , 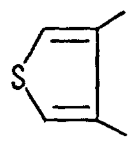 ;

(v) 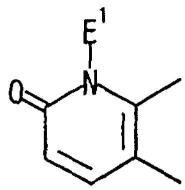 ;

(vi) 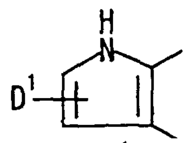 , 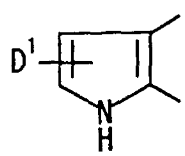 ;

(vii) 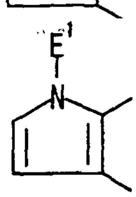 , 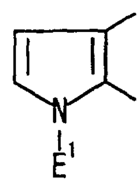 or

(viii) 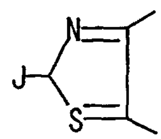 , 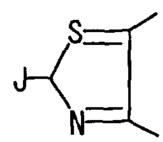 (A-6)

wherein $D^1$ is a hydrogen atom or a halogen atom (for example, fluorine, chlorine and bromine atoms, etc.), $E^1$ is a $C_{1-4}$ alkyl group (for example, methyl, ethyl, propyl and isopropyl groups, etc.) and a compound having a partial chemical structure shown in (ii) forms a quaternary ammonium salt with a halogen iron (for example, $Cl^-$, $Br^-$ and $I^-$), a sulfate ion, a hydroxy ion and the like; G represents a hydrogen atom, a $C_{1-4}$ alkyl group (for example, methyl, ethyl, propyl and isopropyl groups, etc.), and J represents a hydrogen atom, a $C_{1-4}$ alkyl group (for example, methyl, ethyl, propyl and isopropyl groups, etc.) or a $C_{6-10}$ aryl group (for example, phenyl group, etc.).

Preferably, Ring A is a 5- or 6-membered nitrogen-containing heterocyclic ring, for example, (i) a 6-membered aromatic nitrogen-containing heterocyclic ring containing 1 or 2 nitrogen atoms in addition to carbon atoms (for example,

pyridine and pyrazine rings, etc.), (ii) a 6-membered non-aromatic heterocyclic ring containing 1 or 2 nitrogen atoms in addition to carbon atoms (for example, tetrahydropyridine, tetrahydropyrimidine and tetrahydropyridazine groups, etc.), etc. A particularly preferred Ring A includes an aromatic nitrogen-containing heterocyclic ring, especially a pyridine ring, etc.

<u>"As to Ring B"</u>

A preferable homocyclic ring as Ring B is a homocyclic ring consisting of carbon atoms which may have substituents, such as, for example, one represented by Formula (B-1):

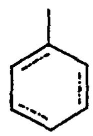 , 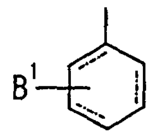 (B-1)

wherein $B^1$ represents a halogen atom, an optionally hydroxy-substituted or halogenated $C_{1-4}$ alkyl group or an optionally halogenated $C_{1-4}$ alkoxy group, a $C_{1-6}$ alkyl-carbonyl group or a carboxyl group, or by Formula (B-2):

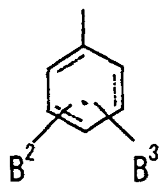 (B-2)

wherein $B^2$ and $B^3$ are same or different and each represents a halogen atom, an optionally halogenated $C_{1-4}$ alkyl group or an optionally halogenated $C_{1-4}$ alkoxy group, or by Formula (B-3):

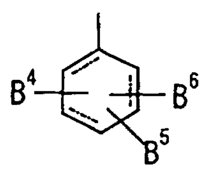 (B-3)

wherein $B^4$, $B^5$ and $B^6$ are same or different and each represents a halogen atom, an optionally halogenated $C_{1-4}$ alkyl group or an optionally halogenated $C_{1-4}$ alkoxy group.

A further preferable homocyclic ring includes a group represented by Formula (B-4):

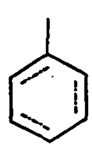 , 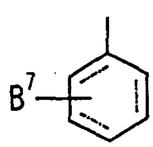 or 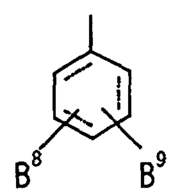 (B-4)

wherein $B^7$, $B^8$ and $B^9$ are same or different and each represents a halogen atom, an optionally halogenated $C_{1-4}$ alkyl group or an optionally halogenated $C_{1-4}$ alkoxy group.

A particularly preferred homocyclic ring includes a group represented by Formula (B-5):

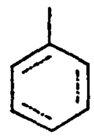 or 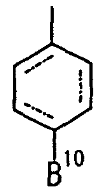 (B-5)

wherein $B^{10}$ represents a halogen atom, an optionally hydroxy-substituted or halogenated $C_{1-4}$ alkyl group, an optionally halogenated $C_{1-4}$ alkoxy group, a $C_{1-6}$ alkyl-carbonyl group or a carboxyl group.

As each of $B^1$ to $B^{10}$ described above, a halogen atom may for example be fluorine, chlorine and bromine atoms, etc., an optionally halogenated $C_{1-4}$ alkyl group may for example be methyl, trifluoromethyl, trichloromethyl, ethyl, 2,2,2-trifluoroethyl, 2,2,2-trichloroethyl, 1,1,2,2-tetrafluoroethyl, pentafluoroethyl, propyl, 2,2,3,3-tetrafluoropropyl and isopropyl groups, etc., and an optionally halogenated $C_{1-4}$ alkoxy group may for example be methoxy, trifluoromethoxy, trichloromethoxy, ethoxy, 2,2,2-trifluoroethoxy, 2,2,2-trichloroethoxy, 1,1,2,2-tetrafluoroethoxy, pentafluoroethoxy, propoxy, 2,2,3,3-tetrafluoropropoxy and isopropoxy groups, etc.

As each of $B^1$ to $B^{10}$ described above, a $C_{1-6}$ alkyl-carbonyl group may for example include formyl and acetyl.

It is also preferable that Ring B is an optionally substituted benzene ring. Such benzene ring may for example be a benzene ring represented by Formula (B-6):

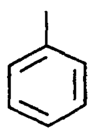 , 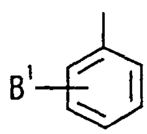 , 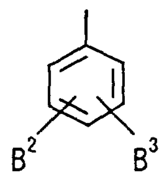 or 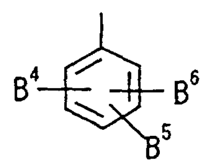 (B-6)

more preferably, one represented by Formula (B-7):

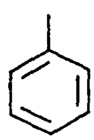 , 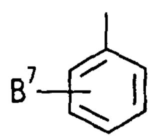 or 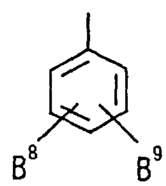 (B-7)

,

and one especially preferred is represented by Formula (B-8):

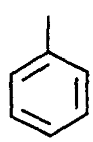 or 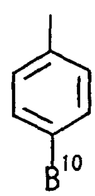 (B-8)

,

wherein each symbol is defined as described above.

Among the substituents in the formulae described above, those especially preferred are:

(1) $B^1$, $B^2$, $B^3$, $B^4$, $B^5$ and $B^6$ are same or different and each of which represents a halogen atom (for example, fluorine and chlorine atoms, etc.), or an optionally halogenated $C_{1-4}$ alkyl group (for example, methyl, trifluoromethyl, ethyl and isopropyl groups, etc.);

(2) $B^1$, $B^2$, $B^3$, $B^4$, $B^5$ and $B^6$ are same or different and each of which represents an optionally halogenated $C_{1-4}$ alkoxy group (for example, methoxy, trifluoromethoxy and ethoxy groups, etc.);

(3) $B^7$, $B^8$ and $B^9$ each of which is a halogen atom (for example, fluorine and chlorine atoms, etc.);
(4) $B^{10}$ is a fluorine atom;
(5) $B^{10}$ is a $C_{1-4}$ alkyl group (for example, methyl group, etc.);
(6) $B^1$ or $B^{10}$ each of which is an optionally hydroxy-substituted $C_{1-6}$ alkyl group (for example, hydroxymethyl, etc.), a $C_{1-6}$ alkyl-carbonyl group (for example, formyl, acetyl, etc.) and a carboxyl group, etc.

A more preferable optionally substituted benzene ring may for example be a phenyl group represented by Formula (B-9):

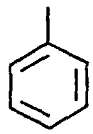 , 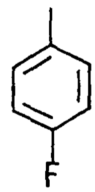 or 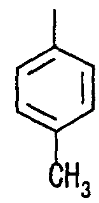 (B-9)

.

An aromatic heterocyclic ring or a non-aromatic heterocyclic ring as Ring B may for example be a 5- or 6-membered aromatic heterocyclic ring or non-aromatic heterocyclic ring such as pyridine, thiophene and pyperidine rings, each of which may have a preferable substituent exemplified with regard to Ring A described above.
When Ring B is an aromatic or non-aromatic heterocyclic group, an especially preferred aromatic or non-aromatic heterocyclic ring may for example be a heterocyclic ring represented by Formula (B-10):

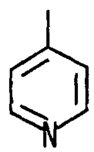 , 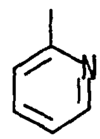 or 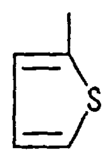 (B-10)

.

When both of or only one of Rings A and B is a heterocyclic ring, such heterocyclic may also be an unsubstituted hetrocyclic ring.

Combination of Ring A and Ring B

A preferable combination (1) of Ring A and Ring B is described below.

(1) One of Ring A and Ring B: a 5- or 6-membered heterocyclic ring (for example, pyridine, pyrazine, thiophene, tetrahydropyridine, piperidine and piperazine rings) which may be substituted by a $C_{1-4}$alkyl group (for example, methyl, ethyl and isopropyl groups, etc.) and which contains 1 or 2 heteroatoms selected from nitrogen and sulfur atoms in addition to carbon atoms;

the other of Ring A and Ring B: a benzene ring which may be substituted by 1 to 3 substituents selected from a halogen atom (for example, fluorine, chlorine and bromine atoms, etc.), an optionally halogenated $C_{1-4}$ alkyl group (for example, methyl, trifluoromethyl, trichloromethyl, ethyl, 2,2,2-trifluoroethyl, pentafluoroethyl, 2,2,2-trichloroethyl, propyl and isopropyl groups, etc.) and an optionally halogenated $C_{1-4}$ alkoxy group (for example, methoxy, trifluoromethoxy, trichloromethoxy, ethoxy, 2,2,2-trifluoroethoxy, pentafluoroethoxy, 2,2,2-trichloroethoxy, propoxy and isopropoxy groups, etc.).

A further preferred combination (2) of Ring A and Ring B is described below.

(2) One of Ring A and Ring B: a 5- or 6-membered aromatic heterocyclic ring which contains 1 or 2 heteroatoms selected from nitrogen and sulfur atoms in addition to carbon atoms (for example, pyridine, pyrazine and thiophene rings, etc.);

the other of Ring A and Ring B: a benzene ring which may be substituted by 1 to 3 substituents selected from a halogen atom (for example, fluorine, chlorine and bromine atoms), an optionally halogenated $C_{1-4}$ alkyl group (for example, methyl, trifluoromethyl, trichloromethyl, ethyl, 2,2,2-trifluoroethyl, pentafluoroethyl, 2,2,2-trichloroethyl, propyl and isopropyl groups) and an optionally halogenated $C_{1-4}$ alkoxy group (for example, methoxy, trifluoromethoxy, trichloromethoxy, ethoxy, 2,2,2-trifluoroethoxy, pentafluoroethoxy, 2,2,2-trichloroethoxy,

propoxy and isopropoxy groups, etc.).

In a particularly prefer-red case, Ring A is an optionally substituted aromatic heterocyclic ring described above (for example, 5- or 6-membered aromatic heterocyclic ring, especially a pyridine ring, etc.), and Ring B is an optionally substituted benzene ring.

"As to Ring C"

In a formula shown above, Ring C is an optionally substituted homocyclic ring or an optionally substituted heterocyclic ring. Such homocyclic or heterocyclic ring may have about 1 to about 5, preferably about 1 to about 3 same or different substituent. Such substituent may be present in any position in such homocyclic or heterocyclic ring.

A homocyclic ring may for example be a "cyclic hydrocarbon (homocyclic ring)" similar to one exemplified with regard to "Ring A and Ring B" including a 3- to 10-membered cyclic hydrocarbon, preferably 5- or 6-membered cyclic hydrocarbon such as benzene, a $C_{3-10}$ cycloalkene (for example, cyclobutene, cyclopentene, cyclohexene, cycloheptene, cyclooctene, etc.) and a $C_{3-10}$ cycloalkane (for example, cyclobutane, cyclopentane, cyclohexane, cycloheptane, cyclooctane, etc.). A preferred homocyclic ring includes a 6-membered homocyclic ring such as benzene, cyclohexene and cyclohexane rings, with a benzene ring being particularly preferred.

A substituent on a homocyclic ring such as a benzene ring described above may for example be a halogen atom (for example, fluorine, chlorine, bromine and iodine atoms), an optionally halogenated $C_{1-10}$ alkyl group (for example, methyl, chloromethyl, difluoromethyl, trichloromethyl, trifluoromethyl, ethyl, 2-bromoethyl, 2,2,2-trifluoroethyl, perfluoroethyl, propyl, isopropyl, 3,3,3-trifluoropropyl,. butyl, isobutyl, t-butyl, perfluorobutyl, pentyl, hexyl, octyl and decyl groups, etc.), an amino-substituted $C_{1-4}$ alkyl group (for example, aminomethyl and 2-aminoethyl groups, etc.), a mono- or di-$C_{1-4}$ alkylamino-substituted $C_{1-4}$ alkyl group (for example, methylaminomethyl, dimethylaminomethyl, 2-methylaminoethyl and 2-dimethylaminoethyl groups, etc.), a carboxy-substituted $C_{1-4}$ alkyl group (for example, carboxymethyl and carboxyethyl groups, etc.), a $C_{1-4}$ alkyl-carbonyl-substituted $C_{1-4}$ alkyl group (for example, methoxycarbonylethyl and ethoxycarbonylethyl groups, etc.), a hydroxyl-substituted $C_{1-4}$ alkyl group (for example, hydroxymethyl and hydroxyethyl groups, etc.), a $C_{1-4}$ alkoxy-carbonyl-substituted $C_{1-4}$ alkyl group (for example, methoxymehyl, methoxyethyl and ethoxyethyl groups, etc.), a $C_{3-10}$ cycloalkyl group (for example, cyclopropyl, cyclobutyl, cyclopentyl, cyclohexyl, cycloheptyl and cyclooctyl groups, etc.), a nitro group, a cyano group, a hydroxyl group, an optionally halogenated $C_{1-10}$ alkoxy group (for example, methoxy, difluoromethoxy, trichloromethoxy, trifluoromethoxy, ethoxy, 2,2,2-trifluoroethoxy, perfluoroethoxy, propoxy, isopropoxy, butoxy, isobutoxy, t-butoxy, perfluorobutoxy, pentyloxy, hexyloxy, octyloxy and decyloxy groups, etc.), an optionally halogenated $C_{1-4}$ alkylthio group (for example, methylthio, difluoromethylthio, trifluoromethylthio, ethylthio, propylthio, isopropylthio and butylthio groups), an amino group, a mono- or di-$C_{1-4}$ alkylamino group (for example, methylamino, ethylamino, propylamino, dimethylamino and diethylamino groups, etc.), a cyclic amino group (for example, a 5- to 9-membered cyclic amino group which may contain 1 to 3 heteroatoms such as oxygen and sulfur atoms in addition to a nitrogen atom, typically, pyrrolidino, piperidino and morpholino groups, etc.), a $C_{1-4}$ alkyl-carbonylamino group (for example, acetylamino, propionylamino and butyrylamino groups, etc.), an aminocarbonyloxy group, a mono- or di-$C_{1-4}$ alkylaminocarbonyloxy group (for example, methylaminocarbonyloxy, ethylaminocarbonyloxy, dimethylaminocarbonyloxy and diethylaminocarbonyloxy, etc.), a $C_{1-4}$ alkylsulfonylamino group (for example, methylsulfonylamino, ethylsulfonylamino and propylsulfonylamino groups, etc.), a $C_{1-4}$ alkoxycarbony group (for example, methoxycarbonyl, ethoxycarbonyl, propoxycarbonyl, butoxycarbonyl and isobutoxycarbonyl groups, etc.), an aralkyloxycarbonyl group (for example, $C_{7-19}$ aralkyloxycarbonyl group such as phenyl-$C_{1-4}$ alkyloxy-carbonyl (e.g., benzyloxycarbonyl group), a carboxyl group, a $C_{1-6}$ alkyl-carbonyl group (for example, methylcarbonyl, ethylcarbonyl and butylcarbonyl groups, etc.), a $C_{3-6}$ cycloalkyl-carbonyl group (for example, cyclohexylcarbonyl group), a carbamoyl group, a mono- or d-$C_{1-4}$ alkylcarbamoyl group (for example, methylcarbamoyl, ethylcarbamoyl, propylcarbamoyl, butylcarbamoyl, diethylcarbamoyl and dibutylcarbamoyl groups) and a $C_{1-6}$ alkylsulfonyl group (for example, methylsulfonyl, ethylsulfonyl and propylsulfonyl groups, etc.), etc.

A homocyclic ring as Ring C may also be substituted by one 5- or 6-membered aromatic monocyclic heterocyclic group (for example, furyl, thienyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl groups, etc.), etc., and such aromatic monocyclic heterocyclic group may be substituted by 1 to 3 optionally halogenated $C_{1-4}$ alkyl groups (for example, methyl, chloromethyl, difluoromethyl, trichloromethyl, trifluoromethyl, ethyl and isopropyl groups; etc.), etc.

A preferred substituent on a homocyclic ring (such as benzene ring, etc.) as Ring C may for example be a halogen atom (for example, fluorine, chlorine and bromine atoms, etc.), an optionally halogenated $C_{1-6}$ alkyl group (for example, methyl, chloromethyl, difluoromethyl, trichloromethyl, trifluoromethyl, ethyl, 2-bromoethyl, 2,2,2-trifluoroethyl, perfluoroethyl, propyl, isopropyl, 3,3,3-trifluoropropyl, butyl, s-butyl, t-butyl and perfluorobutyl groups, etc.), a nitro group, a hydroxyl group, an optionally halogenated $C_{1-6}$ alkoxy group (for example, methoxy, difluoromethoxy, trifluoromethoxy, ethoxy, 2,2,2-trifluoroethoxy, perfluoroethoxy, propoxy, isopropoxy, 3,3,3-trifluoropropoxy and butoxy groups, etc.), an amino group, a mono- or di-$C_{1-4}$ alkylamino-substituted $C_{1-4}$ alkyl group (for example, methylaminomethyl, dimethylaminomethyl, 2-methylaminoethyl and 2-dimethylaminoethyl groups, etc.), a mono- or di $C_{1-4}$ alkylamino group (for example, methylamino, ethylamino, dimethylamino and diethylamino groups, etc.), a $C_{1-4}$ alkoxycarbonyl group (for example, methoxycarbonyl and ethoxycarbonyl groups), a carboxyl group and a carbamoyl group.

Among those listed above, ahalogen atom (for example, fluorine, chlorine and bromine atoms), an optionally halogenated $C_{1-4}$ alkyl group (for example, methyl, chloromethyl, difluoromethyl, trichloromethyl, trifluoromethyl, ethyl, 2-bromoethyl, 2,2,2-trichloroethyl, 2,2,2-trifluoroethyl, perfluoroethyl, propyl, isopropyl and t-butyl groups, etc.), an optionally halogenated $C_{1-4}$ alkoxy group (for example, methoxy, trifluoromethoxy, ethoxy, 2,2,2-trichloroethoxy, 2,2,2-trifluoroethoxy, perfluoroethoxy and propoxy groups, etc.), a di-$C_{1-4}$ alkylamino group (for example, dimethylamino and diethylamino groups, etc.), a $C_{1-3}$ acyloxy group (for example, acetoxy group) and a hydroxyl group are preferred. The number of these substituents is preferably about 1 to about 3.

Those especially preferred are a halogen atom (for example, fluorine, chlorine and bromine atoms, etc.), an optionally halogenated $C_{1-4}$ alkyl group (for example, methyl, chloromethyl, difluoromethyl, trichloromethyl, trifluoromethyl, ethyl, 2-bromoethyl, 2,2,2-trichloroethyl, 2,2,2-trifluoroethyl, perfluoroethyl, propyl, isopropyl and t-butyl groups, etc.) and an optionally halogenated $C_{1-4}$ alkoxy group (for example, methoxy, trifluoromethoxy, ethoxy, 2,2,2-trichloroethoxy, 2,2,2-trifluoroethoxy, perfluoroethoxy and propoxy groups, etc.).

A "heterocyclic ring" in an "optionally substituted heterocyclic ring" may for example be a 5- to 10-membered heterocyclic ring containing 1 to 4 of one or two kinds of heteroatoms selected from nitrogen, oxygen and sulfur in addition to carbon atoms. Such heterocyclic ring may typically be (1) a 5- or 6-membered aromatic monocyclic heterocyclic ring such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl;

(2) a 9- or 10-membered aromatic condensed ring such as benzofuranyl, isobenzofuranyl, benzo [b] thienyl, indolyl, isoindolyl, 1H-indazolyl, benzoimidazolyl, benzoxazolyl, 1,2-benzoisoxazolyl, benzothiazolyl, 1,2-benzoisothiazolyl, 1H-benzotriazolyl, quinolyl, isoquinolyl, cinnolynyl, quinazolynyl, quinoxalynyl, phthalazinyl, naphthylidinyl, purinyl, puteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenathridinyl, phenathrolinyl, indolidinyl, pyrrolo[1,2-b]pyridazinyl, pyrazolo[1,5-a]pyridyl, imidazo[1,2-b]pyridazinyl, imidazo[1,2-a]pyrimidinyl, 1,2,4-triazolo[4,3-a]pyridyl and 1,2,4-triazolo[4,3-b]pyridazinyl; or,

(3) a 5- to 10-membered non-aromatic heterocyclic ring such as oxylanyl, azethidinyl, oxethanyl, thiethanyl, pyrrolidinyl, tetrahydrofuryl, piperidinyl, tetrahydropyranyl, morpholinyl, thiomorpholinyl and pyrazinyl.

Among the heterocyclic rings listed in (1) to (3) described above, a 5- or 6-membered heterocyclic ring containing 1 to 3 heteroatoms such as nitrogen, oxygen and sulfur atoms in addition to carbon atoms is employed widely. Such heterocyclic ring may for example be furyl, thienyl, pyrrolyl, oxazolyl, isoxazolyl, imidazolyl, pyrazolyl, pyridyl, pyridazinyl, quinolyl, isoquinolyl, thiazolyl, thiadiazolyl and thiophenyl.

A substituent which may be present on a heterocyclic ring may for example be the substituents similar to those mentioned with regard to an "optionally substituted homocyclic ring" described above.

A more preferred Ring C includes an optionally substituted benzene ring (especially a substituent-substituted benzene ring), such as a benzene ring which may be substituted by 1 to 3 substituents selected from a halogen atom, an optionally halogenated $C_{1-4}$ alkyl group, an optionally halogenated $C_{1-4}$ alkoxy group, a di-$C_{1-4}$ alkylamino group, a $C_{1-3}$ acyloxy group and a hydroxyl group (especially a benzene ring substituted by a substituent described above). Typically, a preferred example of Ring C is an optionally substituted benzene ring represented by Formula (C-1):

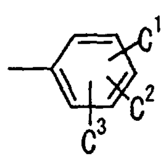

(C-1)

wherein $C^1$, $C^2$ and $C^3$ are same or different and each represents a hydrogen atom, a halogen atom, an optionally halogenated $C_{1-4}$ alkyl group, an optionally halogenated $C_{1-4}$ alkoxy group, a mono- or di-$C_{1-4}$ alkylamino group, a $C_{1-3}$ acyloxy group or a hydroxyl group, or by Formula (C-2):

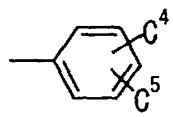

(C-2)

wherein $C^4$ and $C^5$ are same or different and each represents a hydrogen atom, a halogen atom, an optionally halogenated $C_{1-4}$ alkyl group or an optionally halogenated $C_{1-4}$ alkoxy group.

A halogen atom, an optionally halogenated $C_{1-4}$ alkyl group, an optionally halogenated $C_{1-4}$ alkoxy group and a mono- or di-$C_{1-4}$ alkylamino group represented here by $C^1$, $C^2$, $C^3$, $C^4$ or $C^5$ is one exemplified above as a halogen atom, an optionally halogenated $C_{1-4}$ alkyl group, an optionally halogenated $C_{1-4}$ alkoxy group and a mono- or di-$C_{1-4}$ alkylamino group.

A more preferred Ring C includes a benzene ring represented by Formulae C-1 and C-2 shown above in which $C^1$ to $C^5$ are the substituents having the following meanings.

(1) $C^1$, $C^2$ and $C^3$ are same or different and each represents a halogen atom, an optionally halogenated $C_{1-4}$ alkyl group or an optionally halogenated $C_{1-4}$ alkoxy group;
(2) $C^1$, $C^2$ and $C^3$ are same or different and each represents a halogen atom or an optionally halogenated $C_{1-4}$ alkyl group;
(3) $C^1$, $C^2$ and $C^3$ are same or different and each represents a halogen atom;
(4) $C^1$, $C^2$ and $C^3$ are same or different and each represents an optionally halogenated $C_{1-4}$ alkyl group;
(5) $C^1$, $C^2$ and $C^3$ are same or different and each represents an optionally halogenated $C_{1-4}$ alkoxy group;
(6) $C^4$ and $C^5$ are same or different and each represents a halogen atom;
(7) $C^4$ and $C^5$ are same or different and each represents an optionally halogenated $C_{1-4}$ alkyl group; or,
(8) $C^4$ and $C^5$ are same or different and each represents an optionally halogenated $C_{1-4}$ alkoxy group.

In the embodiments (1) to (8) described above, an "optionally halogenated $C_{1-4}$ alkyl group", an "optionally halogenated $C_{1-4}$ alkoxy group" and a "halogen atom" may for example be the respective groups and atoms described above.

A further preferred example of Ring C may for example be a benzene ring represented by Formula C-2 shown above in which $C^4$ and $C^5$ are the substituents having the following meanings.

(a) One of $C^4$ and $C^5$ is a hydrogen atom, and the other is a methoxy group;
(b) $C^4$ and $C^5$ are chlorine atoms;
(c) one of $C^4$ and $C^5$ is a methoxy group, and the other is an isopropyl group;
(c) one of $C^4$ and $C^5$ is a methoxy group, and the other is an isopropyl group;
(d) one of $C^4$ and $C^5$ is a methoxy group, and the other is a 1-methoxy-1-methylethyl group; or,
(e) $C^4$ and $C^5$ are trifluoromethyl groups.

"As to Ring Z"

In a formula shown above, Ring Z is an optionally substituted nitrogen-containing heterocyclic ring. A substituent on Ring Z may for example be any of various substituents, such as alkyl group (for example, a straight or branched alkyl group having 1 to 6, preferably 1 to 4 carbon atoms such as methyl, ethyl, propyl, isopropyl, butyl, isobutyl, sec-butyl and tert-butyl groups, etc.), an alkenyl group (for example, an alkenyl group having 2 to 6, preferably 2 to 4 carbon atoms such as ethenyl, propenyl, isopropenyl, butenyl, isobutenyl and sec-butenyl groups, etc.), an alkynyl group (for example, an alkynyl group having 2 to 6, preferably 2 to 4 carbon atoms such as ethynyl, propynyl, isopropynyl, butynyl, isobutynyl and sec-butynyl groups, etc.), a cycloalkyl group (for example, a $C_{3-8}$ cycloalkyl group, preferably a $C_{3-6}$ cycloalkyl group such as cyclopropyl, cyclobutyl, cyclopentyl and cyclohexyl groups, etc.), a cycloalkyl-alkyl group (for example, a $C_{3-6}$ cycloalkyl-$C_{1-4}$ alkyl group such as cyclopropylmethyl, cyclopropylethyl and cyclohexylmethyl groups, etc.), an aryl group (for example, an aryl group having 6 to 14, preferably 6 to 10 carbon atoms such as phenyl, 1-naphthyl, 2-naphthyl, anthryl and phenanthryl groups, etc., particularly a phenyl group), a nitro group, a cyano group, a hydroxyl group, a $C_{1-4}$ alkoxy group (for example, methoxy, ethoxy, propoxy, isopropoxy and butoxy groups, etc.), a $C_{1-4}$ alkylthio group (for example, methylthio, ethylthio and propylthio groups, etc.), an amino group, a mono- or di-$C_{1-4}$ alkylamino group (for example, methylamino, ethylamino, propylamino, dimethylamino and diethylamino groups, etc.), a cyclic amino group (for example, a 5- to 9-membered cyclic amino group which may contain 1 to 3 heteroatoms such as oxygen and sulfur atoms in addition to nitrogen atoms, typically, for example, pyrrolidino, piperidino, morpholino and thiomorpholino groups, etc.), a $C_{1-4}$ alkyl-carbonylamino group (for example, acetylamino, propionylamino and

butyrylamino groups, etc.), a $C_{1-4}$ alkylsulfonylamino group (for example, methylsulfonylamino and ethylsulfonylamino groups, etc.), a $C_{1-4}$ alkoxy-carbonyl group (for example, methoxycarbonyl, ethoxycarbonyl and propoxycarbonyl groups, etc.), a carboxyl group, a $C_{1-6}$ alkyl-carbonyl group (for example, methylcarbonyl, ethylcarbonyl and propylcarbonyl groups, etc.), a carbamoyl group, a mono- or di-$C_{1-4}$ alkylcarbamoyl group (for example, methylcarbamoyl and ethylcarbamoyl groups), a $C_{1-6}$ alkylsulfonyl group (for example, methylsulfonyl, ethylsulfonyl and propylsulfonyl groups, etc.), an oxo group, a thioxo group and the like. The number of these substituents may vary depending on the size of Ring Z, within the range from 1 to 5, preferably 1 to 2.

Ring Z may be a heterocyclic ring which contains, in addition to Y and a nitrogen atom N, at least one heteroatom selected from nitrogen, oxygen and sulfur atoms, but is more preferably an optionally oxo-derivatized ring.

Ring Z includes a nitrogen-containing heterocyclic ring represented by Formula (Z-1):

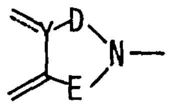

(Z-1)

wherein D and E are the groups which, together with the nitrogen atom adjacent to E, form a Ring Z described above.

At least one of D and E as constituents of Ring Z may for example be an optionally oxo-derivatized alkylene group, an oxyalkylene group and an iminoalkylene group. Preferably, each of D and E is frequently an optionally oxo-derivatized alkylene group and an oxyalkylen group. An optionally oxo-derivatized alkylene group, an oxyalkylene group and an iminoalkylene group represented by D and E preferably has carbon atoms in a number which gives Ring Z which forms a 5- to 12-membered, preferably 5- to 9-membered ring. The number of carbon atoms of the alkylene group represented by D may be different from that by E.

A preferred example of D includes an optionally oxo-derivatized $C_{1-7}$ alkylene group (especially an optionally oxo-derivatized $C_{1-5}$ alkylene group), a $C_{1-7}$ oxyalkylene group (especially a $C_{1-5}$ oxyalkylene group) and a $C_{1-7}$ iminoalkylene group (especially a $C_{1-5}$ iminoalkylene group). A more preferred example of D includes an alkylene group represented by Formula:$-(CH_2)_m-$ wherein m is 1 to 7, an oxyalkylene group represented by Formula:$-O-(CH_2)_p-$ wherein p is an integer of 1 to 7 and an iminoalkylene group represented by Formula:$-NH-(CH_2)_q-$ wherein q is an integer of 1 to 7. In the formulae shown above, each of m and p is preferably 1 to 5, especially 2 to 5.

A preferred example of E includes an optionally oxo-derivatized $C_{1-3}$ alkylene group, especially, an optionally oxo-derivatized alkylene group having 1 to 2 carbon atoms, particularly an optionally oxo-derivatized methylene group.

The number of oxo groups by which Ring Z described above can be substituted is not particularly limited, may vary depending on the size of Ring Z within the range from 1 to 3, while the number of oxo groups is 1 to about 2 when Ring Z is a 5- to 10-membered ring. An oxo group may be a substituent on at least one of D and/or E. In a preferred example of Ring Z, an oxo group is a substituent on E.

In a preferred example of Ring Z, D is an alkylene or oxyalkylene group having 1 to 5, particularly 2 to 5 carbon atoms, and E is an oxo-derivatized alkylene having 1 to 2 carbon atoms, especially >C=O. A preferred example of Ring Z includes a 5- to 9-membered nitrogen-containing heterocyclic ring represented by Formula (Z-2):

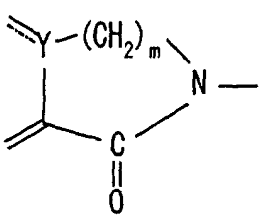

or

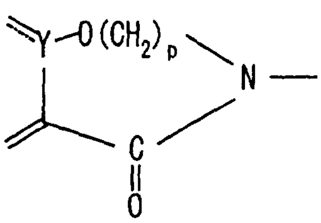

(Z-2)

wherein each of m and p represents an integer of 1 to 5.

<u>"As to n"</u>

In a formula shown above, n represents an integer of 1 to 6, more preferably 1 to 3, most preferably 1 or 2. Particularly, n is 1.

As to Compound (I) and (Ia)

In a compound represented by Formula (I) and Formula (Ia) shown above, the combination of Ring M, —X=Y<, $R^a$, $R^b$, Ring A, Ring B, Ring C, Ring Z and n is not particularly limited, and any suitable combination can be used to build Compound (I) or (Ia). Preferred Compound (I) or (Ia) can be built by combining Ring M, —X=Y<, $R^a$, $R^b$, Ring A, Ring B, Ring C, Ring Z and n which are the preferred embodiments described above.

Among the compounds represented by Formula (I), especially by Formula (Ia), preferred Compound (1) includes the following compound and a pharmaceutically acceptable salt thereof.

A compound wherein:

one of Ring A and Ring B is a 5- or 6-membered heterocyclic ring containing 1 or 2 heteroatoms selected from nitrogen and sulfur atoms in addition to carbon atoms and the other is a benzene ring, wherein each of Ring A and Ring B may have 1 or 2 substituents selected from a halogen atom and an optionally halogenated $C_{1-4}$ alkyl group;
Ring C is a benzene ring which may have 1 to 3 substituents selected from a halogen atom, an optionally halogenated $C_{1-6}$ alkyl group (preferably a $C_{1-4}$ alkyl group) and an optionally halogenated $C_{1-6}$ alkoxy group (preferably a $C_{1-4}$ alkoxy group);
D as a constituent of Ring Z is - $(CH_2)_m$- wherein m denotes an integer of 1 to 7 or -O-$(CH_2)_p$- wherein p is an integer of 1 to 7;
E as a constituent of Ring Z is >C=O;
—X=Y Y< is -CO-N< or -N=C<; and,
n is 1,

as well as a pharmaceutically acceptable salt thereof.

A "5- or 6-membered heterocyclic ring" described above may for example be pyridine, pyrazine, pyrrole, thiophene, thiazole, tetrahydropyrazine and piperidine, and one exemplified typically as Ring A is a heterocyclic ring represented by Formula (A-5) shown above and one exemplified as Ring B is a benzene ring represented by Formulae (B-7) and (B-8), especially Formula (B-10) shown above.

A "halogen atom" described above may for example be fluorine, chlorine and bromine atoms, etc., and an "optionally halogenated $C_{1-4}$ alkyl group" may for example be methyl, chloromethyl, difluoromethyl, trichloromethyl, trifluoromethyl, ethyl, 2-bromoethyl, 2,2,2-trifluoroethyl, perfluoroethyl, propyl, 3,3,3-trifluoropropyl, isopropyl, 2-trifluoromethylethyl, butyl, 4,4,4-trifluorobutyl, isobutyl, sec-butyl and tert-butyl groups, etc., and an "optionally halogenated $C_{1-6}$ alkyl group" may for example be pentyl and hexyl groups, etc. in addition to those exemplified above as alkyl and halogenated alkyl groups.

An "optionally halogenated $C_{1-4}$ alkoxy group" may for example be methoxy, difluoromethoxy, trifluoromethoxy, ethoxy, 2,2,2-trifluoroethoxy, perfluoroethoxy, propoxy, isopropoxy, butoxy, 4,4,4-trifluorobutoxy, isobutoxy, sec-butoxy and tert-butoxy groups, and an "optionally halogenated $C_{1-6}$ alkoxy group" may for example be pentyloxy and hexyloxy groups in addition to those exemplified above as alkoxy and halogenated alkoxy groups.

Among the compounds represented by Formula (I), especially by Formula (Ia), preferred Compound (2) includes the following compound and a pharmaceutically acceptable salt thereof.

A compound wherein:

Ring A is a 5- or 6-membered heterocyclic ring containing one nitrogen atom and one sulfur atom in addition to carbon atoms, such as, for example, a heterocyclic ring represented by Formula (A-7):

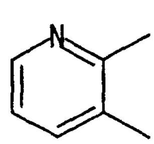 , 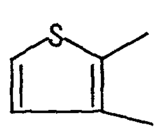 (A-7)

Ring B is a benzene ring which may have 1 to 3 substituents selected from a halogen atom and an optionally halogenated $C_{1-4}$ alkyl group;
Ring C is a benzene ring which may have 1 to 3 substituents selected from a halogen atom, an optionally halogenated $C_{1-4}$ alkyl group and an optionally halogenated $C_{1-4}$ alkoxy group;
D as a constituent of Ring Z is -$(CH_2)_m$- wherein m represents an integer of 1 to 7 or -O-$(CH_2)_p$- wherein p is an integer of 1 to 7;
E as a constituent of Ring Z is >C=O;

—X=Y< is -CO-N<; and,
n is 1,

as well as a pharmaceutically acceptable salt thereof.

A "halogen atom", an "optionally halogenated $C_{1-4}$ alkyl group" and an "optionally halogenated $C_{1-4}$ alkoxy group" may for example be the atoms and the groups exemplified with regard to Compound (1) described above.

One exemplified more preferably is a compound wherein:

$R^a$ and $R^b$ are same or different and each represents a hydrogen atom or [1] a $C_{1-6}$ alkoxy group, [2] a $C_{1-6}$ alkylthio group, [3] an amino group, [4] a $C_{1-7}$ acylamino group, [5] a mono- or di-$C_{1-6}$ alkylamino group, [6] a $C_{3-10}$ cyclic amino group, [7] an optionally $C_{1-6}$ alkyl group-substituted 5- or 6-membered cyclic amino group, [8] a $C_{1-6}$ alkylsulfonylamino group or [9] an optionally $C_{1-6}$ alkylcarbonyloxy group-substituted $C_{1-6}$ alkyl group; or,
$R^a$ and $R^b$ are taken together to form a pyridine ring which may have 1 to 3 substituents selected from a halogen atom or a $C_{1-4}$ alkyl group;
Ring B is a benzene ring which may have 1 to 3 substituents selected from [1] a halogen atom, [2] an optionally halogenated $C_{1-4}$ alkyl group or [3] an optionally halogenated $C_{1-4}$ alkoxy group;
Ring C is a benzene ring which may have 1 to 3 substituents selected from [1] a halogen atom, [2] an optionally halogenated $C_{1-4}$ alkyl group, [3] an optionally halogenated $C_{1-4}$ alkoxy group, [4] an optionally $C_{1-4}$ alkyl group-substituted amino group, [5] a $C_{1-3}$ acyloxy group or [6] a hydroxyl group;
Ring Z is a 5- to 10-membered nitrogen-containing heterocyclic group which may be substituted by a $C_{1-4}$ alkyl group or a hydroxyl group or which may be oxo-derivatized;
— X=Y< is -CO-N< or -N=C<; and,
n is 1,

as well as a pharmaceutically acceptable salt thereof.

A preferred example of Compounds (I) and (Ia) includes a compound represented by Formula:

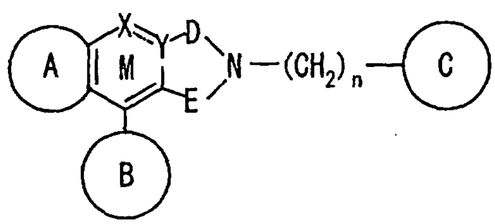

wherein each of D and E is an optionally oxo-derivatized alkylene group and other symbols are defined as described above, as well as a salt thereof.

Preferably, each of D and E is a $C_{1-3}$ alkylene group which may be substituted by one oxo group.

A further preferred example of Compounds (I) and (Ia) includes a compound represented by Formula:

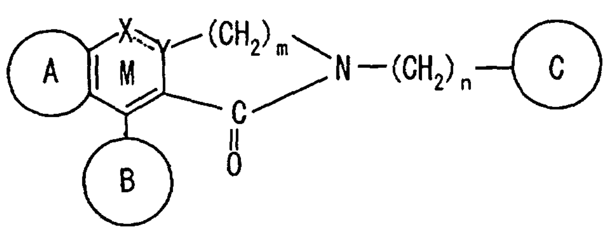

wherein m is an integer of 1 to 7 and other symbols are defined as described above,
as well as a pharmaceutically acceptable salt thereof.

Preferably, m is an integer of 2 to 5.

In a formula describe above, it is preferred that each of $R^a$ and $R^b$ represents a hydrogen atom or a substituent selected from the group consisting of (I) a halogen atom, (II) a $C_{1-6}$ alkyl group which may have 1 to 5 substituents selected from (i) a hydroxy group, (ii) a $C_{1-6}$ alkoxy group, (iii) a $C_{1-6}$ alkylthio group, (iv) an amino group, (v) a $C_{1-7}$ acylamino group, (vi) a mono- or di-$C_{1-6}$ alkylamino group, (vii) a mono- or di-$C_{3-8}$ cycloalkylamino group, (viii) a 5- to 9-membered cyclic amino group which may be substituted by a $C_{1-6}$ alkyl and which may contain 1 to 3 heteroatoms

selected from oxygen and sulfur atoms in addition to nitrogen atoms, (ix) a $C_{1-4}$ alkylsulfonylamino group, (x) a $C_{1-6}$ alkylcarbonyloxy group and (xi) a halogen atom, (III) a 5- to 9-membered (preferably 6-membered) cyclic amino group which may be substituted by a $C_{1-6}$ alkyl and which may contain 1 to 3 (preferably 1 or 2) heteroatoms selected from oxygen and sulfur atoms in addition to nitrogen atoms, (IV) a carboxyl group, (V) a carbamoyl group and (VI) a mono- or di-$C_{1-6}$ alkylcarbamoyl group; or $R^a$ and $R^b$ are taken together to form Ring A;

Ring A is a 5- to 9-membered aromatic heterocyclic ring (preferably a pyridine ring) which may be substituted by a $C_{1-6}$ alkyl group and which may contain 1 to 3 of one or two kinds of heteroatoms selected from nitrogen, sulfur and oxygen in addition to carbon atoms;
Ring B is a $C_{6-14}$ aryl group (preferably a benzene ring) which may be substituted by a substituent selected from the group consisting of (i) an optionally hydroxy group-substituted $C_{1-6}$ alkyl group, (ii) a $C_{1-6}$ alkylcarbonyl group (including formyl) and (iii) a carboxyl group;
Ring C is a $C_{6-14}$ aryl group (preferably a benzene ring) which may be substituted by 1 to 3 substituents selected from the group consisting of (i) a halogen atom, (ii) an optionally halogenated $C_{1-10}$ alkyl group and (iii) a $C_{1-10}$ alkoxy group;
Ring Z is a 5- to 12-membered heterocyclic ring which may be substituted by 1 to 3 substituents selected from the group consisting of (i) a $C_{1-6}$ alkyl group, (ii) a hydroxy group and (iii) an oxo group and which may contain at least one heteroatom selected from the group consisting of nitrogen, oxygen and sulfur atoms in addition to Y and nitrogen atoms.

A compound represented by Formula (I) and (Ia) shown above may form a salt, which is preferably a pharmaceutically acceptable salt when it is used as a pharmaceutical.

A pharmaceutically acceptable salt may for example be a salt with an inorganic acid such as hydrochloric acid, sulfuric acid, phosphoric acid, diphosphoric acid, hydrobromic acid and nitric acid, or a salt with an organic acid such as acetic acid, malic acid, maleic acid, fumaric acid, tartaric acid, succinic acid, citric acid, lactic acid, methanesulfonic acid, p-toluenesulfonic acid, palmitic acid, salicylic acid and stearic acid.

Compounds (I) and (Ia) and the salts thereof employed in the invention include respective stereoisomers such as cis- and trans-isomers, racemates and optically active forms such as R and S forms. Depending on the size of Ring Z or other moieties, a conformation-based isomers may exist and such isomers are also included in Compounds (I) and (Ia) and the salts thereof.

Preferred examples of Compounds (I) and (Ia) are listed below.

(1) 7-[3,5-Bis(trifluoromethyl)benzyl]-6,7,8,9-tetrahydro-5-(4-methylphenyl)-6,11-dioxo-11H-pyrazino[2,1-g][1,7]naphthylidine (hereinafter sometimes abbreviated as Compound No.1)
(2) 7-[3,5-Bis(trifluoromethyl)benzyl]-6,7,8,9,10,12-hexahydro-5-(4-methylphenyl)-6,12-dioxo-[1,4]diazepino[2,1-g] [1,7]naphthylidine (hereinafter sometimes abbreviated as Compound No.2)
(3) 7-[3,5-Bis(trifluoromethyl)benzy-1]-6,7,8,9,10,11-hexahydro-5-(4-methylphenyl)-6,13-dioxo-13H-[1,4]diazosino[2,1-g][1,7]naphthylidine
(4) 6,7,8,9,10,12-Hexahydro-7-(2-methoxybenzyl)-5-(4-methylphenyl)-6,12-dioxo[1,4]diazepino[2,1-g][1,7]naphthylidine
(5) 6,7,8,9,10,11-Hexahydro-7-(2-methoxybenzyl)-5-(4-methylphenyl)-6,13-dioxo-13H-[1,4]diazosino[2,1-g][1,7]naphthylidine
(6) 7-[3,5-Bis(trifluoromethyl)benzyl]-6,7,8,9,10,11,12,14-octahydro-5-(4-methylphenyl)-6,14-dioxo[1,4]diazonino[2,1-g][1,7]naphthylidine
(7) 7-[3,5-Bis(trifluoromethyl)benzyl]-6,7,8,9,10,12-hexahydro-6,12-dioxo-5-phenyl[1,4]diazepino[2,1-g][1,7]naphthylidine
(8) 7-[3,5-Bis(trifluoromethyl)benzyl]-6,7,8,9,10,11-hexahydro-6,13-dioxo-5-phenyl-13H-[1,4]diazosino[2,1-g][1,7]naphthylidine
(9) 7-[3,5-Bis(trifluoromethyl)benzyl]-6,7,8,9,10,11-hexahydro-5-(4-methylphenyl)-6,13-dioxo-13H-[1,4]diazosino[1,2-b] [2,7]naphthylidine
(10) 7-[3,5-Bis(trifluoromethyl)benzyl]-1,2,3,4,6,7,8,9,10,11-decahydro-2-methyl-5-(4-methylphenyl)-6,13-dioxo-13H-[1,4]diazosino[1,2-b][2,7]naphthylidine
(11) 4-[3,5-Bis(trifluoromethyl)benzyl]2,3,4,5-tetrahydro-5-oxo-6-phenylpyrido[3,2-f][1,4]oxazepine
(12) (9R)-7-[3,5-Bis(trifluoromethyl)benzyl]-6,7,8,9,10,12-hexahydro-9-methyl-5-(4-methylphenyl)-6,12-dioxo[1,4]diazepino[2,1-g] [1,7]naphthylidine
(13) (9S)-7-[3,5-Bis(trifluoromethyl)benzyl]-6,7,8,9,10,12-hexahydro-9-methyl-6,12-dioxo-5-phenyl[1,4]diazepino[2,1-g] [1,7]naphthylidine
(14) (9S)-7-[3,5-Bis(trifluoromethyl)benzyl]-6,7,8,9,10,12-hexahydro-9-methyl-5-(4-methylphenyl)-6,12-dioxo

[1,4]diazepino[2,1-g] [1,7]naphthylidine
(15) (±)-7-[3,5-Bis(trifluoromethyl)benzyl]-6,7,8,9,10,11-hexahydro-9-methyl-6,13-dioxo-5-phenyl-13H-[1,4]diazosino[2,1-g] [1,7]naphthylidine
(16) (±)-7-[3,5-Bis(trifluoromethyl)benzyl]-6,7,8,9,10,11-hexahydro-9-methyl-5-(4-methylphenyl)-6,13-dioxo-13H-[1,4]diazosino[2,1-g][1,7]naphthylidine
(17) (9R)-7-[3,5-Bis(trifluoromethyl)benzyl]-6,7,8,9,10,11-hexahydro-9-methyl-6,13-dioxo-5-phenyl-13H-[1,4]diazosino[2,1-g][1,7]naphthylidine
(18) (9R)-7-[3,5-Bis(trifluoromethyl)benzyl]-6,7,8,9,10,11-hexahydro-9-methyl-5-(4-methylphenyl)-6,13-dioxo-13H-[1,4]diazosino[2,1-g][1,7]naphthylidine (hereinafter sometimes abbreviated as-Compound No.3)
(19) (9S)-7-[3,5-Bis(trifluoromethyl)benzyl]-6,7,8,9,10,11-hexahydro-9-methyl-6,13-dioxo-5-phenyl-13H-[1,4]diazosino[2,1-g] [1,7]naphthylidine
(20) (9S)-7-[3,5-Bis(trifluoromethyl)benzyl]-6,7,8,9,10,11-hexahydro-9-methyl-5-(4-methylphenyl)-6,13-dioxo-13H-[1,4]diazosino[2,1-g] [1,7]naphthylidine
(21) 4-[3,5-Bis(trifluoromethyl)benzyl]-2,3,4,5-tetrahydro-5-oxo-6-phenyl-lH-pyrido[2,3-e] [1,4]diazepine
(22) 5-[3,5-Bis(trifluoromethyl)benzyl]-2,3,4,5-tetrahydro-6-oxo-7-phenyl-6H-pyrido[2,3-b] [1,5]oxazosine
(23) 4-[3,5-Bis(trifluoromethyl)benzyl]-2,3,4,5-tetrahydro-7-methyl-5-oxo-6-phenylpyrido[3,2-f][1,4]oxazepine
(24) 5-[3,5-Bis(trifluoromethyl)benzyl]-2,3,4,5-tetrahydro-8-methyl-6-oxo-7-phenyl-6H-pyrido[2,3-b][1,5]oxazosine
(25) (±)-7-[3,5-Bis(trifluoromethyl)benzyl]-6,7,8,9,10,12-hexahydro-9-hydroxy-5-(4-methylphenyl)-6,12-dioxo[1,4]diazepino[2,1-g][1,7]naphthylidine
(26) 7-Benzyl-6,7,8,9,10,12-hexahydro-6,12-dioxo-5-phenyl[1,4]diazepino[2,1-g][1,7]naphthylidine
(27) 7-Benzyl-6,7,8,9,10,11-hexahydro-6,13-dioxo-5-phenyl-13H-[1,4]diazosino[2,1-g][1,7]naphthylidine
(28) 7-Benzyl-6,7,8,9,10,11,12,14-oxtahydro-6,14-dioxo-5-phenyl[1,4]diazonino[2,1-g][1,7]naphthylidine
(29) 7-(3,4-Dichlorobenzyl)-6,7,8,9,10,12-hexahydro-6,12-dioxo-5-phenyl[1,4]diazepino[2,1-g][1,7]naphthylidine
(30) 7-(3,4-Dichlorobenzyl)-6,7,8,9,10,11-hexahydro-6,13-dioxo-5-phenyl-13H-[1,4]diazosino[2,1-g][1,7]naphthylidine
(31) (S)-5-[3,5-Bis(trifluoromethyl)benzyl]-2,3,4,5-tetrahydro-3,8-dimethyl-6-oxo-7-phenyl-6H-pyrido[2,3-b][1,5]oxazosine
(32) (R)-5-[3,5-Bis(trifluoromethyl)benzyl]-2,3,4,5-tetrahydro-3,8-dimethyl-6-oxo-7-phenyl-6H-pyrido[2,3-b][1,5]oxazosine
(33) 4-[3,5-Bis(trifluoromethyl)benzyl]-2,3,4,5-tetrahydro-8-methyl-5-oxo-5-phenylpyrido [3,2-f][1,4]oxazepine
(34) 5-[3,5-Bis(trifluoromethyl)benzyl]-2,3,4,5-tetrahydro-9-methyl-6-oxo-7-phenyl-6H-pyrido[2,3-b][1,5]oxazosine
(35) 4-[3,5-Bis(trifluoromethyl)benzyl]-2,3,4,5-tetrahydro-8-methyl-5-oxo-6-phenylpyrido[3,2-f][1,4]oxazepine 9-oxide
(36) 5-[3,5-Bis(trifluoromethyl)benzyl]-2,3,4,5-tetrahydro-9-methyl-6-oxo-7-phenyl-6H-pyrido[2,3-b][1,5]oxazosine 10-oxide
(37) 8-Acetoxymethyl-4-[3,5-bis(trifluoromethyl)benzyl]-2,3,4,5-tetrahydro-5-oxo-6-phenylpyrido[3,2-f][1,4]oxazepine
(38) 9-Acetoxymethyl-5-[3,5-bis(trifluoromethyl)benzyl]-2,3,4,5-tetrahydro-6-oxo-7-phenyl-6H-pyrido[2,3-b][1,5]oxazosine
(39) 4-[3,5-Bis(trifluoromethyl)benzyl]-8-chloromethyl-2,3,4,5-tetrahydro-5-oxo-6-phenylpyrido[3,2-f][1,4]oxazepine
(40) 4-[3,5-Bis(trifluoromethyl)benzyl]-2,3,4,5-tetrahydro-8-methoxymethyl-5-oxo-6-phenylpyrido[3,2-f][1,4]oxazepine
(41) 4-[3,5-Bis(trifluoromethyl)benzyl]-2,3,4,5-tetrahydro-8-(2-methylethyl)-5-oxo-6-phenylpyrido[3,2-f][1,4]oxazepine
(42) 4-[3,5-Bis(trifluoromethyl)benzyl]-2,3,4,5-tetrahydro-8-methylthiomethyl-5-oxo-6-phenylpyrido[3,2-f] [1,4]oxazepine
(43) 8-Aminomethyl-4-[3,5-bis(trifluoromethyl)benzyl]-2,3,4,5-tetrahydro-5-oxo-6-phenylpyrido[3,2-f][1,4]oxazepine
(44) 4-[3,5-Bis(trifluoromethyl)benzyl]-2,3,4,5-tetrahydro-8-methylaminomethyl-5-oxo-6-phenylpyrido[3,2-f][1,4]oxazepine
(45) 4-[3,5-Bis(trifluoromethyl)benzyl]-8-dimethylamimomethyl-2,3,4,5-tetrahydro-5-oxo-6-phenylpyrido[3,2-f][1,4]oxazepine
(46) 4-[3,5-Bis(trifluoromethyl)benzyl]-8-cyclopropylaminomethyl-2,3,4,5-tetrahydro-5-oxo-6-phenylpyrido[3,2-f][1,4]oxazepine
(47) 4-[3,5-Bis(trifluoromethyl)benzyl]-2,3,4,5-tetrahydro-8-(N-methylpiperazinomethyl)-5-oxo-6-phenylpyrido[3,2-f][1,4]oxazepine

(48) 8-Acetylaminomethyl-4-[3,5-bis(trifluoromethyl)benzyl]-2,3,4,5-tetrahydro-5-oxo-6-phenylpyrido[3,2-f][1,4]oxazepine

(49) 4-[3,5-Bis(trifluoromethyl)benzyl]-2,3,4,5-tetrahydro-8-methanesulfonylaminomethyl-5-oxo-6-phenylpyrido[3,2-f][1,4]oxazepine

(50) 6-[3,5-Bis(trifluoromethyl)benzyl]-5,6,7,8-hexahydro-3,5-dimethyl-5,10-dioxo-4-phenylpyrido[2,3-f][1,4]diazosine

(51) 6-[3,5-Bis(trifluoromethyl)benzyl]-5,6,7,8,9,10-hexahydro-9-methyl-5,10-dioxo-4-phenylpyrido[2,3-f][1,4]diazosine

(52) 6-Benzyl-5,6,7,8,9,10-hexahydro-3,9-dimethyl-5,10-dioxo-4-phenylpyrido[2,3-f][1,4]diazosine

(53) 6-[3,5-Bis(trifluoromethyl)benzyl]-9-ethyl-5,6,7,8,9,10-hexahydro-3-methyl-5,10-dioxo-4-phenylpyrido[2,3-f][1,4]diazosine

(54) 6-[3,5-Bis(trifluoromethyl)benzyl]-6,7,8,9,10,11-hexahydro-3,10-dimethyl-5,11-dioxo-4-phenyl-5H-pyrido[2,3-g][1,5]diazonine

(55) 4-[3,5-Bis(trifluoromethyl)benzyl]-8-hydroxymethyl-2,3,4,5-tetrahydro-5-oxo-6-phenylpyrido[3,2-f] [1,4]oxazepine

(56) 4-[3,5-Bis(trifluoromethyl)benzyl]-2,3,4,5-tetrahydro-5-oxo-6-phenylpyrido[3,2-f][1,4]oxazepine-8-carboxylic acid

(57) 4-[3,5-Bis(trifluoromethyl)benzyl]-2,3,4,5-tetrahydro-5-oxo-6-phenylpyrido[3,2-f][1,4]oxazepine-8-carboxamide

(58) 4-[3,5-Bis(trifluoromethyl)benzyl]-N-methyl-2,3,4,5-tetrahydro-5-oxo-6-phenylpyrido[3,2-f][1,4]oxazepine-8-carboxamide

(59) 4-[3,5-Bis(trifluoromethyl)benzyl]-N,N-dimethyl-2,3,4,5-tetrahydro-5-oxo-6-phenylpyrido[3,2-f] [1,4]oxazepine-8-carboxamide

(60) 4-[3,5-Bis(trifluoromethyl)benzyl]-N-n-butyl-2,3,4,5-tetrahydro-5-oxo-6-phenylpyrido[3,2-f][1,4]oxazepine-8-carboxamide

(61) 4-[3,5-Bis(trifluoromethyl)benzyl]-2,3,4,5-tetrahydro-5-oxo-6-phenyl-8-piperidinocarbonylpyrido[3,2-f][1,4]oxazepine

(62) 4-[3,5-Bis(trifluoromethyl)benzyl]-2,3,4,5-tetrahydro-8-morpholinocarbonyl-5-oxo-6-phenylpyrido[3,2-f][1,4]oxazepine

(63) 4-[3,5-Bis(trifluoromethyl)benzyl]-2,3,4,5-tetrahydro-8-[1-(4-methylpiperazinyl)carbonyl] -5-oxo-6-phenylpyrido[3,2-f][1,4]oxazepine

(64) 2,3,4,5-Tetrahydro-5-oxo-6-phenyl-4-(3,4,5-trimethoxybenzyl)pyrido[3,2-f][1,4]oxazepine

(65) 4-(3,4-Dichlorobenzyl)-2,3,4,5-tetrahydro-5-oxo-6-phenylpyrido[3,2-f][1,4]oxazepine

(66) 4-(3,4-Dimethoxybenzyl)-2,3,4,5-tetrahydro-5-oxo-6-phenylpyrido[3,2-f][1,4]oxazepine

(67) 4-Benzyl-2,3,4,5-tetrahydro-5-oxo-6-phenylpyrido[3,2-f][1,4]oxazepine

(68) 2,3,4,5-Tetrahydro-6-oxo-7-phenyl-5-(3,4,5-trimethoxybenzyl)-6H-pyrido[2,3-b][1,5]oxazosine

(69) (S)-5-Benzyl-2,3,4,5-tetrahydro-3-methyl-6-oxo-7-phenyl-6H-pyrido[2,3-b][1,5]oxazosine

(70) (R)-5-Benzyl-2,3,4,5-tetrahydro-3-methyl-6-oxo-7-phenyl-6H-pyrido[2,3-b][1,5]oxazosine

(71) (S)-5-[3,5-Bis(trifluoromethyl)benzyl]-2,3,4,5-tetrahydro-3-methyl-6-oxo-7-phenyl-6H-pyrido[2,3-b][1,5]oxazosine

(72) (R)-5-[3,5-Bis(trifluoromethyl)benzyl]-2,3,4,5-tetrahydro-3-methyl-6-oxo-7-phenyl-6H-pyrido[2,3-b][1,5]oxazosine

(73) 7-Benzyl-6,7,8,9,10,11-hexahydro-5-(4-methylphenyl)-6,13-dioxo-13H-[1,4]diazosino[2,1-g][1,7]naphthylidine

(74) (9R)-7-Benzyl-6,7,8,9,10,11-hexahydro-9-methyl-5-(4-methylphenyl)-6,13-dioxo-13H-[1,4]diazosino[2,1-g][1,7]naphthylidine

(75) (9S)-7-Benzyl-6,7,8,9,10,11-hexahydro-9-methyl-5-(4-methylphenyl)-6,13-dioxo-13H-[1,4]diazosino[2,1-g][1,7]naphthylidine

(76) (9R)-6,7,8,9,10,11-Hexahydro-9-methyl-5-(4-methylphenyl)-6,13-dioxo-7-(3,4,5-trimethoxybenzyl)-13H-[1,4]diazosino[2,1-g][1,7]naphthylidine

(77) (9S)-6,7,8,9,10,11-Hexahydro-9-methyl-5-(4-methylphenyl)-6,13-dioxo-7-(3,4,5-trimethoxybenzyl)-13H-[1,4]diazosino[2,1-g][1,7]naphthylidine

(78) (9R)-7-(3,5-Dimethoxybenzyl)-6,7,8,9,10,11-hexahydro-9-methyl-5-(4-methylphenyl)-6,13-dioxo-13H-[1,4]diazosino[2,1-g][1,7]naphthylidine

(79) 4-Benzyl-2,3,4,5-tetrahydro-5-oxo-6-(4-methylphenyl)pyrido[3,2-f][1,4]oxazepine

(80) 4-[3,5-Bis(trifuoromethyl)benzyl]-2,3,4,5-tetrahydro-5-oxo-6-(4-methylphenyl)pyrido[3,2-f][1,4]oxazepine

(81) (S)-5-Benzyl-2,3,4,5-tetrahydro-3-methyl-7-(4-methylphenyl)-6-oxo-6H-pyrido[2,3-b][1,5]oxazosine

(82) (S)-5-[3,5-Bis(trifluoromethyl)benzyl]-2,3,4,5-tetrahydro-3-methyl-7-(4-methylphenyl)-6-oxo-6H-pyrido[2,3-b][1,5]oxazosine

(83) (9R)-7-[3,5-Bis(trifluoromethyl)benzyl]-6,7,8,9,10,11-hexahydro-5-(4-hydroxymethylphenyl)-9-methyl-6,13-dioxo-13H-[1,4]diazosino[2,1-g][1,7]naphthylidine

(84) (9R)-7-[3,5-Bis(trifluoromethyl)benzyl]-5-(4-formylphenyl)-6,7,8,9,10,11-hexahydro-9-methyl-6,13-dioxo-13H-[1,4]diazosino[2,1-g][1,7]naphthylidine

(85) (9R)-7-[3,5-Bis(trifluoromethyl)benzyl]-5-(4-formylphenyl)-6,7,8,9,10,11-hexahydro-9-methyl-6,13-dioxo-13H-[1,4]diazosino[2,1-g][1,7]naphthylidine

(86) (9R)-7-[3,5-Bis(trifluoromethyl)benzyl]-5-(4-carboxyphenyl)-6,7,8,9,10,11-hexahydro-9-methyl-6,13-dioxo-13H-[1,4]diazosino[2,1-g][1,7]naphthylidine

(87) (9R)-7-[3,5-Bis(trifluoromethyl)benzyl]-6,7,8,9,10,11-hexahydro-9-methyl-5-(4-methylphenyl)-6,13-dioxo-13H-[1,4]diazosino[2,1-g][1,7]naphthylidine N-oxide

(88) (9R)-7-[3,5-Bis(trifluoromethyl)benzyl]-6,7,8,9,10,11-hexahydro-5-(4-hydroxymethylphenyl)-9-methyl-6,13-dioxo-13H-[1,4]diazosino[2,1-g][1,7]naphthylidine N-oxide

(89) (9R)-7-[3,5-Bis(trifluoromethyl)benzyl]-5-(4-carboxyphenyl)-6,7,8,9,10,11-hexahydro-9-methyl-6,13-dioxo-13H-[1,4]diazosino[2,1-g][1,7]naphthylidine N-oxide

(90) (9S)-[10,10,11,11-$^2H_4$]-7-[3,5-Bis(trifluoromethyl)benzyl]-8,9,10,11-tetrahydro-5-(4-hydroxymethylphenyl)-9-methyl-7H-[1,4]diazosino[2,1-g][1,7]naphthylidine-6,13-dione ($d_4$ form of the compound of (83) listed above)

(91) (9S)-[10,10,11,11-$^2H_4$]-7-[3,5-Bis(trifluoromethyl)benzyl]-5-(4-formylphenyl)-8,9,10,11-tetrahydro-9-methyl-7H-[1,4]diazosino[2,1-g] [1,7]naphthylidine-6,13-dione ($d_4$ form of the compound of (84) listed above)

(92) (9S)-[10,10,11,11-$^2H_4$]-7-[3,5-Bis(trifluoromethyl)benzyl]-5-(4-carboxyphenyl) -8,9,10,11-tetrahydro-9-methyl-7H-[1,4]diazosino[2,1-g] [1,7]naphthylidine-6,13-dione ($d_4$ form of the compound of (86) listed above)

(93) (9R)-7-[3,5-Di(benzyloxy)benzyl]-6,7,8,9,10,11-hexahydro-9-methyl-5-(4-methylphenyl)-6,13-dioxo-13H-[1,4]diazosino[2,1-g][1,7]naphthylidine

(94) (9R)-7-(3,5-Dihydroxybenzyl)-6,7,8,9,10,11-hexahydro-9-methyl-5-(4-methylphenyl)-6,13-dioxo-13H-[1,4]diazosino[2,1-g][1,7]naphthylidine

(95) (9R)-7-(3,5-Diethoxybenzyl)-6,7,8,9,10,11-hexahydro-9-methyl-5-(4-methylphenyl)-6,13-dioxo-13H-[1,4]diazosino[2,1-g] [1,7]naphthylidine

(96) (9R)-7-[3,5-Di(1-methylethyloxy)benzyl]-6,7,8,9,10,11-hexahydro-9-methyl-5-(4-methylphenyl)-6,13-dioxo-13H-[1,4]diazosino[2,1-g] [1,7]naphthylidine

(97) (9R)-7-(3,5-Dimethoxybenzyl)-6,7,8,9,10,11-hexahydro-9-methyl-6,13-dioxo-5-phenyl-13H-[1,4]diazosino [2,1-g] [1,7]naphthylidine

(98) (9R)-7-[3,5-Bis(trifluoromethyl)benzyl]-5-(4-chlorophenyl)-6,7,8,9,10,11-hexahydro-9-methyl-6,13-dioxo-13H-[1,4]diazosino[2,1-g] [1,7]naphthylidine

(99) (9R)-7-[3,5-Bis(trifluoromethyl)benzyl]-5-(3,4-dichlorophenyl)-6,7,8,9,10,11-hexahydro-9-methyl-6,13-dioxo-13H-[1,4]diazosino[2,1-g][1,7]naphthylidine

(100) (9R)-7-(3,5-Dimethoxybenzyl)-5-(3,4-dichlorophenyl)-6,7,8,9,10,11-hexahydro-9-methyl-6,13-dioxo-13H-[1,4]diazosino[2,1-g] [1,7]naphthylidine

(101) (9R)-7-(3,5-Dimethylbenzyl)-6,7,8,9,10,11-hexahydro-9-methyl-5-(4-methylphenyl)-6,13-dioxo-13H-[1,4]diazosino[2,1-g][1,7]naphthylidine

(102) (9R)-7-(3,5-Dichlorobenzyl)-6,7,8,9,10,11-hexahydro-9-methyl-5-(4-methylphenyl)-6,13-dioxo-13H-[1,4]diazosino[2,1-g][1,7]naphthylidine

(103) (9R)-5-(3,4-Dichlorophenyl)-7-(3,5-dimethylbenzyl)-6,7,8,9,10,11-hexahydro-9-methyl-6,13-dioxo-13H-[1,4]diazosino[2,1-g][1,7]naphthylidine

(104) (9R)-7-(3,5-Dimethoxybenzyl)-5-(4-fluorophenyl)-6,7,8,9,10,11-hexahydro-9-methyl-6,13-dioxo-13H-[1,4]diazosino[2,1-g][1,7]naphthylidine

(105) (9R)-5-(4-Chlorophenyl)-7-(3,5-dimethoxybenzyl)-6,7,8,9,10,11-hexahydro-9-methyl-6,13-dioxo-13H-[1,4]diazosino[2,1-g][1,7]naphthylidine

(106) (9R)-7-[3,5-Bis(trifluoromethyl)benzyl]-5-(4-fluorophenyl)-6,7,8,9,10,11-hexahydro-9-methyl-6,13-dioxo-13H-[1,4]diazosino[2,1-g][1,7]naphthylidine

(107) (±)-7-[3,5-Bis(trifluoromethyl)benzyl]-9-ethyl-6,7,8,9,10,11-hexahydro-5-(4-methylphenyl)-6,13-dioxo-13H-[1,4]diazosino[2,1-g][1,7]naphthylidine

(108) (±)-7-[3,5-Bis(trifluoromethyl)benzyl]-6,7,8,9,10,11-hexahydro-9-(1-methylethyl)-5-(4-methylphenyl)-6,13-dioxo-13H-[1,4]diazosino[2,1-g][1,7]naphthylidine

(109) (±)-5-(3,4-Dichlorophenyl)-7-(3,5-dimethoxybenzyl)-9-ethyl-6,7,8,9,10,11-hexahydro-6,13-dioxo-13H-[1,4]diazosino[2,1-g][1,7]naphthylidine

(110) (±)-5-(3,4-Dichlorophenyl)-7-(3,5-dimethoxybenzyl)-6,7,8,9,10,11-hexahydro-9-(1-methylethyl)-6,13-dioxo-13H-[1,4]diazosino[2,1-g][1,7]naphthylidine

Method for producing compounds and salts thereof

Compounds (I) and (Ia) or salts thereof can be produced in accordance with the method described in JP-A-9-263585 or JP-A-10-109989.

The compounds of (1) to (82) listed above can be produced as described in Examples in JP-A-9-263585. The compounds of (83) to (92) listed above can be produced as described in Examples in JP-A-10-109989. The compounds of (93) to (110) listed above can be produced as directed in Reference Examples 1 to 182 (especially Reference Example 165 to 182) described below.

Any of Compounds (I) and (Ia) or salts thereof has a low toxicity and is safe.

Accordingly, Compounds (I) and (Ia) or salts thereof are useful in inhibiting a vomiting in a mammalian animal (for example, hamster, cat, ferret, dog, cattle, sheep, monkey and human). The vomiting includes nausea, feeling sick and emesis. The vomiting also include an acute vomiting, a delayed vomiting and a prodromal vomiting.

Compounds (I) and (Ia) or salts thereof are useful in inhibiting a vomiting induced by a cancer-treating radiotherapy, a toxic substance, a toxin, a metabolic disorder (e.g., gastritis), a pregnancy-induced morning sickness, a rotary vertigo, a kinesia (e.g., motion sickness, etc), a postoperative disease, a gastrointestinal disorder, a gastrointestinal hypokinesia, a visceral pain (e.g., myocardial infarction, peritonitis, etc.), a migraine, an intercranial hypertension and an intercranial hypotention such as a mountain sickness. They are useful extremely in inhibiting a vomiting induced by an agent having an emetic effect listed below.

[Examples of agents having emetic effect]

(1) Anticancer drugs (anti-tumor drugs): for example, cyclophosphamide, carmustine, lomustine, chlorambucil, busulfan, melfalan, mechlorethamine, vincaalkaloids (e.g., etoposide, vinblastine, vincristine, etc.), ergot alkaloids (e.g., ergot alkaloid, bromocriptine, etc.), fumagillin derivatives (e.g., (3R, 4S, 5S, 6R)-5-methoxy-4-[(2R,3R)-2-methyl-3-(3-methyl-2-butenyl)oxylanyl]-1-oxaspiro[2.5]octo-6-yl(chloroacetyl)carbamate and the like), methotrexate, emetine, musutine, cisplatin, dacarbazine, procarbazine, dactinomycin, doxorubicin, mitomycin-C, bleomycin, asparaginase, daunorubicin, floxuridine, cytarabine, fluorouracil, mercaptopurine, mitotrane, procarbazine, streptozocin, tamoxifen, thioguanine and busulfan, etc.;

(2) antibiotics: for example, erythromycin and its derivatives (e.g., erythromycin and its derivatives such as erythromycin A, B, C and D (e.g., N-demethyl-N-isopropyl-8,9-anhydroerythromycin A-6, 9-hemiacetal and the like), clarithromycin and the like), aminoglycoside (e.g., supreptomycin, neomycin, gentamycin), actinomycin, adriamycin and cycloheximide;

(3) morphine and its derivative (e.g., opioid analgesics such as morphine and its salts, male erectile dysfunction (impotence)-treating agent such as apomorphine and its salts and Parkinson's disease-treating agent (dopamine $D_2$ receptor aginist) and the like);

(4) others, diarrhea-treating agent such as opiate receptor agonist (e.g., loperamide), anti-neoplastic agent (e.g., hydroxyurea), anti-asthmatic agent such as a phosphodieterase IV inhibitor (e.g., rolipram,), histamine, pilocarpine, protoveratrine, levodopa, theophylline, hydroxycarbamide, thio-TEPA, carboplatin, epirubicin and the like.

A drug having an emetic effect includes a drug containing a combination of several (preferably 2 to 3) substances listed above.

Compounds (I) and (Ia) or salts thereof are employed preferably against a vomiting caused by a substance described above, especially morphine and derivatives or salts thereof.

While apomorphine or its salt is employed as a therapeutic agent for treating a male erectile deficiency, the male erectile deficiency may not only be a psychogenic erectile dysfunction but also be an erectile deficiency caused by diabetes, a circulatory disease such as a heart disease, arteriosclerosis, senility, a hormone-based pharmaceutical, castration and prostate extraction. Accordingly, apomorphine or its salt may be used in combination with a hormone-based pharmaceutical (e.g., leuprorelin acetate, etc.).

A salt of morphine or its derivative may for example be a pharmaceutically acceptable salt with an inorganic acid such as hydrochloric acid, sulfuric acid and phosphoric acid as well as a pharmaceutically acceptable salt with an organic acid such as tartaric acid, bitartaric acid, citric acid, succinic acid and maleic acid.

A salt of morphine or apomorphine is preferably a pharmaceutically acceptable acid addition salt with hydrochloric acid and sulfuric acid.

A pharmaceutical according to the invention includes (1) a pharmaceutical composition containing Compound (I), (Ia) or salts thereof and a drug having an emetic effect and (2) a product in which Compound (I), (Ia) or salts thereof is formulated separately from a drug having an emetic effect.

A pharmaceutical according to the invention can be produced by mixing the active ingredients of Compound (I), (Ia) or salts thereof and a drug having an emetic effect separately or all at once with a pharmaceutically acceptable carrier, and then administered orally or parenterally as a solid formulation such as a powder, a granule, a tablet and a capsule, a liquid formulation such as a syrup, an emulsion and a solution for injection (including subcutaneous, intravenous and intramuscular injection and dripping infusion), a sustained-release formulation such as a sublingual tablet, a buccal formulation, a pastille and a microcapsule, an instantly-disintegrating oral dosage form and a suppository.

A pharmaceutically acceptable carrier described above may be any of various organic and inorganic carriers used customarily as pharmaceutical materials, which may be incorporated as an excipient, a glidant, a binder and a disintegrant in a solid formulation as well as a solvent, a solubilizing aid, a suspending agent, an osmotic agent, a buffering agent and a painkiller in a liquid formulation. If necessary, a further pharmaceutical additive such as a preservative, an antioxidant, a colorant and a sweetener-may also be added.

A preferred example of the excipient described above-includes lactose, sugar, D-mannitol, starch, crystalline cellulose, light silicic anhydride, calcium carbonate and calcium phosphate, etc. A preferred example of the glidant described above includes stearic acid, magnesium stearate, calcium stearate, talc and colloidal silica, etc. A preferred example of the binder described above includes crystalline cellulose, sugar, D-mannitol, dextrin, hydroxypropyl cellulose, hydroxypropylmethyl cellulose, polyvinyl pyrrolidone, gum arabic and gelatin. A preferred example of the disintegrant described above includes starch, carboxymethyl cellulose, calcium carboxymethyl cellulose, sodium croscalmelose and sodium carboxymethyl starch, etc. A preferred example of the solvent described above includes water for injection, physiological saline, alcohol, propylene glycol, macrogol, sesame oil and corn oil. A preferred example of the solubilizing aid described above includes polyethylene glycol, propylene glycol, D-mannitol, benzyl benzoate, ethanol, trisaminomethane, cholesterol, triethanolamine, sodium carbonate and sodium citrate. A preferred example of the suspending agent described above includes a surfactant such as stearyl triethanolamine, sodium lauryl sulfate, laurylaminopropionic acid, lecithin, benzalkonium chloride, benzethonium chloride and glyceryl monostearate; and a hydrophilic polymer such as polyvinyl alcohol, polyvinyl pyrrolidone, sodium carboxymethyl cellulose, methyl cellulose, hydroxymethyl cellulose, hydroxyethyl cellulose and hydroxypropyl cellulose. A preferred example of the osmotic agent described above includes sodium chloride, glycerin and D-mannitol, etc. A preferred example of the buffering agent described above includes buffer solutions of phosphates, acetates and citrates. A preferred example of the painkiller described above includes benzyl alcohol, etc. A preferred example of the preservative described above includes p-oxybenzoates, chlorobutanol, benzyl alcohol, phenethyl alcohol, dehydroacetic acid and sorbic acid. A preferred example of the antioxidant described above includes sulfites and ascorbic acid, etc.

A pharmaceutical according to the invention may further contain, in addition to the ingredients described above and for the purpose of reducing the possibility of a vomiting, preventing a vomiting and promoting an anti-emetic effect, an anti-dopaminergic agent, a serotonin antagonist [e.g., ondansetron (Zofran) or its sustained-release formulation (Zofran Zydis), graniserton (Kytril), azasetron (Serotone), ramosetron (Nasea), metoclopramide (Primperan) and its sustained-release formulation (Pramidin), tropisetron (Novoban), mosapride (Gsmotin) and dolasetron (Anemet) and the like, as well as palonosetron (RS-42358-197), itasetron (U-98079A), indisetron (N-3389), KAE-393, R-zacopride (SL-920241), relisetron (F-0930-RS), E-3620 and Ro-93777 and the like], a histamine antagonist, a parasympathetic nerve suppressing agent, an anti-emetic agent (e.g., metopimazine, trimethobenzamide, benzquinamide hydrochloride, diphenidol hydrochloride, etc.), an adrenocorticosteroids and its salts such as a glucosteroids [e.g., cortisone acetate (Cortone), hydrocortisone (Cortril, Hydrocorton) or its sodium phosphate salt (Hydrocortisone soluble)-, dexamethasone (Corson, decaderon, Decaderon S) or its acetates (such as Decadron A aqueous suspension for injection), sodium phosphate salt (Decadron, Decadron for injection, Decadron S) or sodium sulfate salt (DEXA-SHELOSON for injection, DEXA-SHELOSON B for injection), betamethazone (Rinderon, Betonelan) or its sodium phosphate salt (Rinderon) or acetate salt (Rinderon suspension), prednisolone (Predonine, Prednisolon tablet) or its acetate (Predonine soluble) or sodium phosphate salt (DOJILON INJECTION), butyl acetate salt (CODELCOTONE) or sodium succinate salt (water-soluble predonine), methylprednisolone (Medrol) or its acetate (Depo-Medrol) or sodium succinate salt (Sol-Medrol), triamcinolone acetate (Kenacort), other glucocorticoid-active steroids], a sedative or tranquilizer such as chlorpromazine. Especially, a combination with a serotonin antagonist such as ondansetron, etc. and/or dexamethasone and its acetate, sodium phosphate salt or sodium sulfate salt may promote the anti-emetic effect of Compound (I), (Ia) or its salt, thus being employed preferably. Thus, a pharmaceutical of the invention encompasses:

(1) a pharmaceutical as a combination of Compound (I), (Ia) or its salt, a drug having an emetic effect and a serotonin antagonist;
(2) a pharmaceutical as a combination of Compound (I), (Ia) or its salt, a drug having an emetic effect and a glucocsteroid; and,
(3) a pharmaceutical as a combination of Compound (I), (Ia) or its salt, a drug having an emetic effect, a serotonin antagonist and a glucosteroid. Such combination is employed preferably in inhibiting a vomiting caused by an anticancer agent such as cisplatin.

Such formulation can be produced by a method known per se which is employed ordinarily in a pharmaceutical formulating process.

Compound (I), (Ia) or its salt and a drug having an emetic effect employed in the invention can be formulated with a dispersant (e.g., Tween 80 (ATLAS POWDER, USA), HCO60 (NIKKO CHEMICALS), polyethylene glycol, carboxymethyl cellulose, sodium alginate, hydroxypropylmethyl cellulose, dextrin, etc.), a stabilizer (e.g., ascorbic acid, sodium pyrosulfite, etc.), a surfactant (e.g., polysorbate 80, macrogol, etc.), a solubilizing agent (e.g., glycerin, ethanol, etc.), an osmotic agent (e.g., sodium chloride, potassium chloride, mannitol, sorbitol, glucose, etc.), a pH modifier (e.g., hydrochloric acid, sodium hydroxide, etc.), a preservative (e.g., ethyl p-oxybenzoate, benzoic acid, methylparabene, propylparabene, benzyl alcohol, etc.), a solubilizer (e.g., concentrated glycerin, meglumine, etc.), a solubilizing aid (e.g., propylene glycol, sugar, etc.), a painkiller (e.g., glucose, benzyl alcohol, etc.) into an aqueous formulation for injection, or dissolved, suspended or emulsified in a vegetable oil such as olive oil, sesame oil, cottonseed oil and corn oil and in a solubilizing aid such as propylene glycol to form an oily formulation, whereby producing an injection formulation.

In order to obtain an oral dosage form, a method known per se is employed to compact Compound (I), (Ia) or its salt or a drug having an emetic effect admixed for example with an excipient (e.g., lactose, sugar, starch, etc.), a disintegrant (e.g., starch, calcium carbonate, etc.), a binder (e.g., starch, gum arabic, carboxymethyl cellulose, polyvinyl pyrrolidone, hydroxypropyl cellulose, etc.) or a glidant (e.g., talc, magnesium stearate, polyethylene glycol 6000, etc.) into a desired shape, which is then subjected to a taste masking, covered with an enteric coating or imparted with a sustained release performance if necessary by means of a coating method known per se, whereby obtaining an oral dosage form. Such coating may for example be hydroxypropylmethyl cellulose, ethyl cellulose, hydroxymethyl cellulose, hydroxypropyl cellulose, polyoxyehtylene glycol, Tween 80, Pluronic F68, cellulose acetate phthalate, hydroxypropylmethyl cellulose phthalate, hydroxymethyl cellulose acetate succinate, Eudragit (Rohm, German, methacrylic/acrylic acid copolymer) and a colorant (e.g., iron oxide red, titanium dioxide, etc.). An oral dosage form may be an instant release formulation or a sustained-release formulation.

In order to obtain for example a suppository, a method known per se is employed to convert Compound (I), (Ia) or its salt and a drug having an emetic effect into an oily or aqueous solid, semi-solid or liquid suppository. An oily base employed in a composition described above may for example be a higher fatty acid glyceride [e.g., cocoa butter, UITEPSOL (DYNAMITE NOVEL, Germany), etc.], a medium fatty acid [e.g., MIGRIOL (DYNAMITE NOVEL, Germany), etc.], or a vegetable oil (e.g., sesame oil, soybean oil, cottonseed oil, etc.). An aqueous base may for example be polyethylene glycol and propylene glycol, and an aqueous gel base may for example be natural gums, cellulose derivatives, vinyl polymers and acrylic acid polymers.

A sustained-release formulation described above may for example be a sustained-release microcapsule.

While a sustained-release microcapsule can be obtained by a method known per se, a sustained-release formulation shown in Section [2] described below is formed and administered in a preferred case.

Compound (I), (Ia) or its salt is preferably formulated as an oral dosage form such as a solid formulation (e.g., powder, granule, tablet, capsule), or as a rectal formulation such as a suppository. An oral dosage form is particularly preferred.

While a drug having an emetic effect may be formulated into a dosage form described above depending on the type of the drug, the drug having an emetic effect, when being present as morphine or its derivative or salt, is formulated preferably into an injection formulation or an oral dosage form such as an instant release formulation (e.g., powder, granule, tablet) or a sustained-release formulation (e.g., microcapsule sustained-release formulation), a sublingual formulation, a buccal formulation, an instantly disintegrating oral formulation and a pastille, etc. More typically, a drug having an emetic effect, when being present as morphine or its salt, is formulated preferably into an injection formulation or an oral dosage form such as an instant release formulation (e.g., powder, granule, tablet) or a sustained-release formulation such as a microcapsule. A drug having an emetic effect, when being present as apomorphine or its salt, is formulated preferably into an oral dosage form such as a sublingual formulation, a buccal formulation and an instant release formulation.

When a pharmaceutical of the invention is used in combination with a serotonin antagonist and/or a glucosteroid described above, the pharmaceutical of the invention may be formulated separately from the serotonin antagonist or the glucosteroid and then used in combination therewith, or it may contain the serotonin antagonist and the glucosteroid whereby being used in combination. It may be used in combination also as a pharmaceutical composition of [1] to [3] described below.

[1] A pharmaceutical composition containing Compound (I), (Ia) or its salt and a serotonin antagonist.
[2]A pharmaceutical composition containing Compound (I), (Ia) or its salt and a glucosteroid antagonist.
[3] A pharmaceutical composition containing Compound (I), (Ia) or its salt, a serotonin antagonist and a glucosteroid.

Any of the pharmaceutical compositions [1] to [3] described above may contain also a compound having an

emetic effect.

A serotonin antagonist and/or a glucosteroid is used preferably as being formulated into an oral dosage form such as a tablet, a powder, a granule and a capsule and an injection formulation (including subcutaneous, intravenous, intramuscular injection and dripping infusion), suppository and patch formulation by a method known per se as is in the cases of Compound (I), (Ia) or its salt and a drug having an emetic effect described above.

The followings are the descriptions with regard to [1] an injection formulation employing morphine or its salt as a drug having an emetic effect and a method for producing the same, [2] a sustained-release or immediate release formulation of a drug having an emetic effect (e.g., morphine or its salt) and a method for producing the same and [3] a sublingual, buccal or instantly-disintegrating oral formulation employing apomorphine or its salt as a drug having an emetic effect and a method for producing the same.

[1] Injection formulation employing morphine or its salt as drug having emetic effect and method for producing the same

A morphine solution for injection obtained by dissolving morphine or its salt in water is employed preferably. Such morphine solution for injection may contain a benzoate and/or a salicylate.

A morphine solution for injection is obtained by dissolving morphine or its salt in water together with a benzoate and/or a salicylate in water as desired.

A benzoate and/or a salicylate described above may be an alkali metal salt such as sodium and potassium salts, an alkaline earth metal salt such as calcium and magnesium salts, an ammonium salt, a meglumine salt as well as a salt of an organic acid such as trometamol.

The concentration of morphine or its salt in a morphine solution for injection is 0.5 to 50 w/v %, preferably about 3 to 20 w/v %. The concentration of a benzoate and/or a salicylate is 0.5 to 50 w/v %, preferably 3 to 20 w/v %.

The formulation may contain additives employed customarily in a morphine solution for injection, such as a stabilizer (ascorbic acid, sodium pyrosulfite and the like), a surfactant (polysorbate 80, macrogol and the like), a solubilizing agent (glycerin, ethanol and the like), a buffering agent (phosphoric acid and its alkali metal salt, citric acid and its alkali metal salt and the like), an osmotic agent (sodium chloride, potassium chloride and the like), a dispersing agent (hydroxypropylmethyl cellulose, dextrin), a pH modifier (hydrochloric acid, sodium hydroxide and the like), a preservative (ethyl p-oxybenzoate, benzoic acid and the like), a solubilizer (concentrated glycerin, meglumine and the like), a solubilizing aid (propylene glycol, sugar and the like), a painkiller (glucose, benzyl alcohol and the like) as appropriate. Any of these additives is added in an amount employed customarily in a formulation for injection.

By adding pH controlling agent, pH of a morphine solution for injection is preferably adjusted to from 2 to 12, more preferably from 2.5 to 8.0.

A morphine solution for injection is obtained by dissolving morphine or its salt and a benzoate and/or a salicylate if desired in water together with the additives listed above if necessary. These components may be dissolved in any order as appropriate similarly to a customary preparation of a formulation for injection.

A morphine solution for injection is preferably warmed, and given as a formulation for injection after sterilizing by filtration or autoclave similarly to a customary formulation for injection. An aqueous solution of morphine for injection is preferably autoclaved at 100 to 121°C for 5 to 30 minutes.

A formulation may be present as a solution imparted with an antibacterial activity for the purpose of using several times in divided doses.

[2] Sustained-release or immediate release formulation and method for producing the same

A sustained-release formulation obtained by coating a core containing a drug (e.g., morphine or its salt) with a water-insoluble material or a swelling polymer as desired is employed preferably.

Especially when a drug employed is morphine or its salt, a sustained-release oral formulation of a single daily dose is preferred.

A water-insoluble material employed as a coating may for example be cellulose ethers such as ethyl cellulose and butyl cellulose, cellulose esters such as cellulose acetate and cellulose propionate, polyvinylesters such as polyvinyl acetate and polyvinyl butyrate, acrylic acid-based polymers such as an acrylic acid/methacrylic acid copolymer, a methyl methacrylate copolymer, an ethoxyethyl methacrylate/cinnamoethyl methacrylate/aminoalkyl methacrylate copolymer, polyacrylic acid, polymethacrylic acid, a metacrylic acid alkylamide copolymer, a poly(methyl methacrylate), a polymethacrylate, a polymethacrylamide, an aminoalkyl methacrylate copolymer, a poly(methacrylic anhydride), a glycidyl methacrylate copolymer, especially, a series of Eudragit such as Eudragit RS-100, RL-100, RS-30D, RL-30D, RL-PO, RS-PO (ethyl acrylate/methyl methacrylate/chlorotrimethyl methacrylate/ethyl ammonium copolymer) and Eudragit NE-30D (methyl methacrylate/ethyl acrylate copolymer), hydrogenated oils such as a hydrogenated castor oil (e.g., LOVELY wax (FREUNT SANGYO)), waxes such as carnauba wax, a fatty acid glycerin ester-and paraffin and a polyglycerin fatty acid ester.

As a swelling polymer, a polymer having an acidic cleavable group and exhibiting a pH-dependent swelling is preferred, and an acidic cleavable group-bearing polymer which undergoes a less swelling at an acidic pH such as in stomach but is swollen extensively at a neutral pH such as in small and large intestines is preferred.
Such polymer having an acidic cleavable group and exhibiting a pH-dependent swelling may for example be a crosslinked polyacrylic acid polymer such as Carbomers 934P, 940, 941, 974P, 980, 1342 and the like, Polycarbophil and Calcium Polycarbophil (BF GOODRICH), HIGHVIS Wakos 103, 104, 105 and 304 (Wako Pure Chemical).

A coating employed in a sustained-release formulation may further contain a hydrophilic material.

Such hydrophilic material may for example be a polysaccharide which may have a sulfate group such as pullulan, dextrin and alkali metal alginates, a polysaccharide having a hydroxyalkyl group or a carboxyalkyl group such as hydroxypropyl cellulose, hydroxypropylmethyl cellulose and sodium carboxymethyl cellulose as well as methyl cellulose, polyvinyl pyrrolidone, polyvinyl alcohol and polyethylene glycol.

The water-insoluble material content in a coating of a sustained-release formulation is about 30 to about 90 % (w/w), preferably about 35 to about 80 % (w/w), more preferably about 40 to about 75 % (w/w), and the swelling polymer content is about 3 to about 30 % (w/w), preferably about 3 to about 15 % (w/w). A coating may further contain a hydrophilic material, the content of which in the coating is about 50 % (w/w) or less, preferably about 5 to about 40 % (w/w), more preferably about 5 to about 35 % (w/w). A % (w/w) referred here means a % by weight based on the coating composition which is the remainder of the coating solution after deleting any solvent (e.g., water and a lower alcohol such as methanol and ethanol, etc.).

A sustained-release formulation is produced, as exemplified below, by preparing a core containing a drug followed by coating a resultant core with a coating solution obtained by melting a water-insoluble material or a swelling polymer or by dissolving or dispersing such material in a solvent.

I. Drug-containing core preparation

While a coated drug-containing core (hereinafter sometimes referred to simply as a core) may be in any nonlimiting shape, it is formed preferably as a particle such as a granule or a fine particle.

When a core is a granule or a fine particle, it has a mean particle size preferable of about 150 to 2,000 μm, more preferably about 500 to 1,400 μm.

A core can be prepared by a standard method. For example, a drug is combined with suitable excipient, binder, disintegrant, glidant, stabilizer and the like, and then subjected to a wet extrusion granulation or a fluidized bed granulation.

The drug content in a core is about 0.5 to about 95 % (w/w), preferably about 5.0 to about 80 % (w/w), more preferably about 30 to about 70 % (w/w).

An excipient contained in a core may for example be a saccharide such as sugar, lactose, mannitol and glucose, starch, crystalline cellulose, calcium phosphate and corn starch. Among these, crystalline cellulose and corn starch are preferred.

A binder may for example be polyvinyl alcohol, hydroxypropyl cellulose, polyethylene glycol, polyvinyl pyrrolidone, Pluronic F68, gum arabic, gelatin and starch, etc. A disintegrant may for example be calcium carboxymethyl cellulose (ECG505), sodium CROSCALMELOSE (Ac-Di-Sol), crosslinked polyvinyl pyrrolidone (Crosspovidone) and a low-substituted hydroxypropyl cellulose (L-HPS). Among these, hydroxypropyl cellulose, polyvinyl pyrrolidone and a low-substituted hydroxypropyl cellulose are preferred. A glidant and an anticoagulant may for example be talc, magnesium stearate. and its inorganic salts, and a lubricant may for example be polyethylene glycol, etc. A stabilizer may for example be an acid such as tartaric acid, citric acid, succinic acid, fumaric acid and maleic acid, etc.

In addition to the methods described above, other methods can be employed to form a core, such as an agitating granulation method wherein an inert carrier particle as a seed for the core is sprayed with a binder dissolved in a suitable solvent such as water and a lower alcohol (e.g., methanol and ethanol) with being supplemented portionwise with a drug or a mixture thereof with an excipient and a glidant as well as a pan coating method, a fluidized bed coating method and a melting granulation method. An inert carrier particle may for example be one prepared from sugar, lactose, starch, crystalline cellulose and waxes, and has a mean particle size preferably of about 100 μm to about 1,500 μm.

In order to separate a drug contained in a core from a coating, the surface of the core may be covered with a protective material. Such protective material may for example be a hydrophilic material described above and a water-insoluble material. A preferred protective material is polyethylene glycol or a polysaccharide having a hydroxyalkyl group or a carboxyalkyl group, more preferably, hydroxypropylmethyl cellulose and hydroxypropyl cellulose. The protective material may contain, as a stabilizer, an acid such as tartaric acid, citric acid, succinic acid, fumaric acid and maleic acid, etc., as well as a glidant such as talc, etc. A protective material, when employed, is coated at a rate of about 1 to about 15 % (w/w), preferably about 1 to about 10 % (w/w), more preferably about 2 to about 8 % (w/w) based on a core.

A protective material can be coated by a standard coating method, and typically a core is sprayed with the protective material by a fluidized bed coating method and a pan coating method.

II. Coating of the core with a coating agent

A sustained-release formulation is produced by coating the core obtained in said I with a coating agent solution in which said water-insoluble substance and a polymer swelling dependent on pH and a hydrophilic substance are dissolved with heating, or dissolved or dispersed in a solvent.
Examples of coating methods of the core with the coating agent solution include a spray coating method and the like.
The composition ratio of the water-insoluble substance, the swelling polymer and the hydrophilic substance in the coating agent solution is appropriately selected so that a content of each component in a coat is said content, respectively.
The coating amount of the coating agent is from about 1 to about 90 % (w/w), preferably from about 5 to about 50 % (w/w), more preferably from about 5 to about 35 % (w/w) for the core (not include the coating amount of the protective agent).
Water or an organic solvent can be used alone or by a mixture solution of both as a solvent of the coating agent solution. The mixture ratio of water to an organic solvent (weight ratio of water/ organic solvent) can be varied within the range of from 1 to 100 %, and is preferably from 1 to about 30 %, when a mixed solution is used. The organic solvents are not specifically limited as long as water-insoluble substances are dissolved. For example, lower alcohol such as methyl alcohol, ethyl alcohol, isopropyl alcohol, n-butyl alcohol, etc., lower alkanone such as acetone, etc., as well as acetonitrile, chloroform, methylene chloride and the like are used. Among them, lower alcohol is preferable, and ethyl alcohol and isopropyl alcohol are most preferable. Water as well as the mixed solution of water and the organic solvent is preferably used as the solvent of the coating agent. At this time, acids such as tartaric acid, citrate acid, succinic acid, fumaric acid, maleic acid, and the like may be added to the coating agent solution for stabilization of the coating agent solution if necessary.
When coated by spray coating, the manipulation can be carried out by standard coating methods, and specifically, can be carried out by spray-coating the core with the coating agent solution, for example, by a liquid layer coating method and a pan coating method. At this time if necessary, talc, titanium oxide, magnesium stearate, calcium stearate, silicic anhydride light and the like as lubricants, and glycerin fatty acid ester, hardened ricinus, triethyl citrate, cetyl alcohol, stearyl alcohol and the like as plasticizers may be added.
After coating with the coating agent, anti-static agents such as talc may be mixed as needed.
Rapid-release formulations may be liquid (solution, suspension, emulsion and the like) or solid (particle, pill, tablet and the like). Oral administration agents or parenteral administration agents such as injection agent are used, but the oral administration agent is preferable.
The rapid-release formulation may usually comprise carriers, additives, and excipients (sometimes described roughly as excipients hereinafter) used popularly in the field of formulation in addition to a drug which is an active component. The formulation excipients are not especially limited as long as excipients are commonly used as formation excipients. For example, excipients for oral solid formulation include lactose, starch, corn starch, crystal cellulose (made by Asahikasei, Ltd., Abisel PH101), powder sugar, granulated sugar, mannitol, silicic anhydride light, magnesium carbonate, calcium carbonate, L-cysteine, etc., and preferably, corn starch and mannitol are included. These excipients can be used by one or more combinations thereof. The content of excipients is for example, from about 4.5 to about 99.4 %, preferably from about 20 to about 98.5, more preferably from about 30 to about 97 % by weight of the total amount of rapid-release formulation.
The content of the drug in the rapid-release formulation can be appropriately selected from the range of from about 0.5 to about 95 %, preferably the range of from about 1 to about 60 % of the total amount of rapid-release formulation.
When the rapid-release formulation is an oral solid formulation, a disintegrant is contained in addition to the typical above mentioned component. As these disintegrants, for example, calcium carboxymethyl cellulose (made by Gotoku Yakuhin, ECG-505), cross carmellose sodium (e.g., Asahikasei Ltd., Akujizol), cross povidone (e.g., BASF Co., Colidon CL), lowly substituted hydroxypropylcellulose (Shin-etsu Kagaku Ltd.), carboxymethyl starch (Matsuya Kagaku Ltd.), sodium carboxymethyl starch (Kimura Sangyo, Ekisupurotabu), partial $\alpha$ starch (Asahikasei Ltd., PCS) are used, and those can be used which disintegrate granules with water absorption and swelling by contact to water, or with channel making between an active component and excipients which compose the core. These disintegrants can be used by one or more combinations thereof. The combined amount of disintegrants is appropriately selected depending on the type and combined amount of the drug used as well as the design of formulation for release, and for example is from about 0.05 to about 30 %, preferably from about 0.5 to about 15 % by weight of the total amount of rapid-release formulation.

When the rapid-release formulation is an oral solid formulation, popularly used additives may be further added to the desired solid formulation in addition to the above composition in case of a oral solid formulation. As these additives, for example, binders (e.g., sucrose, gelatin, gum acacia, methylcellulose, hydroxypropylcellulose, hydroxypropyl methylcellulose, carboxymethyl cellulose, polyvinyl pyrrolidone, pullulane, dextrin, etc.), lubricants (e.g., polyethylene glycol, magnesium stearate, talc, silicic anhydride light (e.g., Aerojil (Nippon Aerojil), surfactants (e.g., anion surfactant of sodium alkyl sulfate, polyoxyethylene fatty acid ester and polyoxyethylene sorbitan ester, non-ion surfactants of polyoxyethylene ricinus derivative, etc.), coloring agents (e.g., tardyestuff, caramel, colcothar, titanium oxide, riboflavins, etc.), and if necessary, taste agents (e.g., sweeting agent and fragrant materials, etc.), absorbents, preservatives, humectants, anti-static agents and the like are used. Also organic acids such as tartaric acid, citric acid, succinic acid, and fumaric acid may be added as stabilizers.

Hydroxypropyl cellulose, polyethylene glycol and polyvinyl pyrrolidone are preferably used as the above binders

The rapid-release formulation can be prepared by mixing said each component, kneading together if necessary, and molding based on common manufacturing techniques of formulation. The above mixing is performed by methods generally used, e.g., mixture and kneading. Specifically, when the rapid-release formulation is molded into particulate form, by the similar technique as that in the formulation process of said sustained-release formulation, it can be prepared by mixing using a vertical granulator, an all-round kneading machine (Hata Tekkou)and a liquid layer particle-making machine FD-5S (Pawreck) followed by making particles by a wet extrusion particle making method and a liquid layer particle-making method.

These obtained rapid-release and sustained-release formulations may be the formulations which are simultaneously or administered with any dose intervals in combination as both are or after separately formulating with appropriate formulation excipients by standard methods, or may be formulated into one oral administration agent (e.g., granulated agent, fine particle agent, tablet, capsule, etc.) as both are or in conjugation with appropriate formulation excipients. Both formulations are formulated into granules or fine particles and filled the same capsule and the like and may be an oral administration formulation.

[3] Apomorphine sublingual tablet, buccal or intraoral rapid-disintegrants and preparation thereof

Sublingual tablets, buccal formulations or intraoral rapid-disintegrants may be solid formulations such as tablets or oral cavity mucous membrane laminating agents (films).

The formulations contained apomorphine and salts thereof and excipients are preferable as apomorphine sublingual tablets, buccal or intraoral rapid-disintegrants. Auxiliary agents such as lubricants, isotonic agents, hydrophilic carriers, water-dispersing polymers, stabilizers may be contained. Also β-cyclodextrin or β-cyclodextrin derivatives (e.g., hydroxypropyl-β-cyclodextrin, etc.) and the like may be contained to facilitate absorption and to enhance in vivo availability.

The above excipients include lactose, sucrose, D-mannitol, starch, crystal cellulose, silicic anhydride light and the like. The lubricants include magnesium stearate, calcium stearate, talc, colloid silica, and especially magnesium stearate and colloid silica are preferable. The isotonic agents include sodium chloride, glucose, fructose, mannitol, sorbitol, lactose, saccharose, glycerine, urea and the like, and especially, mannitol is preferable. The hydrophilic carriers include swelling hydrophilic carriers such as crystal cellulose, ethyl cellulose, cross-linking polyvinyl pyrrolidone, silicic anhydride light, silicic acid, dicalcium phosphate, calcium carbonate, and the like, and especially crystal cellulose(e.g., microcrystal cellulose, etc.) is preferable. The water-dispersing polymers include gums (e.g., tragacanth gum, acacia gum, guar gum), alginate salts (e.g., sodium alginate), cellulose derivatives (e.g., methyl cellulose, carboxymethyl cellulose, hydroxymethyl cellulose, hydroxypropyl cellulose, hydroxypropylmethyl cellulose), gelatin, water-soluble starch, polyacrylic acid (e.g., carbomer), polymethacylic acid, polyvinyl alcohol, polyethylene glycol, polyvinyl pyrrolidone, polycarbofil, ascorbate palmitate salts and the like, and hydroxypropylmethyl cellulose, polyacrylic acid, alginate salt, gelatin, carboxymethyl cellulose, polyvinyl pyrrolidone, polyethylene glycol are preferable. Hydroxypropylmethyl cellulose is most preferable. The stabilizers include cysteine, thiosorbitol, tartaric acid, citric acid, sodium carbonate, ascorbic acid, glycine, sodium sulfite and the like, and citric acid and ascorbic acid are most preferable.

Apomorphine sublingual tablet, buccal or intraoral rapid-disintegrants can be produced by mixing apomorphine, or salts thereof and excipients by per se well-known methods. Additionally, auxiliary agents such as aforementioned lubricants, isotonic agents, hydrophilic carriers, water-dispersing polymers, stabilizers, coloring agents, sweeting agents and preservatives may be mixed. Sublingual tablets, buccal tablets or intraoral rapid-disintegrant tablets are obtained by mixing the aforementioned components simultaneously or with time intervals followed by forming tablets with pressure and klap. In order to obtain appropriate hardness, solvents such as water and alcohol may be used for humidification and wetting if necessary before and after a process of tablet klap molding, and after molding tablets may be dried to be produced.

When oral cavity mucous membrane laminating agents (films) are molded, apomorphine and salts thereof,

aforementioned water-dispersing polymer (preferably, hydroxypropyl cellulose, hydroxypropylmethyl cellulose), excipients and the like are dissolved in solvent such as water, and a resultant solution is flowed casting to be a film. Additionally, additives such as plasticizers, stabilizers, anti-oxidant agents, preservatives, coloring agents, buffering agents, sweeting agents may be added. Glycols such as polyethylene glycol and propylene glycol may be contained to give an appropriate elasticity to a film, and biological adhesive polymers (e.g., polycarbofil, and carbopole) may be contained to enhance adhesion of films to mucosal linings of oral cavity. Flow casting is achieved by pouring the solution onto non-adhesive surface and spreading it with an applicator such as a doctor blade at a homogenous thickness (preferably from about 10 to 1000 μm) followed by drying the solution to form films. These formed films may be dried at room temperature or by heating, and cut off by a desired surface area.

The preferred intraoral rapid-disintegrants include a solid rapid dispersing agent consisting of retiform bodies of apomorphine or salts thereof with a water-soluble or water-dispersing carrier which is inactive with apomorphine or salts thereof. The retiform bodies are obtained by sublimating solvents from the solid composition comprising of a solution in which apomorphine or a salt thereof is dissolved in an appropriate solvent.

It is preferable that a matrix forming agent and a secondary component in addition to apomorphine or a salt thereof are comprised in the intraoral rapid-disintegrants composition.

The matrix forming agents comprise animal or plant proteins such as gelatins, dextrins and soybeans, wheat and ispaghul (psyllium) seed protein; gum materials such as gum acacia, guar gum, agarose and xanthane; polysaccharides; alginic acids; carboxymethyl celluloses; carrageenans; dextrans; pectins; synthetic polymers such as polyvinyl pyrrolidone; materials derived from gelatin-gum acacia complex. Additionally, sugars such as mannitol, dextrose, lactose, galactose, and trehalose; cyclic sugars such as cyclodextrin; inorganic salts such as sodium phosphate, sodium chloride and aluminium silicate; amino acids with carbon atom numbers of from 2 to 12 such as glycine, L-alanine, L-aspartic acid, L-glutamic acid, L-hydroxyproline, L-isoleucine, L-leucine and L-phenylalanine are included.

One or more types of matrix forming agents can be introduced into the solution or the suspension before solid formation. The matrix forming agent may exist in the presence or absence of surfactants. Matrix forming agents can support to maintain diffusion states of apomorphine or a salt thereof in the solution or the suspension, in addition to matrix formation.

Secondary components such as preservatives, anti-oxidant agents, surfactants, viscosity improvers, coloring agents, pH modifiers, pepper-uppers, sweeting agents or eating quality masking agents may be contained in the composition. Suitable coloring agents include red, black and yellow ferric oxides and FD &C dyes such as FD & C blue No. 2 and FD & C red No. 40 of Ellis and Eberhardt Company. Suitable flavorings comprise mint, raspberry, glycyrrhiza, orange, lemon, grapefruit, caramel, vanilla, cherry and grape flavors and combinations thereof. Suitable pH modifiers comprise citric acid, tartaric acid, phosphoric acid, hydrochloric acid and maleic acid. Suitable sweeting agents comprise aspartame, acesulfame K and thaumatin. Suitable eating quality masking agents comprise sodium bicarbonate, ion exchange resin, cyclodextrin enclosure compounds, absorptive substances and microcapsuled apomorphine.

Apomorphine or a salt thereof is usually comprised from about 0.1 to about 50 %, preferably from about 0.1 to about 30 % of the formulation by weight. The formulations (aforementioned sublingual tablet, buccal etc) in which apomorphine or a salt thereof can be dissolved in water at 90% or more for from about 1 to about 60 minutes, preferably for from about 1 to about 15 minutes, more preferably for from about 2 to about 5 minutes, are preferable. Also the intraoral rapid disintegrates which are disintegrated within from 1 to 60 seconds, preferably from 1 to 30 seconds, more preferably from 1 to 10 seconds after putting into the mouth are preferable.

The content of the above excipients is from about 10 to about 99 %, preferably from about 30 to about 90 % by weight of the total formulation. The content of β-cyclodextrin or a derivative thereof is from 0 to about 30 % by weight of the total formulation. The content of lubricants is from about 0.01 to about 10 %, preferably from about 1 to about 5 % by weight of the total formulation. The content of isotonic agents is from about 0.1 to about 90 %, preferably from about 10 to about 70% by weight of the total formulation. The content of hydrophilic carriers is from about 0.1 to about 50 %, preferably from about 10 to about 30 % by weight of the total formulation. The content of water-dispersing polymers is from about 0.1 to about 30 %, preferably from about 10 to about 25 % by weight of the total formulation. The content of stabilizer is from about 0.1 to about 10 %, preferably from about 1 to about 5 % by weight of the total formulation. The above formulation may further contain additives such as coloring agents, sweeting agents, preservatives and the like if necessary.

Apomorphine formulation may be used by combining phosphodiesterase V inhibitors such as sildenafile, prostaglandin formulations such as vasomax, and α-blocker formulations such as zonagen, to enhance treatment benefits of male erectile dysfunction of apomorphine or salts thereof.

Besides, if male erectile dysfunction of patients is ascribed to diabetes, apomorphine formulations are sometimes more effective by using by combining with curative medicines for diabetes such as biguanide agents and sulfonylurea agents such as pioglitazone, troglitazone, rosiglitazone, insulin, metoformin and the like.

The daily dosage of the medicine of the present invention is not specifically limited, but is varied depending on degree of symptoms, age of subject, sex, body weight, sensitivity difference, timing of administration, interval, nature

of medical formulation, preparation, type, types of active components and the like. As Compound (I) and (Ia) or the salt thereof, the dosage is not specifically limited within the range as far as side effects are not critical, but usually from about 0.005 to 100 mg, preferably from about 0.05 to 50 mg, more preferably from about 0.2 to 30 mg per 1 kg of body weight in mammals by oral administration, and usually this is administered by dividing from 1 to 3 times a day.

For drugs having emetic action, any dosages can be set within the range in which side effects are not critical. For the drug having emetic action, the daily dosage is not specifically limited, but is varied depending on degree of symptoms, age of subject, sex, body weight, sensitivity difference, timing of administration, interval, nature of medical formulation, preparation, type, types of active components and the like. The dose of the drug is usually, for example, from about 0.001 to 2000 mg, preferably from about 0.01 to 500 mg, more preferably from about 0.1 to 100 mg per 1 kg of body weight in mammals by oral administration, and usually this is administered by dividing from 1 to 4 times a day.

(1) When the drug having emetic action is morphine or salts thereof, for example, when pain of patients with cancer is prevented or treated with the once a day formulation, the dosage of morphine or salts thereof per day per patient is usually from about 4 to 1000 mg, preferably about 20 to 600 mg, more preferably about 50 to 500 mg. However, the dosage over the above range can be administered if necessary so far as safety can be assured.

(2) When the drug having emetic action is apomorphine or a salt thereof, for example, when impotence or male erectile dysfunction is treated, the one dosage is usually from about 0.01 to 300 mg, preferably from about 0.01 to 100 mg, more preferably from about 0.01 mg to 30 mg per 1 kg of body weight in mammals by oral administration.

Apomorphine or a salt thereof is preferably administered orally (preferably sublingual) from about 5 to 60 minutes, preferably from about 15 to 20 minutes before sex relations so as to maintain given apomorphine levels in circulating blood and midbrain during sex relations (e.g., sexual conjugation).

When the medicine of the present invention is used by combination with serotonin antagonist and/or glucosteroid, any amounts can be set for serotonin antagonist and/or glucosteroid within the range in which side effects are not critical. As serotonin antagonist or glucosteroid, the daily dosage is usually from about 0.005 to 50 mg, preferably from about 0.01 to 30 mg, more preferably from about 0.01 to 10 mg per 1 kg of body weigh in mammals, respectively, and this is administered by dividing from 1 to 3 a day.

When the medicine of the present invention is administered, they may be administered simultaneously, however, the drug having emetic action may be precedently administered and Compound (I) and (Ia) or the salt thereof may be administered before or after emetic episode. Or Compound (I) and (Ia) or the salt thereof may be administered followed by administration of the drug having emetic action. When the medicines are administered with an interval, the interval is varied depending on administered active components, formulation, and administration methods. For example, when the drug having emetic action is precedently administered, the method is included in which Compound (I) and (Ia) or the salt thereof are administered within from 1 minutes to 3 days, preferably within from 10 minutes to a day, more preferably within from 15 minutes to an hour after the compound having emetic action is administered. When Compound (I) and (Ia) or the salt thereof are precedently administered, the method is included in which the compound having emetic action is administered within from a minute to a day, preferably within from 10 minutes to 6 hours, more preferably within 15 minutes to an hour after Compound (I) and (Ia) or the salt thereof are administered.

When the medicine of the present invention is used by combination with serotonin antagonist and/or glucosteroid, serotonin antagonist and/or glucosteroid may be administered simultaneously with or separately from Compound (I) and (Ia) or salts thereof. When administered with an interval, for example when the drug having emetic action is precedently administered, the method is included in which they are administered within from a minute to 3 days, preferably within from 10 minutes to a day, more preferably within 15 minutes to an hour after the drug having emetic action is administered.

As a preferred administration method, for example, the drug formulated as oral administration having emetic action is administered orally at from about 0.001 to 200 mg, and after about 15 minutes, Compound (I) and (Ia) or salts thereof formulated as oral administration is administered orally at from about 0.005 to 100 mg for a daily dosage.

In the medicine of the present invention, the content of Compound(I) and (Ia) or salts thereof is varied depending on dosage forms, however usually is from about 0.01 to 100 %, preferably from about 0.1 to 50 %, more preferably from about 0.5 to 20 % by weight of the total formulation. When the medicine of the present invention is used by combination with serotonin antagonist and/or glucosteroid, the content of serotonin antagonist and/or glucosteroid is from about 0.001 to 100 %, preferably about 0.01 to 50 %, more preferably from about 0.5 to 20 % by weight of the total formulation.

Compound(I) and (Ia) or salts thereof are useful as anti-emesis agents, and especially, can inhibit emesis caused by drugs having emetic action, safely and rapidly at low doses.

The invention will be described in more details below based on the Reference Examples and examples, but the invention is not limited by these examples, and the examples may be changed within the scope without departing from the scope of the invention.

Elution by column chromatography in the Reference Examples was performed under observation by TLC (thin layer chromatography) unless otherwise specified. In TLC observation, $60F_{254}$ made by Merck was used as a TLC plate, and the solvent used as elution solvent in column chromatography was used as a running solvent. UV detector was used for detection. Silica gel 60 (70-230 meshes) supplied by Merck was used as silica gel for a column chromatography. Room temperature usually means temperatures from about 10°C to 35°C. Sodium sulfate or magnesium sulfate was used to dry the extract solutions.
The abbreviations in the Reference Examples mean as follows;

NMR : nuclear magnetic resonance analysis
EI-MS: electron impact mass spectrometry
SI-MS: secondary electron ion mass spectrometry
DMF: dimethyl formamide, THF: tetrahydrofuran, DMSO: dimethyl sulfoxide, Hz: hertz, J: coupling constant, m: multiplet, q: quartet, t: triplet, d: doublet, s: singlet, b: broad, like: proximity

[Reference Examples]

(Reference Example 1)

N-[3,5-bis(Trifluoromethyl)benzyl]-7,8-dihydro-7-(2-hydroxyethyl)-5-(4-methyl phenyl)-8-oxo-6-pyrido[3,4-b] pyridine carboxamide

(Process 1)

Under atmosphere of nitrogen, iodine (catalytic amount) was added to a suspension of magnesium (2.4 g) in THF (30 ml) with stirring, then a solution of 4-bromotoluene (17.1 g) in THF (20 ml) was dropped, and stirred for an hour. This mixture was added to a solution of 2, 3-pyridine carboxyl acid anhydride (12.7 g) in THF (50 ml) with stirring and holding temperature at 0 to 5°C, stirred for 30 min, and then stirred at room temperature for an hour. After evaporating solvents from the reaction mixture, water was added to the residue and pH was adjusted to 1.0 with hydrochloric acid. This mixture was extracted with dichloromethane, washed with water, dried, and the solvents were evaporated. To the residue, dichloromethane (about 10 ml) was added, then isopropyl ether (about 70 ml) was added, and was stirred for 16 hours at room temperature to yield 3-(4-methylbenzoyl)-2-pyridine carboxylic acid as colorless crystals.
Melting point: 168-170°C (re-crystallization from dichloromethane-ethyl acetate)
NMR (200MHz, $CDCl_3$) ppm : 2.41(3H, s), 7.24(2H, d, J=8.4Hz), 7.62(2H, d, J=8.4Hz), 7.70(1H, dd, J=8.0, 4.8Hz), 7.85(1H, dd, J=8.0, 1.5Hz), 8.77(1H, dd, J=4.8, 1.5Hz)

(Process 2)

A mixture of the compound (6.0 g) obtained in the process 1, DMF (catalytic amount), thionyl chloride (10 ml), THF (50 ml), and dichloroethane (50 ml) was refluxed for 3 hours. After evaporating the solvents, the residue was dissolved in dichloromethane (100 ml). To this solution, iminodiacetonitrile (3.0 g) and triethylamine (10 ml) were added and were stirred at room temperature for 16 hours. After the reaction mixture was washed subsequently with water, diluted hydrochloric acid, sodium hydrogen carbonate solution and water and dried, the solvent was evaporated. Then, N,N-bis(cyanomethyl)-3-(4-methylbenzoyl)-2-pyridine carboxamide was obtained as maple crystals (4.3 g).
Melting point: 166-168°C (re-crystallization from ethyl acetate - ethyl ether)
NMR (200MHz, $CDCl_3$)ppm : 2.44(3H, s), 4.55(2H, s), 4.69(2H, s), 7.31(2H, d, J=8.1Hz), 7.56(1H, dd, J=7.9, 4.9Hz), 7.69(2H, d, J=8.1Hz), 7.94 (1H, dd, J=7.9, 1.6Hz), 8.78(1H, dd, J=4.9, 1.6Hz)
Anal. Calcd for $C_{18}H_{14}N_4O_2$
Calculated (%): C 67.92, H 4.43, N 17.60
Found (%): C 67.76, H 4.54, N 17.62

(Process 3)

A mixture of the compound (0.86 g) obtained in the process 2, 1,8-diazabicyclo[5,4,0]-7-undecene (DBU) (1 ml) and toluene (40 ml) was refluxed with heating for an hour. After the reaction mixture was diluted with ethyl acetate, washed subsequently with water, diluted acid, sodium hydrogen carbonate solution and water, the solvent was evaporated. Then, 7-cyanomethyl-7,8-dihydro-5-(4-methylphenyl)-8-oxo-6-pyrido[3,4-b] pyridine carbonitrile was obtained as maple crystals (765 mg). Melting point: 229-231°C (re-crystallization from ethyl acetate)
NMR (200MHz, $CDCl_3$)ppm : 2.48(3H, s), 5.28(2H, s), 7.31(2H, d, J=8.2Hz), 7.40(2H, d, J=8.2Hz), 7.64(1H, dd, J=8.2,

4.4Hz), 7.80(1H, dd, J=8.2, 1.4Hz), 9.06(1H, dd, J=4.4, 1.4Hz)
Anal. Calcd for $C_{18}H_{12}N_4O\cdot0.2H_2O$
Calculated (%): C 71.14, H 4.11, N 18.43
Found (%): C 71.20, H 4.26, N 18.20

(Process 4)

A mixture of the compound (2.35 g) obtained in the process 3, hydrochloric acid (25 ml) and acetic acid (25 ml) was refluxed with heating for 1.5 hours. After evaporation of the solvents, the water was added, and the mixture was extracted with ethyl acetate. After this extract solution was washed with saturated brine and dried, the solvent was evaporated. Then 7-carboxymethyl-7,8-dihydro-5-(4-methylphenyl)-8-oxo-6-pyrido[3,4-b] pyridine carbonitrile was obtained as a colorless crystal (1.62 g).
Melting point: 253-254°C (re-crystallization from ethyl acetate)
NMR (200MHz, $CDCl_3$)ppm : 2.46(3H, s), 5.22(2H, s), 6.64(1H, bs, -$CO_2$H), 7.32(2H, d, J=8.2Hz), 7.37(2H, d, J=8.2Hz), 7.62(1H, dd, J=8.4, 4.4Hz), 7.82(1H, d, J=8.4Hz), 9.09(1H, d, J=4.4Hz)
Anal. Calcd for $C_{18}H_{13}N_3O_3\cdot0.1H_2O$
Calculated (%): C 67.33, H 4.14, N 13.09
Found (%): C 67.28, H 4.19, N 13.00

(Process 5)

Hydroxy benzotriazole (770 mg) and 1,3-dicyclohexyl carbodiimide (1.23 g) were added to a solution of the compound (1.54 g) obtained in Process 4 in THF (50 ml) and stirred at room temperature for 3 hours. Then sodium borohydride (550 mg) was added to this reaction mixture and stirred at room temperature for 20 min. After this reaction mixture was diluted with ethyl acetate, washed with water and dried, the solvent was evaporated. Dichloromethane was added to the residue, and the insoluble parts were filtered out, followed by evaporation of the solvent. Hydrochloric acid (50ml) was added to the residue, and refluxed with heating for 16 hours. After evaporation of the solvents, ice-cold water was added to the residue. This solution was alkalinized using potassium carbonate solution and extracted with ethyl acetate. The extract was washed with water and dried, and the solvent was evaporated. Then, 6,8,9,11-tetrahydro-5-(4-methylphenyl)-6,11-dioxo[1,4]oxysadino[3,4-g][1,7]naphthylidine was obtained as colorless crystals (0.86 g).
Melting point: 247-249°C (re-crystallization from ethyl acetate)
NMR (200MHz, $CDCl_3$) ppm : 2.45(3H, s), 4.48-4.72(4H, m), 7.12(2H, d, J=8.0Hz), 7.32(2H, d, J=8.0Hz), 7.55(1H, dd, J=8.4, 4.4Hz), 7.68 (1H, dd, J=8.4, 1.6Hz), 9.01(1H, dd, J=4.4, 1.6Hz)
Anal. Calcd for $C_{18}H_{14}N_2O_3\cdot0.2H_2O$
Calculated (%): C 69.76, H 4.68, N 9.04
Found (%): C 69.64, H 4.86, N 8.95

(Process 6)

A mixture of the compound (410 mg) obtained in process 5 and 3,5-bis(trifluoromethyl) benzylamine (1.2 g) was heated at 150°C for 2.5 hours under an atmosphere of argon. After cooling down to room temperature, isopropyl ether was added to yield the title compound as colorless crystals (441 mg).
Melting point : 123-125°C (re-crystallization from ethyl acetate)
NMR (200MHz, $CDCl_3$) ppm : 2.28 (3H, s), 3.71(2H, m), 3.97 (2H, m), 4.46(2H, d, J=5.2Hz), 7.00-7.20(4H, m), 7.37 (1H, dd, J=8.4, 4.2Hz), 7.52(1H, dd, J=8.4, 1.6Hz), 7.66(2H, s), 7.76(1H, s), 8.51 (1H, bs), 8.61(1H, dd, J=4.2, 1.6Hz)
Anal. Calcd for $C_{27}H_{21}N_3O_3F_6$
Calculated (%): C 59.02, H 3.85, N 7.65
Found (%): C 58.95, H 3.95, N 7.52

(Reference Example 2)

N-[3,5-bis(Trifluoromethyl)benzyl]-7-(3-chloropropyl)-7,8-dihydro-5-(4-methylphenyl)-8-oxo-6-pyrido[3,4-b]pyridine carboxamide

(Process 1)

A mixture of 3-(4-methylbenzoyl)-2-pyridine carboxylic acid (13.9 g), bromomalonic acid diethyl ester (15.4

g) triethylamine (9.1 ml) and THF (120 ml) was refluxed with heating for 6 hours. After evaporation of the solvents, ethyl acetate was added to the residue. This solution was washed subsequently with water, diluted hydrochloric acid and saturated brine, and dried followed by evaporation of the solvents. DBU (4.2 ml) was added to the solution of the residue (oil) (20.5 g) in THF (120ml) at -78°C. After this mixture was stirred at 0°C for 15 min, the solvent was concentrated. After the concentrated solution was poured into 2N-HCl, its pH was adjusted at about 10 using sodium hydrogen carbonate, and then the solution was extracted with ethyl acetate. The extract was washed with water and dried followed by evaporation of solvents. Then, 5,6-dihydro-5-hydroxy-5-(4-methylphenyl)-8-oxo-8H-pyrano[3,4-b]pyridine-6, 6-dicarboxylic acid diethyl ester was obtained as colorless crystals (14.1 g).
Melting point: 148-149°C (re-crystallization from ethyl acetate-isopropyl ether)
NMR (200MHz, $CDCl_3$) ppm : 1.06(3H, t, J=7.1Hz), 1.21(3H, t, J=7.1Hz), 2.31(3H, s), 3.95-4.30(4H, m), 4.65(1H, s), 7.15(2H, d, J=8.3Hz), 7.55(1H, dd, J=8.0, 4.8Hz), 7.65(2H, d, J=8.3Hz), 8.47(1H, dd, J=8.0, 1.4Hz), 8.86(1H, dd, J=4.8, 1.4Hz)
Anal. Calcd for $C_{21}H_{21}NO_7$
Calculated (%): C 63.15, H 5.30, N 3.51
Found (%): C 63.09, H 5.16, N 3.47

The present compound was obtained by the process described below.

A mixture of 3-(4-methylbenzoyl)-2-pyridine carboxylic acid (3.0 g), DMF (one drop), thionyl chloride (4.5 ml) and THF (30 ml) was refluxed with heating for 2 hours. After evaporation of the solvents, the obtained crystal residue was dissolved in THF (50 ml). Hydroxy malonic acid diethyl ester (4.1 g) was added to this solution with stirring at -10°C, then sodium hydride (60 % oil) (646 mg) was gradually added. After this reaction mixture was stirred at -10°C for 30 min, added to a mixture of ethyl acetate (100 ml) and water (100 ml). The organic layer was separated, and the aqueous layer was extracted with ethyl acetate. The organic layer and the extract were combined, and washed with water and brine, and dried followed by evaporation of the solvents. Then the above compound was obtained as colorless crystals (4.0 g). Physico-chemical data of this compound is identical to those of the above compound.

(Process 2)

A mixture of the compound (14.1 g) obtained from the process 1, acetic acid (100 ml) and hydrochloric acid (100 ml) was refluxed with heating for 3 hours. After evaporation of the solvents, water was added to the residue. Then 5-(4-methylphenyl)-8-oxo-8H-pyrano[3,4-b] pyridine-6-carboxylic acid was obtained as a colorless crystal (8.45 g)
Melting point: 274-277°C (changed to brown color at around 240°C) (re-crystallization from THF - isopropyl ether)
NMR (200MHz, $CDCl_3$-DMSO-$d_6$) ppm : 2.43 (3H, s), 6.10(1H, bs, -$CO_2H$), 7.16(2H, d, J=8.0Hz), 7.29(2H, d, J=8.0Hz), 7.50-7.70(2H, m), 8.94(1H, m)
Anal. Calcd for $C_{16}H_{11}NO_4 \cdot 0.1H_2O$
Calculated (%) : C 67.89, H 3.99, N 4.95
Found (%): C 67.70, H 4.06, N 4.83

(Process 3)

A solution of 5-(4-methylphenyl)-8-oxo-8H-pyrano[3,4-b] pyridine-6-carboxylic acid (150 mg) in THF (5 ml) was dropped into a mixture of 3-bromopropylamine hydrobromide (1.5 g), triethylamine (2.0 ml) and methanol (5 ml), and stirred at room temperature for 2 hours followed by evaporation of the solvents. Hydrochloric acid (10 ml) was added to the residue, and stirred at room temperature for 14 hours, then concentrated. The pH of the concentrated solution was adjusted at 1 using 1N-NaOH. The precipitated crystals were collected by filtration and washed with water. Then 7-(3-bromopropyl)-7,8-dihydro-5-(4-methylphenyl)-8-oxo-6-pyrido[3,4-b] pyridine carboxylic acid was obtained as colorless crystals (131 mg).
Melting point: 194-196°C (re-crystallization from THF - isopropyl ether)
NMR (200MHz, $CDCl_3$-DMSO-$d_6$)ppm : 2.30-2.60 (2H, m), 2.42(3H, s), 3.54(2H, t, J=6.8Hz), 4.29(2H, t, J=7.2Hz), 5.42(1H, bs, -$CO_2H$), 7.20-7.40(4H, m), 7.50(1H, m), 7.64(1H, d,J=8.0Hz), 8.88(1H, m)

(Process 4)

A mixture of the compound (110 mg) obtained from the process 3, DMF (catalytic amount), thionyl chloride (0.3 ml), 1,2-dichloromethane (3 ml) and THF (3 ml) was refluxed with heating for 40 min followed by evaporation of the solvents. A mixture of THF (5 ml), 3,5-bis(trifluoromethyl) benzylamine (82 mg), triethylamine (0.12 ml) and THF (2 ml) was added to the residue, and was stirred at room temperature for 2 hours. Ethyl acetate was added to the reaction mixture, and was washed subsequently with water, diluted hydrochloric acid, sodium hydrogen carbonate solution and water, then was dried followed by evaporation of the solvents. Then the title compound was obtained as

colorless crystals (79 mg).
Melting point: 227-229°C (re-crystallization from ethyl acetate - isopropyl ether)
NMR (200MHz, $CDCl_3$) ppm : 2.10-2.40 (2H, m), 2.27(3H, s), 3.4-3.7(4H, m) , 4.49(2H, d, J=5.8Hz), 7.07(2H, d, J=7.6Hz), 7.24(2H, d, J=7.6Hz), 7.40(1H, dd, J=8.4, 4.2Hz), 7.54(1H, dd, J=8.4, 1.4Hz), 7.67(2H, s), 7.78(1H, s), 8.06 (1H, bt), 8.70(1H, dd,J=4.2, 1.4Hz)
Anal. Calcd for $C_{28}H_{22}N_3O_2ClF_6 \cdot 0.2H_2O$
Calculated (%): C 57.43, H 3.86, N 7.18
Found (%) : C 57.29, H 3.98, N 7.07

(Reference Example 3)

N-[3,5-bis(Trifluoromethyl)benzyl]-5-(4-methylphenyl)-8-oxo-8H-pyrano[3,4-b]pyridine-6-carboxamide

Using the compound obtained from the process 2 of the Reference Example 2 and 3,5-bis(trifluoromethyl) benzylamine, the reaction and treatment were performed as in the case of the process 4 of the Reference Example 2. Then, the title compound was obtained as colorless crystals.
Melting point: 182-183°C (re-crystallization from ethyl acetate - ether)
NMR (200MHz, $CDCl_3$) ppm : 2.44 (3H, s), 4.63(2H, d, J=6.4Hz), 7.17(2H, d, J=8.1Hz), 7.34(2H, d, J=8.1Hz), 7.50-7.65 (3H, m), 7.73(2H, s), 7.80(1H, s), 8.96(1H, m)
Anal. Calcd for $C_{25}H_{16}N_2O_3F_6$
Calculated (%): C 59.30, H 3.18, N 5.53
Found (%): C 59.42, H 3.30, N 5.45

(Reference Example 4)

N-(2-Methoxybenzyl)-5-(4-methylphenyl) -8-oxo-8H-pyrano[3,4-b] pyridine-6-carboxamide

Using the compound obtained from the process 2 of the Reference Example 2 and 2-methoxybenzyl amine, the reaction and treatment were performed as in the case of the process 4 of the Reference Example 2. Then the title compound was obtained as colorless crystals.
Melting point: 189-190°C (re-crystallization from ethyl acetate)
NMR (200MHz, $CDCl_3$) ppm : 2.44 (3H, s), 3.90(3H, s), 4.48(2H, d, J=5.8Hz), 6.85-6.95(2H, m), 7.10-7.35(6H, m), 7.43(1H, bt), 7.50-7.63(2H, m), 8.93(1H, m)

(Reference Example 5)

N-[3,5-bis(Trifluoromethyl)benzyl]-7,8-dihydro-7-(4-hydroxybutyl)-5-(4-methylphenyl)-8-oxo-6-pyrido[3,4-b] pyridine carboxamide

4-Amino-1-butanol (0.5 ml) was added at 0°C to a solution of the compound (200 mg) obtained from the Reference Example 3 in THF (5 ml) - methanol (10 ml), and stirred at room temperature for an hour. The solvents were evaporated from the reaction mixture, and hydrochloric acid (15 ml) was added to the residue, then was stirred at room temperature for 14 hours. After the reaction solution was poured into potassium carbonate solution -ice, the solution was extracted with ethyl acetate. The extract solution was washed with water and dried followed by evaporation of the solvents. Then the title compound was obtained as colorless crystals (144 mg).
Melting point: 187-188°C (re-crystallization from ethyl acetate - isopropyl ether)
NMR (200MHz, $CDCl_3$) ppm:1.3-1.5(2H, m), 1.6-1.9(2H, m), 2.29(3H, s), 2.82(1H, bs), 3.55(2H, t, J=5.7Hz), 3.69(2H, m), 4.48(2H, d, J=5.8Hz), 7.08(2H, d, J=8.1Hz), 7.21(2H, d, J=8.1Hz), 7.29 (1H, dd, J=8.4, 4.2Hz), 7.52 (1H, dd, J=8.4, 1.4Hz), 7.68 (2H, s), 7.78(1H, s), 8.39(1H, bt), 8.61(1H, dd, J=4.2, 1.4Hz)

(Reference Example 6)

7,8-Dihydro-7-(3-hydroxypropyl)-N-(2-methoxybenzyl)-5-(4-methylphenyl)-8-oxo-6-pyrido[3,4-b]pyridinecarboxamide

Using the compound obtained from the Reference Example 4 and 3-amino-1-propanol, the reaction and treatment were performed as in the case of the Reference Example 5. Then, the titled compound was obtained. This compound was used in the reaction of the Reference Example 58 without purification.

(Reference Example 7)

7,8-Dihydro-7-(4-hydroxybutyl)-N-(2-methoxybenzyl)-5-(4-methylphenyl)-8-oxo-6-pyrido[3,4-b]pyridinecarboxamide

Using the compound obtained from the Reference Example 4 and 4-amino-1-butanol, the reaction and treatment were performed as in the case of the Reference Example 5. Then, the title compound was obtained as colorless crystals.
Melting point: 205-206°C (re-crystallization from methanol - ethyl ether)
NMR (200MHz, $CDCl_3$) ppm : 1.57(2H, m), 1.95(2H, m), 2.33(3H, s), 2.71(1H, bs), 3.66(2H, t, J=6.0Hz), 3.75(3H; s), 4.00-4.15(2H, m), 4.29(2H, d, J=6.2Hz), 6.59(1H, bt), 6.71-6.92(3H, m), 7.04-7.30 (5H, m), 7.41(1H, dd, J=8.4, 4.4Hz), 7.62(1H, dd, J=8.4, 1.4Hz), 8.82(1H, dd, J=4.4, 1.4Hz)

(Reference Example 8)

N-[3,5-bis(Trifluoromethyl)benzyl-7,8-dihidro-7-(5-hydroxypentyl)-5-(4-methylphenyl)-8-oxo-6-pyrido[3,4-b]pyridine carboxamide

Using the compound obtained from the Reference Example 3 and 5-amino-1-pentanol, the reaction and treatment were performed as in the case of the Reference Example 5. Then, the title compound was obtained as colorless crystals.
Melting point: 136-137°C (re-crystallization from ethyl acetate - ethyl ether)
NMR (200MHz, $CDCl_3$) ppm:1.10-1.35(2H, m), 1.35-1.55(2H, m), 1.6-1.9(2H, m), 2.28(3H, s), 3.50-3.70(4H, m), 4.47 (2H, d, J=5.8Hz), 7.06(2H, d, J=8.0Hz), 7.19(2H, d, J=8.0Hz), 7.35(1H, dd, J=8.3, 4.4Hz), 7.50(1H, d, J=8.3, 1.4Hz), 7.69(2H, s), 7.78(1H, s), 8.29(1H, bt), 8.64(1H, dd, J=4.4, 1.4Hz)

(Reference Example 9)

N-[3,5-bis(Trifluoromethyl)benzyl]-7,8-dihydro-7-(3-hydroxypropyl)-8-oxo-5-phenyl-6-pyrido[3,4-b] pyridinecarboxamide

(Process 1)

Using 3-benzoyl-2-pyridinecarboxylic acid instead of 3-(4-methylbenzoyl)-2-pyridinecarboxylic acid in the process 1 of the Reference Example 2, the reaction and treatment were performed as in the case of the process 1 of the Reference Example 2. Then 5,6-dihydro-5-hydroxy-8-oxo-5-phenyl-8H-pyrano[3,4-b]pyridine-6, 6-dicarboxylic acid diethyl ester was obtained as colorless crystals.
Melting point: 146-147°C (re-crystallization from ethyl acetate - isopropyl ether)
NMR (200MHz, $CDCl_3$) ppm:1.07(3H, t, J=7.2Hz), 1.18(3H, t, J=7.2Hz), 4.00-4.25(4H, m), 4.70(1H, s), 7.30-7.40(3H, m), 7.56(1H, dd, J=8.0, 4.8Hz)., 7.74-7.85(2H, m), 8.48(1H, dd, J=8.0, 1.5Hz), 8.87(1H, dd, J=4.8, 1.5Hz)

(Process 2)

Using the compound obtained from the process 1, acetic acid and hydrochloric acid, the reaction and treatment were performed as in the case of the process 2 of the Reference Example 2. Then, 8-oxo-5-phenyl-8H-pyrano[3,4-b] pyridine-6-carboxylic acid was obtained as colorless crystals.
Melting point: 288-290°C (re-crystallization from THF - methanol - ether)
NMR (200MHz, DMSO-$d_6$) ppm : 7.28-7.60(6H, m), 7.81(1H, dd, J=8.2, 4.4Hz), 8.95(1H, dd, J=4.4, 1.6Hz)

(Process 3)

Using the compound obtained from the process 2 and 3,5-bis(trifluoromethyl)benzylamine, the reaction and treatment were performed as in the case of the Reference Example 3. Then, N-[3,5-bis(trifluoromethyl)benzyl]-8-oxo-5-phenyl-8H-pyrano[3,4-b]pyridine-6-carboxamide was obtained as colorless crystals.
Melting point: 182-183°C (re-crystallization from ethyl acetate - isopropyl ether)
NMR (200MHz, $CDCl_3$) ppm: 4.61(2H, t, J=6.2Hz), 7.24-7.34(2H, m), 7.49-7.67(6H, m), 7.72(2H, s), 7.79(1H, s), 8.96 (1H, dd, J=4.2, 1.6Hz)

(Process 4)

Using the compound obtained from the process 3 and 3-amino-1-propanol, the reaction and treatment were performed as in the case of the Reference Example 5. Then the title compound was obtained as colorless crystals.
Melting point: 129-130°C (re-crystallization from ethyl acetate - ethyl ether)
NMR (200MHz, $CDCl_3$) ppm:1.91(2H, m), 3.45(2H, t, J=5.4Hz), 3.70(2H, m), 4.46(2H, d, J=6.0Hz), 7.2-7.4(5H, m), 7.44(1H, dd, J=8.4, 4.4Hz), 7.59(1H, dd, J=8.4, 1.6Hz), 7.61(2H, s), 7.78(1H, s), 8.25(1H, bt), 8.70(1H, dd, J=4.4, 1.6Hz)

(Reference Example 10)

N-[3,5-bis(Trifluoromethyl)benzyl]-7,8-dihydro-7-(4-hydroxybutyl)-8-oxo-5-phenyl-6-pyrido[3,4-b]pyridine carboxamide

Using the compound obtained from the process 3 of the Reference Example 9 and 4-amino-1-butanol, the reaction and treatment were performed as in the case of the Reference Example 5. Then, the title compound was obtained as colorless crystals.
Melting point: 155-157°C (re-crystallization from ethyl acetate - isopropyl ether)
NMR (200MHz, $CDCl_3$) ppm : 1.45(2H, m), 1.83(2H, m), 3.58(2H, t, J=5.8Hz), 3.73(2H, m), 4.44(2H, d, J=5.8Hz), 7.2-7.4(6H, m), 7.54(1H, dd, J=8.1, 1.1Hz), 7.60(2H, s), 7.77(1H, s), 8.05(1H, bt), 8.66(1H, dd, J=4.1, 1.1Hz)

(Reference Example 11)

N-[3,5-bis(Trifluoromethyl)benzyl]-1,2-dihydro-2-(4-hydroxybutyl)-4-(4-methylphenyl)-1-oxo-3-pyrido[3,4-c] pyridinecarboxamide

(Process 1)

Using 4-(4-methylbenzoyl)-3-pyridinecarboxylic acid instead of 3-(4-methylbenzoyl)-2-pyridinecarboxylic acid in the process 1 of the Reference Example 2, the reaction and treatment were performed as in the case of the process 1 of the Reference Example 2. Then, 3,4-dihydro-4-hydroxy-4-(4-methylphenyl)-1-oxo-1H-pyrano[3,4-c]pyridine-3,3-dicarboxylic acid diethyl ester was obtained as a yellow oil.
NMR (200MHz, $CDCl_3$) ppm : 1.08(3H, t, J=7.1Hz), 1.21(3H, t, J=7.2Hz), 2.31(3H, s), 4.00-4.40(4H, m) , 4.72(1H, bs), 7.14(2H, d, J=8.4Hz), 7.64(2H, d, J=8.4Hz), 8.05(1H, d, J=5.3Hz), 8.85(1H, d, J=5.3Hz), 9.12(1H, s)

(Process 2)

Using the compound obtained from the process 1, acetic acid and hydrochloric acid, the reaction and treatment were performed as in the case of the process 2 of the Reference Example 2. Then, 4-(4-methylphenyl)-1-oxo-1H-pyrano[3,4-c]pyridine-3-carboxylic acid was obtained as colorless crystals.
Melting point: 254-256°C (re-crystallization from THF - isopropyl ether)
NMR (200MHz, $CDCl_3$+$d_6$-DMSO) ppm:2.43(3H, s), 5.31 (1H, bs, COOH), 7.04(1H, d, J=5.5Hz), 7.16(2H, d, J=7.8Hz), 7.29(2H, d, J=7.8Hz), 8.81(1H, d, J=5.5Hz), 9.54(1H, s)

(Process 3)

Using the compound obtained from the process 2 and 3,5-bis(trifluoromethyl)benzylamine, the reaction and treatment were performed as in the case of the Reference Example 3. Then, N-[3,5- bis(trifluoromethyl)benzyl)-4-(4-methylphenyl)-1-oxo-1H-pyrano[3,4-c]pyridine-3-carboxamide was obtained as colorless crystals.
Melting point: 188-189°C (re-crystallization from ethyl acetate - isopropyl ether)
NMR (200MHz, $CDCl_3$) ppm:2.44(3H, s), 4.61(2H, d, J=6.2Hz), 7.05(1H, d, J=5.3Hz), 7.15(2H, d, J=8.1Hz), 7.32(2H, d, J=8.1Hz), 7.43(1H, bt), 7.7.0(2H, s), 7.80(1H, s), 8.85(1H, d, J=5.3Hz), 9.56(1H, s)

(Process 4)

Using the compound obtained from the process 3 and 4-amino-1-butanol, the reaction and treatment were performed as in the case of the Reference Example 5. Then, the title compound was obtained as colorless crystals.
Melting point: 128-131°C (re-crystallization from ethyl acetate - isopropyl ether)
NMR (200MHz, $CDCl_3$) ppm:1.45-1.70(2H, m), 1.75-2.05(2H, m), 2.31(3H, s), 3.65(2H, t, J=5.9Hz), 3.98(2H, m), 4.36(2H, d, J=6.0Hz), 7.00(1H, d, J=5.7Hz), 7.12(2H, d, J=8.1Hz), 7.18(2H, d, J=8.1Hz), 7.56(2H, s), 7.80(1H, s), 8.47(1H, d, J=5.7Hz), 9.41(1H, s)

(Reference Example 12)

N-[3,5-bis(Trifluoromethyl)benzyl]-2-chloro-N-(2-hydroxyethyl)-4-phenyl-3-pyridine-carboxamide

(Process 1)

3,5-bis(Trifluoromethyl)benzylbromide (1.1 ml) was added to a solution of 2-aminoethanol (3.6 ml) in THF (30 ml) under ice cooling. After this mixture was stirred at room temperature, ethyl acetate (30 ml) was added. The mixture was washed with water and saturated sodium chloride solution, and the organic layer was dried followed by evaporation of the solvents. Then, N-[3,5- bis(trifluoromethyl)benzyl]-N-(2-hydroxyethyl)amine was obtained as colorless crystals (1.38 g) Melting point: 107-108°C (re-crystallization from ethanol - ethyl ether)
NMR (200MHz, $CDCl_3$) ppm:2.83(2H, t, J=5.4Hz), 3.72(2H, t, J=5.4Hz), 3.96(2H, s), 7.78(1H, s), 7.82(2H, s)

(Process 2)

Thionyl chloride (0.7 ml) and DMF (catalytic amount) were added to a solution of 2-chloro-4-phenyl-3-pyridinecarboxylic acid (318 mg) in THF (10 ml), and refluxed with heating for 4 hours. After evaporation of the solvents, the residue was dissolved in THF (5 ml). This solution was added to the mixture of N-[3,5- bis(trifluoromethyl)benzyl]-N-(2-hydroxyethyl)amine (391 mg) obtained from the process 1, trimethylamine (0.57 ml) and THF (10 ml) under ice cooling, and stirred at room temperature for 2 hours. After evaporation of the solvents, water was added to the residue, and extracted with ethyl acetate. The extract was washed with water, and dried followed by evaporation of the solvents. The residue was separated and purified by a column chromatography on silica gel (hexane : ethyl acetate = 1 : 1). Then, the title compound was obtained as an oil (551 mg) (the ratio of cis to trans isomers for amido bond was about 2 : 1).
NMR (200MHz, $CDCl_3$) ppm : 2.82-3.92(4H, m), 4.16(1H×1/3, d, J=16.0Hz), 4.41(1H×1/3, d, J=16.0Hz), 4.73(1H×2/3, d, J=15.0Hz), 4.87(1H×2/3, d, J=15.0Hz), 7.20-8.85(9H, m), 8.43(1H, m)

(Reference Example 13)

(S) -N-[3,5-bis(Trifluoromethyl)benzyl]-7,8-dihydro-7-(3-hydroxy-2-methylpropyl)-5-(4-methylphenyl)-8-oxo-6-pyrido [3,4-b]pyridine carboxamide

(S) -3-Amino-2-methyl-1-propanol (307 mg) was added to a solution of the compound (1.0 g) obtained from the Reference Example 3 in THF (10 ml) - methanol (7.5 ml), and stirred at room temperature for 14 hours. After diluted hydrochloric acid was added to the reaction mixture, extracted with ethyl acetate. The extract solution was washed with saturated brine, and then dried followed by evaporation of the solvents. Acetonitrile (3 ml), toluene (21 ml) and DBU (0.42 ml) were added to the residue, and refluxed with heating for an hour. After the reaction solution was cooled, then diluted with ethyl acetate, and washed subsequently with water, diluted hydrochloric acid and saturated brine. The organic layer was dried and the solvent was evaporated. Then the title compound was obtained as colorless crystals.
Melting point: 123-125°C (After once melting, solidification again), 215-216°C (melting again) (re-crystallization from ethyl acetate - isopropyl ether)
$[\alpha]_D$: +11.1° (c = 0.350, $CHCl_3$)
NMR (200MHz, $CDCl_3$) ppm : 0.79(3H, d, J=7.0Hz), 2.13(1H, m), 2.28(3H, s), 3.10-3.70(4H, m), 4.48(2H, d, J=6.2Hz), 7.00-7.25(4H, m), 7.43(1H, dd, J=8.4, 4.2Hz), 7.59(1H, dd, J=8.4, 1.6Hz), 7.69(2H, s), 7.79(1H, s), 8.38(1H, bt), 8.70 (1H, dd, J=4.2, 1.6Hz)
Anal. Calcd for $C_{29}H_{25}N_3O_3F_6 \cdot 0.5H_2O$
Calculated (%): C 59.39, H 4.47, N 7.16
Found (%): C 59.64, H 4.31, N 7.01

(Reference Example 14)

(R) -N-[3,5-bis(Trifluoromethyl)benzyl]-7,8-dihydro-7-(3-hydroxy-2-methylpropyl)-8-oxo-5-phenyl-6-pyrido[3,4-b] pyridine carboxamide

Using the compound obtained from the process 3 of the Reference Example 9 and (R) -3-amino-2-methyl-1-propanol, the reaction and treatment were performed as in the case of the Reference Example 13. Then the title compound was obtained as colorless crystals.
Melting point: 101-103°C (re-crystallization from ethyl ether - isopropyl ether)
NMR (200MHz, $CDCl_3$) ppm:0.77(3H, d, J=6.6Hz), 2.14(1H, m), 3.10-3.70(4H,m), 4.47 (2H,d, J=5.8Hz), 7.1-7.4(5H, m), 7.45(1H, dd, J=8.4, 4.2Hz), 7.60(1H, d, J=8.4Hz), 7.65(2H, s), 7.78(1H, s), 8.60(1H, bt), 8.69(1H, d, J=4.2Hz)
$[\alpha]_D$:-5.4° (c = 0.512, $CHCl_3$)

(Reference Example 15)

(R) -N-[3,5-bis(Trifluoromethyl)benzyl]-7,8-dihydro-7-(3-hydroxy-2-methylpropyl)-5-(4-methylphenyl)-8-oxo-6-pyrido [3,4-b]pyridine carboxamide

Using the compound obtained from the Reference Example 3 and (R) -3-amino-2-methyl-1-propanol, the reaction and treatment were performed as in the case of the Reference Example 13. Then the title compound was obtained as colorless crystals.
Melting point: 123-125°C (After once melting, solidification again), 215-216°C (melting again) (re-crystallization from ethyl acetate - isopropyl ether)
NMR (200 MHz $CDCl_3$) ppm: Identical to the spectra of the compound of the Reference Example 13.
$[\alpha]_D$: -9.0° (C = 0.346, $CHCl_3$)

(Reference Example 16)

(±) -N-[3,5-bis(Trifluoromethyl)benzyl]-7,8-dihydro-7-(4-hydroxy-3-methylbutyl)-8-oxo-5-phenyl-6-pyrido[3,4-b] pyridinecarboxamide

Using the compound obtained from the process 3 of the Reference Example 9 and 4-amino-2-methyl-1-butanol, the reaction and treatment were performed as in the case of the Reference Example 13. Then, the title compound was obtained as colorless crystals.
Melting point: 217-219°C (re-crystallization from ethyl acetate - isopropyl ether)

(Reference Example 17)

(±) -N-[3,5-bis(Trifluoromethyl)benzyl]-7,8-dihydro-7-(4-hydroxy-3-methylbutyl)-5-(4-methylphenyl)-8-oxo-6-pyrido [3,4-b]pyridinecarboxamide

Using the compound obtained from the Reference Example 3 and 4-amino-2-methyl-1-butanol, the reaction and treatment were performed as in the case of the Reference Example 13. Then the title compound was obtained as colorless crystals.
Melting point: 129-130°C (After once melting, solidification again), 188-190°C (melting again) (re-crystallization from ethyl acetate - isopropyl ether)
NMR (200MHz, $CDCl_3$) ppm: 0.79(3H, d, J=6.6Hz), 1.4-1.8(3H, m), 2.28(3H, s), 3.03(1H, t, J=6.6Hz, -OH), 3.2-3.7 (4H, m), 4.49(2H, d, J=5.8Hz), 7.0-7.3(4H, m), 7.30(1H, dd, J=8.4, 4.4Hz), 7.53(1H, dd, J=8.4, 1.4Hz), 7.68(2H, s), 7.78(1H, s), 8.48(1H, t, J=6.0Hz), 8.61(1H, dd, J=4.4, 1.4Hz)

(Reference Example 18)

(R) -N-[3,5-bis(Trifluoromethyl)benzyl]-7,8-dihydro-7-(4-hydroxy-3-methylbutyl)-8-oxo-5-phenyl-6-pyrido[3,4-b] pyridine carboxamide

Using the compound obtained from the process 3 of the Reference Example 9 and (R)-4-amino-2-methyl-1-butanol tetrahydropyranyl (THP), the reaction and treatment were performed as in the case of the Reference Example 13. Then, THP ether of the title compound was obtained as a pale orange oil. The THP group of this compound was

removed by reacting with p-toluene sulfonic acid in methanol at room temperature. Then the title compound was obtained as colorless crystals.
Melting point: 213-215°C (re-crystallization from ethyl acetate - ethyl ether)
NMR (200MHz, $CDCl_3$) ppm: Identical to the spectra of the compound of the Reference Example 16.
$[\alpha]_D$: + 1.0° (C=0.219, $CHCl_3$)

By the way, (R)-4-amino-2-methyl-1-butanol THP ether was prepared by the following method.

Using (S) - (+) -3-hydroxy-2-methylpropionic acidmethyl ester as a starting material, (R) -4-hydroxy-3-methyl butanenitrile THP ether was prepared by reaction according to the method described in the references [Kenji Mori, Tetrahedron Vol. 39: p3107-p3109 (1983): a synthetic method for enantiomeric isomers has been described, or H. Mattes et al., Journal of Medical Chemistry, Vol. 30: p1948-p1951 (1987)]. [colorless oil, NMR (200 MHz, $CDCl_3$) ppm: 1.09 (3H×1/2, d, J = 6.8 Hz), 1.10 (3H×1/2, d, J=6.8Hz), 1.5-1.9 (6H, m), 2.1-2.6 (3H, m), 3.17-3.88 (4H, m), 4.60 (1H, b)].

The solution of this compound (22.7 g) in ethyl ether (100 ml) was gradually added to the suspension of lithium aluminium hydride (3.7 g) in ethyl ether (200 ml) with intensively stirring at 0°C. Subsequently, the reaction mixture was stirred at room temperature for an hour, cooled again with ice water, and then, water (3 ml), 15 % sodium hydroxide solution (3 ml) and water (100ml) were added with stirring. The precipitate was separated by filtration using Celite, and washed with ethyl acetate. The filtrate was combined with the washing solution, and they were washed with potassium carbonate solution and brine followed by dry and evaporation of the solvent. Then, (R)-4-amino-2-methyl-1-butanol THP ether was obtained as colorless oil (21.7 g).
NMR (200 MHz, $CDCl_3$) ppm: 0.94 (3H×1/2, d, J = 6.8 Hz), 0.95 (3H×1/2, d, J = 6.8 Hz), 1.2-1.9 (11H, m), 3.13-3.90 (6H, m), 4.57 (1H, b).

(Reference Example 19)

(R) -N-[3,5-bis(Trifluoromethyl)benzyl]-7,8-dihydro-7-(4-hydroxy-3-methylbutyl)-5-(4-methylphenyl)-8-oxo-6-pyrido [3,4-b]pyridine carboxamide

Using the compound obtained from the Reference Example 3 and (R) -4-amino-2-methyl-1-butanol THP ether. the reaction and treatment were performed as in the case of the Reference Example 13. Then, THP ether of the title compound was obtained as a pale orange oil. The THP group of this compound was removed by reacting with p-toluene sulfonic acid in methanol at room temperature. Then, the title compound was obtained as colorless crystals.
Melting point: 123-125°C (After once melting, solidification again), 205-206°C (melting again) (re-crystallization from ethyl acetate - isopropyl ether)
NMR (200MHz, $CDCl_3$) ppm: Identical to the spectra of the compound of the Reference Example 17.
$[\alpha]_D$: + 1.2° (C=0.471, $CHCl_3$)

(Reference Example 20)

S -N-[3,5-bis(Trifluoromethyl)benzyl]-7,8-dihydro-7-(4-hydroxy-3-methylbutyl)-8-oxo-5-phenyl-6-pyrido[3,4-b]pyridine carboxamide

Using (S) -4-amino-2-methyl-1-butanol THP ether instead of (R) -4-amino-2-methyl-1-butanol THP ether in the Reference Example 18, the reaction and treatment was performed as in the case of the Reference Example 18. Then, the title compound was obtained as colorless crystals.
Melting point: 213-214°C (re-crystallization from ethyl acetate - ethyl ether)
NMR (200MHz, $CDCl_3$) ppm: Identical to the spectra of the compound of the Reference Example 16.
$[\alpha]_D$: - 1.5° (C=0.492, $CHCl_3$)

(Reference Example 21)

(S) -N-[3,5-bis(Trifluoromethyl)benzyl]-7,8-dihydro-7-(4-hydroxy-3-methylbutyl)-5-(4-methylphenyl)-8-oxo-6-pyrido [3,4-b]pyridine carboxamide

Using (S) -4-amino-2-methyl-1-butanol THP ether instead of (R) -4-amino-2-methyl-1-butanol THP ether in the Reference Example 19, The reaction and treatment were performed as in the case of the Reference Example 19. Then, the title compound was obtained as colorless crystals.
Melting point: 213 to 215°C (After once melting, solidification again), 207-208°C (melting again) (re-crystallization from ethyl acetate - isopropyl ether)

NMR (200MHz, $CDCl_3$) ppm: Identical to the spectra of the compound of the Reference Example 17.
$[\alpha]_D$: - 2.7° (C=0.391, $CHCl_3$)

(Reference Example 22)

N-(2-Aminoethyl)-N-[3,5-bis(trifluoromethyl)benzyl]-2-chloro-4-phenyl-3-pyridine carboxamide

(Process 1)

Thionyl chloride (0.15 ml) and DMF (catalytic amount) were added to a solution of 2-chloro-4-phenyl-3-pyridine carboxylic acid (145 mg), and refluxed with heating for 2 hours. Following evaporation of the solvent, the residue was dissolved in THF (5 ml). This solution was added to a mixture of N-[3,5-bis(trifluoromethyl)benzyl]-N'-tert-butoxycarbonyl ethylene diamine (240 mg), triethyl amine (0.26 ml) and THF (10 ml), and stirred at room temperature for 3 hours. N-[3,5-bis(trifluoromethyl)benzyl]-N'-tert-butoxycarbonyl ethylene diamine was prepared as an oil by after obtaining N-[3,5-bis(trifluoromethyl)benzyl]ethylene diamine as an oil by reacting ethylene diamine with methane sulfonic acid-3,5-bis(trifluoromethyl)benzyl ester in THF, further by reacting this compound with di-tert- butyl bicarbonate in THF in the presence of triethylamine.

Following evaporation of the solvents from the reaction mixture, water was added to the residue, which was extracted with ethyl acetate. After washing the extract solution with water and drying, the solvents were evaporated to give N-[3,5-bis(trifluoromethyl)benzyl]-N-[2-(tert-butoxycarbonyl amino)ethyl]-2-chloro-4-phenyl-3-pyridinecarboxamide as a pale yellow oil.
NMR (200MHz, $CDCl_3$)ppm : 1.20-1.60 (total 9H, m), 2.70-4.90 (total 7H, m), 7.20-8.00(total 9H, m), 8.46(1H, d, J=5.2Hz)

(Process 2)

4N-HCl in ethyl acetate solution (10 ml) was added to the compound obtained in the process 1, and stirred at room temperature for 30 min. Following evaporation of the solvents, sodium hydrogen carbonate solution was added to the residue, which was extracted with ethyl acetate. After washing the extract solution with water and drying, the solvents were evaporated to give the title compound as a yellow oil (349 mg). NMR (200MHz, $CDCl_3$)ppm : 2.30-3.70 (4H, m), 4.15(1H×2/5, d, J=16.2Hz), 4.38(1H×2/5, d, J=16.2Hz), 4.65 (1H×3/5, d, J=15.2Hz), 4.84 (1H×3/5, d, J=15.2Hz), 7.20-7.60(6H, m), 7.65-7.80 (6H, m), 8.47 (1H, m)

(Reference Example 23)

N-[3,5-bis(Trifluoromethyl)benzyl] -2-chloro-N-(3-hydroxypropyl)-4-phenyl-3-pyridinecarboxamide

(Process 1)

Using 3-amino-1-propanol instead of 2-amino ethanol in the process 1 of the Reference Example 12, the reaction and treatment were performed as in the case of the process 1 of the Reference Example 12. Then, N-[3,5-bis (trifluoromethyl) benzyl]-N-(3-hydroxypropyl)amine as colorless crystals.
Melting point: 57-58°C (re-crystallization from ethyl ether - hexane)
NMR (200MHz, $CDCl_3$) ppm : 1.77 (2H, quintet, J=5.8Hz), 2.89(2H, t, J=5.8Hz), 3.82(2H, t, J=5.8Hz), 3.93(2H, s), 7.89 (3H, s)
Anal. Calcd for $C_{12}H_{13}NOF_6$
Calculated (%): C 47.85, H 4.35, N 4.65
Found (%): C 47.76, H 4.32, N 4.65

(Process 2)

Using amine obtained in the process 1 instead of N-[3,5-bis(trifluoromethyl)benzyl]-N-(2-hydroxyethyl)amine in the process 2 of the Reference Example 12, the reaction and treatment were performed as in the case of the process 2 of the Reference Example 12. Then, the title compound was obtained as colorless crystals (the ratio of cis to trans isomers for amino bonds: about 3 : 1).
Melting point: 121-122°C (re-crystallization from ethyl acetate - isopropyl ether)
NMR (200MHz,$CDCl_3$)ppm : 1.00-1.70(2H, m), 2.75-3.20(2H, m), 3.35-3.55(3H, m), 4.06 (1H×1/4, d, J=16.2Hz), 4.31 (1H×1/4, d, J=16.2Hz), 4.65(1H×3/4, d, J=15.2Hz), 4.76(1H×3/4, d, J=15.2Hz), 7.20-7.55(6H, m), 7.72(2H, s), 7.80(1H, s), 8.47(1H, d, J=5.2Hz)
Anal. Calcd for $C_{24}H_{19}N_2O_2F_6Cl$
Calculated (%): C 55.77, H 3.71, N 5.42
Found (%): C 55.65, H 3.70, N 5.57

(Reference Example 24)

N-[3,5-bis(Trifluoromethyl)benzyl]-2-chloro-N-(2-hydroxyethyl)-5-methyl-4-phenyl-3-pyridine carboxamide

Using 2-chloro-5-methyl-4-phenyl-3-pyridine carboxylic acid instead of 2-chloro-4-phenyl-3-pyridine carboxylic acid in the process 2 of the Reference Example 12, the reaction and treatment were performed as in the case of the process 2 of the Reference Example 12. Then, the title compound was obtained as colorless crystals.
Melting point: 146-148°C (re-crystallization from ethyl acetate - isopropyl ether)
NMR (200MHz, $CDCl_3$) ppm:2.09 (3H, s), 3.02 (1H, dt, J=15.0, 5.6Hz), 3.25 (1H, dt, J=15.0, 5.6Hz), 3.60(2H, m), 4.57 (1H, d, J=15.2Hz), 4.79(1H, d, J=15.2Hz), 7.05-7.50(5H, m), 7.62(2H, s) 7.76(1H, s), 8.33(1H, s)

(Reference Example 25)

N-[3,5-bis(Trifluoromethyl)benzyl]-2-chloro-N-(3-hydroxypropyl)-5-methyl-4-phenyl-3-pyridine carboxamide

Using 2-chloro-5-methyl-4-phenyl-3-pyridine carboxylic acid instead of 2-chloro-4-phenyl-3-pyridine carboxylic acid in the process 2 of the Reference Example 12 and-using N-[3,5-bis(trifluoromethyl)benzyl]-N-(3-hydroxypropyl) amine obtained in the process 1 of the Reference Example 23 instead of N-[3,5-bis(trifluoromethyl)benzyl]-N-(2-hydroxyethyl)amine, the reaction and treatment were performed as in the case of the process 2 of the Reference Example 12. Then, the title compound was obtained as a pale yellow oil.
NMR (200MHz, $CDCl_3$) ppm : 1.10-1.80(2H, m), 2.06(3H×1/2, s), 2.08(3H×1/2, s), 2.80-3.30(3H, m), 3.35-3.70(1H, m), 4.08(1H ×1/2, d, J=16.4Hz), 4.39(1H×1/2, d, J=15.0Hz), 4.47(1H×1/2, d, J=16.4Hz), 4.70(1H×1/2, d, J=15.0Hz), 6.90-7.62(7H, m), 7.72(1H×1/2, s), 7.77(1H×1/2, s), 8.28(1H×1/2, s), 8.31(1H ×1/2, s)

(Reference Example 26)

(±) -N-[3,5-bis(Trifluoromethyl)benzyl]-7,8-dihydro-7-(2,3-dihydroxypropyl) -5-(4-methylphenyl)-8-oxo-6-pyrido[3,4-b] pyridinecarboxamide

Using the compound obtained in the Reference Example 3 and (±) -3-amino-1,2-propanediol, the reaction and treatment was performed as in the case of the Reference Example 13. Then the title compound was obtained as pale yellow foams.
NMR (200MHz, $CDCl_3$) ppm : 2.20(3H, s), 3.50(2H, m), 4.02-4.30 (5H, m), 4.75(1H, b), 5.35(1H, b), 6.92-7.46(6H, m), 7.55(2H, s), 7.70(1H, s), 8.63(1H, m), 8.83(1H, b)

(Reference Example 27)

N-Benzyl-8-oxo-5-phenyl-8H-pyrano-[3,4-b]pyridine-6-carboxamide

Using the compound obtained in the process 2 of the Reference Example 9 and benzyl amine, the reaction and treatment were performed as in the case of the Reference Example 3. Then, the title compound was obtained as colorless crystal.
Melting point: 188-189°C (re-crystallization from acetone - ethyl ether).
NMR (200MHz, $CDCl_3$) ppm:4.48(2H, d, J=5.4Hz), 7.2-7.4(8H, m), 7.49-7.65(5H, m), 8.95(1H, dd, J=4.4, 2.0Hz)

(Reference Example 28)

N-(3,4-Dichlorobenzyl)-8-oxo-5-phenyl-8H-pyrano[3,4-b]pyridine-6-carboxamide

Using the compound obtained in the process 2 of the Reference Example 9 and 3,4-dichloro benzyl amine, the reaction and treatment were performed as in the case of the Reference Example 3. Then, the title compound was obtained as colorless crystals.
Melting point: 198-200°C (re-crystallization from acetone - ethyl ether).

NMR (200MHz, $CDCl_3$) ppm:4.44 (2H, d, J=6.0Hz), 7.10 (1H, dd, J=8.2, 2.0Hz), 7.25-7.70(10H, m), 8.96(1H, dd, J=4.3, 1.7Hz)

(Reference Example 29)

N-[3,5-bis(Trifluoromethyl)benzyl]-2-chloro-N-[(S)-3-hydroxy-2-methylpropyl]-5-methyl-4-phenyl-3-pyridine carboxamide

3,5-bis(Trifluoromethyl)benzyl methane sulfonate was reacted with (S) -3-amino-2-methylpropanol in THF to give N-[3,5-bis(trifluoromethyl)benzyl]-N-[(S)-3-hydroxy-2-methylpropyl]amine as a colorless oil.
NMR (200MHz, $CDCl_3$) ppm:0.86(3H, d, J=6.8Hz), 1.98(1H, m), 2.63(1H, dd, J=9.4, 11.8Hz), 2.70-2.90(3H, m), 3.56 (1H, dd, J=8.6, 10.6Hz), 3.71(1H, ddd, J=1.4, 4.0, 10.6Hz), 3.87(1H, d, J=13.8Hz), 3.98(1H, d, J=13.8Hz), 7.79(3H, s)
Using 2-chloro-5-methyl-4-phenyl-3-pyridinecarboxylic acid and the above N-[3,5-bis(trifluoromethyl)benzyl]-N-[(S)-3-hydroxy-2-methylpropyl]amine instead of 2-chloro-4-phenyl-3-pyridine carboxylic acid and N-[3,5-bis(trifluoromethyl)benzyl]-N-(2-hydroxyethyl)amine in the process 2 of the Reference Example 12, respectively, the reaction and treatment were performed as in the case of the process 2 of the Reference Example 12. Then, the title compound was obtained as a colorless oil.
NMR (200MHz, $CDCl_3$) ppm : 0.60-0.82 (3H, m), 1.50-2.00 (1H,m), 2.00-2.15(3H, m), 2.15-3.92(4H, m), 4.05-4.92(2H, m), 7.00-7.85(8H, m), 8.34(1H, m)

(Reference Example 30)

N-[3,5-bis(Trifluoromethyl)benzyl]-2-chloro-N-[(R)-3-hydroxy-2-methypropyl]-5-methyl-4-phenyl-3-pyridine carboxamide

Using N-[3,5-bis(trifluoromethyl)benzyl]-N-[(R)-3-hydroxy-2-methylpropyl]amine instead of N-[3,5-bis(trifluoromethyl)benzyl]-N-[(S)-3-hydroxy-2-methylpropyl]amine in the Reference Example 29, the reaction and treatment were performed as in the case of the Reference Example 29. Then, the title compound was obtained as a colorless oil. The NMR spectra (200 MHz, $CDCl_3$) of this product were identical to the spectra of the compound obtained in the Reference Example 29.

(Reference Example 31)

N-[3,5-bis(Trifluoromethyl)benzyl]-2-chloro-N-(2-hydroxyethyl)-6-methyl-4-phenyl-3-pyridinecarboxamide

(Process 1)

A mixture of ethyl 2-chloro-6-methyl-4-phenyl-3-pyridine carboxylate (15.43 g), ethanol (70 ml) and an aqueous solution of 4N-sodium hydroxide (70 ml) was refluxed with heating for 2.5 hours. Following concentration of the reaction solution, the concentrated solution was acidified (pH 3) using hydrochloric acid, which was extracted with ethyl acetate. After the extract solution was washed with saturated brine and dried, the solvents were evaporated to give 2-chloro-6-methyl-4-phenyl-3-pyridinecarboxylic acid as colorless crystals (11.2 g).
Melting point: 191-194°C (re-crystallization from ethyl acetate - isopropyl ether)
NMR (200MHz, $CDCl_3$) ppm : 2.59(3H, s), 7.16(1H, s), 7.45(5H, s), 9.53(1H, b)

(Process 2)

Using 2-chloro-6-methyl-4-phenyl-3-pyridinecarboxylic acid obtained in the process 1 instead of 2-chloro-4-phenyl-3-pyridinecarboxylic acid in the process 2 of the Reference Example 12, the reaction and treatment were performed as in the case of the process 2 of the Reference Example 12. Then, the title compound was obtained as a pale yellow oil. NMR (200MHz, $CDCl_3$)ppm:1.95-3.80(4H, m), 2.58(3H, s), 4.15(1H X2/5, d, J=16.2Hz), 4.41(1H×2/5, d, J=16.2Hz), 4.75(1HX3/5, d, J=15.0Hz), 4.85(1HX3/5, d, J=15.0Hz), 7.15(1HX3/5, s), 7.17(1HX2/5, d, J=15.0Hz), 7.23-7.58(5H, m), 7.74(2H, s), 7.78(1H, s)

(Reference Example 32)

N-[3,5-bis(Trifluoromethyl)benzyl]-2-chloro-N-(3-hydroxypropyl)-6-methyl-4-phenyl-3-pyridine carboxamide

Using 2-chloro-6-methyl-4-phenyl-3-pyridinecarboxylic acid and N-[3,5-bis(trifluoromethyl)benzyl]-N-(3-hydroxypropyl)amine instead of 2-chloro-4-phenyl-3-pyridinecarboxylic acid and N-[3,5-bis(trifluoromethyl)benzyl]-N-(2-hydroxyethyl)amine in the process 2 of the Reference Example 12, respectively, the reaction and treatment were performed as in the case of the process 2 of the Reference Example 12. Then, the title compound was obtained as a pale yellow oil.
NMR (200MHz, $CDCl_3$) ppm : 1.15-1.65 (2H, m), 2.59(3H, s), 2.75-3.20(2H, m), 4.06(1HX2/5, d, J=15.4Hz), 4.31 (1H×2/5, d, J=15.4Hz), 4.65(1HX3/5, d, J=15.2Hz), 4.74(1HX3/5, d, J=15.2Hz), 7.16(1H, s), 7.20-7.60 (5H,m), 7.72 (2H, s), 7.78(1H, s)

(Reference Example 33)

N-[3,5-bis(Trifluoromethyl)benzyl]-N-(2-hydroxyethyl)-5-methyl-2-methylaminocarbonyl-4-phenyl-3-pyridine carboxamide

(Process 1)

A mixture of 5-methyl-4-phenyl-2,3-pyridine dicarboxylic acid diethyl ester [13.0 g, melting point; 73-74°C (re-crystallization from ethyl ether - isopropyl ether)] prepared according to the method described in Japanese Patent Laid-Open No. 62-106081, potassium hydroxide (20 g) and an aqueous solution of 70 % ethanol (200 ml) was refluxed with heating for 3 hours. Following evaporation of the solvents, the residue was diluted with water, and washed with ethyl ether. The aqueous layer was acidified at pH 2 to 3 using 2N-hydrochloric acid, and extracted with ethyl acetate. After the extract solution was washed with saturated brine and dried, the solvents were evaporated to give 5-methyl-4-phenyl-2,3-pyridine dicarboxylic acid as pale yellow crystals (3.06 g).
Melting point; 187-188°C (re-crystallization from THF - ethyl ether)
NMR (200MHz, DMSO-$d_6$) ppm:2.10(3H, s), 7.20-7.30 (2H, m), 7.40-7.55(3H, m), 8.65(1H, s)

(Process 2)

The compound (2.9 g) obtained in the process 1 was refluxed with heating in acetic acid anhydride (50 ml) for 2 hours. After evaporation of the solvent, ethanol (50 ml) was added to the residue and stirred at room temperature for 4 hours. After evaporation of the solvent, the residue was dissolved in ethyl acetate. After this solution was washed with saturated brine and dried, the solvents were evaporated to give a mixture (about 3 : 2) of 2-ethyl ester of and 3-ethyl ester of 5-methyl-4-phenyl-2,3-pyridine dicarboxylic acid as a pale yellow oil (3.39 g).

(Process 3)

To a solution of the oil (1.94 g) obtained in the process 2 in THF (30 ml) was added DMF (three drops) and oxalyl chloride (2.0 ml), and stirred at room temperature for 30 min. After evaporation of the solvents, the residue was dissolved in THF (40 ml). N-[3,5-bis(trifluoromethyl)benzyl]-N-(2-hydroxyethyl)amine (2.2 g) and triethyl amine (2.0 ml) were added to this solution and stirred at room temperature for 16 hours. Following dilution with ethyl acetate, the reaction mixture was washed subsequently with water, diluted hydrochloric acid, aqueous solution of potassium carbonate and saturated brine and dried. After evaporation of the solvents, the residue was purified by column chromatography using silica gel (hexane-ethyl acetate =1 : 2) to give N-[3,5-bis (trifluoromethyl)benzyl]-2-ethoxycarbonyl-N-(2-hydroxyethyl)-5-methyl-4-phenyl-3-pyridinecarboxamide as colorless crystals (1.31 g).
Melting point: 138-139°C (re-crystallization from ethyl acetate - isopropyl ether)
NMR (200MHz, $CDCl_3$) ppm : 1.45(3H×1/4, t, J=7.1Hz), 1.46(3H× 3/4, t, J=7.1Hz), 2.18(3H×3/4, s), 2.19(3H×1/4, s), 2.82(1H, m), 3.2-3.7(3H, in), 4.15-4.62(4H, m), 7.05-7.80(8H, m), 8.65(1H ×3/4, s), 8.68 (1H×1/4, s)

(Process 4)

A solution of 40% methylamine - methanol (15 ml) was added to a solution the compound (377 mg) obtained in the process 3 in THF (5 ml), and stirred at room temperature for 16 hours. The solvents were evaporated to give the title compound as a pale yellow oil (370 mg).
NMR (200MHz, $CDCl_3$)ppm : 2.12 (3H×2/3, s), 2.14(3H×1/3, s), 2.83(1H, m), 3.03(3H×1/3, d, J=5.2Hz), 3.04(3H×2/3,

d, J=4.8Hz), 3.25-3.80(3H, m), 4.30(1H×2/3, d, J=15Hz), 4.36(1H ×1/3, d, J=15Hz), 4.59(1H×1/3, d, J=15Hz), 4.86 (1H×2/3, d, J=15Hz), 7.0-7.9(8H, m), 8.02(1H×2/3, bd), 8.17(1H×1/3, bd), 8.46(1H, s)

(Reference Example 34)

N-[3,5-bis(Trifluoromethyl) benzyl]-N-(2-hydroxyethyl)-2-methylaminocarbonyl-4-phenyl-3-pyridinecarboxamide

(Process 1)

Using 4-phenyl-2,3-pyridine dicarboxylic acid diethyl ester [Japanese Patent Laid-Open No. 62-106081] instead of 5-methyl-4-phenyl-2,3-pyrideine dicarboxylic acid diethyl ester in the process 1 of the Reference Example 33, the reaction and treatment were performed as in the case of the process 1 of the Reference Example 33. Then, 4-phenyl-2,3-pyridine carboxylic acid was obtained as a pale yellow crystal.
Melting point; 146-148°C (re-crystallization from THF - isopropyl ether)
NMR (200MHz, $CDCl_3$+DMSO-$d_6$) ppm: 7.3-7.6(6H, m), 8.69 (1H, d, J=5.0Hz)

(Process 2)

Using the compound obtained in the process 1, the reaction and treatment were performed as in the case of the process 2 of the Reference Example 33 to yield a mixture of 2-ethyl ester of and 3-ethyl ester (about 3 : 2) of 4-phenyl-2,3-pyridine carboxylic acid as a pale brown oil.

(Process 3)

Using the compound obtained in the process 2, the reaction and treatment were performed as in the case of the process 3 of the Reference Example 33 to give N-[3,5-bis(trifluoromethyl)benzyl]-2-ethoxycarbonyl-N- (2-hydroxyethyl)-4-phenyl-3-pyridinecarboxamide as a pale yellow oil.
NMR (200MHz, $CDCl_3$) ppm : 1.48 (3H, t, J=7.1Hz), 2.71(1H, m), 3.1-3.7(3H, m), 4.1-4.9(4H, m), 7.18-7.52(6H, m), 7.65-7.82(3H, m), 8.78(1H×3/4, d, J=4.8Hz), 8.80(1H×1/4, d, J=4.8Hz)

(Process 4)

Using the compound obtained in the process 3, the reaction and treatment were performed as in the case of the process 4 of the Reference Example 33 to yield the title compound as a pale yellow oil.
NMR (200MHz, $CDCl_3$) ppm : 2.73(1H, m), 3.05 (3H×1/3, d, J=5.0Hz), 3.06(3H×2/3, d, J=5.0Hz), 3.1-3.9(3H, m), 4.29 (1H×1/3, d, J=16Hz), 4.52(1H×2/3, d, J=15Hz), 4.54(1H×1/3, d, J=16Hz), 4.93(1H×2/3, d, J=15Hz), 7.0-7.9(9H, m), 7.95(1H×2/3, bd), 8.19(1H×1/3, bd), 8.59(1H, d, J=5.2Hz)

(Reference 35)

N-Benzyl-2-ethoxycarbonyl-N-(2-hydroxyethyl)-5-methyl-4-phenyl-3-pyridinecarboxamide

Using the oil obtained in the process 2 of the Reference Example 33 and N-benzyl-N-(2-hydroxyethyl) amine, the reaction and treatment were performed as in the case of the process 3 of the Reference Example 33. Then, the title compound was obtained as a pale yellow oil.
NMR (200MHz, $CDCl_3$) ppm : 1.44 (3H×1/4, t, J=7.2Hz), 1.46(3H× 3/4, t, J=7.1Hz), 2.15(3H×3/4, s), 2.19(3H×1/4, s), 2.6-3.7(4H, m), 3.96(1H×3/4, d, J=15Hz), 4.00(1H×1/4, d, J=15Hz), 4.4-4.6(2H+1H×1/4, m), 5.37(1H×3/4, d, J=15Hz), 6.48(2H× 3/4, m), 6.82(2H×1/4, m) 7.0-7.6(8H, m), 8.65(1H×3/4, s), 8.66(1H×1/4, s)

(Reference Example 36)

N-[3,5-bis(Trifluoromethyl)benzyl]-N-(3-hydroxypropyl)-5-methyl-2-methylamino carbonyl-4-phenyl-3-pyridinecarboxamide

(Process 1)

Using the oil obtained in the process 2 of the Reference Example 33 and N-[3,5-bis(trifluoromethyl)benzyl]-N-(3-hydroxypropyl)amine, the reaction and treatment were performed as in the case of the process 3 of the Reference

Example 33. Then, N-[3,5-bis(trifluoromethyl)benzyl]-2-ethoxycarbonyl-N-(3-hydroxypropyl)-5-methyl-4-phenyl-3-pyridine carboxamide was obtained as a pale yellow oil.
NMR (200MHz, $CDCl_3$)ppm : 1.44(3H×1/4, t, J=7.1Hz), 1.45(3H× 3/4, t, J=7.1Hz), 1.60(2H, m), 2.17(3H×3/4, s), 2.18 (3H×1/4, s), 2.7-3.7(4H, m), 3.96(1H×1/4, d, J=16Hz), 4.35-4.60(3H+ 1H×3/4, m), 7.10-7.80(8H, m), 8.64(1H×3/4, s), 8.68(1H×1/4, s)

(Process 2)

Using the compound obtained in the process 1, the reaction and treatment were performed as in the case of the process 4 of the Reference Example 33 to yield the title compound as a pale yellow oil.
NMR (200MHz, $CDCl_3$) ppm : 1.3-1.9(2H, m), 2.11(3H×3/5, s), 2.14(3H×2/5, s), 2.7-3.8(4H, m), 3.02(3H×3/5, d, J=5.2Hz), 3.03(3H×2/5, d, J=5.2Hz), 4.04(1H×2/5, d, J=16Hz), 4.28(1H ×3/5, d, J=15Hz), 4.46(1H×2/5, d, J=16Hz), 4.82(1H×3/5, d, J=15Hz), 7.0-7.6(5H, m), 7.63(2H×3/5, s), 7.67(2H×2/5, s), 7.73(1H, s), 7.96(1H×3/5, bd), 8.06 (1H×2/5, bd), 8.45(1H× 3/5, s), 8.48(1H×2/5, s)

The compounds in the Reference Examples from 37 to 45 were obtained as each pale yellow oil by reaction and treatment substantially similar to the process 2 of the Reference Example 12, using 2-chloro-4-phenyl-3-pyridine carboxylic acid and N-substituted-N-(substituted)benzylamine{N-(2-hydroxyethyl)-N-(3,4,5-trimethoxybenzyl)amine, N-(3,4-dichlorobenzyl) -N- (2-hydroxyethyl) amine, N- (3,4-dimethoxybenzyl) -N-(2-hydroxyethyl) amine, N-benzyl-N-(2-hydroxyethyl)amine, N-(2-hydroxypropyl)-N-(3,4,5-trimethoxybenzyl)amine, N-benzyl-N-[(S)-3-hydroxy-2-methylpropyl]amine, N-benzyl-N-[(R)-3-hydroxy-2-methylpropyl]amine, N-[3,5-(bis-trifluoromethyl)benzyl]-N-[(S)-3-hydroxy-2-methylpropyl]amine and N-[3,5-(bis-trifluoromethyl)benzyl]-N-[(R)-3-hydroxy-2-methylpropyl] amine, respectively}. Their physicochemical data are described below.

(Reference Example 37)

2-Chloro-N- (2-hydroxyethyl)-4-phenyl-N-(3,4,5-trimethoxy benzyl)-3-pyridinecarboxamide

NMR (200MHz, $CDCl_3$)ppm : 2.05-2.50(2H, m), 2.80-4.00(12H, m), 4.00-4.40(1H×3/2, m), 4.93(1H×1/2, d, J=14.2Hz), 6.22(2H× 1/2, s), 6.55(2H×1/2, s), 7.25-7.70(6H, m), 8.42(1H×1/2, d, J=6.2Hz), 8.48(1H×1/2, d, J=5.8Hz) (Mixture of amide rotational isomers of 1:1)

(Reference Example 38)

2-Chloro-N-(3,4-dichlorobenzyl) -N-(2-hydroxyethyl)-4-phenyl-3-pyridinecarboxamide

NMR (200MHz, $CDCl_3$)ppm : 1.80-3.85(5H, m), 3.96(1H×4/9, d, J=16.0Hz), 4.24(1H×4/9, d, J=16.0Hz), 4.44 (1H×5/9, d, J=15.2Hz), 4.92(1H×5/9, d, J=15.2Hz), 6.50-6.85(2H, m), 7.10-7.70(7H, m), 8.46(1H, m)
(Mixture of amide rotational isomers of 5:4)

(Reference Example 39)

2-Chloro-N-(3,4-dimethoxybenzyl) -N-(2-hydroxyethyl)-4-phenyl-3-pyridine carboxamide

NMR (200MHz, $CDCl_3$)ppm : 2.70-4.30(12H, m) , 4.53(1HX1/2, d, J=14.8Hz), 4.74 (1H×1/2, d, J=14.8Hz), 6.30-7.00 (3H, m), 7.20-7.65(6H, m), 8.39(1H×1/2, d, J=5.0Hz), 8.46(1HX1/2, d, J=5.2Hz)
(Mixture of amide rotational isomers of 1:1)

(Reference Example 40)

N-Benzyl-2-chloro-N-(2-hydroxyethyl)-4-phenyl-3-pyridine carboxamide

NMR (200MHz, $CDCl_3$)ppm : 2.27(1H×1/2, b), 2.60(1H×1/2, b), 2.75-3.15(1H, m), 3.25-3.65(3H, m), 3.90 (1H×1/2, d, J=15.4Hz), 4.26(1H×1/2, d, J=15.4Hz), 4.49(1H×1/2, d, J=15.0Hz), 4.95(1H X 1/2, d, J=15.0Hz), 6.74 (2H×1/2, m), 6.92(2H×1/2, m), 7.10-7.65(9H, m), 8.42(1H, m)
(Mixture of amide rotational isomers of 1:1)

(Reference Example 41)

2-Chloro-N-(2-hydroxypropyl)-4-phenyl-N-(3,4,5-trimethoxybenzyl)-3-pyridine carboxamide

NMR (200MHz, $CDCl_3$)ppm : 1.10-2.30(3H, m), 2.70-4.30(14H+1H ×3/7, m), 4.88(1H×4/7, d, J=14.8Hz), 6.18(2H×4/7, s), 6.52(2H ×3/7, s), 7.20-7.60(6H, m), 8.47(1H, m)
(Mixture of amide rotational isomers of 4:3)

(Reference Example 42)

N-Benzyl-2-chloro-N-[(S)-3-hydroxy-2-methylpropyl]-4-phenyl-3-pyridine carboxamide

NMR (200MHz, $CDCl_3$) ppm : 0.50-0.85 (3H, m), 1.40-1.85(1H, m), 2.20-3.75(5H,m), 3.80-5.15(2H, m), 6.60-7.65(11H, m), 8.42(1H, m)
(Mixture of amide rotational isomers of 2:1)

(Reference Example 43)

N-Benzyl-2-chloro-N-[(R)-3-hydroxy-2-methylpropyl]-4-phenyl-3-pyridine carboxamide

NMR (200MHz, $CDCl_3$) ppm: Identical to the spectra of the compound of the Reference Example 42.

(Reference Example 44)

N-[3,5-(bis Trifluoromethyl)benzyl]-2-chloro-N-[(S)-3-hydroxy-2-methylpropyl]-4-phenyl-3-pyridine carboxamide

NMR (200MHz, $CDCl_3$)ppm : 0.53(3H×1/4, d, J=7.0Hz), 0.63(3H× 1/4, d, J=7.0Hz), 0.75(3H×1/4, d, J=6.8Hz), 0.81(3H×1/4, d, J=6.8Hz), 1.50-1.90 (1H, m), 2.42-3.80 (5H, m), 4.00-4.95(2H, m), 7.10-7.90(9H, m), 8.42 (1H, m)
(Mixture of amide rotational isomers of 1:1)

(Reference Example 45)

N-[3,5-(bis-Trifluoromethyl)benzyl]-2-chloro-N-[(R)-3-hydroxy-2-methylpropyl]-4-phenyl-3-pyridine carboxamide

NMR (200MHz, $CDCl_3$) ppm: Identical to the spectra of the compound of the Reference Example 44.

(Reference Example 46)

N-Benzyl-7,8-dihydro-7-(4-hydroxybutyl)-5-(4-methylphenyl)-8-oxo-6-pyrido[3,4-b]pyridine carboxamide

(Process 1)

Using the compound obtained in the process 2 of the Reference Example 2 and benzyl amine, the reaction and treatment substantially similar to the case of the process 4 of tie Reference Example 2 were performed. Then, N-benzyl-5-(4-methylphenyl)-8-oxo-8H-pyrano[3,4-b]pyridine-6-carboxamide was obtained as colorless crystals.
Melting point; 208-209°C (re-crystallization from acetone - isopropyl ether)
NMR (200MHz, $CDCl_3$)ppm:2.45(3H, s), 4.48(2H, d, J=5.6Hz), 7.10-7.40(10H, m), 7.58(2H, m), 8.94(1H, dd, J=3.6, 2.2Hz)

(Process 2)

Using the compound obtained in the process 1 and 4-amino-1-butanol, the reaction and treatment substantially similar to the case of the Reference Example 13 were performed. Then the title compound was obtained as colorless crystals.
Melting point; 205-207°C (re-crystallization from acetone - isopropyl ether)
NMR (200MHz, $CDCl_3$) ppm : 1.48(2H, m), 1.83(2H, m), 2.45(3H, s), 2.86(1H, b), 3.57(2H, t, J=5.9Hz), 3.85(2H, m), 4.34(2H, d, J=6.0Hz), 6.8-7.1(2H, m), 7.10-7.35(8H, m), 7.50(1H, m), 7.55(1H, dd, J=8.4, 1.4Hz), 8.60(1H, dd, J=4.0,

1.4Hz)

(Reference Example 47)

(R)-N-Benzyl-7,8-dihydro-7-(4-hydroxy-3-methylbutyl)-5-(4-methylphenyl)-8-oxo-6-pyrido[3,4-b]pyridinecarboxamide

Using the compound obtained in the process 1 of the Reference Example 46 and THP ether of (R)-4-amino-2-methyl-1-butanol, the reaction and treatment substantially similar to the case of the Reference Example 19 were performed. Then, the title compound was obtained as colorless crystals.
Melting point; 226-227°C (re-crystallization from acetone - ethyl ether)
NMR (200MHz, $CDCl_3$)ppm : 0.81 (3H, d, J=6.6Hz), 1.5-2.0 (3H, m), 2.44(3H, s), 3.20-3.55(3H, m), 3.93(2H, m), 4.31 (2H, d, J=5.4Hz), 6.75-6.90(2H, m), 7.1-7.3(8H, m), 7.39(1H, dd, J=8.2, 4.2Hz), 7.61(1H, d, J=8.2Hz), 8.68(1H, d, J=4.2Hz)

(Reference Example 48)

(S)-N-Benzyl-7,8-dihydro-7-(4-hydroxy-3-methylbutyl)-5-(4-methylphenyl)-8-oxo-6-pyrido[3,4-b]pyridine carboxamide

Using the compound obtained in the process 1 of the Reference Example 46 and THP ether of (S)-4-amino-2- methyl-1-butanol, the reaction and treatment substantially similar to the case of the Reference Example 19 were performed. Then, the title compound was obtained as colorless crystals.
Melting point; 226-227°C (re-crystallization from acetone - ethyl ether)
NMR (200MHz, $CDCl_3$) ppm: Identical to the spectra of the compound of the Reference Example 47.

(Reference Example 49)

(R)-7,8-Dihydro-7-(4-hydroxy-3-methylbutyl)-5-(4-methylphenyl)-8-oxo-N-(3,4,5-trimethoxy benzyl)-6-pyrido[3,4-b] pyridine carboxamide

(Process 1)

Using the compound obtained in the process 2 of the Reference Example 2 and 3,4,5-trimethoxybenzylamine, the reaction and treatment substantially similar to the case of the process 4 of the Reference Example 2 were performed. Then, 5-(4-methylphenyl)-8-oxo-N-(3,4,5-trimethoxybenzyl)-8H-pyrano[3,4-b]pyridine-6-carboxamide was obtained as colorless crystals.
Melting point; 195-196°C (re-crystallization from acetone - isopropyl ether)
NMR (200MHz, $CDCl_3$)ppm:2.45(3H, s), 3.84(3H, s), 3.85(6H, s), 4.40(2H, d, J=5.8Hz), 6.50(2H, s), 7.17(2H, d, J=8.0Hz), 7.27(1H, b), 7.32(2H, d, J=8.0Hz), 7.58(2H, m), 8.94(1H, dd, J=4.0, 2.2Hz)

(Process 2)

Using the compound obtained in the process 1 and THP ether of (R)-4-amino-2-methyl-1-butanol, the reaction and treatment substantially similar to the case of the Reference Example 19 were performed. Then, the title compound was obtained as colorless crystals.
Melting point; 194-195°C (re-crystallization from acetone - ethyl ether)
NMR (200MHz, $CDCl_3$)ppm : 0.84(3H, d, J=6.8Hz), 1.5-2.0(3H, m), 2.38(3H, s), 3.2-3.6(3H, m), 3.65-3.95(2H, m), 3.80 (6H, s), 3.82(3H, s), 4.23(2H, d, J=6.0Hz), 6.40(2H, s), 7.05-7.40 (4H, m), 7.32(1H, dd, J=8.2, 4.2Hz), 7.56(1H, dd, J=8.2, 1.6Hz), 7.80(1H, m), 8.63(1H, dd, J=4.2, 1.6Hz)

(Reference Example 50)

(S)-7,8-Dihydro-7-(4-hydroxy-3-methylbutyl)-5-(4-methylphenyl)-8-oxo-N-(3,4,5-trimethoxybenzyl)-6-pyrido[3,4-b] pyridine carboxamide

Using the compound obtained in the process 1 of the Reference Example 49 and THP ether of (S)-4-amino-2-methyl-1-butanol, the reaction and treatment substantially similar to the case of the Reference Example 19 were performed. Then, the title compound was obtained as colorless crystals.
Melting point; 194-195°C (re-crystallization from acetone - ethyl ether)

NMR (200MHz, $CDCl_3$) ppm: Identical to the spectra of the compound of the Reference Example 49.

(Reference Example 51)

(R)-N-(3,5-Dimethoxybenzyl)-7,8-dihydro-7-(4-hydroxy-3-methylbutyl) -5-(4-methylphenyl)-8-oxo-6-pyrido[3,4-b] pyridine carboxamide

(Process 1)

Using the compound obtained in the process 2 of the Reference Example 2 and 3,5-dimethoxy benzyl amine, the reaction and treatment substantially similar to the case of the process 4 of the Reference Example 2 were performed. Then, N-(3,5-dimethoxybenzyl)-5-(4-methylphenyl)-8-oxo-8H-pyrano[3, 4-b]pyridine-6-carboxamide was obtained as colorless crystals.
Melting point; 154-155°C (re-crystallization from ethyl acetate - isopropyl ether)
NMR (200MHz, $CDCl_3$) ppm : 2.45 (3H, s), 3.78(6H, s), 4.41(2H, d, J=5.4Hz), 6.41(3H, m), 7.17 (2H, d, J=8.0Hz), 7.23 (1H, b), 7.33 (2H, d, J=8.0Hz), 7.58(2H, m), 8.94(1H, dd, J=4.0, 2.2Hz)

(Process 2)

Using the compound obtained in the process 1 and THP ether of (R)-4-amino-2-methyl-1-butanol, the reaction and treatment substantially similar to the case of the Reference Example 19 were performed. Then, the title compound was obtained as colorless crystals.
Melting point; 169-172°C (re-crystallization from acetone - isopropyl ether)
NMR (200MHz, $CDCl_3$)ppm : 0. 85(3H, d, J=6.8Hz), 1.62(1H, m), 1.79(2H, m), 2.40(3H, s), 3.11(1H, b), 3.25-3.60(2H, m), 3.76(6H, s), 3.86(2H, m), 4.23(2H, d, J=5.6Hz), 6.25(2H, d, J=2.2Hz), 6.35(1H, t, J=2.2Hz), 7.15-7.35(4H, m), 7.30 (1H, dd, J=8.4, 4.2Hz), 7.44(1H, m), 7.56(1H, dd, J=8.4, 1.6Hz), 8.65(1H, dd, J=4.2, 1.6Hz)
The compounds in the Reference Examples from 52 to 54 and 144 were obtained as each pale yellow oil by the reaction and treatment substantially similar to the case of the process 2 of the Reference Example 12, using 2-chloro-4-(4-methylphenyl)-3-pyridinecarboxylic acid [prepared by following condensation from 2-cyano-3-methyl-3-(4-methylphenyl)propenoic acid ethyl ester with N,N-dimethyl acetamide dimethyl acetal, being cyclized using hydrogen chloride and alkali-hydrolyzing ester group : melting point; 205 to 208°C (decomposition)] and N-substituted-N-(substituted)benzylamine, N-benzyl-N-(2-hydroxyethyl)amine, N-[3,5-bis(trifluoromethyl)benzyl]-N-(2-hydroxyethyl) amine, N-benzyl-N-[(S)-3-hydroxy-2-methylpropyl]amine and N-[3,5-bis(trifluoromethyl)benzyl]-N-[(S)-3-hydroxy-2-methylpropyl]amine, respectively}. Their physicochemical data are described below.

(Reference Example 52)

N-Benzyl-2-chloro-N-(2-hydroxyethyl)-4-(4-methylphenyl)-3-pyridinecarboxamide

NMR (200MHz, $CDCl_3$) ppm : 2.43 (3H×1/2, s), 2.46(3H×1/2, s), 2.70-3.80(total 4H, m), 3.90(1H×1/2, d, J=15.4Hz), 4.24(1H ×1/2, d, J=15.4Hz), 4.51 (1H×1/2, d, H=15.2Hz), 4.94(1H×1/2, d, J=15.2Hz), 6.74(1H, m), 6.97 (1H, m), 7.10-7.55(8H, m), 8.40(1H, m)
(Mixture of amide rotational isomers of 1:1)

(Reference Example 53)

N-[3,5-bis(Trifluoromethyl)benzyl]-2-chloro-N-(2-hydroxyethyl)-4-(4-methylphenyl)-3-pyridine carboxamide

NMR (200MHz, $CDCl_3$) ppm : 2.36 (3H×7/11, s), 2.44 (3H×4/11, s), 2.80-3.80(total 4H, m), 4.16(1H×4/11, d, J=16.2Hz), 4.41(1H ×4/11, d, J=16.2Hz), 4.77(1H×7/11, d, H=15.0Hz), 4.90(1HX 7/11, d, J=15.0Hz), 7.10-7.50(6H, m), 7.76(2H, m), 8.42(1H, m)
(Mixture of amide rotational isomers of 7:4)

(Reference Example 54)

N-Benzyl-2-chloro-N-[(S)-3-hydroxy-2-methylpropyl]-4-(4-methylphenyl)-3-pyridine carboxamide

NMR (200MHz, $CDCl_3$)ppm : 0.59(3H×1/4, d, J=7.0Hz), 0.66(3H×1/4, d, J=7.0Hz), 0.77(3H×1/4, d,

J=3.8Hz), 0.80(3H×1/4, d, J=3.8Hz), 1.40-1.90(1H, m), 2.30-2.50(3H, m), 2.50-3.80(total 5H, m), 3.80-4.42(2H×3/4, m), 5.05(2H×1/4, m), 6.60-7.50(total 10H, m), 8.40(1H, m)
(Mixture of amide rotational isomers of 1:1)

(Reference Example 55)

7-[3,5-bis(trifluoromethyl)benzyl]-6,7,8,9-tetrahydro-5-(4-methylphenyl)-6,11-dioxo-11H-pyradino[2,1-g] [1,7] naphthylidine

A mixture of the compound (200 mg) obtained in the Reference Example 1, triethyl amine (0.20 ml), methane sulfonyl chloride (0.10 ml) and dichloromethane (10 ml) was stirred at 0°C for 2 hours. Ethyl acetate was added to the reaction mixture, which was washed with water, and dried. Then, the solvents were evaporated to give N-[3,5-bis (trifluoromethyl)benzyl]-7,8-dihydro-7-(2-methanesulfonyloxyethyl) -5-(4-methylphenyl)-8-oxo-6-pyrido[3,4-b]pyridine carboxamide. After this compound was dissolved in DMF (5 ml), sodium hydride (60 % oil) (30mg) was added, and stirred at room temperature for 1.5 hours. The reaction mixture was diluted with ethyl acetate, washed sequentially with water, diluted hydrochloric acid and water, dried, and then the solvents were evaporated to give the title compound as colorless crystals (109 mg).
Melting point; 270-271°C (re-crystallization from ethyl acetate - ethyl ether)
NMR (200MHz, $CDCl_3$) ppm : 2.46(3H, s), 3.67(2H, t like, J=5.4Hz), 4.51(2H, t like, J=5.4Hz), 4.81(2H, s), 7.13(2H, d, J=8.1Hz), 7.33(2H, d, J=8.1Hz), 7.52(1H, dd, J=8.4, 4.4Hz), 7.64(1H, dd, J=8.4, 1.6Hz), 7.70(2H, s), 7.84(1H, s), 8.97(1H, dd, J=4.4, 1.6Hz)
Anal. Calcd for $C_{27}H_{19}N_3O_2F_6$
Calculated (%): C 61.02, H 3.60, N 7.91
Found (%): C 61.07, H 3.50, N 7.85

(Reference Example 56)

7-[3,5-bis(Trifluoromethyl)benzyl]-6,7,8,9,10,12-hexahydro-5-(4-methylphenyl)-6,12-dioxo[1,4]diazepino[2,1-g] [1,7] naphthylidine

A mixture of N-[3,5-bis(trifluoromethyl)benzyl]-7-(3-chloropropyl)-7,8-dihydro-5-(4-methylphenyl)-8-oxo-6-pyrido[3,4-b]pyridine carboxamide (66 mg) obtained the Reference Example 2, sodium hydride (60 % oil) (84 mg) and THF (3 ml) was stirred at room temperature for 14 hours. Following addition of 2N-HCl to the reaction mixture, it was alkalized using an aqueous solution of potassium carbonate, and then extracted with ethyl acetate. The extract solution was washed with water, dried, and the solvents were evaporated to give the title compound as colorless crystals (35 mg).
Melting point; 194-195°C (re-crystallization from ethyl acetate - isopropyl ether)
NMR (200MHz, $CDCl_3$)ppm : 2.16(2H, m), 2.42(3H, s), 3.25-3.70(3H, m), 4.12(1H, d, J=15Hz), 5.34(1H, d, J=15Hz), 5.52(1H, m), 6.93(1H, d, J=8.2Hz), 7.20(1H, d, J=8.2Hz), 7.30-7.45(2H, m), 7.51(1H, dd, J=8.4, 4.4Hz), 7.62(2H, s), 7.70(1H, dd, J=8.4, 1.6Hz), 7.84(1H, s), 8.93(1H, dd, J=4.4, 1.6Hz)
Anal. Calcd for $C_{28}H_{21}N_3O_2F_6$
Calculated (%): C 61.65, H 3.88, N 7.70
Found (%): C 61.29, H 4.06, N 7.61

(Reference Example 57)

7-[3,5-bis(Trifluoromethyl)benzyl]-6,7,8,9,10,11,-hexahydro-5-(4-methylphenyl)-6,13-dioxo-13H-[1,4]diazocino[2,1-g] [1,7]naphthylidine

Using the compound obtained in the Reference Example 5, the reaction and treatment were performed as in the case of the Reference Example 55. Then, the title compound was obtained as colorless crystals.
Melting point; 192-193°C (re-crystallization from ethyl acetate - isopropyl ether)
NMR (200MHz, $CDCl_3$) ppm : 1.7-2.5 (4H, m), 2.37(3H, s), 3.25(1H, m), 3.40-3.72(2H, m), 4.01(1H, d, J=15Hz), 5.13 (1H, dd, J=14, 5.4Hz), 5.46(1H, d, J=15Hz), 6.85(1H, d, J=7.9Hz), 7.05(1H, d, J=7.9Hz), 7.26(1H, d, J=7.8Hz), 7.34 (1H, d, J=7.8Hz), 7.42-7.60(2H, m), 7.47(2H, s), 7.81(1H, s), 8.92(1H, m)

(Reference Example 58)

6,7,8,9,10,12-Hexahydro-7-(2-methoxybenzyl)-5-(4-methylphenyl)-6,12-dioxo[1,4]diazepino[2,1-g][1,7]naphthylidine

Using the compound obtained in the Reference Example 6, the reaction and treatment were performed as in the case of the Reference Example 55. Then, the title compound was obtained as colorless crystals.
Melting point; 264-266°C (re-crystallization from ethyl acetate - ethyl ether)
NMR (200MHz, $CDCl_3$) ppm : 1. 7-2.1 (2H, m), 2.43(3H, s), 3.25-3.52(3H, m), 3.84(3H, 5), 4.67(2H, s), 5.39(1H, dd, J=14, 5.8Hz), 6.85-7.00(3H, m), 7.10-7.22(2H, m), 7.22-7.44(3H, m), 7.48(1H, dd, J=8.4, 4.4Hz), 7.72(1H, dd, J=8.4, 1.4Hz), 8.90(1H, dd, J=4.4, 1.4Hz)

(Reference 59)

6,7,8,9,10,11-Hexahydro-7-(2-methoxybenzyl)-5-(4-methylphenyl)-6,13-dioxo-13H-[1,4]diazocino[2,1-g] [1,7] naphthylidine

Using the compound obtained in the Reference Example 7, the reaction and treatment were performed as in the case of the Reference Example 55. Then, the title compound was obtained as colorless crystals.
Melting point; 235-236°C (re-crystallization from ethyl acetate)
NMR (200MHz, $CDCl_3$) ppm:1.6-2.3(4H, m), 2.46(3H, s), 3.15-3.30(1H, m), 3.38-3.65(2H, m), 3.80(3H,s), 4.24(1H, d, J=15Hz), 5.04(1H, d, J=15Hz), 5.13(1H, dd, J=15, 6.4Hz), 6.25(1H, dd, J=7.6, 1.4Hz), 6.63(1H, dt, $J_d$=0.5Hz, $J_t$=7.6Hz), 6.82(1H, d, J=7.4Hz), 6.96(1H, dd, J=7.6, 2.0Hz), 7.11-7.34(3H, m), 7.38-7.47(1H, m), 7.47(1H, dd, J=8.3, 4.3Hz), 7.62(1H, dd, J=8.3, 1.7Hz), 8.90(1H, dd, J=4.3, 1.7Hz)

(Reference Example 60)

7-[3,5-bis(Trifluoromethyl)benzyl]-6,7,8,9,10,11,12,14-octahydro-5-(4-methylphenyl)-6,14-dioxo[1,4]diazonino[2,1-g] [1,7]naphthylidine

Using the compound obtained in the Reference Example 8, the reaction and treatment were performed as in the case of the Reference Example 55. Then, the title compound was obtained as colorless crystals.
Melting point; 177-179°C (re-crystallization from ethyl acetate - isopropyl ether)
NMR (200MHz, $CDCl_3$) ppm:1.45-1.95(4H, m), 2.10(2H, m), 2.33(3H, s), 3.06-3.24(1H, m), 3.32-3.56(2H, m), 3.86 (1H, d, J=15Hz), 4.95(1H, dt, Jd=15Hz, Jt=4.8Hz), 5.38(1H, d, J=15Hz), 6.86(1H, dd, J=8.0, 1.5Hz), 7.00(1H, d, J=8.0Hz), 7.17(1H, d, J=8.2Hz), 7.29(1H, dd, J=8.2, 1.5Hz), 7.40-7.54(2H, m), 7.44(2H, s), 7.79(1H, s), 8.89(1H, dd, J=3.8, 2.0Hz)

(Reference Example 61)

7-[3,5-bis(Trifluoromethyl)benzyl]-6,7,8,9,10,12-hexahydro-6,12-dioxo-5-phenyl[1,4]diazepino[2, 1-g][1,7] naphthylidine

Using the compound obtained in the Reference Example 9, the reaction and treatment were performed as in the case of the Reference Example 55. Then, the title compound was obtained as colorless crystals.
Melting point; 244-245°C (re-crystallization from ethyl acetate - THF - ethyl ether)
NMR (200MHz, $CDCl_3$) ppm : 2.00-2.25(2H, m), 3.25-3.70(3H, m), 4.15(1H, d, J=15Hz), 5.30(1H, d, J=15Hz), 5.52 (1H, m), 7.05(1H, d, J=7.4Hz), 7.3-7.7(6H,m), 7.62(2H, s), 7.84(1H, s), 8.93(1H, dd, J=4.2, 1.6Hz)

(Reference Example 62)

7-[3,5-bis(Trifluoromethyl)benzyl]-6,7,8,9,10,11-hexahydro-6,13-dioxo-5-phenyl-13H-[1,4]diazocino[2,1-g][1,7] naphthylidine

Using the compound obtained in the Reference Example 10, the reaction and treatment were performed as in the case of the Reference Example 55. Then, the title compound was obtained as colorless crystals.
Melting point; 205-206°C (re-crystallization from ethyl acetate - ethyl ether)
NMR (200MHz, $CDCl_3$) ppm:1.70-2.35(4H, in), 3.18-3.36(1H, m), 3.4-3.7(2H, m), 3.98(1H, d, J=15Hz), 5.14(1H, dd, J=14, 5.8Hz), 5.43(1H, d, J=15Hz), 6.94(1H, d, J=7.3Hz), 7.19 (1H, t, J=7.3Hz), 7.3-7.6(5H, m), 7.44(2H, s), 7.79(1H,

s), 8.91(1H, dd, J=4.0, 1.8Hz)

(Reference Example 63)

7-[3,5-bis(Trifluoromethyl)benzyl]-6,7,8,9,10,11-hexahydro-5-(4-methylphenyl)-6,13-dioxo-13H-[1,4] diazocino[1,2-b] [2,7]naphthylidine

Using the compound obtained in the Reference Example 11, the reaction and treatment were performed as in the case of the Reference Example 55. Then, the title compound was obtained as colorless crystals.
Melting point; 231-233°C (re-crystallization from THF-isopropyl ether)
NMR (200MHz, $CDCl_3$) ppm:1.7-2.3(4H, m), 2.37(3H, s), 3.2-3.7(3H, m), 4.00(1H, d, J=15Hz), 5.05(1H, dd, J=15, 6.2Hz), 5.44(1H, d, J=15Hz), 6.83(1H, dd, J=7.8, 1.6Hz), 6.98(1H, d, J=5.4Hz), 7.04(1H, d, J=7.8Hz), 7.25(1H, d, J=7.8Hz), 7.33(1H, dd, J=7.8, 1.6Hz), 7.46(2H, s), 7.81(1H, s), 8.64(1H, d, J=5.4Hz), 9.68(1H, s)

(Reference Example 64)

7-[3,5-bis(Trifluoromethyl)benzyl]-1,2,3,4,6,7,8,9,10,11-decahydro-2-methyl-5-(4-methylphenyl)-6,13-dioxo-13H-[1,4] diazocino[1,2-b] [2,7]naphthylidine

A mixture of the compound (250 mg) obtained in the Reference Example 63, iodomethane (3 ml) and ethyl acetate (6 ml) was refluxed with heating for 1.5 hours. After evaporation of the solvents, the residue was dissolved in methanol (15 ml). Sodium borohydride (50 mg) was added to this solution with stirring at 0°C, and the mixture was stirred at 0°C for an hour, then concentrated. Ethyl acetate was added to the concentrated solution, which was washed with water and dried. The solvents were evaporated. The residue was dissolved in methanol (15 ml), and 10% palladium carbonate (50 % hydrated) (100mg) was added, and then stirred for three hours under a hydrogen atmosphere. The catalyst was filtrated to remove, and the solvents were evaporated from the filtrate. The residue was purified by a column chromatography on silica gel (ethyl acetate → ethyl acetate : methanol =4 :1) to give the title compound as pale yellow crystals (150 mg).
Melting point; 233-235°C (re-crystallization from THF-ethyl acetate- isopropyl ether)
NMR (200MHz, $CDCl_3$) ppm : 1.7-2.6(8H, m), 2.31(3H, s), 2.47(3H, s), 3.1-3.8(5H, m), 3.95(1H, d, J=15Hz), 4.93(1H, dd, J=14, 6.2Hz), 5.41(1H, d, J=15Hz), 6.72(1H, d, J=7.8Hz), 6.98(1H, d, J=7.8Hz), 7.19(2H, s), 7.42(2H, s), 7.78(1H, s)

(Reference Example 65)

4-[3,5-bis(Trifluoromethyl) benzyl]-2,3,4,5-tetrahydro-5-oxo-6-phenylpyrido[3,2-f][1,4]oxazepine

Sodium hydride (60 % oil) (60 mg) was added to a solution of N-[3,5-bis(trifluoromethyl)benzyl]-2-chloro-N-(2-hydroxyethyl)-4-phenyl-3-pyridine carboxamide (in the Reference Example 12) (348 mg) in THF (15 ml) and th mixture was stirred for 2 hours under reflux with heating. Ethyl acetate was added to the reaction mixture, which was washed with water and dried. The solvents were evaporated to give the title compound as colorless crystals (278 mg).
Melting point; 200-201°C (re-crystallization from ethanol - hexane)
NMR (200MHz, $CDCl_3$) ppm:3.70(2H, t, J=5.8Hz), 4.47(2H, t, J=5.8Hz), 4.88(2H, s), 7.24(1H, d, J=5.2Hz), 7.25-7.55 (5H, m), 7.80(2H, s), 7.86(1H, s), 8.44(1H, d, J=5.2Hz)
EI-MS m/z : 466($M^+$) [$(C_{23}H_{16}N_2O_2F_6)^+$]

(Reference Example 66)

(9R)-7-[3,5-bis(Trifluoromethyl)benzyl]-6,7,8,9,10,12-hexahydro-9-methyl-5-(4-methylphenyl)-6,12-dioxo[1,4] diazepino[2,1-g] [1,7]naphthylidine

A mixture of the compound (700 mg) obtained in the Reference Example 13, triethyl amine (0.41ml), methanesulfonyl chloride (0.224 ml) and THF (15 ml) was stirred at room temperature for 30 min, and then a saturated aqueous solution of sodium potassium (15 ml) was added, and further stirred at room temperature for 30 min. The reaction mixture was extracted with ethyl acetate. After the extract was washed with diluted hydrochloric acid and saturated brine and dried, the solvents were evaporated. The residue was dissolved in THF (15 ml), and then sodium hydride (60 % oil) (76 mg) was added to the mixture and the mixture was stirred at room temperature for 1.5 hours. This reaction mixture was diluted with ethyl acetate, and washed with diluted hydrochloric acid, an aqueous solution of sodium carbonate and saturated brine, then, dried. The solvents were evaporated. The residue was purified by

column chromatography on silica gel (ethyl acetate : methanol = 9: 1) to give the title compound as colorless crystals (408 mg).
Melting point; 179-180°C (re-crystallization from ethyl acetate - isopropyl ether)
NMR (200MHz, $CDCl_3$)ppm:1.05(3H×2/3, d, J=7.0Hz), 1.22(3H×1/3, d, J=7.0Hz), 2.39(3H×1/3, s), 2.42(3H×2/3, s), 2.52(1H, m), 3.0-3.3(2H, m), 3.48(1H×2/3, dd, J=14, 4.6Hz), 3.71(1H×1/3, dd, J=16, 5.2Hz), 4.06(1H×1/3, d, J=15Hz), 4.12(1H×2/3, d, J=15Hz), 5.28-5.65(2H, m), 6.83(1H×1/3, d, J=7.4Hz), 6.96(1H ×2/3, d, J=7.6Hz), 7.09(1H×1/3, d, J=7.4Hz), 7.20(1H×2/3, d, J=7.6Hz), 7.35(2H, m), 7.42-7.75(4H, m), 7.83(1H, s), 8.92 (1H, d, J=3.6Hz)
Anal. Calcd for $C_{29}H_{23}N_3O_2F_6$
Calculated (%) : C 62.25, H 4.14, N 7.51
Found (%): C 62.00, H 4.08, N 7.24
$[\alpha]_D$ : -60.2° (C = 0.348, MeOH)

(Reference Example 67)

(9S)-7-[3, 5-bis (Trifluoromethyl)benzyl]-6,7,8,9,10,12-hexahydro-9-methyl-6,12-dioxo-5-phenyl[1,4]diazepino[2,1-g][1,7]naphthylidine

Using the compound obtained in the Reference Example 14, the reaction and treatment were performed as in the case of the Reference Example 66. Then, the title compound was obtained as colorless crystals.
Melting point; 150-152°C (re-crystallization from ethyl acetate- isopropyl ether)
NMR (200MHz, $CDCl_3$) ppm : 1.06 (3H×2/3, d, J=7.0Hz), 1.21(3H×1/3, d, J=7.0Hz), 2.50(1H, m), 3.05-3.30(2H, m), 3.49(1H×2/3, dd, J=14, 4.6Hz), 3.72(1H×1/3, dd, J=16, 5.4Hz), 4.07(1H×1/3, d, J=15Hz), 4.14(1H×2/3, d, J=15Hz), 5.25-5.62(2H, m), 6.94(1H ×1/3, d, J=7.6Hz), 7.08(1H×2/3, d, J=7.4Hz), 7.2-7.7(8H, m), 7.83(1H, s), 8.93(1H, dd, J=4.3, 1.7Hz)
Anal. Calcd for $C_{28}H_{21}N_3O_2F_6$
Calculated (%): C 61.65, H 3.88, N 7.70
Found (%): C 61.33, H 3.89, N 7.51
$[\alpha]_D$: +69.8° (C = 0.353, MeOH)

(Reference Example 68)

(9S)-7-[3,5-bis(Trifluoromethyl)benzyl]-6,7,8,9,10,12-hexahydro-9-methyl-5-(4-methylphenyl)-6,12-dioxo[1,4]diazepino[2,1-g][1,7]naphthylidine

Using the compound obtained in the Reference Example 15, the reaction and treatment were performed as in the case of the Reference Example 66. Then, the title compound was obtained as colorless crystals.
Melting point; 179-180°C (re-crystallization from ethyl acetate- isopropyl ether)
NMR (200 MHz, $CDCl_3$) ppm: Identical to the spectra of the compound in the Reference Example 60.
Anal. Calcd for $C_{29}H_{23}N_3O_2F_6$
Calculated (%): C 62.25, H 4.14, N 7.51
Found (%): C 61.94, H 4.16, N 7.24
$[\alpha]_D$: +58.2° (C = 0.353, MeOH)

(Reference Example 69)

(±)-7-[3,5-bis(Trifluoromethyl)benzyl]-6,7,8,9,10,11-hexahydro-9-methyl-6,13-dioxo-5-phenyl-13H-[1,4]diazocino[2,1-g] [1,7]naphthylidine

Methanesulfonyl chloride (0.29 ml) was added to a solution of the compound (830 mg) obtained in the Reference Example 16 and triethyl amine (0.56 ml) in THF (15 ml) with stirring under ice-cooling. This mixture was stirred under ice-cooling for 50 min followed by the addition of a saturated aqueous solution of sodium hydrogen carbonate (15 ml), and further stirred at room temperature for 40 min. The reaction mixture was extracted with ethyl acetate, and the extract solution was washed with diluted hydrochloric acid and saturated brine, and then dried followed by evaporation of the solvents. After the residue was dissolved in THF (25 ml), sodium hydride (60 % oil) (90 mg) was added, and stirred for an hour under reflux with heating. This reaction mixture was diluted with ethyl acetate and washed with diluted hydrochloric acid, an aqueous solution of sodium carbonate and saturated brine followed by drying. Then, the solvents were evaporated to give the title compound (460 mg) as colorless crystals.
Melting point; 257-258°C (re-crystallization from ethyl acetate- ethyl ether)

NMR (200MHz, $CDCl_3$) ppm : 0.92(3H, d, J=6.6Hz), 1.73(1H, m), 1.95-2.40(2H, m), 2.98(1H, d, J=15Hz), 3.30-3.65 (2H, m), 3.97(1H, d, J=15Hz), 5.11(1H, dd, J=14, 5.9Hz), 5.43(1H, d, J=15Hz), 6.93(1H, d, J=7.6Hz), 7.19(1H, dd, J=7.6, 7.0Hz), 7.3-7.6(7H, m), 7.81(1H, s), 8.91(1H, dd, J=4.0, 2.0Hz)
Anal. Calcd for $C_{29}H_{23}N_3O_2F_6$
Calculated (%): C 62.25, H 4.14, N 7.51
Found (%): C 61.93, H 4.05, N 7.57

(Reference Example 70)

(±)-7-[3,5-bis(Trifluoromethyl)benzyl] -6,7,8,9,10,11-hexahydro-9-methyl-5-(4-methylphenyl)-6,13-dioxo-13H-[1,4] diazocino[2,1-g][1,7]naphthylidine

Using the compound obtained in the Reference Example 17, the reaction and treatment were performed as in the case of the Reference Example 69. Then, the title compound was obtained as colorless crystals.
Melting point; 280-281°C (re-crystallization from ethyl acetate- THF- isopropyl ether)
NMR (200MHz, $CDCl_3$)ppm : 0.91(3H, d, J=6.8Hz), 1.73(1H, m), 1.95-2.40(2H, m), 2.37(3H, s), 2.97(1H, d, J=15Hz), 3.35-3.62(2H, m), 3.99(1H, d, J=15Hz), 5.10(1H, dd, J=14, 5.3Hz), 5.46(1H, d, J=15Hz), 6.83(1H, dd, J=7.8, 1.6Hz), 7.05(1H, d, J=7.8Hz), 7.25(1H, d, J=7.8Hz), 7.34(1H, dd, J=7.8, 1.6Hz), 7.46(1H, dd, J=8.4, 4.2Hz), 7.47(2H, s), 7.55 (1H, dd, J=8.4, 1.8Hz), 7.81(1H, s), 8.91(1H, dd, J=4.2, 1.8Hz)
Anal. Calcd for $C_{30}H_{25}N_3O_2F_6$
Calculated (%): C 62.83, H 4.39, N 7.33
Found (%): C 62.61, H 4.021, N 7.12

(Reference Example 71)

(9R)-7-[3,5-bis(Trifluoromethyl)benzyl]-6,7,8,9,10,11-hexahydro-9-methyl-6,13-dioxo-5-phenyl-13H-[1,4]diazocino [2,1-g][1,7]naphthylidine

Using the compound obtained in the Reference Example 18, the reaction and treatment were performed as in the case of the Reference Example 69. Then, the title compound was obtained as. colorless crystals.
Melting point; 245-247°C (re-crystallization from ethyl acetate- isopropyl ether)
NMR (200 MHz, $CDCl_3$) ppm: Identical to the spectra of the compound in the Reference Example 69.
$[\alpha]_D$: +133.8° (C = 0.51, MeOH)
Anal. Calcd for $C_{29}H_{23}N_3O_2F_6$
Calculated (%): C 62.25, H 4.14, N 7.51
Found (%) : C 62.13, H 4.13, N 7.40

(Reference Example 72)

(9R)-7-[3,5-bis(Trifluoromethyl)benzyl]-6,7,8,9,10,11-hexahydro-9-methyl-5-(4-methylphenyl)-6,13-dioxo-13H-[1,4] diazocino[2,1-g] [1,7]naphthylidine

Using the compound obtained in the Reference Example 19, the reaction and treatment were performed as in the case of the Reference Example 69. Then, the title compound was obtained as colorless crystals.
Melting point; 226-228°C (re-crystallization from ethyl acetate- isopropyl ether)
NMR (200 MHz, $CDCl_3$) ppm: Identical to the spectra of the compound in the Reference Example 70.
$[\alpha]_D$ : +109.4° (C = 0.541, MeOH)
Anal. Calcd for $C_{30}H_{25}N_3O_2F_6$
Calculated (%): C 62.83, H 4.39, N 7.33
Found (%): C 62.55, H 4.56, N 7.10

(Reference Example 73)

(9S)-7-[3,5-bis(Trifluoromethyl)benzyl]-6,7,8,9,10,11-hexahydro-9-methyl-6,13-dioxo-5-phenyl-13H-[1,4]diazocino [2,1-g][1,7]naphthylidine

Using the compound obtained in the Reference Example 20, the reaction and treatment were performed as in the case of the Reference Example 69. Then, the title compound was obtained as colorless crystals.

Melting point; 242-244°C (re-crystallization from ethyl acetate- isopropyl ether)
NMR (200 MHz, $CDCl_3$) ppm: Identical to the spectra of the compound in the Reference Example 69.
$[\alpha]_D$ : -130.4° (C = 0.496, MeOH)
Anal. Calcd for $C_{29}H_{23}N_3O_2F_6$
Calculated (%) : C 62.25, H 4.14, N 7.51
Found (%) : C 62.07, H 4.15, N 7.36

(Reference Example 74)

(9S)-7-[3,5-bis(Trifluoromethyl)benzyl]-6,7,8,9,10,11-hexahydro-9-methyl-5-(4-methylphenyl)-6,13-dioxo-13H-[1,4]diazocino[2,1-g][1,7]naphthylidine

Using the compound obtained in the Reference Example 21, the reaction and treatment were performed as in the case of the Reference Example 69. Then, the title compound was obtained as colorless crystals.
Melting point; 227-228°C (re-crystallization from ethyl acetate- isopropyl ether)
NMR (200 MHz, $CDCl_3$) ppm: Identical to the spectra of the compound in the Reference Example 70.
$[\alpha]_D$: -107.1° (C = 0.521, MeOH)
Anal. Calcd for $C_{30}H_{25}N_3O_2F_6$
Calculated (%) : C 62.83, H 4.39, N 7.33
Found (%) : C 62.55, H 4.40, N 7.13

(Reference Example 75)

4-[3,5-bis(Trifluoromethyl)benzyl]-2,3,4,5-tetrahydro-5-oxo-6-phenyl-1H-pyrido[2,3-e][1,4]diazepine

A mixture of the compound (370 mg) obtained in the Reference Example 22, potassium carbonate anhydride (200 mg) and xylene (10 ml) was stirred for 9 hours under reflux with heating. After the reaction mixture was cooled, water was added and then the mixture was extracted with ethyl acetate. The extract was washed with water followed by drying. The solvent was evaporated to give the title compound as colorless crystals. Melting point; 242-243°C (re-crystallization from ethyl acetate- isopropyl ether)
NMR (200MHz, $CDCl_3$) ppm : 3.60-3.80 (4H, m), 4.81(2H, s), 4.86(1H, s), 6.87(1H, d, J=5.2Hz), 7.30-7.50(6H, m), 7.79(2H, s), 7.85(1H, s), 8.21(1H, d, J=5.2Hz)
Anal. Calcd for $C_{23}H_{17}N_3OF_6$
Calculated (%): C 59.36, H 3.68, N 9.03
Found (%): C 59.24, H 3.66, N 9.06
EI-MS m/z: 465 ($M^+$) [$(C_{23}H_{17}N_3OF_6)^+$]

(Reference Example 76)

5-[3,5 bis(Trifluoromethyl)benzyl]-2,3,4,5-tetrahydro-6-oxo-7-phenyl-6H-pyrido[2,3-b][1,5]oxazosine

Using the compound obtained in the Reference Example 23, the reaction and treatment were performed as in the case of the Reference Example 65. Then, the title compound was obtained as colorless crystals.
Melting point; 188-189°C (re-crystallization from ethyl acetate- isopropyl ether)
NMR (200MHz, $CDCl_3$) ppm:1.65-1.88(1H, m), 2.18-2.45(1H, m), 3.36(1H, dd, J=15.2Hz), 3.73(1H, m), 4.17(1H, d, J=15.2Hz), 4.32(1H, dt, J=12.6, 3.6Hz), 4.67(1H, ddd, J=12.6, 5.6, 2.4Hz), 5.50(1H, d, J=15.2Hz), 7.16(1H, d, J=5.2Hz), 7.20-7.45(5H, m), 7.71(2H, s), 7.83(1H, s), 8.41(1H, d, J=5.2Hz)
Anal. Calcd for $C_{24}H_{18}N_2O_2F_6$
Calculated (%): C 60.00, H 3.78, N 5.83
Found (%): C 59.92, H 3.76, N 5.89

(Reference Example 77)

4-[3,5 bis(Trifluoromethyl)benzyl]-2,3,4,5-tetrahydro-7-methyl-5-oxo-6-phenyl pyrido[3,2-f][1,4]oxazepine

Using the compound obtained in the Reference Example 24, the reaction and treatment were performed as in the case of the Reference Example 65. Then, the title compound was obtained as colorless crystals.
Melting point; 179-181°C (re-crystallization from ethyl acetate- isopropyl ether)

NMR (200MHz, $CDCl_3$) ppm:2.13(3H, s), 3.57(2H, t, J=5.8Hz), 4.42(2H, t, J=5.8Hz), 4.80(2H, s), 7.16(2H, m), 7.47 (3H, m), 7.65(2H, s), 7.81(1H, s), 8.32(1H, s)

(Reference Example 78)

5-[3,5 bis(Trifluoromethyl)benzyl]-2,3,4,5-tetrahydro-8-methyl-6-oxo-7-phenyl-6H-pyrido[2,3-b][1,5]oxazosine

Using the compound obtained in the Reference Example 25, the reaction and treatment were performed as in the case of the Reference Example 65. Then, the title compound was obtained as colorless crystals.
Melting point; 180-182°C (re-crystallization from ethyl acetate- isopropyl ether)
NMR (200MHz, $CDCl_3$) ppm : 1.71 (1H, m), 2.07(3H, m), 2.28(1H, m), 3.24(1H, dd, J=15.2, 3.8Hz), 3.64(1H, dd, J=15.2, 12.0Hz), 4.05(1H, d, J=15.6Hz), 4.27(1H, dt, J=12.6, 3.8Hz), 4.63(1H, ddd, J=12.6, 5.4, 2.0Hz), 5.45(1H, d, J=15.6Hz), 7.38(5H, m), 7.54(2H, s), 7.78(1H, s), 8.29(1H, s)

(Reference Example 79)

(±)-7-[3,5 bis(Trifluoromethyl)benzyl]-6,7,8,9,10,12-hexahydro-9-hydroxy-5-(4-methylphenyl) -6,12-dioxo[1,4] diazepino[2,1-g] [1,7]naphthylidine

Using the compound obtained in the Reference Example 26, the reaction and treatment were performed as in the case of the Reference Example 56. Then, the title compound was obtained as colorless crystals.
Melting point; 282-283°C (re-crystallization from acetone-ethyl ether)
NMR (200MHz,$CDCl_3$) ppm : 2.43 (3H, s), 3.35-3.63(3H, m), 4.02(1H ×3/8, d, J=3.5Hz, -OH), 4.21(1H×3/8, d, J=15Hz), 4.30(1H× 5/8, d, J=3.5Hz, -OH), 4.38(1H×5/8, d, J=15Hz), 4.60(1H, m), 5.24(1H×3/8, d, J=15Hz), 5.61 (1H×5/8, d, J=15Hz), 5.68(1H, m), 6.92(1H, t-like, J=3.8Hz), 7.19-7.86(8H, m), 8.95(1H, d, J=4Hz)
Anal. Calcd for $C_{28}H_{21}N_3O_3F_6 \cdot 1/4H_2O$
Calculated (%): C 59.42, H 3.83, N 7.42
Found (%): C 59.45, H 3.74, N 7.39
EI-MS m/z: 561 ($M^+$) [$(C_{28}H_{21}N_3O_3F_6)^+$]

(Reference Example 80)

7-Benzyl-6,7,8,9,10,12-hexahydro-6,12-dioxo-5-phenyl[1,4]diazepino[2,1-g][1,7]naphthylidine

7-Benzyl-7,8-dihydro-7-(3-hydroxypropyl)-8-oxo-5-phenyl-6-pyrido[3,4-b]pyridinecarboxamide was prepared by reacting and treating as in the case of the Reference Example 13 using the compound obtained in the Reference Example 27 and 3-amino-1-propanol. Using this compound, the reaction and treatment were performed as in the case of the Reference Example 12. Then, the title compound was obtained as colorless crystals. Melting point; 210-212°C (re-crystallization from ethyl acetate- ethyl ether)
NMR (200MHz, $CDCl_3$) ppm:1.7-2.2(2H, m), 3.2-3.6 (3H, m), 4.30 (1H, d, J=14Hz), 4.89(1H, d, J=14Hz), 5.43(1H, dd, J=14, 5.7Hz), 7.0-7.7 (11H, m), 7.70(1H, dd, J=8.4, 1.6Hz), 8.92(1H, dd, J=4.4, 1.6Hz)

(Reference Example 81)

7-Benzyl-6,7,8,9,10,11-hexahydro-6,13-dioxo-5-phenyl-13H-[1,4]diazocino[2,1-g] [1,7]naphthylidine

7-Benzyl-7,8-dihydro-7-(4-hydroxybutyl)-8-oxo-5-phenyl-6-pyrido[3,4-b]pyridine carboxamide was prepared by reacting and treating as in the case of the Reference Example 16 using the compound obtained in the Reference Example 27 and 4-amino-1-butanol. Using this compound, the reaction and treatment were performed as in the case of the Reference Example 69. Then, the title compound was obtained as colorless crystals. Melting point; 243-244°C (re-crystallization from acetone-ethyl ether)
NMR (200MHz, $CDCl_3$) ppm : 1.6-2.3(4H, m), 3.15(1H, m), 3.35-3.65(2H, m), 3.76(1H, d, J=15Hz), 5.15(1H, dd, J=14, 5.7Hz), 5.42(1H, d, J=15Hz), 6.64(2H, d, J=6.2Hz), 7.0-7.3(4H, m), 7.3-7.7(6H, m), 8.91(1H, dd, J=4.2, 1.8Hz)

(Reference Example 82)

7-Benzyl-6,7,8,9,10,11,12,14-octahydro-6,14-dioxo-5-phenyl-[1,4]diazonino[2,1-g][1,7]naphthylidine

7-Benzyl-7,8-dihydro-7-(5-hydroxypentyl)-8-oxo-5-phenyl-6-pyrido[3,4-b]pyridine carboxamide was prepared by reacting and treating as in the case of the Reference Example 16 using the compound obtained in the Reference Example 27 and 5-amino-1-pentanol. Using this compound, the reaction and treatment were performed as in the case of the Reference Example 69. Then, the title compound was obtained as colorless crystals.
Melting point; 224-226°C (re-crystallization from ethyl acetate - ethyl ether)
NMR (200MHz, $CDCl_3$) ppm: 1.3-1.9 (4H, m), 2.09(2H, m), 2.85-3.05(1H, m), 3.15-3.40(1H, m), 3.50(1H, dt, Jd=15Hz, Jt=6.4Hz), 3.64(1H, d, J=15Hz), 4.97(1H, dt, Jd=15Hz, Jt=4.8Hz), 5.48(1H, d, J=15Hz), 6.43(2H, d, J=7.2Hz), 7.05-7.25 (4H, m), 7.3-7.7(6H, m), 8.91(1H, dd, J=4.2, 1.8Hz)

(Reference Example 83)

7-(3,4-Dichlorobenzyl)-6,7,8,9,10,12-hexahydro-6,12-dioxo-5-phenyl[1,4]diazepino[2,1-g][1,7]naphthylidine

7-(3,4-Dichlorobenzyl)-7,8-dihydro-7-(3-hydroxypropyl)-8-oxo-5-phenyl-6-pyrido[3,4-b]pyridine carboxamide was prepared by reacting and treating as in the case of the Reference Example 13 using the compound obtained in the Reference Example 28 and 3-amino-1-propanol. Using this compound, the reaction and treatment were performed as in the case of the Reference Example 66. Then, the title compound was obtained as colorless crystals.
Melting point; 224-226°C (re-crystallization from ethyl acetate - ethyl ether)
NMR (200MHz, $CDCl_3$) ppm:1.9-2.3(2H, m), 3.2-3.6(3H, m), 4.01(1H, d, J=15Hz), 5.05(1H, d, J=15Hz), 5.49(1H, dd, J=13, 5.0Hz), 6.9-7.1(2H, m), 7.25(1H, m), 7.38(1H, d, J=8.6Hz), 7.3-7.8(6H, m), 8.93(1H, d, J=4.0Hz)

(Reference Example 84)

7-(3,4-Dichlorobenzyl)-6,7,8,9,10,11-hexahydro-6,13-dioxo-5-phenyl-13H-[1,4]diazocino[2,1-g][1,7]naphthylidine

7-(3,4-Dichlorobenzyl)-7,8-dihydro-7-(4-hydroxybutyl)-8-oxo-5-phenyl-6-pyrido[3,4-b]pyridine carboxamide was prepared by reacting and treating as in the case of the Reference Example 16 using the compound obtained in the Reference Example 28 and 4-amino-1-butanol. Using this compound, the reaction and treatment were performed as in the case of the Reference Example 15. Then, the title compound was obtained as colorless crystals.
Melting point; 236-238°C (re-crystallization from acetone - ethyl ether)
NMR (200MHz, $CDCl_3$) ppm:1.7-2.3(4H, m), 3.14(1H, m), 3.39-3.60(2H, m) 3.70(1H, d, J=15Hz), 5.14(1H, dd, J=15, 5.9Hz), 5.35(1H, d, J=15Hz), 6.35(1H, dd, J=8.4, 2.0Hz), 7.02(2H, m), 7.18(1H, d, J=8.4Hz), 7.3-7.6(6H, m), 8.91(1H, dd, J=4.0, 1.8Hz)

(Reference Example 85)

(S)-5-[3,5 bis(Trifluoromethyl)benzyl]-2,3,4,5-tetrahydro-3,8-dimethyl-6-oxo-7-phenyl-6H-pyrido[2,3-b][1,5]oxazosine

Using the compound obtained in the Reference Example 29, the reaction and treatment were performed as in the case of the Reference Example 65. Then, the title compound was obtained as colorless crystals.
Melting point; 147-148°C (re-crystallization from ethyl acetate- hexane)
NMR (200MHz, $CDCl_3$) ppm:0.83(3H, d, J=7.4Hz), 2.07(3H, s), 2.39(1H, m), 2.97(1H, d, J=15.4Hz), 3.48(1H, m), 3.87 (1H, dd, J=10.4, 12.4Hz), 4.06(1H, d, J=15.6Hz), 4.59(1H, dd, J=5.2, 12.4Hz), 5.44(1H, d, J=15.4Hz), 7.37(5H, s), 7.53(2H, s), 7.78(1H, s), 8.29(1H, s)
Anal. Calcd for $C_{26}H_{22}N_2O_2F_6$
Calculated (%): C 61.42, H 4.36, N 5.51
Found (%): C 61.30, H 4.52, N 5.70
$[\alpha]_D^{20}$ : -106.8° (C = 0.257, $CHCl_3$)

(Reference Example 86)

(R)-5-[3,5 bis(Trifluoromethyl)benzyl]-2,3,4,5-tetrahydro-3,8-dimethyl-6-oxo-7-phenyl-6H-pyrido[2,3-b][1,5]oxazosine

Using the compound obtained in the Reference Example 30, the reaction and treatment were performed as

in the case of the. Reference Example 65. Then, the title compound was obtained as colorless crystals.
Melting point; 147-149°C (re-crystallization from ethyl acetate- hexane)
NMR (200MHz, $CDCl_3$) ppm: Identical to the spectra of the compound of the Reference Example 85.
Anal. Calcd for $C_{26}H_{22}N_2O_6$
Calculated (%): C 61.42, H 4.36, N 5.51
Found (%): C 61.26, H 4.33, N 5.69
$[\alpha]_D^{20}$ : +102.5° (C = 0.573, $CHCl_3$)

(Reference Example 87)

4-[3,5 bis(Trifluoromethyl)benzyl]-2,3,4,5-tetrahydro-8-methyl-5-oxo-6-phenyl pyrido[3,2-f][1,4]oxazepine

Using the compound obtained in the Reference Example 31, the reaction and treatment were performed as in the case of the Reference Example 65. Then, the title compound was obtained as colorless crystals.
Melting point; 151-153°C (re-crystallization from ethyl acetate- isopropyl ether)
NMR (200MHz, $CDCl_3$) ppm:2.58(3H, s), 3.69(2H, t, J=5.4Hz), 4.47(2H, t, J=5.4Hz), 4.87(2H, s), 7.11(1H, s), 7.17-7.56 (5H, m), 7.80(2H, s), 7.86(1H, s)
Anal. Calcd for $C_{24}H_{18}N_2O_2F_6 \cdot 1/4H_2O$
Calculated (%): C 59.44, H 3.85, N 5.78
Found (%): C 59.42, H 3.82, N 5.84

(Reference Example 88)

5-[3,5-bis(Trifluoromethyl)benzyl]-2,3,4,5-tetrahydro-9-methyl-6-oxo-7-phenyl-6H-pyrido[2,3-b][1,5]oxazosine

Using the compound obtained in the Reference Example 32, the reaction and treatment were performed as in the case of the Reference Example 65. Then, the title compound was obtained as colorless crystals.
Melting point; 164-165°C (re-crystallization from ethyl acetate- isopropyl ether)
NMR (200MHz, $CDCl_3$) ppm : 1.79(1H, m), 2.30(1H, m), 2.56(3H, s), 3.35(1H, m), 3.77(1H, m), 4.14(1H, d, J=15.2Hz), 4.31(1H, m), 4.65(1H, m), 5.49(1H, d, J=15.2Hz), 7.02(1H, s), 7.20-7.50(5H, m), 7.72(2H,s), 7.83(1H,s)
Anal. Calcd for $C_{25}H_{20}N_2O_2F_6$
Calculated (%): C 60.73, H 4.08, N 5.67
Found (%): C 60.43, H 4.04, N 5.74

(Reference Example 89)

4-[3,5 bis(Trifluoromethyl)benzyl]-2,3,4,5-tetrahydro-8-methyl-5-oxo-6-phenylpyrido[3,2-f][1,4]oxazepine 9-oxide

m-Chloroperbenzoic acid (870 mg) was added to a solution of the compound (1.20 g) obtained in the Reference Example 87 in chloroform (30 ml), and the mixture was stirred at room temperature for 20 hours. The solvents were evaporated, and an aqueous solution of potassium carbonate was added to the residual, which was extracted with ethyl acetate. The extract solution was washed with aqueous solution of potassium carbonate and dried. Then, the solvent was evaporated to give the title compound as colorless crystals (1.10 g).
Melting point; 181-183°C (re-crystallization from THF-isopropyl ether)
NMR (200MHz, $CDCl_3$) ppm: 2.62(3H, s), 3.72(2H, m), 4.65(2H, m), 4.89(2H, s), 7.18(1H, s), 7.20-7.50(5H, m), 7.79 (2H, s), 7.87(1H, s)
Anal. Calcd for $C_{24}H_{18}N_2O_3F_6$
Calculated (%) : C 57.03, H 3.79, N 5.54
Found (%): C 57.15, H 3.77, N 5.16

(Reference Example 90)

5-[3,5-bis(Trifluoromethyl)benzyl]-2,3,4,5-tetrahydro-9-methyl-6-oxo-7-phenyl-6H-pyrido[2,3-b][1,5]oxazosine 10-oxide

Using the compound obtained in the Reference Example 88, the reaction and treatment were performed as in the case of the Reference Example 89. Then, the title compound was obtained as colorless crystals (727 mg).
Melting point; 116-118°C (re-crystallization from ethanol - ethyl ether)

NMR (200MHz, $CDCl_3$)ppm : 1.60-1.82(1H, m), 2.42(1H, m), 2.61(3H, s), 3.43(1H, dd, J=6.0, 17.0Hz), 3.81(1H, m), 4.18(1H, d, J=15.4Hz), 4.25(1H, m), 4.78(1H, dd, J=5.2, 12.6Hz), 5.52(1H, d, J=15.4Hz), 7.16(1H, s), 7.18-7.50(5H, m), 7.72(2H, s), 7.84(1H, s)
Anal. Calcd for $C_{25}H_{20}N_2O_3$. 1/4$H_2O$
Calculated (%): C 58.31, H 4.01, N 5.44
Found (%): C 58.17, H 4.38, N 5.31

(Reference Example 91)

8-Acetoxymethyl-4-[3,5-bis(trifluoromethyl)benzyl]-2,3,4,5-tetrahydro-5-oxo-6-phenylpyrido[3,2-f][1,4]oxazepine

A mixture of the compound (939 mg) obtained in the Reference Example 89 and acetic anhydride (25 ml) was refluxed with heating for 20 min. Following evaporation of the solvent, an aqueous solution of potassium carbonate was added to the residual, which was then extracted with ethyl acetate. The extract solution was washed with water and dried. Then the solvents were evaporated to give the title compound as colorless crystals (740 mg).
Melting point; 122-124°C (re-crystallization from ethyl acetate - isopropyl ether)
NMR (200MHz, $CDCl_3$) ppm : 2.18 (3H, s), 3.71(2H, t, J=5.6Hz), 4.50(2H, t, J=5.6z), 4.88(2H, s), 5.21(2H, s), 7.18-7.50 (6H, m), 7.79(2H, s), 7.87(1H, s)
Anal. Calcd for $C_{26}H_{20}N_2O_4F_6$
Calculated (%): C 58.00, H 3.74, N 5.20
Found (%): C 57.60, H 4.02, N 5.09

(Reference Example 92)

9-Acetoxymethyl-5-[3,5 bis(trifluoromethyl)benzyl]-2,3,4,5-tetrahydro-6-oxo-7-phenyl-6H-pyrido[2,3-b][1,5]oxazosine

Using the compound obtained in the Reference Example 90, the reaction and treatment were performed as in the case of the Reference Example 91. Then, the title compound was obtained as colorless crystals (479 mg).
Melting point; 156-157°C (re-crystallization from ethyl acetate - isopropyl ether)
NMR (200MHz, $CDCl_3$)ppm:1.60-1.95(1H, m), 2.00-2.20(1H, m), 2.17(3H, s), 3.36(1H, m), 3.75(1H, m), 4.14(1H, d, J=15.2Hz), 4.31(1H, m), 4.61(1H, m), 5.20(2H, s), 5.48(1H, d, J=15.2Hz), 7.18(1H, s), 7.20-7.50(5H, m), 7.70(2H, s), 7.83(1H, s)
Anal. Calcd for $C_{27}H_{22}N_2O_4F_6$
Calculated (%): C 58.70, H 4.01, N 5.07
Found (%): C 58.81, H 4.11, N 5.17

(Reference Example 93)

4-[3,5-bis(Trifluoromethyl)benzyl]-8-chloromethyl-2,3,4,5-tetrahydro-5-oxo-6-phenylpyrido[3,2-f] [1,4]oxazepine

Phosphorous oxychloride (1.24 ml) and triethyl amine (1.85 ml) were simultaneously dropped into a solution of the compound (4.40 g) obtained in the Reference Example 89 in dichloromethane (100 ml) with stirring. After this mixture was refluxed with heating for an hour, the solvents were evaporated. An aqueous solution of potassium carbonate was added to the residual, which was then extracted with ethyl acetate-THF. The extract solution was washed with water and dried. The solvents were evaporated to give the title compound as colorless crystals (1.44 g).
Melting point; 183-184°C (re-crystallization from ethyl acetate - isopropyl ether)
NMR (200MHz, $CDCl_3$) ppm : 3.73 2H, t, J=5.4Hz), 4.51(2H, t, J=5.4Hz), 4.66(2H, s), 4.89(2H, s), 7.27(1H, s), 7.30-7.55 (5H, m), 7.81(2H, s), 7.88(1H, s)
Anal. Calcd for $C_{24}H_{17}N_2O_2F_6Cl$
Calculated (%) : C 55.99, H 3.33, N 5.44
Found (%) : C 55.75, H 3.53, N 5.27

(Reference Example 94)

4-[3,5-bis(Trifluoromethyl)benzyl]-2,3,4,5-tetrahydro-8-methoxymethyl-5-oxo-6-phenylpyrido[3,2-f][1,4]oxazepine

A mixture of the compound (151 mg) obtained in the Reference Example 93, THF (2 ml), methanol (1 ml) and 28% sodium methoxide-methanol solution (1 ml) was stirred at room temperature for 2 hours. Following evaporation

of the solvents, water was added to the reside, which was then extracted with ethyl acetate. The extract was washed with water and dried. The solvents were evaporated to yield the title compound as colorless crystals (118 mg).
Melting point; 139-140°C (re-crystallization from ethyl acetate - isopropyl ether)
NMR (200MHz, $CDCl_3$) ppm : 3.51 (3H, s), 3.71(2H, t, J=5.6Hz), 4.49(2H, t, J=5.6Hz), 4.58(2H, s), 4.89(2H, s), 7.27 (1H, s), 7.30-7.52(5H, m), 7.81(2H, s), 7.87(1H, s)
Anal. Calcd for $C_{25}H_{20}N_2O_3F_6$
Calculated (%): C 58.83, H 3.95, N 5.49
Found (%) : C 58.73, H 3.95, N 5.57

(Reference Example 95)

4-[3,5-bis(Trifluoromethyl)benzyl]-2,3,4,5-tetrahydro-8-(1-methylethyloxy) -5-oxo-6-phenylpyrido[3,2-f][1,4]oxazepine

A mixture of the compound (150 mg) obtained in the Reference Example 93, THF (1 ml), isopropanol (10 ml) and sodium hydride (60 % oil) (120 mg) was stirred at room temperature for 3 hours. Following evaporation of the solvents, water was added to the residue, which was then extracted with ethyl acetate. The extract solution was washed with water and dried followed by evaporation of the solvents. The residue was purified by column chromatography on silica gel (hexane - ethyl acetate =1:1) to give the title compound as colorless crystals (74 mg). Melting point; 134-136°C (re-crystallization from ethyl acetate - hexane)
NMR (200MHz, $CDCl_3$) ppm : 1.26(6H, d, J=6.0Hz), 3.60-3.90 (3H,m), 4.48(2H, t, J=5.4Hz), 4.63(2H, s), 4.89(2H, s), 7.27(1H, s), 7.30-7.55(5H, m), 7.81(2H, s), 7.87(1H, s)
Anal. Calcd for $C_{27}H_{24}N_2O_3F_6$
Calculated (%): C 60.22, H 4.49, N 5.20
Found (%): C 60.00, H 4.61, N 5.07

(Reference Example 96)

4-[3,5-bis(Trifluoromethyl)benzyl]-2,3,4,5-tetrahydro-8-methylthiomethyl-5-oxo-6-phenylpyrido[3,2-f][1,4]oxazepine

A mixture of the compound (125 mg) obtained in the Reference Example 93, methanol (5 ml) and an aqueous solution of 15 % sodium methyl mercaptan (1 ml) was stirred at room temperature for 10 min. Following evaporation of the solvents, water was added to the residue, which was then extracted with ethyl acetate. The extract solution was washed with water and dried. The solvents were evaporated to give the title compound as colorless crystals (103 mg).
Melting point; 165-166°C (re-crystallization from ethyl acetate - isopropyl ether)
NMR (200MHz,$CDCl_3$)ppm:2.14(3H, s), 3.72(2H, t, J=5.4Hz), 3.79(2H, s), 4.49(2H, t, J=5.4Hz), 4.89(2H, s), 7.30-7.50 (5H, m), 7.34(1H, s), 7.81(2H, s), 7.87(1H, s)
Anal. Calcd for $C_{25}H_{20}N_2O_2SF_6$. 1/6$H_2O$
Calculated (%): C 56.71, H 3.87, N 5.29
Found (%): C 56.67, H 3.87, N 5.23

(Reference Example 97)

8-Aminomethyl-4-[3,5 bis(trifluoromethyl)benzyl]-2,3,4,5-tetrahydro-5-oxo-6-phenylpyrido[3,2-f][1,4]oxazepine

A mixture of the compound (500 mg) obtained in the Reference Example 93, THF (10 ml) and 25% aqueous ammonia (10 ml) was heated at 120°C for an hour in a dip pipe. After cooling, the solvents were evaporated, and water was added to the residue, which was then extracted with ethyl acetate. The extract solution was washed with water and dried. The solvents were evaporated to give the title compound as colorless crystals (443 mg).
Melting point; 188-191°C (re-crystallization from THF - ethyl ether)
NMR (200MHz, $CDCl_3$) ppm : 3.71(2H, t, J=5.6Hz), 4.00(2H, s), 4.50(2H, t, J=5.6Hz), 4.89(2H, s), 7.20-7.60(6H, m), 7.81(2H, s), 7.87(1H, s)
Anal. Calcd for $C_{24}H_{19}N_3O_2F_6$
Calculated (%): C 58.19, H 3.87, N 8.48
Found (%): C 58.36, H 3.81, N 8.00

(Reference Example 98)

4-[3,5-bis(Trifluoromethyl)benzyl]-2,3,4,5-tetrahydro-8-methylaminomethyl-5-oxo-6-phenylpyrido[3,2-f][1,4] oxazepine

A mixture of the compound (150 mg) obtained in the Reference Example 93 and 40 % methylamine - methanol solution (10 ml) was stirred at room temperature for 30 min. Following evaporation of the solvents, water was added to the residue, which was then extracted with ethyl acetate. The extract solution was washed with water and dried. The solvents were evaporated to give the title compound as colorless crystals (115 mg).
Melting point; 152-154°C (re-crystallization from ethyl acetate - isopropyl ether)
NMR (200MHz, $CDCl_3$) ppm : 2.50(3H, s), 3.70(2H, t, J=5.6Hz), 3.89(2H, s), 4.48(2H, t, J=5.6Hz), 4.88(2H, s), 7.22-7.50 (6H, m), 7.80(2H, s), 7.86(1H, s)
Anal. Calcd for $C_{25}H_{21}N_3O_2F_6$
Calculated (%): C 58.94, H 4.15, N 8.25
Found (%): C 58.71, H 4.25, N 8.35

(Reference Example 99)

4-[3,5-bis(Trifluoromethyl)benzyl]-8-dimethylaminomethyl-2,3,4,5-tetrahydro-5-oxo-6-phenylpyrido[3,2-f][1,4] oxazepine

Dimethyl amine (1 ml) was added to a solution of the compound (150 mg) obtained in the Reference Example 93 in THF (3 ml), and stirred at room temperature for 30 min. Following evaporation of the solvents, water was added to the residue, which was then extracted with ethyl acetate. The extract solution was washed with water and dried. The solvents were evaporated to give the title compound as colorless crystals (128 mg).
Melting point; 186-188°C (re-crystallization from ethyl acetate - isopropyl ether)
NMR (200MHz,$CDCl_3$)ppm : 2.33(6H, s), 3.60(2H, s), 3.71(2H, t, J=5.6Hz) , 4.49(2H, t, J=5.6Hz), 4.89(2H, s), 7.26 (1H, s), 7.30-7.50(5H, m), 7.81(2H, s), 7.86(1H, s)
Anal. Calcd for $C_{26}H_{23}N_3O_2F_6$
Calculated (%): C 59.66, H 4.43, N 8.03
Found (%): C 59.43, H 4.49, N 7.84

(Reference Example 100)

4-[3,5-bis(Trifluoromethyl)benzyl]-8-cyclopropyl aminomethyl-2,3,4,5-tetrahydro-5-oxo-6-phenylpyrido[3,2-f][1,4] oxazepine

Cyclopropyl amine (0.5 ml) was added to a solution of the compound (155 mg) obtained in the Reference Example 93 in THF (10 ml), and the mixture was refluxed with heating for 15 hours under stirring. Following evaporation of the solvents, water was added to the residue, which was then extracted with ethyl acetate. The extract solution was washed with water and dried followed by evaporation of the solvents. The residue was purified by column chromatography on silica gel (ethyl acetate : methanol = 9:1) to give the title compound as colorless crystals (127 mg).
Melting point; 129-131°C (re-crystallization from ethyl acetate - hexane)
NMR (200MHz, $CDCl_3$) ppm : 0.44(4H, m), 2.19(1H, m), 3.69(2H, t, J=5.6Hz) , 3.97(2H, s), 4.48(2H, t, J=5.6Hz) , 4.87 (2H, s), 7.25(1H, s), 7.26-7.55(5H, m), 7.79(2H, s), 7.86(1H, s)
Anal. Calcd for $C_{27}H_{23}N_3O_2F_6\cdot 1/6H_2O$
Calculated (%): C 60.22, H 4.37, N 7.80
Found (%): C 59.98, H 4.40, N 7.85

(Reference Example 101)

4-[3,5-bis(Trifluoromethyl)benzyl]-2,3,4,5-tetrahydro-8-(N-methylpiperazinomethyl)-5-oxo-6-phenylpyrido[3,2-f] [1,4] oxazepine

N-methyl piperazine (1 ml) was added to a solution of the compound (150 mg) obtained in the Reference Example 93 in THF (1 ml), and stirred at room temperature for 15 hours. Following evaporation of the solvents, water was added to the residue, which was then extracted with ethyl acetate. The extract solution was washed with water and dried. The solvents were evaporated to give the title compound as colorless crystals (105 mg).

Melting point; 181-182°C (re-crystallization from ethyl acetate - isopropyl ether)
NMR (200MHz, $CDCl_3$)ppm : 2.30(3H, s), 2.48 (4H, br), 2.59(4H, br), 3.68(2H, s), 3.71(2H, t, J=5.6Hz), 4.48(2H, t, J=5.6Hz), 4.89(2H, s), 7.27(1H, s), 7.30-7.50(5H, m), 7.81(2H, m), 7.87(1H, s)
Anal. Calcd for $C_{26}H_{28}N_4O_2F_6$
Calculated (%): C 60.20, H 4.88, N 9.68
Found (%): C 59.96, H 5.00, N 9.51

(Reference Example 102)

8-Acetylaminomethyl-4-[3,5-bis(trifluoromethyl)benzyl]-2,3,4,5-tetrahydro-5-oxo-6-phenylpyrido[3,2-f][1,4]oxazepine

Acetic anhydride (1 ml) was added to a solution of the compound (150 mg) obtained in the Reference Example 97 in pyridine (3 ml), and the mixture was stirred at room temperature for 20 min. The solvents were evaporated, and ethyl acetate was added to the residue. This mixture was washed with 1N-hydrochloric acid and water and dried. Then, the solvents were evaporated to give the title compound as colorless crystals (113 mg).
Melting point; 223-224°C (re-crystallization from THF - ethyl ether)
NMR (200MHz, $CDCl_3$)ppm:2.07(3H, s), 3.72(2H, t, J=5.4Hz) , 4.49(2H, t, J=5.4Hz), 4.56(2H, d, J=5.4Hz), 4.88(2H, s), 6.62(1H, br), 7.21(1H, s), 7.22-7.55(5H, m), 7.80(2H, s), 7.87(1H, s)
Anal. Calcd for $C_{26}H_{21}N_3O_3F_6$
Calculated (%): C 58.10, H 3.94, N 7.82
Found (%): C 58.06, H 3.97, N 7.99

(Reference Example 103)

4-[3,5-bis(Trifluoromethyl)benzyl]-2,3,4,5-tetrahydro-8-methanesulfonyl amino methyl-5-oxo-6-phenylpyrido[3,2-f][1,4]oxazepine

Triethylamine (0.085 ml) and methanesulfonyl chloride (0.050 ml) were added to a solution of the compound (150 mg) obtained in the Reference Example 97 in THF (5 ml) and stirred at room temperature for an hour. The solvents were evaporated, and ethyl acetate was added to the residue. This mixture was washed with an aqueous solution of potassium carbonate and water, and then dried. The solvents were evaporated to give the title compound as colorless crystals (108 mg).
Melting point; 194-195°C (re-crystallization from THF - isopropyl ether)
NMR (200MHz, $CDCl_3$)ppm:2.99(3H, s), 3.72(2H, t, J=5.4Hz), 4.44(2H, d, J=6.0Hz), 4.48(2H, t, J=5.4Hz), 4.88(2H, s), 5.55(1H, t, J=6.0Hz), 7.26(1H, s), 7.27-7.50(5H, m), 7.80(2H, s), 7.87(1H, s)
Anal. Calcd for $C_{25}H_{21}N_3O_4SF_6 \cdot 1/2H_2O$
Calculated (%): C 51.55, H 3.80, N 7.21
Found (%): C 51.43, H 3.78, N 7.07

(Reference Example 104)

6-[3,5-bis(Trifluoromethyl)benzyl]-5,6,7,8,9,10-hexahydro-3,9-dimethyl-5,10-dioxo-4-phenylpyrido[2,3-f] [1,4] diazocine

Triethyl amine (0.42 ml) and methanesulfonyl chloride (0.24 ml) were added to a solution of the compound (370 mg) obtained in the Reference Example 33 in THF (15 ml) under cooling and stirred at room temperature for an hour. A saturated solution of sodium hydrogen carbonate (15 ml) was added to the reaction solution, and the mixture was stirred at room temperature for further 40 min, and then extracted with ethyl acetate. The extract solution was washed with diluted hydrochloric acid and saturated brine and dried followed by evaporation of the solvents. After the residue was dissolved in THF (30 ml), sodium hydride (60 % oil) (84 mg) was added to the mixture and the mixture was refluxed with heating for 40 min. This reaction mixture was diluted with ethyl acetate and washed with diluted hydrochloric acid, an aqueous solution of sodium carbonate and saturated brine followed by drying. The solvents were evaporated to give the title compound as colorless crystals (213 mg).
Melting point; 203-205°C (re-crystallization from ethyl acetate - isopropyl ether)
NMR (200MHz, $CDCl_3$) ppm : 1.72(1H, dd, J=15, 7.2Hz), 2.18(3H, s), 2.75(1H, m), 3.04 (3H, s), 3.54(3H, m), 4.09(1H, dd, J=14, 7.2Hz), 7.2-7.6(5H, m), 7.48(2H, s), 7.74(1H, s), 8.69(1H, s)
Anal. Calcd for $C_{26}H_{21}N_3O_2F_6 \cdot 0.2H_2O$
Calculated (%): C 59.48, H 4.11, N 8.00

Found (%): C 59.39, H 4.13, N 7.83
EI-MS m/z: 521 ($M^+$) [$(C_{26}H_{21}N_3O_2F_6)^+$]

(Reference Example 105)

6-[3,5 bis(Trifluoromethyl)benzyl]-5,6,7,8,9,10-hexahydro-9-methyl-5,10-dioxo-4-phenylpyrido[2,3-f] [1,4]diazocine

Using the compound obtained in the Reference Example 34, the reaction and treatment were performed as in the case of the Reference Example 104. Then, the title compound was obtained as colorless crystals.
Melting point; 167-169°C (re-crystallization from ethyl acetate- isopropyl ether)
NMR (200MHz, $CDCl_3$) ppm : 2.05 (1H, m), 2.87(1H, m), 3.10(3H, s), 3.36(1H, d, J=14Hz), 3.48(1H, d, J=14Hz), 3.97 (1H, m), 4.26(1H, dd, J=15, 7.1Hz), 7.35 (3H, m), 7.53 (5H, m), 7.71 (1H, s), 8.81(1H, d, J=5.0Hz)
Anal. Calcd for $C_{25}H_{19}N_3O_2F_6$
Calculated (%): C 59.18, H 3.77, N 8.28
Found (%): C 58.90, H 3.81, N 8.05

(Reference Example 106)

6-Benzyl-5,6,7,8,9,10-hexahydro-3,9-dimethyl-5,10-dioxo-4-phenylpyrido[2,3-f][1,4]diazocine

N-Benzyl-N-(2-hydroxyethyl)-5-methyl-2-methylaminocarbonyl-4-phenyl-3-pyridine carboxamide was prepared by reacting and treating as in the process 4 of the Reference Example 33 using the compound obtained in the Reference Example 35 and methylamine. Using this compound, the reaction and treatment were performed as in the case of the Reference Example 104 to give the title compound as colorless crystals.
Melting point; 183 to 184°C (re-crystallization from ethyl acetate- isopropyl ether)
NMR (200MHz, $CDCl_3$) ppm : 1.46(1H, dd, J=15, 8.1Hz), 2.17(3H, s), 2.69(1H, m), 3.02(3H, s), 3.27(1H, d, J=13Hz), 3.44(1H, d, J=13Hz), 3.56(1H, m), 4.00(1H, m), 7.01(2H, m), 7.2-7.6(8H, m), 8.68(1H, s)
Anal. Calcd for $C_{24}H_{23}N_3O_2$
Calculated (%): C 74.78, H 6.01, N 10.90
Found (%): C 74.52, H 6.13, N 10.82

(Reference Example 107)

6-[3,5-bis(Trifluoromethyl)benzyl]-9-ethyl-5,6,7,8,9,10-hexahydro-3-methyl-5, 10-dioxo-4-phenylpyrido[2,3-f][1,4] diazocine

N-[3,5 bis(Trifluoromethyl)benzyl]-2-ethylaminocarbonyl-N-(2-hydroxyethyl)-5-methyl-4-phenyl-3-pyridinecarboxamide was prepared by reacting and treating as in the process 4 of the Reference Example 33 using the compound obtained in the process 3 of the Reference Example 33 and ethyl amine. Using this compound, the reaction and treatment were performed as in the case of the Reference Example 104 to yield the title compound as colorless crystals.
Melting point; 228-229°C (re-crystallization from ethyl acetate- isopropyl ether)
NMR (200MHz, $CDCl_3$) ppm:1.33(3H, t, J=7.0Hz), 1.51(1H, dd, J=15, 7.6Hz), 2.18(3H, s), 2.72(1H, m), 3.39(1H, m), 3.42(1H, d, J=14Hz), 3.57(1H, d, J=14Hz), 3.57(1H, m), 3.77(1H, in), 4.03(1H, dd, J=15, 7.6Hz), 7.2-7.6(5H, m), 7.48 (2H, s), 7.74(1H,s), 8.69(1H, s)
Anal. Calcd for $C_{27}H_{23}N_3O_2F_6$
Calculated (%): C 60.56, H 4.33, N 7.85
Found (%): C 60.28, H 4.51, N 7.65

(Reference Example 108)

6-[3,5 bis(Trifluoromethyl)benzyl]-6,7,8,9,10,11-hexahydro-3,10-dimethyl-5,11-dioxo-4-phenyl-5H-pyrido[2,3-g][1,5] diazonine

Using the compound obtained in the Reference Example 36, the reaction and treatment were performed as in the case of the Reference Example 104. Then, the title compound was obtained as colorless crystals.
Melting point; 247-249°C (re-crystallization from ethyl acetate- isopropyl ether)
NMR (200MHz, $CDCl_3$) ppm:1.2-1.4(1H, m), 1.8-2.3(1H, m), 2.15(3H, s), 3.0-3.6(4H, m), 3.04(3H, s), 3.91(1H, d,

J=15Hz), 5.32(1H, d, J=15Hz), 7.0-7.5(7H, m), 7.75(1H, s), 8.59(1H, s)
Anal. Calcd for $C_{27}H_{23}N_3O_2F_6$
Calculated (%): C 60.56, H 4.33, N 7.85
Found (%): C 60.41, H 4.46, N 7.87
EI-MS m/z: 535 ($M^+$) [$(C_{27}H_{23}N_3O_2F_6)^+$]

(Reference Example 109)

4-[3,5-bis(Trifluoromethyl)benzyl]-8-hydroxymethyl-2,3,4,5-tetrahydro-5-oxo-6-phenylpyrido[3,2-f][1,4]oxazepine

A mixture of 8-acetoxymethyl-4-[3,5 bis(trifluoromethyl)benzyl]-2,3,4,5-tetrahydro-5-oxo-6-phenylpyrido [3,2-f][1,4]oxazepine (Reference Example 91) (4.51 g), ethanol (50 ml) and 4N-NaOH (50 ml) was stirred at room temperature for 1.5 hours. The solvents were evaporated, and water was added to the residue. The pH of this mixture was adjusted to about 8 with diluted hydrochloric acid, and the mixture was extracted with ethyl acetate. The extract solution was washed with water and dried. The solvents were evaporated to give the title compound as colorless crystals (4.10 g).
Melting point; 199-201°C (re-crystallization from ethyl acetate- isopropyl ether)
NMR(200MHz, CDCl3) ppm:3.10(1H, b), 3.71(2H, t, J=5.6Hz), 4.50(2H, t, J=5.6Hz), 4.78(2H, s), 4.88(2H,. s), 7.20-7.50 (6H, m), 7.80(2H, m), 7.87(1H, s)

(Reference Example 110)

4-[3,5-bis(Trifluoromethyl)benzyl]-2,3,4,5-tetrahydro-5-oxo-6-phenylpyrido[3,2-f][1,4]oxazepine-8-carboxylic acid

A mixture of 4-[3,5-bis(trifluoromethyl)benzyl]-8-hydroxymethyl-2,3,4,5-tetrahydro-5-oxo-6-phenylpyrido [3,2-f] [1,4]oxazepine (Reference Example 109) (3.49g), 2N-NaOH (100 ml) and potassium permanganate (2.22g) was stirred at room temperature for 45 hours. A saturated aqueous solution of sodium thiosulfate (10 ml) was added to the reaction mixture, of which pH was adjusted to about 3 with hydrochloric acid, and then the mixture was extracted with ethyl acetate - THF (1:2). The extract solution was washed with saturated brine and dried. The solvents were evaporated to give the title compound as colorless crystals (2.74 g).
Melting point; 199-123°C (re-crystallization from methanol - ethyl ether)
NMR(200MHz, DMSO-$d_6$) ppm:3.94(2H, b), 4.46(2H, b), 4.91(2H, s), 7.25-7.55(5H, m), 7.90(1H, s), 8.06(2H, s), 8.12 (1H, s)

(Reference Example 111)

4-[3,5-bis(Trifluoromethyl)benzyl]-2,3,4,5-tetrahydro-5-oxo-6-phenylpyrido[3,2-f][1,4]oxazepine-8-carboxamide

A mixture of 4-[3,5-bis(trifluoromethyl)benzyl]-2,3,4,5-tetrahydro-5-oxo-6-phenylpyrido[3,2-f][1,4]oxazepine-8-carboxylic acid (Reference Example 110) (220 mg), THF (15 ml), DMF (catalytic amount) and thionyl chloride (0.087 ml) was stirred for 2.5 hours under reflux with heating. The solvents were evaporated, and the residue was dissolved in THF (10 ml). Aqueous ammonia (2 ml) was added to this solution, and this mixture was stirred at room temperature for an hour followed by concentration of the solvents. Water was added to the concentrate, which was extracted with ethyl acetate. The extract solution was washed with water and dried. The solvents were evaporated to give the title compound as colorless crystals (163 mg).
Melting point; 221-222°C (re-crystallization from ethyl acetate- isopropyl ether)
NMR(200MHz, $CDCl_3$) ppm:3.74(2H, t, J=5.6Hz), 4.50(2H, t, J=5.6Hz), 4.92(2H, s), 5.80(1H, b), 7.30-7.55(6H, m), 7.83(2H, s), 7.89(1H, s), 8.20(1H, s)
The compounds of Reference Examples from 112 to 117 were obtained by reacting 4-[3,5 bis(trifluoromethyl) benzyl]-2,3,4,5-tetrahydro-5-oxo-6-phenylpyrido[3,2-f] [1,4]oxazepine-8-carboxylic acid (Reference Example 110) with substituted amines (methyl amine, dimethyl amine, n-butyl amine, piperidine, morpholine, 1-methy piperazine) through acid chloride and treating as in the case of the Reference Example 111. Their physicochemical data are described below.

(Reference Example 112)

4-[3,5-bis(Trifluoromethyl)benzyl]-N-methyl-2,3,4,5-tetrahydro-5-oxo-6-phenylpyrido[3,2-f][1,4]oxazepine-8-carboxamide

Melting point; 145°C (decomposition) (re-crystallization from THF - ethyl ether)
NMR(200MHz, $CDCl_3$) ppm:3.04(3H, d, J=5.2Hz), 3.73(2H, t, J=5.4Hz), 4.48(2H, t, J=5.4Hz), 4.09(2H, s), 7.25-7.60 (5H, m), 7.65-7.95(1H, b), 7.81(2H, s), 7.87(1H, s), 8.17(1H, s)

(Reference Example 113)

4-[3,5-bis(Trifluoromethyl)benzyl]-N,N-dimethyl-2,3,4,5-tetrahydro-5-oxo-6-phenylpyrido[3,2-f][1,4]oxazepine-8-carboxamide

Melting point; 235-236°C (re-crystallization from ethyl acetate- isopropyl ether)
NMR(200MHz, $CDCl_3$) ppm:3.11(3H, s), 3.15(3H, s), 3.72(2H, t, J=5.6Hz), 4.47(2H, t, J=5.6Hz), 4.90(2H, s), 7.25-7.50 (5H, m), 7.60(1H, s), 7.82(2H, s), 7.88(1H, s)

(Reference Example 114)

4-[3,5-bis(Trifluoromethyl)benzyl]-N-n-butyl-2,3,4,5-tetrahydro-5-oxo-6-phenylpyrido[3,2-f] [1,4]oxazepine-8-carboxamide

Melting point; 194-196°C (re-crystallization from ethyl acetate- isopropyl ether)
NMR(200MHz, $CDCl_3$) ppm : 0.96(3H, t, J=7.2Hz), 1.20-1.80(6H, m), 4.78(2H, m), 3.73(2H, t, J=5.6Hz), 4.49(2H, t, J=5.6Hz), 4.91(2H, s), 7.30-7.58(5H, m), 7.82(2H, s), 7.88(1H, s), 8.18(1H, s)

(Reference Example 115)

4-[3,5 bis (Trifluoromethyl) benzyl)-2,3,4,5-tetrahydro-5-oxo-6-phenyl-8-piperidinocarbonylpyrido[3,2-f][1,4]oxazepine

Melting point; 218-220°C (re-crystallization from THF - isopropyl ether)
NMR(200MHz, $CDCl_3$) ppm:1.60(2H, b), 1.69(4H, b), 3.44(2H, t, J=5.6Hz), 3.72(4H, m), 4.46(2H, t, J=5.6Hz), 4.89 (2H, s), 7.20-7.60(6H, m), 7.81(2H, s), 7.87(1H, s)

(Reference Example 116)

4-[3,5-bis(Trifluoromethyl)benzyl]-2,3,4,5-tetrahydro-8-morpholino carbonyl-5-oxo-6-phenylpyrido[3,2-f][1,4] oxazepine

Melting point; 265-266°C (re-crystallization from ethyl acetate- isopropyl ether)
NMR(200MHz, $CDCl_3$) ppm:3.55-3.90(10H, m), 4.46(2H, t, J=5.6Hz), 4.89(2H, s), 7.25-7.52(5H, m), 7.59(1H, s), 7.81 (2H, s), 7.88(1H, s)

(Reference Example 117)

4-[3,5-bis(Trifluoromethyl)benzyl]-2,3,4,5-tetrahydro-8-[1-(4-methylpiperazinyl)carbonyl]-5-oxo-6-phenylpyrido[3,2-f] [1,4]oxazepine

Melting point; 196-198°C (re-crystallization from THF - isopropyl ether)
NMR (200MHz, $CDCl_3$) ppm: 2.35(3H, s), 2.45(2H, m), 2.54(2H, m), 3.61(2H, m), 3.72(2H, t, J=5.6Hz), 3.84(2H, m), 4.46(2H, t, J=5.6Hz), 4.89(2H, s), 7.25-7.50(5H, m), 7.54(1H, s), 7.81(2H, s), 7.88(1H, s)

The compounds of the Reference Examples from 118 to 126 were obtained as colorless crystals by reacting (cyclizing reaction in THF in the presence of sodium hydride) and treating substantially as in the case of the Reference Example 65 using the compounds in the Reference Examples from 37 to 45, respectively. Each physicochemical data are shown below.

(Reference Example 118)

2,3,4,5-Tetrahydro-5-oxo-6-phenyl-4-(3,4,5-trimethoxybenzyl)pyrido[3,2-f][1,4]oxazepine

Melting point; 177-179°C (re-crystallization from acetone - ethyl ether)
NMR(200MHz, $CDCl_3$) ppm : 3.70(2H, t, J=5.6Hz), 3.85(6H,s), 3.87(3H, s), 4.34(2H, t, J=5.6Hz), 4.72(2H, s), 6.60(2H, s), 7.24(1H, d, J=5.2Hz), 7.30-7.55(5H, m), 8.42(1H, d, J=5.2Hz)

(Reference Example 119)

4-(3,4-Dichlorobenzyl)-2,3,4,5-tetrahydro-5-oxo-6-phenylpyrido[3,2-f] [1,4]oxazepine

Melting point; 189-192°C (re-crystallization from THF - ethyl ether)
NMR(200MHz, $CDCl_3$) ppm:3.67(2H, t, J=5.4Hz), 4.42(2H, t, J=5.4Hz), 4.71(2H, s), 7.10-7.70(9H, m), 8.43 (1H, d, J=5.2Hz)

(Reference Example 120)

4-(3,4-Dimethoxybenzyl) -2,3,4,5-tetrahydro-5-oxo-6-phenylpyrido[3,2-f][1,4]oxazepine

Melting point; 175-176°C (re-crystallization from THF - ethyl ether)
NMR(200MHz, $CDCl_3$) ppm:3.67(2H, t, J=5.4Hz), 3.85(3H, s), 3.91(3H, s), 4.29(2H, t, J=5.4Hz), 4.72(2H, s), 6.80-7.00 (3H, m), 7.22(1H, d, J=5.2Hz), 7.30-7.50(5H, m), 8.40(1H, d, J=5.2Hz)

(Reference Example 121)

4-Benzyl-2,3,4,5-tetrahydro-5-oxo-6-phenylpyrido[3,2-f][1,4]oxazepine

Melting point; 209-211°C (re-crystallization from methanol - ethyl ether)
NMR (200MHz, $CDCl_3$) ppm:3.64(2H, t, J=5.6Hz), 4.33(2H, t, J=5.6Hz), 4.77(2H, s), 7.22(1H, d, J=5.2Hz), 7.30-7.55 (5H, m), 8.39(1H, d, J=5.2Hz)

(Reference Example 122)

2,3,4,5-Tetrahydro-6-oxo-7-phenyl-5-(3,4,5-trimethoxybenzyl)-6H-pyrido[2,3-b][1,5]oxazosine

Melting point; 155-156°C (re-crystallization from ethyl acetate - ethyl ether)
NMR(200MHz, $CDCl_3$) ppm:1.65-1.85(1H, m), 2.29(1H, m), 3.40-3.75(2H, m), 3.77(6H, s), 3.87(3H, s), 4.07(1H, d, J=14.2Hz), 4.27(1H, m), 4.66(1H, m), 5.22(1H, d, J=14.2Hz), 6.53(2H, s), 7.15(1H, d, J=5.2Hz), 7.35(5H, m), 8.40(1H, d, J=5.2Hz)

(Reference Example 123)

(S)-5-Benzyl-2,3,4,5-tetrahydro-3-methyl-6-oxo-7-phenyl-6H-pyrido[2,3-b][1,5]oxazosine

Melting point; 139-141°C (re-crystallization from ethyl acetate - isopropyl ether)
NMR(200MHz, $CDCl_3$) ppm:0.84(3H, d, J=7.0Hz), 2.43(1H, m), 3.12(1H, d, J=14.8Hz), 3.39(1H, dd, J=15.4, 10.2Hz), 3.72-4.00(2H, m), 4.60(1H, dd, J=12.4, 5.2Hz), 5.51(1H, d, J=14.8Hz), 7.16(1H, d, J=5.0Hz), 7.20-7.50(10H, m), 8.39 (1H,d d, J=5.0Hz)

(Reference Example 124)

(R)-5-Benzyl-2,3,4,5-tetrahydro-3-methyl-6-oxo-7-phenyl-6H-pyrido[2,3-b] [1,5]oxazosine

Melting point; 139-141°C (re-crystallization from ethyl acetate - isopropyl ether)
NMR (200MHz, $CDCl_3$) ppm: Identical to the spectra of the compound of the Reference Example 123.

(Reference Example 125)

(S)-5-[3,5-bis(Trifluoromethyl)benzyl]-2,3,4,5-tetrahydro-3-methyl-6-oxo-7-phenyl-6H-pyrido[2,3-b][1,5]oxazosine

Melting point; 142-143°C (re-crystallization from ethyl acetate - isopropyl ether)
NMR(200MHz, $CDCl_3$) ppm:0.87(3H, d, J=7.0Hz), 2.46(1H, m), 3.10(1H, d, J=15.4Hz), 3.59(1H, dd, J=15.0, 10.6Hz), 3.92(1H, dd,. J=12.6, 10.4Hz),. 4.20(1H, d, J=15.4Hz), 4.63(1H, dd, J=12.6, 5.2Hz), 5.50(1H, d, J=15.4Hz), 7.18(1H, d, J=5.0Hz), 7.20-7.50(5H, m), 7.72(2H, s), 7.84(1H, s), 8.43(1H, d, J=5.0Hz)

(Reference Example 126)

(R)-5-[3,5-bis(Trifluoromethyl)benzyl]-2,3,4,5-tetrahydro-3-methyl-6-oxo-7-phenyl-6H-pyrido[2,3-b][1,5]oxazosine

Melting point; 142-143°C (re-crystallization from ethyl acetate - isopropyl ether)
NMR (200MHz, $CDCl_3$) ppm: Identical to the spectra of the compound of the Reference Example 125.

(Reference Example 127)

7-Benzyl-6,7,8,9,10,11-hexahydro-5-(4-methylphenyl)-6,13-dioxo-13H-[1,4]diazocino[2,1-g] [1,7]naphthylidine

Using the compound obtained in the Reference Example 46, the reaction and treatment were performed as in the case of the Reference Example 69. Then, the title compound was obtained as colorless crystals.
Melting point; 239-241°C (re-crystallization from ethyl acetate- isopropyl ether)
NMR (200MHz, $CDCl_3$) ppm : 1.6-2.1(4H, m), 2.50(3H, s), 3.14(1H, dd, J=15, 3.8Hz), 3.3-3.7(2H, m), 3.77(1H, d, J=15Hz), 5.14(1H, dd, J=14, 5.9Hz), 5.42(1H, d, J=15Hz), 6.67(2H, d, J=7.0Hz), 6.92(1H, dd, J=7.6, 1.8Hz), 7.1-7.5 (6H, m), 7.46(1H, dd, J=8.4, 4.4Hz), 7.60(1H, dd, J=8.4, 1.8Hz), 8.90(1H, dd, J=4.4, 1.8Hz)

(Reference Example 128)

(9R)-7-Benzyl-6,7,8,9,10,11-hexahydro-9-methyl-5-(4-methylphenyl)-6,13-dioxo-13H-[1,4]diazocino[2,1-g] [1,7] naphthylidine

Using the compound obtained in the Reference Example 47, the reaction and treatment were performed as in the case of the Reference Example 69. Then, the title compound was obtained as colorless crystals.
Melting point; 218-220°C (re-crystallization from ethyl acetate- isopropyl ether)
NMR (200MHz, $CDCl_3$) ppm:0.85(3H, d, J=7.0Hz), 1.50-1.75(1H, m), 1.90-2.35(2H, m), 2.50(3H, s), 2.89(1H, d, J=15Hz), 3.26(1H, dd, J=14, 10Hz), 3.59(1H, dd, J=14, 11Hz), 3.75(1H, d, J=15Hz), 5.10(1H, dd, J=14, 6.1Hz), 5.42 (1H, d, J=15hz), 6.69(2H, d, J=6.8Hz), 6.91(1H, dd, J=7.8, 1.8Hz), 7.1-7.5(6H, m), 7.46(1H, dd, J=8.4, 4.2Hz), 7.60 (1H, dd, J=8.4, 1.8Hz), 8.90(1H, dd, J=4.2, 1.8Hz)

(Reference Example 129)

(9S)-7-Benzyl-6,7,8,9,10,11-hexahydro-9-methyl-5-(4-methylphenyl)-6,13-dioxo-13H-[1,4] diazocino[2,1-g][1,7] naphthylidine

Using the compound obtained in the Reference Example 47, the reaction and treatment were performed as in the case of the Reference Example 69. Then, the title compound was obtained as colorless crystals.
Melting point; 218-220°C (re-crystallization from ethyl acetate- isopropyl ether)
NMR (200MHz, $CDCl_3$) ppm: Identical to the spectra of the compound of the Reference Example 128.

(Reference Example 130)

(9R)-6,7,8,9,10,11-Hexahydro-9-methyl-5-(4-methylphenyl)-6,13-dioxo-7-(3,4,5-trimethoxybenzyl)-13H-[1,4] diazocino [2,1-g][1,7]naphthylidine

Using the compound obtained in the Reference Example 49, the reaction and treatment were performed as in the case of the Reference Example 69. Then, the title compound was obtained as colorless crystals.
White powder

NMR (200MHz, $CDCl_3$) ppm : 0.90(3H, d, J=6.6Hz), 1.5-1.8(1H, m), 1.9-2.5(2H, m), 2.41(3H, s), 3.11(1H, d, J=15Hz), 3.35(1H, dd, J=15, 11Hz), 3.56(1H, dd, J=14, 11Hz), 3.7-3.9(1H, m), 3.75(6H, s), 3.87(3H, s), 5.07(1H, dd, J=14, 5.9Hz), 5.19(1H, d, J=15Hz), 6.30(2H, s), 6.77(1H, d, J=8.0Hz), 6.97(1H, d, J=8.0Hz), 7.29(1H, d, J=8.2Hz), 7.37(1H, d, J=8.2Hz), 7.45(1H, dd, J=8.4, 4.0Hz), 7.58(1H, dd, J=8.4, 1.4Hz), 8.89(1H, dd, J=4.0, 1.4Hz)

(Reference Example 131)

(9S)-6,7,8,9,10,11-Hexahydro-9-methyl-5-(4-methylphenyl)-6,13-dioxo-7-(3,4,5-trimethoxybenzyl)-13H-[1,4]diazocino[2,1-g][1,7]naphthylidine

Using the compound obtained in the Reference Example 50, the reaction and treatment were performed as in the case of the Reference Example 69. Then, the title compound was obtained as colorless crystals.
White powder
NMR (200MHz, $CDCl_3$) ppm: Identical to the spectra of the compound of the Reference Example 130.

(Reference Example 132)

(9R)-7-(3,5-Dimethoxybenzyl)-6,7,8,9,10,11-hexahydro-9-methyl-5-(4-methylphenyl)-6,13-dioxo-13H-[1,4]diazocino[2,1-g][1,7]naphthylidine

Using the compound obtained in the Reference Example 51, the reaction and treatment were performed as in the case of the Reference Example 69. Then, the title compound was obtained as colorless crystals.
Melting point; 206-208°C (re-crystallization from ethanol - isopropyl ether)
NMR (200MHz, $CDCl_3$) ppm : 0.87 (3H, d, J=7.0Hz), 1.67(1H, m), 1.9-2.4(2H, m), 2.42(3H, s), 3.05(1H, d, J=15Hz), 3.24-3.40(1H, m), 3.45-3.85(2H, m), 3.74(6H, s), 5.08(1H, dd, J=14, 5.8Hz), 5.26 (1H, d, J=14Hz), 6.12(2H,d, J=2.0Hz), 6.38 (1H, t, J=2.0Hz), 6.84 (1H, d, J=7.0Hz), 7.09 (1H, d, J=7.0Hz), 7.29 (1H, d, J=9.2Hz), 7.38 (1H, d, J=9.2Hz), 7.46 (1H, dd, J=8.2, 4.2Hz), 7.62 (1H, dd, J=8.2, 1.6Hz), 8.89 (1H, dd, J=4.2, 1.6Hz)

The compounds in the Reference Examples from 133 to 136 were obtained as colorless crystals by reacting (cyclizing reaction in THF in the presence of sodium hydride) and treating substantially as in the case of the Reference Example 65 using the compounds in the Reference Examples from 52 to 54 and 144, respectively. Each physicochemical data are shown below.

(Reference Example 133)

4-Benzyl-2,3,4,5-tetrahydro-5-oxo-6-(4-methylphenyl)pyrido[3,2-f][1,4]oxazepine

Melting point; 203-204°C (re-crystallization from methanol - ethyl ether)
NMR(200MHz, $CDCl_3$)ppm:2.41(3H, s), 3.64(2H, t, J=5.4Hz), 4.32(2H, t, J=5.4Hz), 4.78(2H, s), 7.21(1H, d, J=5.2Hz), 7.25(4H, s), 7.38(5H, s), 8.37(1H, d, J=5.2Hz)

(Reference Example 134)

4-[3,5-bis(Trifluoromethyl)benzyl]-2,3,4,5-tetrahydro-5-oxo-6-(4-methylphenyl)pyrido[3,2-f][1,4]oxazepine

Melting point; 212-213°C (re-crystallization from acetone - isopropyl ether)
NMR(200MHz, $CDCl_3$)ppm:2.40(3H, s), 3.70(2H, t, J=5.6Hz), 4.47(2H, t, J=5.6Hz), 4.89(2H, s), 7.24(total 5H, m), 7.81 (2H, s), 7.87(1H, s), 8.41(1H, d, J-5.2Hz)

(Reference Example 135)

(S)-5-Benzyl-2,3,4,5-tetrahydro-3-methyl-7-(4-methylphenyl)-6-oxo-6H-pyrido[2,3-b][1,5]oxazosine

Melting point; 148-149°C (re-crystallization from acetone - ethyl ether)
NMR (200MHz, $CDCl_3$) ppm : 0.83(3H, d, J=7.4Hz), 2.30-2.60 (1H, b), 2.42(3H, s), 3.11(1H, d, J=15.4Hz), 3.40(1H, dd, J=15.4, 10.4Hz), 3.75-4.00(2H, m), 4.59(1H, dd, J=12.4, 4.8Hz), 5.50(1H, d, J=15.0Hz), 7.15(1H, d, J=4.8Hz), 7.20-7.40(total 9H, m), 8.37(1H, d, J=4.8Hz)

(Reference Example 136)

(S)-5-[3, 5-bis(Trifluoromethyl)benzyl]-2,3,4,5-tetrahydro-3-methyl-7-(4-methylphenyl)-6-oxo-6H-pyrido[2,3-b][1,5] oxazosine

Melting point; 159-160°C (re-crystallization from acetone - isopropyl ether)
NMR (200MHz, $CDCl_3$) ppm: 0.86(3H, d, J=7.0Hz), 2.20-2.60 (1H, b), 2.37(3H, s), 3.09(1H, d, J=15.4Hz), 3.58(1H, dd, J=15.4, 10.4Hz), 3.89(1H, t, J=11.6Hz), 4.18(1H, d, J=15.4Hz), 4.62(1H, dd, J=12.2, 5.2Hz), 5.53(1H, d, J=15.4Hz), 7.17(total 5H, m), 7.72(2H, s), 7.84(1H, s), 8.40(1H, d, J=5.2Hz)

(Reference Example 137)

(9R)-7-[3,5-bis(Trifluoromethyl)benzyl]-6,7,8,9,10,11-hexahydro-5-(4-hydroxymethylphenyl) -9-methyl-6,13-dioxo-13H-[1,4]diazocino[2,1-g][1,7]naphthylidine

Streptomyces subrutilus IFO 13388 was cultured at 28°C for 14 days on agarose slants at pH 7.3 containing 0.4 % yeast extract, 1 % wheat germ extract, 0.4 % glucose and 2 % agarose (ISP medium No.2). The other medium containing 0.5 % glucose, 5 % dextrin, 3.5 % soy bean meal and 0.7 % calcium carbonate was prepared. Its 40 ml was placed into Erlenmeyer flask of 200 ml and autoclaved at 120°C for 20 min. One platinum loop of said cultured bacterial cells was inoculated into this medium and cultured with shaking at 28°C for 48 hours. The obtained cultured medium was dispensed by 1 ml into 13 flasks containing the same medium and cultured with shaking at 28°C for 48 hours. Each 0.4 ml of a solution of (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g] [1,7]naphthylidine (104 mg) obtained in the Reference Example 72 in DMSO (5.2 ml) was added to this cultured medium, and reacted with shaking at 28°C for 48 hours. After reaction, the medium was adjusted to pH 4 with 2N-sulfuric acid and extracted with 500 ml of ethyl acetate.
The extract solution was concentrated and then purified by column chromatography on silica gel (ethyl acetate - methanol = 9:1) to give the title compound as colorless crystals (30 mg) Melting point; 240-241°C (re-crystallization from ethyl acetate - ethyl ether)
NMR (200 MHz, $CDCl_3$) ppm: 0.91 (3H, d, J= 6.6Hz), 1.5-1.9 (2H, m), 1.95-2.40 (2H, m), 2.98 (1H, d, J=15 Hz), 3.35-3.65 (2H, m), 4.02 (1H, d, J = 15 Hz), 4.75 (2H, s), 5.10 (1H, dd, J = 15, 5.5 Hz), 5.46 (1H, d, J=15 Hz), 6.97 (1H, d, J = 8.0 Hz), 7.26 (1H, d, J = 8.0 Hz), 7.4-7.6 (6H, m), 7.81 (1H, s), 8.93 (1H, dd, J = 4.0 and 1.8 Hz)
Anal. Calcd for $C_{30}H_{25}N_3O_3F_6$
Calculated (%) : C 60.12, H 4.27, N 7.13
Found (%): C 61.15, H 4.21, N 7.03
EI-MS m/z: 589 ($M^+$) [$(C_{30}H_{25}N_3O_3F_6)^+$]

(Reference Example 138)

(9R)-7-[3,5-bis(Trifluoromethyl)benzyl]-5-(4-formylphenyl)-6,7,8,9,10,11-hexahydro-9-methyl-6,13-dioxo-13H-[1,4] diazocino[2,1-g][1,7]naphthylidine

Streptomyces tanashiensis subsp. cephalomyceticus IFO 13929 was cultured at 28°C for 14 days on agarose slants at pH 7.3 containing 0.4% yeast extract, 1 % wheat germ extract, 0.4 % glucose and 2 % agarose (ISP medium No.2). The other medium containing 0.5 % glucose, 5 % dextrin, 3.5 % soy bean meal and 0.7 % calcium carbonate was prepared. Its 40 ml was placed into Erlenmeyer flask of 200 ml and autoclaved at 120°C for 20 min. One platinum loop of said cultured bacterial cells was inoculated into this medium and cultured with shaking at 28°C for 48 hours. The obtained cultured medium was dispensed by 1 ml into 25 flasks containing the same medium and cultured with shaking at 28°C for 48 hours. Each 0.4 ml of a solution of (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthylidine (200 mg) obtained in the Reference Example 72 in DMSO (10 ml) was added to this cultured medium, and reacted with shaking at 28°C for 48 hours. After reaction, the medium was adjusted to pH 4 with 2N-sulfuric acid and extracted with 1 L of ethyl acetate.
The extract solution was concentrated and then purified by column chromatography on silica gel (ethyl acetate - methanol = 9:1) to give the title compound as colorless crystal. The structure of this product was determined by comparing NMR spectra with those of the compound prepared in the Reference Example 139.

(Reference Example 139)

(9R)-7-[3,5-bis(Trifluoromethyl)benzyl]-5-(4-formylphenyl)-6,7,8,9,10,11-hexahydro-9-methyl-6,13-dioxo-13H-[1,4] diazocino[2,1-g][1,7]naphthylidine

A mixture of (9R)-7-[3,5-bis(Trifluoromethyl)benzyl]-6,7,8,9,10,11-hexahydro-5-(4-hydroxymethylphenyl)-9-methyl-6,13-dioxo-13H-[1,4]diazocino[2,1-g][1,7]naphthylidine (109 mg) obtained in the Reference Example 137, manganese dioxide (1.3 g) and dichloromethane (13 ml) was stirred at room temperature for 2 hours, and then the precipitate was filtrated by using Celite. The filtrate was concentrated to give the title compound as colorless crystals (70 mg).
Melting point; 213-214°C (re-crystallization from ethyl acetate - ethyl ether)
NMR (200 MHz, $CDCl_3$) ppm: 0.95 (3H, d, J = 6.6 Hz), 1.60-1.85 (1H, m) ,1.95-2.40 (2H, m), 3.05 (1H, d, J = 15 Hz), 3.35-3.65 (2H, m), 4.00 (1H, d, J = 15 Hz), 5.11 (1H, dd, J=14, 5.7 Hz), 5.38 (1H, d, J = 15 Hz), 7.12 (1H, d ,J = 8.4 Hz), 7.40-7.55 (4H, m), 7.60-7.75 (2H, m), 7.78 (1H, m), 7.96 (1H, d, J = 8.0 Hz), 8.94 (1H, m), 10.03 (1H, s)
Anal. Calcd for $C_{30}H_{23}N_3O_3F_6$
Calculated (%): C 61.33, H 3.95, N 7.15
Found (%) : C 61.25, H 4.04, N 7.11
EI-MS m/z: 587 ($M^+$) [$(C_{30}H_{23}N_3O_3F_6)^+$]

(Reference Example 140)

(9R)-7-[3,5-bis(Trifluoromethyl)benzyl]-5-(4-carboxyphenyl)-6,7,8,9,10,11-hexahydro-9-methyl-6,13-dioxo-13H-[1,4] diazocino[2,1-g][1,7]naphthylidine

Streptomyces lavenduligriseus IFO 13405 was cultured at 28°C for 14 days on agarose slants at pH 7.3 containing 0.4 % yeast extract, 1 % wheat germ extract, 0.4 % glucose and 2 % agarose (ISP medium No.2). The other medium containing 0.5 % glucose, 5 % dextrin, 3.5 % soy bean meal and 0.7 % calcium carbonate was prepared. Its 40 ml was placed into Erlenmeyer flask of 200 ml and autoclaved at 120°C for 20 min. One platinum loop of said cultured bacterial cells was inoculated into this medium and cultured with shaking at 28°C for 48 hours. The obtained cultured medium was dispensed by 1 ml into 25 flasks containing the same medium and cultured with shaking at 28°C for 48 hours. Each 0.4 ml of a solution of (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthylidine (200 mg) in DMSO (10 ml) obtained in the Reference Example 72 was added to this cultured medium, and reacted with shaking at 28°C for 48 hours. After reaction, the medium was adjusted to pH 4 with 2N-sulfuric acid and extracted with 1 L of ethyl acetate.

The extract solution was concentrated and the concentrate was extracted with 1N sodium hydroxide solution. The extract solution was adjusted to pH 3 to 4 and extracted with ethyl acetate. The extract solution was washed with saturated brine and dried followed by evaporation of the solvents. The residue was purified by column chromatography on silica gel (ethyl acetate - methanol = 9:1) to give the title compound as colorless crystals (58 mg).
Melting point; 300-301°C (re-crystallization from ethyl acetate - ethyl ether)
NMR (200 MHz, $CDCl_3$) ppm: 0.96 (3H, d, J = 6.6 Hz), 1.76 (1H, m), 1.95-2.40 (2H, m), 3.09 (1H, d, J = 15 Hz), 3.40-3.65 (2H, m), 4.02 (1H, d, J = 15 Hz), 5.12 (1H, dd, J = 14, 5.6 Hz), 5.36 (1H, d, J = 15 Hz), 7.04 (1H, dd, J = 8.0, 1.6 Hz), 7.45-7.65 (5H, m), 7.83 (1H, s), 7.91 (1H, d, J = 8.4 Hz), 8.20 (1H, dd, J = 8.4, 1.8 Hz), 8.97 (1H, dd, J = 3.6, 2.2 Hz)
EI-MS m/z: 603 ($M^+$) [$(C_{30}H_{23}N_3O_4F_6)^+$]

(Reference Example 141)

(9R)-7-[3,5-bis(Trifluoromethyl)benzyl]-6,7,8,9,10,11-hexahydro-9-methyl-5-(4-methylphenyl)-6,13-dioxo-13H-[1,4] diazocino[2,1-g][1,7]naphthylidine N-oxide

A mixture of (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7] naphthylidine (1.60 g) obtained in the Reference Example 72, m-chloro perbenzoic acid (1.2 g) and dichloromethane (20 ml) was stirred at room temperature for 3 hours, and then the solvent was evaporated. The residue was dissolved in ethyl acetate and washed with an aqueous solution of potassium carbonate and saturated brine followed by drying. The solvents were evaporated to give the title compound as pale yellow crystals (0.73 g).
Melting point; 237-239°C (re-crystallization from ethyl acetate - ethyl ether)
NMR (200MHz, $CDCl_3$) ppm: 0.91 (3H, d, J = 6.6 Hz), 1.73 (1H, m), 1.9-2.4 (2H, m), 2.36 (3H, s), 2.97 (1H, d, J=15

Hz), 3.3-3.5 (2H, m), 3.97 (1H, d, J = 15 Hz), 4.97 (1H, dd, J = 14, 5.2 Hz), 5.41 (1H, d, J = 15 Hz), 6.82 (2H, m), 7.04 (1H, d, J = 7.4 Hz), 7.15-7.35 (3H, m), 7.44 (2H, s), 7.81 (1H, s), 8.31 (1H, dd, J = 5.4, 0.8 Hz)
Anal. Calcd for $C_{30}H_{25}N_3O_3F_6$
Calculated (%): C 61.12, H 4.27, N 7.13
Found (%) : C 60.77, H 4.55, N 7.00

(Reference Example 142)

(9R)-7-[3,5-bis(Trifluoromethyl)benzyl]-6,7,8,9,10,11-hexahydro-5-(4-hydroxymethylphenyl)-9-methyl-6,13-dioxo-13H-[1,4]diazocino[2,1-g][1,7]naphthylidine N-oxide

A mixture of (9R)-7-[3,5-bis(trifluoromethyl)benzyl]-6,7,8,9,10,11-hexahydro-5-(4-hydroxymethylphenyl)-9-methyl-6,13-dioxo-13H-[1,4]diazocino[2,1-g][1,7]naphthylidine (100 mg) obtained in the Reference Example 137, 4-dimethyl aminopyridine (catalytic amount), acetic anhydride (0.1ml) and pyridine (3 ml) was stirred at room temperature for 16 hours, and then the solvents were evaporated. Ethyl acetate was added to the residue, which was then washed with diluted hydrochloric acid and saturated brine followed by drying. The solvents were evaporated and the residue was dissolved in dichloromethane (10 ml). m-Chloro perbenzoic acid (102 mg) was added to this solution, which was stirred at room temperature for an hour. The reaction solution was diluted with dichloromethane, and then washed with 0.1 N sodium hydroxide solution followed by drying. The solvents were evaporated. Methanol (10 ml) and 1N sodium hydroxide solution (2 ml) were added to the residue. After stirring at room temperature for 30 min, water was added to the mixture, which was extracted with ethyl acetate. The extract solution was washed with saturated brine and dried. The solvents were evaporated to give the title compound as pale yellow crystals (28 mg).
Melting point; 240-243°C (re-crystallization from ethyl acetate - ethyl ether)
NMR (200 MHz, $CDCl_3$) ppm: 0.91 (3H, d, J= 6.2 Hz), 1.5-2.4 (4H, m), 2.98 (1H, d, J = 15 HZ), 3.44 (2H, m), 4.00 (1H, d, J = 15 Hz), 4.74 (2H, s), 4.98 (1H, m), 5.40 (1H, d, J = 15 Hz), 6.88 (1H, m), 7.26 (1H, d, J = 7.4 Hz), 7.45 (6H, m), 7.81 (1H, s), 8.32 (1H, m)

(Reference Example 143)

(9R)-7-[3,5-bis(Trifluoromethyl)benzyl]-5-(4-carboxyphenyl)-6,7,8,9,10,11-hexahydro-9-methyl-6,13-dioxo-13H-[1,4]diazocino[2,1-g][1,7]naphthylidine N-oxide

Ethyl ether (10 ml) of diazomethane (prepared using N-nitroso methylurea and potassium hydroxide) was added to a solution of (9R)-7-[3,5-bis(trifluoromethyl)benzyl]-5-(4-carboxyphenyl)-6,7,8,9,10,11-hexahydro-9-methyl-6,13-dioxo-13H-[1,4]diazocino[2,1-g][1,7]naphthylidine (200 mg) obtained in the Reference Example 140 in THF (20 ml) and stirred at room temperature for 30 min. After evaporating the solvents, the residue was dissolved in dichloromethane (20 ml). m-Chloro perbenzoic acid (0.6 g) was added to this solution by a small portion and the mixture was stirred at room temperature for 2 hours. The reaction solution was diluted with dichloromethane and washed with 1N sodium hydroxide solution and water followed by drying. The solvents were evaporated. Methanol (20 ml) and 1N sodium hydroxide solution (5 ml) were added to the residue, which was refluxed with heating for 30 min. After evaporating the solvents, water was added to the residue, which was then, adjusted to pH 2 to 3 using diluted hydrochloric acid. This solution was extracted with ethyl acetate, and the extract was washed with saturated brine followed drying. The solvents were evaporated to give the title compound as pale yellow crystals (88 mg).
NMR (200 MHz, $CDCl_3$) ppm: 0.95 (3H, d, J = 6.6 Hz), 1.75 (1H, m), 1.9-2.4 (2H, m), 3.08 (1H, d, J = 15 Hz), 3.47 (2H, m), 4.00 (1H, d, J = 15 Hz), 5.00 (1H, dd, J = 14, 6.0 Hz), 5.29 (1H, d, J = 15 Hz), 6.80 (1H, d, J = 8.4 Hz), 7.02 (1H, d, J = 7.3 Hz), 7.28 (1H, dd, J = 7.0, 6.6 Hz), 7.46 (2H, s), 7.54 (1H, d, J = 7.0 Hz), 7.81 (1H, s), 7.88 (1H, d, J = 7.3 Hz), 8.16 (1H, d, J = 8.4 Hz), 8.39 (1H, d, J = 6.6 Hz)

(Reference Example 144)

N-[3,5-bis(Trifluoromethyl)benzyl]-2-chloro-N-[(S)-3-hydroxy-2-methylpropyl]-4-(4-methylphenyl)-3-pyridine carboxamide

NMR(200MHz, $CDCl_3$) ppm : 0. 54 (3H×1/4, d, J=7.0Hz), 0.63 (3H×1/4, d, J=7.0Hz), 0.79(3H×1/4, d, J=7.0Hz), 0.84(3H×1/4, d, J=7.0Hz), 1.50-1.90(1H, m), 2.25-2.45(3H, m), 2.45-3.90(total 5H, m), 4.05-4.45(1H, m), 4.50-4.95(1H, m), 7.00-7.20(1H, m), 7.20-7.50(total 5H, m), 7.70-7.85(2H, m), 8.42(1H, m)
(Mixture of amide rotational isomers 1:1)

(Reference Example 145)

(9R)-[10,10,11,11,$^2H_4$]-7-[3,5-bis(Trifluoromethyl)benzyl]-8,9,10,11-tetrahydro-9-methyl-5-(4-methylphenyl)-7H-[1,4] diazocino[2,1-g][1,7]naphthylidine-6,13-dion ($d_4$ isomer of the compound in the Reference Example 72)

(Process 1)

Ethyl ether (60 ml) solution of methyl (S)-(+)-3-hydroxy-2-methylpropionate THP ether (39.5 g) (prepared according to the method described in a reference "Kenji Mori, Tetrahedron Vol. 39: p3107-p3109 (1983) : a synthetic method for enantiomeric isomers is described") was gradually added to a suspension of lithium aluminium deuteride (5.80 g) in ethyl ether (200 ml) at 0°C with vigorously stirring. Then, the reaction mixture was stirred at room temperature for an hour, and then cooled again with ice-water. A mixture of 1N sodium hydroxide solution (24 ml) and THF (24 ml) was added with stirring. The precipitate was filtrated by using Celite. The filtrate was dried and then the solvents were evaporated to give (S)-[1,1-$^2H_2$]-2-methyl-1,3-propanediol mono THP ether as a colorless oil (35.6 g).
NMR(200MHz, $CDCl_3$)ppm : 0.90(3H×1/2, d, J=7.0 Hz), 0.91(3H× 1/2, d, J=7.0 Hz), 1.4-1.9 (6H, m), 2.01 (1H, m), 2.74 (1H, bs), 3.3-3.6 (3H, m), 3.89 (1H, m), 4.58 (1H, b)

(Process 2)

p-Toluene sulfonyl chloride (39 g) was added to a solution of the compound (35.6 g) obtained in the process 1 in pyridine (150 ml) with stirring under cooling. This mixture was stirred at room temperature overnight, diluted with ethyl ether and washed with water, diluted hydrochloric acid and brine followed by drying. The solvents were evaporated. The residue was dissolved in DMSO (150 ml), sodium cyanide (13 g) was added thereto, and the mixture was stirred at room temperature for 6 hours. The mixture was diluted with hexane, washed with water, and dried. Then the solvents were evaporated to yield (R)-[2,2-$^2H_2$]-4-hydroxy-3-methylbutanenitrile THP ether as a pale yellow oil (19.1 g)
NMR(200MHz, $CDCl_3$) ppm : 1.09 (3H×1/2, d, J=6 Hz), 1.10 (3H× 1/2, d, J=6.8 Hz), 1.5-1.9 (6H, m), 2.16 (1H, m), 3.16-3.88 (4H, m), 4.60 (1H, b)

(Process 3)

A solution of the compound (19.1 g) obtained in the process 2 in ethyl ether (80 ml) was gradually added to a suspension of lithium aluminium deuteride (4.85 g) in ethyl ether (200 ml) at 0°C with vigorously stirring. Then, the reaction mixture was stirred at room temperature for an hour, and then cooled again with ice-water. A mixture of 1N sodium hydroxide solution (20 ml) and THF (20 ml) was added with stirring. The precipitate was filtrated by using Celite. The filtrate was dried and then the solvents were evaporated to give (R)-[3,3,4,4-$^2H_4$]-4-amino-2-methyl-1-butanol THP ether as a pale yellow oil (19.3 g).
NMR(200MHz, $CDCl_3$)ppm: 0.93 (3H×1/2, d, J=6.8 Hz), 0.94(3H ×1/2, d, J=6.8 Hz), 1.2-1.9 (9H, m), 3.13-3.27 (1H, m), 3.45-3.64 (2H, m), 3.86 (1H, m), 4.57 (1H, b)

(Process 4)

(R)-N-[3,5-bis(trifluoromethyl)benzyl]-7,8-dihydro-7-([1,1,2,2,$^2H_4$]-4-hydroxy-3-methylbutyl)-5-(4-methylphenyl)-8-oxo-1,7-naphthylidine-6-carboxamide was obtained as colorless crystals by reacting and treating as in the case of the Reference Example 5 using the compounds obtained in the process 3 and the Reference Example 3.
Melting point; 205-207°C (re-crystallization from ethyl acetate - ethyl ether)
NMR(200MHz, $CDCl_3$)ppm: 0.78 (3H, d, J = 7.0 Hz), 1.55 (1H, m), 2.28 (3H, s), 3.14 (1H, b, OH), 3.2-3.5 (2H, m), 4.50 (2H, d, J = 6.0 Hz), 7.0-7.3 (4H, m), 7.29 (1H, dd, J = 8.4, 4.4 Hz), 7.54 (1H, dd, J=8.4, 1.6Hz), 7.69 (2H, s), 7.78 (1H, s), 8.55 (1H, t, J = 6.0 Hz), 8.61 (1H, dd, J = 4.4, 1.6 Hz)
Anal. Calcd for $C_{30}H_{23}D_4F_6N_3O_3 \cdot 1/2H_2O$
Calculated (%): C 59.60, H 4.00, D 1.33, N 6.95
Found (%): C 59.94, H 3.82, D 1.29, N 7.13

(Process 5)

Using the compound obtained in the process 4, the reaction and treatment were performed as in the case of the Reference Example 69. Then, the title compound was obtained as colorless crystals.
Melting point; 227-229°C (re-crystallization from ethyl acetate - methanol - ethyl ether)
NMR(200MHz, $CDCl_3$)ppm : 0.91 (3H, d, J = 7.0Hz), 2.08 (1H, m), 2.37 (3H, s), 2.97 (1H, dd, J =15, 1.4 Hz), 3.45 (1H,

dd, J =15, 11 Hz), 3.99 (1H, d, J = 15 Hz), 5.46 (1H, d, J = 15 Hz), 6.83 (1H, dd, J = 7.8, 1.8 Hz), 7.05 (1H, d, J = 7.8 Hz), 7.26 (1H, d, J = 7.8 Hz), 7.34 (1H, dd, J = 7.8, 1.8 Hz), 7.47 (1H, dd, J = 8.6, 4.4 Hz), 7.48 (2H, s), 7.56 (1H, dd, J = 8.6, 1.8 Hz), 7.82 (1H, s), 8.91 (1H, dd, J = 4.1, 1.8 Hz)
Anal. Calcd for $C_{30}H_{21}D_4F_6N_3O_2$
Calculated (%): C 62.39, H 3.66, D 1.39, N 7.28
Found (%): C 62.41, H 3.65, D 1.35, N 7.21
EI-MS m/z: 577($M^+$), 558, 350, 313
$[\alpha]_D$: +116.6° (c = 0.541, MeOH)

(Reference Example 146)

(9R)-[10,10,11,11,$^2H_4$]-7-[3,5-bis(Trifluoromethyl)benzyl]-8,9,10,11-tetrahydro-5-(4-hydroxymethylphenyl)-9-methyl-7H-[1,4] diazocino[2,1-g] [1,7]naphthylidine-6,13-dion ($d_4$ isomer of the compound in the Reference Example 137)

Using the compound obtained in the Reference Example 145, the reaction and treatment were performed as in the case of the Reference Example 137. Then, the title compound was obtained as colorless crystals.
Melting point; 241-242°C (re-crystallization from ethyl acetate - methanol - ethyl ether)
NMR(200MHz, $CDCl_3$)ppm : 0.91(3H, d, J=7.0Hz), 1.85(1H, m), 2.09(1H, in), 2.98(1H, dd, J=15, 1.4Hz), 3.47(1H, dd, J=15, 11Hz), 4.02(1H, d, J= 15Hz) , 4.75 (2H, d, J=4.4Hz), 5.45(1H, d, J=15Hz), 6.97(1H, d, J=8.0Hz), 7.25(1H, d, J=8.0Hz), 7.4-7.6(6H, m), 7.81(1H, s), 8.91(1H, dd, J=4.0, 2.2Hz)
Anal. Calcd for $C_{30}H_{21}D_4F_6N_3O_3 \cdot 1/2H_2O$
Calculated (%) : C 59.80, H 3.68, D 1.34, N 6.97
Found (%): C 59.76, H 3.78, D 1.40, N 6.73
EI-MS m/z: 593($M^+$), 554, 519, 366, 313
$[\alpha]_D$: +94.2° (c = 0.538, MeOH)

(Reference Example 147)

(9R)-[10,10,11,11-$^2H_4$]-7-[3,5-bis(Trifluoromethyl)benzyl]-5-(4-formylphenyl)-8,9,10,11-tetrahydro-9-methyl-7H-[1,4] diazocino[2,1-g][1,7]naphthylidine-6,13-dion ($d_4$ isomer of the compound in the Reference Example 138)

Using the compound obtained in the Reference Example 146, the reaction and treatment were performed as in the case of the Reference Example 138 or 139. Then, the title compound was obtained as colorless crystals.
Melting point; 215-216°C (re-crystallization from ethyl acetate - ethyl ether)
NMR(200MHz, $CDCl_3$)ppm: 0.95(3H, d, J=7.0Hz), 2.10(1H, m), 3.05(1H, dd, J=15, 1.4Hz), 3.47(1H, dd, J=15, 11Hz), 4.00(1H, d, J=15Hz), 5.39(1H, d, J=15Hz), 7.12(1H, dd, J=8.0, 1.8Hz), 7.40-7.55(4H, m), 7.62-7.74(2H, m), 7.79(1H, s), 7.97(1H, dd, J=8.0, 1.8Hz), 8.94 (1H, dd, J=4.0, 1.8Hz), 10.04 (1H, s)
Anal. Calcd for $C_{30}H_{19}D_4F_6N_3O_3$
Calculated (%) : C 60.91, H 3.24, D 1.36, N 7.10
Found (%): C 60.89, H 3.31, D 1.36, N 6.99
EI-MS m/z: 591($M^+$), 572, 517, 364, 313
$[\alpha]_D$: +106.1° (c = 0.510, MeOH)

(Reference Example 148)

(9R)-[10,10,11,11-$^2H_4$]-7-[3,5-bis(trifluoromethyl)benzyl]-5-(4-carboxylphenyl)-8,9,10,11-tetrahydro-9-methyl-7H-[1,4] diazocino[2,1-g][1,7]naphthylidine-6,13-dion ($d_4$ isomer of the compound in the Reference Example 140)

Potassium permanganate (1.7 g) was gradually added to a mixture of the compound (2.0 g) obtained in the Reference Example 146, t-butanol (80 ml) and 0.3N sodium hydroxide solution (55 ml) at 0°C with stirring. The mixture was stirred at room temperature for an hour, then ethanol (10 ml) was added, and it was further stirred at room temperature for 30 min. The precipitate was filtrated by using Celite and washed with ethanol. The filtrate and washings were combined and the solvents were evaporated. The residue was dissolved in 1N sodium hydroxide solution. This solution was washed with a mixed solution of ethyl ether and THF, acidified using 2N hydrochloric acid, and then extracted with ethyl acetate. The extract was washed with brine and dried. Then, the solvents were evaporated to give the title compound as a colorless crystal (1.71 g). Melting point; 302-303°C (re-crystallization from ethyl acetate - methanol - ethyl ether)
NMR(200MHz, $CDCl_3$) ppm: 0.95(3H, d, J=6.6Hz), 2.09(1H, m), 3.09(1H, d, J=14Hz), 3.49(1H, dd, J=14, 9.4Hz), 4.02(1H, d, J=15Hz), 5.36(1H, d,J=15Hz), 7.04(1H, d, J=8.0Hz), 7.50(4H, m), 7.59(1H, d, J=8.4Hz), 7.82(1H, s), 7.91(1H, d, J=8.4Hz), 8.20(1H, d, J=8.0Hz), 8.97(1H, dd, J=3.6, 2.2Hz)
Anal. Calcd for $C_{30}H_{19}D_4F_6N_3O_4 \cdot 1/2H_2O$
Calculated (%): C 58.44, H 3.27, D 1.31, N 6.82
Found (%) : C 58.47, H 3.21, D 1.32, N 6.89
EI-MS m/z: 607 ($M^+$), 568, 533, 380, 313
$[\alpha]_D$: +123.2° (c = 0.545, MeOH)

(Reference Example 149)

(R)-N-[3,5-Di(benzyloxy)benzyl]-7,8-dihydro-7-(4-hydroxy-3-methylbutyl)-5-(4-methylphenyl)-8-oxo-6-pyrido[3,4-b]pyridinecarboxamide

(Process 1)

N-[3,5-di(benzyloxy)benzyl]-5-(4-methylphenyl)-8-oxo-8H-pyrano[3,4-b]pyridine-6-carboxamide was obtained as colorless crystals by reacting and treating as in the case of the process 4 of the Reference Example 2 using the compound obtained in the process 2 of the Reference Example 2 and 3,5-di(benzyloxy)benzylamine.
Melting point; 177-179°C (re-crystallization from ethyl acetate- ethyl ether)
NMR (200MHz, $CDCl_3$) ppm : 2.43 (3H, s), 4.40 (2H, d, J = 5.8 Hz), 5.01 (4H, s), 6.51 (3H, m), 7.1-7.5 (15H, m), 7.57 (2H, m), 8.94 (1H, dd, J = 3.6, 2.2 Hz)
Anal. Calcd for $C_{37}H_{30}N_2O_5$
Calculated (%): C 76.27, H 5.19, N 4.81
Found (%): C 76.36, H 5.11, N 4.78

(Process 2)

Using the compound obtained in the process 1 and THP ether of (R)-4-amino-2-methyl-1-butanol, the reaction and treatment were performed as in the case of the Reference Example 19. Then, the title compound was obtained as colorless crystals.
Melting point; 168-170°C (re-crystallization from ethyl acetate- ethyl ether)
NMR (200MHz, $CDCl_3$) ppm : 0.83 (3H, d, J = 6.6 Hz), 1.5-1.9 (3H, m), 2.30 (3H, s), 2.99 (1H, m), 3.2-3.6 (2H, m), 3.75-3.95 (2H, m), 4.21 (2H, d, J = 6.0 Hz), 4.98 (4H, s), 6.33 (2H, d, J = 1.8 Hz), 6.50 (1H, bt), 7.1-7.5 (16H, m), 7.58 (1H, d, J = 8.4 Hz), 8.67 (1H, dd, J = 4.4, 1.2 Hz)
Anal. Calcd for $C_{42}H_{41}N_3O_5 \cdot 0.3H_2O$
Calculated (%) : C 74.93, H 6.23, N 6.24
Found (%): C 74.80, H 6.25, N 6.28

(Reference Example 150)

(R)-N-(3,5-Dimethoxybenzyl)-7,8-dihydro-7-(4-hydroxy-3-methylbutyl)-8-oxo-5-phenyl-6-pyrido[3,4-b]pyridine carboxamide

(Process 1)

N-(3,5-dimethoxybenzyl)-8-ozo-5-phenyl-8H-pyrano[3,4-b]pyridine carboxamide was obtained as colorless crystals by reacting and treating as in the case of the process 4 of the Reference Example 2 using the compound obtained in the process 2 of the Reference Example 9 and 3,5-dimethoxybenzylamine.
Melting point; 126-127°C (re-crystallization from ethyl acetate- ethyl ether)
NMR (200MHz, $CDCl_3$) ppm : 3.78 (6H, s), 4.40 (2H, d, J = 5.8 Hz), 6.41 (3H, m), 7.20-7.35 (3H, m), 7.45-7.65 (5H, m), 8.95 (1H, dd, J = 4.0, 1.4 Hz)
Anal. Calcd for $C_{24}H_{20}N_2O_5$
Calculated (%): C 69.22, H 4.84, N 6.73
Found (%): C 69.27, H 4.72, N 6.83

(Process 2)

Using the compound obtained in the process 1 and THP ether of (R)-4-amino-2-methyl-1-butanol, the reaction

and treatment were performed as in the case of the Reference Example 19. Then, the title compound was obtained as colorless crystals.
Melting point; 150-151°C (re-crystallization from ethyl acetate- ethyl ether)
NMR (200MHz, $CDCl_3$) ppm : 0.83 (3H, d, J = 6.6 Hz), 1.5-1.9 (3H, m), 3.09 (1H, m), 3.2-3.6 (2H, m), 3.6-3.9 (2H, m), 3.76 (6H, s), 4.20 (2H, d, J = 5.8 Hz), 6.27 (2H, d, J = 2.2 Hz), 6.35 (1H, t, J = 2.2 Hz), 7.2-7.7 (8H, m), 8.61 (1H, dd, J = 4.4, 1.4 Hz)
Anal. Calcd for $C_{29}H_{31}N_3O_5$
Calculated (%): C 69.44, H 6.23, N 8.38
Found (%): C 69.11, H 6.04, N 8.51

(Reference Example 151)

(R)-N-[3,5-bis(Trifluoromethyl)benzyl]-5-(4-chlorophenyl)-7,8-dihydro-7-(4-hydroxy-3-methylbutyl)-8-oxo-6-pyrido [3,4-b]pyridinecarboxamide

(Process 1)

5-(4-Chlorophenyl)-8-oxo-8H-pyrano[3,4-b] pyridine-6-carboxylic acid was obtained as colorless crystals by reacting and treating as in the case of the process 1 and 2 of the Reference Example 2 using 3-(4-chlorobenzoyl)-2-pyridinecarboxylic acid instead of 3-(4-methylbenzoyl)-2-pyridinecarboxylic acid in the process 1 of the Reference Example 2.
NMR (200MHz, $CDCl_3$+DMSO-$d_3$) ppm : 7.24 (2H, d, J= 8.0 Hz), 7.47 (2H, d, J = 8.0 Hz), 7.50 (1H, d, J = 8.0 Hz), 7.63 (1H, dd, J = 8.0, 4.4 Hz), 8.96 (1H, d, J = 4.4 Hz)

(Process 2)

N-[3,5-bis(trifluoromethyl)benzyl]-5-(4-chlorophenyl)-8-oxo-8H-pyrano[3,4-b]pyridine-6-carboxamide was obtained as colorless crystals by reacting and treating as in the case of the process 4 of the Reference Example 2 using the compound obtained in the process 1 and 3,5-bis(trifluoromethyl)benzylamine.
Melting point; 217-219°C (re-crystallization from ethyl acetate- isopropyl ether)
NMR (200MHz, $CDCl_3$) ppm : 4.63 (2H, d, J = 6.2 Hz), 7.23 (2H, d, J = 8.2 Hz), 7.51 (2H, d, J = 8.2 Hz), 7.53 (1H, dd, J = 8.4, 1.6 Hz), 7.65 (1H, dd, J = 8.4, 4.4 Hz), 7.6-7.8 (1H, m), 7.73 (2H, s), 7.80 (1H, s), 8.98 (1H, dd, J = 4.4, 1.6 Hz)

(Process 3)

The title compound was obtained as colorless crystals by reacting and treating as in the case of the Reference Example 19 using the compound obtained in the process 2 and THP ether of (R)-4-amino-2-methyl-1-butanol.
Melting point; 259-261°C (re-crystallization from ethyl acetate- isopropyl ether)
NMR (200MHz, $CDCl_3$) ppm : 0.78 (3H, d, J = 6.2 Hz), 1.4-1.8 (3H, m), 3.02 (1H, m), 3.2-3.7 (4H, m), 4.48 (2H, m), 7.1-7.4 (5H, m), 7.46 (1H, d, J = 8.0 Hz), 7.72 (2H, s), 7.85 (1H, s), 8.60 (2H, m)

(Reference Example 152)

(R)-N-[3,5-bis(Trifluoromethyl)benzyl]-5-(3,4-dichlorophenyl)-7,8-dihydro-7-(4-hydroxy-3-methylbutyl)-8-oxo-6-pyrido [3,4-b]pyridine carboxamide

(Process 1)

5-(3,4-dichlorophenyl)-8-oxo-8H-pyrano[3,4-b]pyridine-6-carboxylic acid was obtained as colorless crystals by reacting and treating as in the case of the process 1 and 2 of the Reference Example 2 using 3-(3,4-dichlorobenzoyl)-2-pyridinecarboxylic acid instead of 3-(4-methylbenzoyl)-2-pyridinecarboxylic acid in the process 1 of the Reference Example 2.
NMR (200MHz, DMSO-$d_6$) ppm : 7.34 (1H, dd, J = 8.2, 1.8 Hz), 7.50 (1H, dd, J = 8.4, 1.4 Hz), 7.66 (1H, d, J = 1.8 Hz), 7.72-7.85 (2H, m), 8.96 (1H, dd, J = 4.4, 1.4 Hz)

(Process 2)

N-[3,5-bis(trifluoromethyl)benzyl]-5-(3,4-dichlorophenyl)-8-oxo-8H-pyrano[3,4-b]pyridine-6-carboxamide

was obtained as colorless crystals by reacting and treating as in the case of the process 4 of the Reference Example 2 using the compound obtained in the process 1 and 3, 5-bis (trifluoromethyl) benzylamine.
Melting point; 220-221°C (re-crystallization from ethyl acetate- isopropyl ether)
NMR (200MHz, $CDCl_3$) ppm : 4.64 (2H, d, J = 6.2 Hz), 7.15 (1H, dd, J = 8.4, 1.8 Hz), 7.39 (1H, d, J = 1.8 Hz), 7.5-7.8 (4H, m), 7.75 (2H, s), 7.81 (1H, s), 8.99 (1H, dd, J = 4.4, 1.4 Hz)

(Process 3)

The title compound was obtained by reacting and treating as in the case of the Reference Example 19 using the compound obtained in the process 2 and THP ether of (R)-4-amino-2-methyl-1-butanol. This product was used for the reaction of the example 7 without purification.

(Reference Example 153)

(R)-5-(3,4-Dichlorophenyl)-N-(3,5-dimethoxybenzyl)-7,8-dihydro-7-(4-hydroxy-3-methylbutyl)-8-oxo-6-pyrido[3,4-b] pyridinecarboxamide

(Process 1)

5-(3,4-Dichlorophenyl)-N-(3,5-dimethoxybenzyl)-8-oxo-8H -pyrano[3,4-b]pyridine-6-carboxamide was obtained as colorless crystals by reacting and treating as in the case of the process 4 of the Reference Example 2 using the compound obtained in the process 1 of the Reference Example 152 and 3,5-dimethoxybenzylamine.
Melting point; 220-221°C (re-crystallization from ethyl acetate- ethyl ether)
NMR (200MHz, $CDCl_3$) ppm : 3.78 (6H, s), 4.41 (2H, d, J = 6.0 Hz), 6.38-6.48 (3H, m), 7.15 (1H, dd, J = 8.4, 1.8 Hz), 7.34-7.82 (5H, m), 8.96 (1H, dd, J = 4.2, 1.6 Hz)
Anal. Calcd for $C_{24}H_{18}N_2O_5Cl_2$
Calculated (%): C 59.40, H 3.74, N 5.77
Found (%): C 59.13, H 3.81, N 5.77

(Process 2)

The title compound was obtained by reacting and treating as in the case of the Reference Example 19 using the compound obtained in the process 1 and THP ether of (R)-4-amino-2-methyl-1-butanol. This product was used for the reaction of the example 8 without purification.

(Reference Example 154)

(R)-N-(3,5-Dimethylbenzyl)-7,8-dihodro-7-(4-hydroxy-3-methylbutyl)-5-(4-methylphenyl)-8-oxo-6-pyrido[3,4-b] pyridinecarboxamide

(Process 1)

N-(3,5-Dimethylbenzyl)-5-(4-methylphenyl)-8-oxo-8H-pyrano[3,4-b]pyridine-6-carboxamide was obtained as colorless crystals by reacting and treating as in the case of the process 4 of the Reference Example 2 using the compound obtained in the process 2 of the Reference Example 2 and 3,5-dimethylbenzylamine.
Melting point; 201-202°C (re-crystallization from ethyl acetate- isopropyl ether)
NMR (200MHz, $CDCl_3$) ppm : 2.30 (6H, s), 2.45 (3H, s), 4.39 (2H, d, J = 5.8 Hz), 6.88 (2H, s), 6.93 (1H, s), 7.17 (2H, d, J = 8.0 Hz), 7.19 (1H, m), 7.33 (2H, d, J = 8.0 Hz), 7.57 (2H, m), 8.93 (1H, dd, J = 4.0, 2.2 Hz)
Anal. Calcd for $C_{25}H_{22}N_2O_3$
Calculated (%): C 75.36, H 5.57, N 7.03
Found (%): C 74.93, H 5.58, N 7.00

(Process 2)

The title compound was obtained as colorless crystals by reacting and treating as in the case of the Reference Example 19 using the compound obtained in the process 1 and THP ether of (R) -4-amino-2-methyl-1-butanol.
Melting point; 193-194°C (re-crystallization from ethyl acetate- isopropyl ether)
NMR (200MHz, $CDCl_3$) ppm : 0.86 (3H, d, J=6.6Hz), 1.5-2.0 (3H, m), 2.25 (6H, s), 2.42 (3H, s), 3.14 (1H, m), 3.2-3.4

(2H, m), 3.89 (2H, m), 4.20 (2H, d, J= 5.4 Hz), 6.61 (2H, s), 6.87 (1H, s), 7.1-7.4 (6H, m), 7.58 (1H, dd, J = 8.4, 1.4 Hz), 8.62 (1H, dd, J = 4.4, 1.4 Hz)
Anal. Calcd for $C_{30}H_{33}N_3O_3 \cdot 0.3H_2O$
Calculated (%): C 73.69, H 6.93, N 8.59
Found (%): C 73.85, H 6.95, N 8.52

(Reference Example 155)

(R)-N-(3,5-Dichlorobenzyl)-7,8-dihydro-7-(4-hydroxy-3-methylbutyl)-5-(4-methylphenyl)-8-oxo-6-pyrido[3,4-b] pyridinecarboxamide

(Process 1)

N-(3,5-Dichlorobenzyl)-5-(4-methylphenyl)-8-oxo-8H-pyrano[3,4-b]pyridine-6-carboxamide was obtained as colorless crystals by reacting and treating as in the case of the process 4 of the Reference Example 2 using the compound obtained in the process 2 of the Reference Example 2 and 3,5-dichlorobenzylamine.
Melting point; 232-233°C (re-crystallization from THF-isopropyl ether)
NMR (200MHz, $CDCl_3$) ppm : 2.45 (3H, s), 4.44 (2H, d, J = 6.4 Hz), 7.1-7.3 (5H, m), 7.34 (2H, d, J = 7.6 Hz), 7.43 (1H, bt), 7.59 (2H, m), 8.95 (1H, dd, J = 4.0, 2.2 Hz)
Anal. Calcd for $C_{23}H_{16}N_2O_3Cl_2$
Calculated (%) : C 62.89, H 3.67, N 6.38
Found (%) : C 62.62, H 3.70, N 36.36

(Process 2)

The title compound was obtained as colorless crystals by reacting and treating as in the case of the Reference Example 19 using the compound obtained in the process 1 and THP ether of (R)-4-amino-2-methyl-1-butanol.
Melting point; 123-125°C (re-crystallization from ethyl acetate- isopropyl ether)
NMR (200MHz, $CDCl_3$) ppm : 0.81 (3H, d, J = 7.0 Hz), 1.5-2.0 (3H, m), 2.39 (3H, s), 3.17 (1H, m), 3.2-3.8 (4H, m), 4.34 (2H, d, J = 6.2 Hz), 6.95 (2H, d, J = 2.0 Hz), 7.1-7.4 (5H, m), 7.31 (1H, dd, J = 8.2, 4.4 Hz), 7.56 (1H, dd, J = 8.2, 1.6 Hz), 8.31 (1H, bt), 8.62 (1H, dd, J = 4.4, 1.6 Hz)
Anal. Calcd for $C_{28}H_{27}N_3O_3Cl_2$
Calculated (%): C 64.13, H 5.19, N 8.01
Found (%): C 63.82, H 5.01, N 7.96

(Reference Example 156)

(R)-5-(3,4-Dichlorophenyl)-N-(3,5-dimethylbenzyl)-7,8-dihydro-7-(4-hydroxy-3-methylbutyl)-8-oxo-6-pyrido[3,4-b] pyridinecarboxamide

(Process 1)

5-(3,4-Dichlorophenyl)-N-(3,5-dimethylbenzyl)-8-oxo-8H-pyrano[3,4-b]pyridine-6-carboxamide was obtained as colorless crystals by reacting and treating as in the case of the process 4 of the Reference Example 2 using the compound obtained in the process 1 of the Reference Example 152 and 3,5-dimethylbenzylamine.
Melting point; 210-211°C (re-crystallization from ethyl acetate-isopropyl ether)
NMR (200MHz, $CDCl_3$) ppm : 2.30 (6H, s), 4.40 (2H, d, J = 5.6 Hz), 6.89 (2H, s), 6.94 (1H, s), 7.16 (1H, dd, J= 8.2, 2.2 Hz), 7.30 (1H, bt), 7.39 (1H, d, J = 2.2 Hz), 7.50 (1H, dd, J = 8.4, 1.6 Hz), 7.60 (1H, d, J = 8.2 Hz), 7.65 (1H, dd, J = 8.4, 4.4 Hz), 8.97 (1H, dd, J = 4.4, 1.6 Hz)
Anal. Calcd for $C_{24}H_{18}N_2O_3Cl_2 \cdot 0.2H_2O$
Calculated (%) : C 63.09, H 4.06, N 6.13
Found (%) : C 63.13, H 3.94, N 6.14

(Process 2)

The title compound was obtained as colorless foam by reacting and treating as in the case of the Reference Example 19 using the compound obtained in the process 1 and THP ether of (R)-4-amino-2-methyl-1-butanol.
NMR (200MHz, $CDCl_3$) ppm : 0.86 (3H, d, J=5.8Hz), 1.4-1.9 (3H, m), 2.29 (6H, s), 2.90 (1H, m), 3.25-3.85 (4H, m),

4.29 (2H, d, J=5.6Hz), 6.72 (2H, s), 6.93 (1H, s), 7.25-7.55 (5H, m), 7.75 (1H, m), 8.60 (1H, m)

(Reference Example 157)

(R)-N-(3,5-Dimethoxybenzyl)-5-(4-fluorophenyl)-7,8-dihydro-7-(4-hydroxy-3-methylbutyl)-8-oxo-6-pyrido[3,4-b] pyridinecarboxamide

(Process 1)

5-(4-Fluorophenyl)-8-oxo-8H-pyrano[3,4-b]pyridine-6-carboxylic acid was obtained as colorless crystals by reacting and treating as in the case of the process 1 and 2 of the Reference Example 2 using 3-(4-fluorobenzoyl)-2-pyridinecarboxylic acid instead of 3-(4-methylbenzoyl)-2-pyridinecarboxylic acid in the process 1 of the Reference Example 2.
NMR (200MHz, DMSO-$d_6$) ppm : 7.25-7.50 (5H, m), 7.81 (1H, dd, J = 8.4, 4.4 Hz), 8.95 (1H, dd, J = 4.4, 1.4 Hz)

(Process 2)

N-(3,5-Dimethoxybenzyl)-5-(4-fluorophenyl)-8-oxo-8H-pyrano[3,4-b]pyridine-6-carboxamidewas obtained as colorless crystals by reacting and treating as in the case of the process 4 of the Reference Example 2 using the compound obtained in the process 1 and 3,5-dimethoxybenzylamine.
Melting point; 193-194°C (re-crystallization from ethyl acetate- isopropyl ether)
NMR (200MHz, $CDCl_3$) ppm : 3.78 (6H, s), 4.41 (2H, d, J = 6.0 Hz), 6.42 (3H, m), 7.2-7.4 (5H, m), 7.52 (1H, dd, J = 8.4, 1.6 Hz), 7.64 (1H, dd, J = 8.4, 4.4 Hz), 8.97 (1H, dd, J = 4.4, 1.6 Hz)
Anal. Calcd for $C_{24}H_{19}N_2O_5F$
Calculated (%): C 66.36, H 4.41, N 6.45
Found (%): C 66.07, H 4.55, N 6.27

(Process 3)

The title compound was obtained as colorless crystals by reacting and treating as in the case of the Reference Example 19 using the compound obtained in the process 2 and THP ether of (R)-4-amino-2-methyl-1-butanol.
Melting point; 170-171°C (re-crystallization from ethyl acetate - ethyl ether - isopropyl ether)
NMR (200MHz, $CDCl_3$) ppm : 0.82 (3H, d, J= 6.6 Hz), 1.5-1.9 (3H, m), 3.06 (1H, m), 3.2-3.6 (2H, m), 3.65-3.85 (2H, m), 3.78 (6H, s), 4.27 (2H, d, J = 6.2 Hz), 6.26 (2H, d, J = 2.2 Hz), 6.36 (1H, t, J = 2.2 Hz), 7.07 (2H, t, J = 8.6 Hz), 7.28 (1H, dd, J = 8.4, 4.0 Hz), 7.35-7.50 (2H, m), 7.46 (1H, dd, J = 8.4, 1.4 Hz), 7.91 (1H, bt), 8.59 (1H, dd, J = 4.0, 1.4 Hz)
Anal. Calcd for $C_{29}H_{30}N_3O_5F$
Calculated (%) : C 67.04, H 5.82, N 8.09
Found (%) : C 66.87, H 5.73, N 8.04

(Reference Example 158)

(R)-5-(4-Chlorophenyl).-N-(3,5-dimethoxybenzyl)-7,8-dihydro-7-(4-hydroxy-3-methylbutyl)-8-oxo-6-pyrido[3,4-b] pyridine carboxamide

(Process 1)

5-(4-Chlorophenyl)-N-(3,5-dimethoxybenzyl)-8-oxo-8H pyrano[3,4-b]pyridine-6-carboxamide was obtained as colorless crystals by reacting and treating as in the case of the process 4 of the Reference Example 2 using the compound obtained in the process 1 of the Reference Example 151 and 3,5-dimethoxybenzylamine.
Melting point; 179-180°C (re-crystallization from ethyl acetate- isopropyl ether)
NMR (200MHz, $CDCl_3$) ppm : 3.78 (6H, s), 4.41 (2H, d, J = 5.8 Hz), 6.42 (3H, m), 7.23 (2H, d, J = 8.4 Hz), 7.34 (1H, bt), 7.51 (2H, d, J = 8.4 Hz), 7.52 (1H, dd, J = 8.2, 1.6 Hz), 7.63 (1H, dd, J = 8.2, 4.4 Hz), 8.97 (1H, dd, J = 4.4, 1.6 Hz)
Anal. Calcd for $C_{24}H_{19}N_2O_5Cl$
Calculated (%) : C 63.93, H 4.25, N 6.21
Found (%) : C 63.70, H 4.37, N 6.11

(Process 2)

The title compound was obtained as colorless crystals by reacting and treating as in the case of the Reference Example 19 using the compound obtained in the process 1 and THP ether of (R)-4-amino-2-methyl-1-butanol.
Melting point; 181-182°C (re-crystallization from ethyl acetate - ethyl ether - isopropyl ether)
NMR (200MHz, $CDCl_3$) ppm : 0.83 (3H, d, J = 6.6 Hz), 1.4-1.8 (3H, m), 2.98 (1H, m), 3.2-3.4 (2H,m), 3.60-3.85 (2H, m), 3.78 (6H, s), 4.27 (2H, d, J = 5.8 Hz), 6.29 (2H, d, J = 2.2 Hz), 6.38 (1H, t, J = 2.2 Hz), 7.22-7.45 (5H, m), 7.45 (1H, dd, J = 8.4, 1.8 Hz), 7.82 (1H, bt), 8.60 (1H, dd, J = 4.0, 1.8 Hz)
Anal. Calcd for $C_{29}H_{30}N_3O_5Cl$
Calculated (%) : C 64.98, H 5.64, N 7.84
Found (%) : C 64.79, H 5.58, N 7.73

(Reference Example 159)

(R)-N-[3,5-bis(Trifluoromethyl)benzyl]-5-(4-fluorophenyl)-7,8-dihodro-7-(4-hydroxy-3-methylbutyl)-8-oxo-6-pyrido [3,4-b]pyridine carboxamide

(Process 1)

N-[3,5-bis(Trifluoromethyl)benzyl]-5-(4-fluorophenyl)-8-oxo-8H-pyrano[3,4-b]pyridine-6-carboxamide was obtained as colorless crystals by reacting and treating as in the case of the process 4 of the Reference Example 2 using the compound obtained in the process 1 of the Reference Example 157 and 3,5-bis(trifluoromethyl)benzylamine.
Melting point; 166-167°C (re-crystallization from ethyl acetate - isopropyl ether)
NMR (200MHz, $CDCl_3$) ppm : 4.63 (2H, d, J = 6.2 Hz) , 7.1-7.3 (4H, m), 7.54 (1H, dd, J = 8.4, 1.6 Hz), 7.6-7.8 (1H, m), 7.65 (1H, dd, J = 8.4, 4.4 Hz), 7.73 (2H, s), 7.80 (1H, s), 8.98 (1H, dd, J = 4.4, 1.6 Hz)
Anal. Calcd for $C_{24}H_{13}N_2O_3F_7$
Calculated (%): C 56.48, H 2.57, N 5.49
Found (%): C 56.52, H 2.68, N 5.47

(Process 2)

The title compound was obtained as colorless crystals by reacting and treating as in the case of the Reference Example 19 using the compound obtained in the process 1 and THP ether of (R)-4-amino-2-methyl-1-butanol.
Melting point; 212-213°C (re-crystallization from ethyl acetate - isopropyl ether)
NMR (200MHz, $CDCl_3$) ppm : 0.77 (3H, d, J = 7.0 Hz), 1.4-1.8 (3H, m), 3.1-3.7 (5H, m), 4.51 (2H, m), 6.84-7.00 (2H, m), 7.25-7.48 (3H, m), 7.48 (1H, dd, J= 8.4, 1.8 Hz), 7.69 (2H, s), 7.82 (1H, s), 8.60 (1H, dd, J = 4.2, 1.8 Hz), 8.76 (1H, bt)
Anal. Calcd for $C_{29}H_{24}N_3O_3F_7$
Calculated (%) : C 58.49, H 4.06, N 7.06
Found (%): C 58.28, H 4.06, N 7.02

(Reference Example 160)

(±)-N-[3,5-bis(Trifluoromethyl)benzyl]-7,8-dihydro-7-(4-hydroxy-3-ethylbutyl)-5-(4-methylphenyl) -8-oxo-6-pyrido [3,4-b]pyridinecarboxamide

The title compound was obtained as a colorless oil by reacting and treating as in the case of the Reference Example 5 using the compound obtained in the Reference Example 3 and (±)-4-amino-2-ethyl-1-butanol.
NMR (200MHz, $CDCl_3$) ppm : 0.86 (3H, t, J=7.0Hz), 1.0-1.4 (3H, m), 1.5-1.9 (2H, m), 2.28 (3H, s), 3.0-3.6 (5H, m), 4.50 (2H, d, J = 6.2 Hz), 7.07 (2H, d, J = 8.6 Hz), 7.1-7.3 (2H, m), 7.31 (1H, dd, J=8.0, 4.2 Hz), 7.55 (1H, dd, J=8.0, 1.4 Hz), 7.68 (2H, s), 7.78 (1H, s), 8.43 (1H, bt), 8.65 (1H, m)

(Reference Example 161)

(±)-N-[3,5-bis(Trifluoromethyl)benzyl]-7,8-dihydro-7-[4-hydroxy-3-(1-methylethyl)butyl)-5-(4-methylphenyl)-8-oxo-6-pyrido[3,4-b]pyridine carboxamide

The title compound was obtained as a colorless oil by reacting and treating as in the case of the Reference Example 5 using the compound obtained in the Reference Example 3 and (±)-4-amino-2-(1-methylethyl)-1-butanol.

NMR (200MHz, $CDCl_3$) ppm : 0.82 (6H, d, J = 6.6 Hz), 1.3-2.0 (4H, m), 2.27 (3H, s), 3.30 (1H, m), 3.52 (4H, m), 4.50 (2H, d, J = 5.8 Hz), 7.07 (2H, d, J = 8.6 Hz), 7.20-7.35 (3H, m), 7.54 (1H, dd, J = 8.4, 1.6Hz), 7.67 (2H, s), 7.78 (1H, s), 8.51 (1H, bt), 8.63 (1H, dd, J = 4.4, 1.6 Hz)

(Reference Example 162)

(±)-5-(3,4-Dichlorophenyl)-N-(3,5-dimethoxybenzyl)-7,8-dihydro-7-(4-hydroxy-3-ethylbutyl)-8-oxo-6-pyrido[3,4-b] pyridine carboxamide

The title compound was obtained by reacting and treating as in the case of the Reference Example 5 using the compound obtained in the process 1 of the Reference Example 153 and (±)-4-amino-2-ethyl-1-butanol. This compound was used for the reaction of the example 17 without purification.

(Reference Example 163)

(±)-5-(3,4-Dichlorophenyl)-N-(3,5-dimethoxybenzyl) -7,8-dihydro-7-[4-hydroxy-3-(1-methylethyl)butyl]-8-oxo-6-pyrido [3,4-b]pyridine carboxamide

The title compound was obtained as a colorless oil by reacting and treating as in the case of the Reference Example 5 using the compound obtained in the process 1 of the Reference Example 153 and (±)-4-amino-2-(1-methylethyl)-1-butanol. This compound was used for the reaction of the example 18 without purification.

(Reference Example 164)

(±)-7-[3,5-bis(Trifluoromethyl)benzyl]-6,7,8,9,10,11-hexahydro-10-methyl-5-(4-methylphenyl)-6,13-dioxo-13H-[1,4] diazocino[2,1-g][1,7]naphthylidine

(Process 1)

(±)-N-[3,5-bis(Trifluoromethyl)benzyl]-7,8-dihydro-7-[4-hydroxy-2-methylbutyl)-5-(4-methylphenyl)-8-oxo-6-pyrido[3,4-b]pyridinecarboxamide was obtained as colorless foam by reacting and treating as in the case of the Reference Example 5 using the compound obtained in the Reference Example 3 and (±)-4-amino-3-methyl-1-butanol.
NMR (200MHz, $CDCl_3$) ppm : 0.81 (3H, d, J = 7.0 Hz), 1.3-2.4 (3H, m), 2.30 (3H, s), 2.95 (1H, m), 3.4-3.9 (4H, m), 4.46 (2H, m), 7.0-7.4 (5H, m), 7.52 (1H, d, J = 7.0 Hz), 7.71 (2H, s), 7.78 (1H, s), 8.56 (1H, bt), 8.64 (1H, d, J = 3.0 Hz)

(Process 2)

Using the compound obtained in the process 1, the reaction and treatment were performed as in the case of the Reference Example 69. Then, the title compound was obtained as colorless powder.
NMR (200MHz, $CDCl_3$) ppm : 1.18 (3H, d, J = 7.0 Hz), 1.4-2.5 (3H, m), 2.37 (3H, s), 3.1-3.8 (3H, m), 4.02 (1H, d, J = 15 Hz), 4.81 (1H, d, J = 14 Hz), 5.46 (1H, d, J = 15 Hz), 6.84 (1H, d, J = 7.2 Hz), 7.06 (1H, d, J = 7.2 Hz), 7.2-7.6 (4H, m), 7.48 (2H, s),7.81 (1H, s), 8.90 (1H, m)

(Reference 165)

(9R)-7-[3,5-Di(benzyloxy)benzyl]-6,7,8,9,10,11-hexahydro-9-methyl-5-(4-methyl phenyl)-6,13-dioxo-13H-[1,4] diazocino (2,1-g][1,7]naphthylidine

Using the compound obtained in the Reference Example 149, the reaction and treatment were performed as in the case of the Reference Example 69. Then, the title compound was as colorless powder.
NMR (200MHz, $CDCl_3$) ppm : 0.82 (3H, d, J = 6.6 Hz), 1.65 (1H, m), 1.8-2.4 (2H, m), 2.15 (3H, s), 3.02 (1H, d, J = 15 Hz ), 3.32 (1H, dd, J = 15, 10 Hz), 3.52 (1H, dd, J=14, 10 Hz), 3.81 (1H, d, J=14Hz), 4.97 (4H, s), 5.04 (1H, dd, J=14, 5.6 Hz), 5.24 (1H, d, J = 14 Hz), 6.28 (2H, d, J = 2.2 Hz), 6.55 (1H, bt), 6.86 (1H, d, J=8.0Hz), 7.03 (1H, d, J = 8.0 Hz), 7.2-7.5 (13H, m), 7.60 (1H, dd, J = 8.4, 1.2 Hz), 8.88 (1H, dd, J = 4.4, 1.2 Hz)
Anal. Calcd for $C_{42}H_{39}N_3O_4 \cdot 0.5H_2O$
Calculated (%): C 76.57, H 6.12, N 6.38
Found (%): C 76.19, H 6.33, N 6.25

(Reference Example 166)

(9R)-7-(3, 5-Dihydroxybenzyl)-6,7,8,9,10,11-hexahydro-9-methyl-5-(4-methylphenyl)-6,13-dioxo-13H-[1,4]diazocino [2,1-g] [1,7]naphthylidine

A mixture of the compound (2.3 g) obtained in the Reference Example 165, 10% palladium-carbon (50 % water) (3.0 g) and methanol (70 ml) was refluxed with heating for 15 hours under hydrogen atmosphere. After the catalyst was filtered out by using Celite, the filtrate was concentrated to give the title compound as colorless powder (1.08 g).
NMR (200MHz, DMSO-$d_6$) ppm : 0.78 (3H, d, J = 6.2 Hz), 1.1-1.6 (1H, m), 1.9-2.5 (2H, m), 2.38 (3H, s), 2.90 (1H, d, J = 15 Hz ), 3.0-3.6 (2H, m), 3.87 (1H, d, J = 14 Hz), 4.7-4.9 (1H, m), 4.84 (1H, d, J = 14 Hz), 5.88 (2H, d, J = 1.8 Hz), 6.17 (1H, bt), 6.9-7.3 (4H, m), 7.5-7.7 (2H, m), 8.84 (1H, m), 9.21 (2H, s)

(Reference Example 167)

(9R)-7-(3,5-Diethoxybenzyl)-6,7,8,9,10,11-hexahydro-9-methyl-5-(4-methylphenyl)-6,13-dioxo-13H-[1,4]diazocino [2,1-g] [1,7] naphthylidine

After a mixture of the compound (200 mg) obtained in the Reference Example 166, sodium hydride (60 % oil) (70 mg) and DMF (7 ml) was stirred at room temperature for 30 min, ethyl iodine was added under ice-cooling, and then it was stirred at room temperature for an hour. The reaction solution was diluted with ethyl acetate, and washed subsequently with water, diluted hydrochloric acid, saturated aqueous solution of sodium hydrogen carbonate and saturated brine. The organic layer was dried and the solvents were evaporated. The residual was purified by column chromatography on silica gel (ethyl acetate : methanol = 9:1) to give the title compound as colorless powder (66.5 mg).
NMR (200MHz, $CDCl_3$) ppm : 0.86 (3H, d, J = 6.6 Hz), 1.43 (6H, t, J = 6.9 Hz), 1.66 (1H, m), 1.9-2.4 (2H, m), 2.42 (3H, s), 3.06 (1H, d, J = 15 Hz), 3.33 (1H, dd, J = 15, 11Hz), 3.56 (1H, dd, J = 14, 11 Hz), 3.81 (1H, d, J = 14 Hz), 3.95 (4H, q, J = 6.9 Hz), 5.06 (1H, dd, J = 14, 5.6 Hz), 5.23 (1H, d, J = 14 Hz), 6.17 (2H, d, J = 2.2 Hz), 6.38 (1H, t, J = 2.2 Hz), 6.88 (1H, dd, J = 7.8, 1.6 Hz), 7.15 (1H, d, J = 7.8 Hz), 7.29 (1H, d, J = 7.8 Hz), 7.36 (1H, dd, J = 7.8, 1.6 Hz), 7.46 (1H, dd, J = 8.4, 4.4 Hz), 7.63 (1H, dd, J = 8.4, 1.4 Hz), 8.90 (1H, dd, J = 4.4, 1.4 Hz)

(Reference Example 168)

(9R)-7-[3,5-Di(1-methylethyloxy)benzyl]-6,7,8,9,10,11-hexahydro-9-methyl-5-(4-methylphenyl)-6,13-dioxo-13H-[1,4] diazocino[2,1-g] [1,7]naphthylidine

The title compound was obtained as colorless powder by reacting and treating as in the case of the Reference Example 167 using the compound obtained in the Reference Example 166 and 2-iodine propane.
NMR (200MHz, $CDCl_3$) ppm : 0.84 (3H, d, J =7.0 Hz), 1.34 (12H, d, J = 6.0 Hz), 1.65 (1H, m), 1.9-2.4 (2H, m), 2.44 (3H, s), 3.06 (1H, d, J=15 Hz), 3.35 (1H, dd, J = 15, 10 Hz), 3.55 (1H, dd, J = 14, 11 Hz), 3.88 (1H, d, J=14 Hz), 4,48 (2H, m), 5.05 (1H, dd, J = 14, 5.7 Hz), 5.19 (1H, d, J = 14 Hz), 6.18 (2H, d, J = 2.2 Hz), 6.37 (1H, t, J = 2.2 Hz), 6.95 (1H, d, J = 7.4 Hz), 7.23 (1H, d, J = 7.6 Hz), 7.29 (1H, d, J = 7.6 Hz), 7.36 (1H, d, J = 7.4 Hz), 7.47 (1H, dd, J = 8.4, 4.2 Hz), 7.65 (1H, dd, J = 8.4, 1.6 Hz), 8.90 (1H, dd, J = 4.2, 1.6 Hz)

(Reference Example 169)

(9R)-7-(3,5-Dimethoxybenzyl)-6,7,8,9,10,11-hexahydro-9-methyl-6,13-dioxo-5-phenyl-13H-[1,4]diazocino[2,1-g][1,7] naphthylidine

Using the compound obtained in the Reference Example 150, the reaction and treatment were performed as in the case of the Reference Example 69. Then, the title compound was obtained as colorless crystals.
Melting point; 199-201°C (re-crystallization from ethyl acetate - ethyl ether)
NMR (200MHz, $CDCl_3$) ppm : 0.87 (3H, d, J = 7.0 Hz), 1.70 (1H, m), 1.90-2.29 (2H, m), 3.05 (1H, d, J = 15 Hz), 3.33 (1H, dd, J = 15, 10 Hz), 3.56 (1H, dd, J = 14, 11 Hz), 3.73 (6H, s), 3.80 (1H, d, J = 14 Hz), 5.08 (1H, dd, J = 14, 5.7 Hz), 5.23 (1H, d, J = 14 Hz), 6.12 (2H, d, J = 2.2 Hz), 6.37 (1H, t, J = 2.2 Hz), 6.97 (1H, d, J = 7.2Hz), 7.23-7.55 (5H, m), 7.59 (1H, dd, J = 8.2, 1.4 Hz), 8.90 (1H, dd, J = 4.2, 1.4 Hz)
Anal. Calcd for $C_{29}H_{29}N_3O_4$
Calculated (%) : C 72.03, H 6.04, N 8.69
Found (%): C 71.94, H 6.09, N 8.93

(Reference Example 170)

(9R)-7-[3,5-bis(Trifluoromethyl)benzyl]-5-(4-chlorophenyl)-6,7,8,9,10,11-hexahydro-9-methyl-6,13-dioxo-13H-[1,4] diazocino[2,1-g] [1,7]naphthylidine

Using the compound obtained in the Reference Example 151, the reaction and treatment were performed as in the case of the Reference Example 69. Then, the title compound was obtained as colorless crystals.
Melting point; 226-227°C (re-crystallization from ethyl acetate - ethyl ether)
NMR (200MHz, $CDCl_3$) ppm : 0.92 (3H, d, J = 6.6 Hz), 1.73 (1H, m), 1.9-2.4 (2H, m), 3.02 (1H, d, J = 16 Hz), 3.35-3.65 (2H, m), 4.01 (1H, d, J = 15 Hz), 5.10 (1H, dd, J = 14, 5.2 Hz), 5.39 (1H, d, J = 15 Hz), 6.88 (1H, d, J = 8.4 Hz), 7.19 (1H, d, J = 8.4 Hz), 7.4-7.6 (6H, m), 7.85 (1H, s), 8.93 (1H, t, J = 3.0 Hz)

(Reference Example 171)

(9R)-7-[3,5-bis(Trifluoromethyl)benzyl] -5-(3,4-dichlorophenyl)-6,7,8,9,10,11-hexahydro-9-methyl-6,13-dioxo-13H-[1,4]diazocino[2,1-g][1,7)naphthylidine

Using the compound obtained in the Reference Example 152, the reaction and treatment were performed as in the case of the Reference Example 69. Then, the title compound was obtained as colorless crystals.
Melting point; 280-282°C (re-crystallization from ethyl acetate - isopropyl ether)
NMR (200MHz, $CDCl_3$) ppm : 0.93 (3H×5/9, d, J = 6.6 Hz), 0.96 (3H×4/9, d, J = 6.2 Hz), 1.67 (1H, m), 1.9-2.4 (2H, m), 3.02 (1H×5/9, d, J = 15 Hz), 3.13 (1H×4/9, d , J = 15 Hz), 3.35-3.65 (2H, m), 4.04 (1H×5/9, d, J = 15 Hz), 4.05 (1H×4/9, d, J = 15 Hz), 5.00-5.18 (1H, m), 5.26 (1H×4/9, d, J = 15 Hz), 5.41 (1H×5/9, d, J = 15 Hz), 6.79 (1H×5/9, dd, J = 8.2, 2.0 Hz), 6.98 (1H×4/9, d, J = 2.0Hz), 7.25-7.65 (6H, m), 7.85 (1H, s), 8.94 (1H, m)

(Reference Example 172)

(9R)-7-(3,5-Dimethoxybenzyl)-5-(3,4-dichlorophenyl)-6,7,8,9,10,11-hexahydro-9-methyl-6,13-dioxo-13H-[1,4] diazocino[2,1-g] [1,7]naphthylidine

Using the compound obtained in the Reference Example 153, the reaction and treatment were performed as in the case of the Reference Example 69. Then, the title compound was obtained as colorless crystals.
Melting point; 207-208°C (re-crystallization from ethyl acetate - ethyl ether)
NMR (200MHz, $CDCl_3$) ppm : 0.88 (3H×1/2, d, J = 6.8 Hz), 0.89 (3H×1/2, d, J = 6. 8 Hz), 1.65 (1H, m), 1.9-2.4 (2H, m), 3.00-3.38 (2H, m), 3.44-3.90 (2H, m), 3.75 (3H, s), 3.77 (3H, s), 5.07 (1H, dd, J = 14, 6.2 Hz), 5.25 (1H, dd, J = 15, 7.4 Hz), 5.99 (1H, d, J = 2.2 Hz), 6.12 (1H, d, J = 2.2 Hz), 6.36 (1H×1/2, t, J = 2.2 Hz), 6.40 (1H×1/2, t, J = 2.2 Hz), 6.79 (1H×1/2, dd, J = 8.0, 2.2 Hz), 7.11 (1H×1/2, d, J = 1.8 Hz), 7.25-7.65 (4H, m), 8.91 (1H, m)
Anal. Calcd for $C_{29}H_{27}N_3O_4Cl_2$
Calculated (%): C 63.05, H 4.93, N 7.61
Found (%): C 62.73, H 5.07, N 7.64

(Reference Example 173)

(9R)-7-(3,5-Dimethylbenzyl)-6,7,8,9,10,11-hexahydro-9-methyl-5-(4-methylphenyl)-6,13-dioxo-13H-[1,4]diazocino [2,1-g][1,7]naphthylidine

Using the compound obtained in the Reference Example 154, the reaction and treatment were performed as in the case of the Reference Example 69. Then, the title compound was obtained as colorless crystals.
Melting point; 200-202°C (re-crystallization from THF - isopropyl ether)
NMR (200MHz, $CDCl_3$) ppm : 0.85 (3H, d, J = 6.6 Hz), 1.67 (1H, m), 1.9-2.4 (2H, m), 2.25 (6H, s), 2.44 (3H, s), 2.97 (1H, d, J = 15 Hz), 3.27 (1H, dd, J = 15, 10Hz), 3.58 (1H, dd, J = 14, 11 Hz), 3.73 (1H, d, J = 15 Hz), 5.08 (1H, dd, J = 14, 5.7 Hz), 5.38 (1H, d, J = 15 Hz), 6.50 (2H, s), 6.92 (2H, m), 7.15 (1H, d, J = 8.0 Hz), 7.35 (1H, d, J = 7.6 Hz), 7.45 (1H, d, J = 7.6 Hz), 7.46 (1H, dd, J = 8.2, 4.2 Hz), 7.58 (1H, dd, J = 8.2, 1.6 Hz), 8.90 (1H, dd, J = 4.2, 1.6 Hz)
Anal. Calcd for $C_{30}H_{31}N_3O_2$
Calculated (%) : C 77.39, H 6.71, N 9.03
Found (%): C 77.01, H 6.75, N 8.95

(Reference Example 174)

(9R)-7-(3,5-Dichlorobenzyl)-6,7,8,9,10,11-hexahydro-9-methyl-5-(4-methylphenyl)-6,13-dioxo-13H-[1,4]diazocino [2,1-g] [1,7]naphthylidine

Using the compound obtained in the Reference Example 155, the reaction and treatment were performed as in the case of the Reference Example 69. Then, the title compound was obtained as colorless crystals.
Melting point; 139-141°C (re-crystallization from ethyl acetate - ethyl ether)
NMR (200MHz, $CDCl_3$) ppm : 0.90 (3H, d, J = 6.6 Hz), 1.70 (1H, m), 1.9-2.4 (2H, m), 2.43 (3H, s), 2.95 (1H, d, J = 15 Hz), 3.38 (1H, dd, J=15, 11 Hz), 3.52 (1H, dd, J=15, 11 Hz), 3.74 (1H, d, J = 15 Hz), 5.08 (1H, dd, J = 15, 5.6 Hz), 5.39 (1H, d, J = 15 Hz), 6.79 (2H, d, J = 1.8 Hz), 6.88 (1H, dd, J = 7.6, 1.8 Hz), 7.21 (1H, d, J = 7.6 Hz), 7.29 (1H, s), 7.31 (1H, d, J = 7.6 Hz), 7.38 (1H, dd, J = 7.6, 1.8 Hz), 7.47 (1H, dd, J = 8.4, 4.4 Hz), 7.59 (1H, dd, J = 8.4, 1.8 Hz), 8.90 (1H, dd, J = 4.4, 1.8 Hz)
Anal. Calcd for $C_{28}H_{25}N_3O_2Cl_2 \cdot 0.3H_2O$
Calculated (%): C 65.71, H 5.04, N 8.21
Found (%): C 65.40, H 4.90, N 8.17

(Reference Example 175)

(9R)-5-(3,4-Dichlorophenyl)-7-(3,5-dimethylbenzyl)-6,7,8,9, 10,11-hexahydro-9-methyl-6,13-dioxo-13H-[1,4] diazocino[2,1-g][1,7]naphthylidine

Using the compound obtained in the Reference Example 156, the reaction and treatment were performed as in the case of the Reference Example 69. Then, the title compound was obtained as colorless powder.
Melting point; 137-139°C
NMR (200MHz, $CDCl_3$) ppm : 0.86 (3H×1/2, d, J = 6.2 Hz), 0.89 (3H×1/2, d, J = 6.0 Hz), 1.5-2.3 (3H, m), 2.25 (3H, s), 2.28 (3H, s) , 2.90-3.84 (4H, m) , 5.07 (1H, dd, J=14, 5.8 Hz), 5.31 (1H, dd, J = 14, 9.2 Hz), 6.50 (1H, s), 6.57 (1H, s), 6.78 (1H ×1/2, dd, J = 8.0, 1.8 Hz), 6.93 (1H, d, J = 6.0 Hz), 7.12 (1H ×1/2, d, J = 1.8 Hz), 7.30 (1H, d, J = 8.8 Hz), 7.4-7.7 (3H, m), 8.92 (1H, m)
Anal. Calcd for $C_{29}H_{27}N_3O_2Cl_2 \cdot 0.3H_2O$
Calculated (%): C 66.24, H 5.29, N 7.99
Found (%): C 66.21, H 5.49, N 7.70

(Reference Example 176)

(9R)-7-(3,5-Dimethoxybenzyl)-5-(4-fluorophenyl)-6,7,8,9,10,11-hexahydro-9-methyl-6,13-dioxo-13H-[1,4]diazocino [2,1-g][1,7]naphthylidine

Using the compound obtained in the Reference Example 157, the reaction and treatment were performed as in the case of the Reference Example 69. Then, the title compound was obtained as colorless crystals.
Melting point; 192-193°C (re-crystallization from ethyl acetate - ethyl ether)
NMR (200MHz, $CDCl_3$) ppm : 0.88 (3H, d, J = 6.8 Hz), 1.5-1.8 (1H, m), 1.9-2.4 (2H, m), 3.03 (1H, d, J = 15 Hz), 3.36 (1H, dd, J = 15, 10 Hz), 3.56 (1H, dd, J = 14, 11 Hz), 3.75 (1H, d, J = 15 Hz), 3.76 (6H, s), 5.08 (1H, dd, J = 14, 5.6 Hz), 5.27 (1H, d, J = 15 Hz), 6.03 (2H, d, J = 2.2 Hz), 6.37 (1H, t, J = 2.2 Hz), 6.9-7.1 (2H, m), 7.19 (1H, m), 7.45-7.60 (3H, m), 8.91 (1H, dd, J = 4.2, 2.0 Hz)
$[a]_D$: +109.4° (c = 0.497%, methanol)
Anal. Calcd for $C_{29}H_{28}N_3O_4F$
Calculated (%) : C 69.45, H 5.63, N 8.38
Found (%): C 69.32, H 5.57, N 8.31

(Reference Example 177)

(9R)-5-(4-Chlorophenyl)-7-(3,5-dimethoxybenzyl)-6,7,8,9,10,11-hexahydro-9-methyl-6,13-dioxo-13H-[1,4]diazocino [2,1-g][1,7] naphthylidine

Using the compound obtained in the Reference Example 158, the reaction and treatment were performed as in the case of the Reference Example 69. Then, the title compound was obtained as colorless crystals.
Melting point; 229-230°C (re-crystallization from ethyl acetate - ethyl ether)

NMR (200MHz, $CDCl_3$) ppm : 0.88 (3H, d, J = 7.0 Hz), 1.5-1.8 (1H, m), 1.9-2.4 (2H, m), 3.06 (1H, d, J = 15 Hz), 3.29 (1H, dd, J = 15, 9.8 Hz), 3.56 (1H, dd, J = 14, 11 Hz), 3.76 (1H, d, J = 15 Hz), 3.77 (6H, s), 5.07 (1H, dd, J = 14, 5.6 Hz), 5.27 (1H, d, J = 15 Hz), 6.08 (2H, d, J = 2.2 Hz), 6.39 (1H, t, J = 2.2 Hz), 6.91 (1H, d, J = 8.4 Hz), 7.28 (1H, d, J = 8.4 Hz), 7.46 (2H, m), 7.48 (1H, dd, J = 8.4, 4.0 Hz), 7.55 (1H, dd, J = 8.4, 1.8 Hz), 8.91 (1H, dd, J = 4.0, 1.8 Hz)
Anal. Calcd for $C_{29}H_{28}N_3O_4Cl \cdot 0.2H_2O$
Calculated (%): C 66.78, H 5.49, N 7.99
Found (%): C 66.78, H 5.54, N 7.88

(Reference Example 178)

(9R)-7-[3,5-bis(Trifluoromethyl)benzyl]-5-(4-fluorophenyl)-6,7,8,9,10,11-hexahydro-9-methyl-6,13-dioxo-13H-[1,4]diazocino[2,1-g][1,7]naphthylidine

Using the compound obtained in the Reference Example 159, the reaction and treatment were performed as in the case of the Reference Example 69. Then, the title compound was obtained as colorless crystals.
Melting point; 234-236°C (re-crystallization from ethyl acetate - ethyl ether)
NMR (200MHz, $CDCl_3$) ppm : 0.93 (3H, d, J = 6.8 Hz), 1.73 (1H, m), 2.0-2.4 (2H, m), 3.00 (1H, d, J = 15 Hz), 3.34-3.62 (2H, m), 3.97 (1H, d, J = 15 Hz), 5.10 (1H, dd, J = 15, 5.2 Hz), 5.42 (1H, d, J = 15 Hz), 6.85-6.95 (2H, m), 7.13 (1H, dt, $J_d$ = 2.2, $J_t$ = 9.0 Hz), 7.40-7.52 (3H, m), 7.45 (2H, s), 7.83 (1H, s), 8.92 (1H, t, J = 2.9 Hz)
Anal. Calcd for $C_{29}H_{22}N_3O_2F$
Calculated (%): C 60.31, H 3.84, N 7.28
Found (%) : C 60.43, H 3.98, N 7.13

(Reference Example 179)

(±)-7-[3,5-bis(Trifluoromethyl)benzyl]-9-ethyl-6,7,8,9,10,11-hexahydro-5-(4-methylphenyl) -6,13-dioxo-13H-[1,4]diazocino[2,1-g][1,7]naphthylidine

Using the compound obtained in the Reference Example 160, the reaction and treatment were performed as in the case of the Reference Example 69. Then, the title compound was obtained as colorless crystals.
Melting point; 258-260°C (re-crystallization from ethyl acetate - isopropyl ether)
NMR (200MHz, $CDCl_3$) ppm : 0.90 (3H, t, J = 7.1 Hz), 1.0-1.4 (2H, m), 1.5-1.9 (2H, m), 2.28 (1H, m), 2.39 (3H, s), 3.04 (1H, d, J = 15 Hz), 3.39 (1H, dd, J =15, 9.6 Hz), 3.52 (1H, dd, J = 15, 11 Hz), 3.97 (1H, d, J=15 Hz), 5.11 (1H, dd, J = 15, 6.6 Hz), 5.48 (1H, d, J = 15 Hz), 6.82 (1H, d, J = 7.6 Hz), 7.06 (1H, d, J = 7.6 Hz), 7.2-7.6 (4H, m), 7.48 (2H, s), 7.82 (1H, s), 8.91 (1H, dd, J = 4.0, 1.8 Hz)
Anal. Calcd for $C_{31}H_{27}N_3O_2F_6$
Calculated (%): C 63.37, H 4.63, N 7.15
Found (%): C 63.24, H 4.67, N 7.29

(Reference Example 180)

(±)-7-[3,5-bis (Trifluoromethyl)benzyl]-6,7,8,9,10,11-hexahydro-9-(1-methylethyl) -5-(4-methylphenyl)-6,13-dioxo-13H-[1,4]diazocino[2,1-g][1,7]naphthylidine

Using the compound obtained in the Reference Example 161, the reaction and treatment were performed as in the case of the Reference Example 69. Then, the title compound was obtained as colorless crystals.
Melting point; 228-229°C (re-crystallization from ethyl acetate - isopropyl ether)
NMR (200MHz, $CDCl_3$) ppm : 0.82 (3H, d, J = 6.6 Hz), 0.87 (3H, d, J = 7.0 Hz), 1.5-2.4 (4H, m), 2.39 (3H, s), 3.06 (1H, d, J = 15 Hz), 3.38 (1H, dd, J = 15, 9.0 Hz), 3.50 (1H, dd, J = 14, 10 Hz), 3.95 (1H, d, J = 15 Hz), 5.14 (1H, dd, J = 14, 5.6 Hz), 5.49 (1H, d, J=15 Hz), 6.83 (1H, dd, J = 7.8, 1.4 Hz), 7.07 (1H, d, J = 7.8 Hz), 7.28 (1H, d, J = 8.0 Hz), 7.35 (1H, dd, J = 8.0, 1.4 Hz), 7.47 (1H, dd, J = 8.4, 4.4 Hz), 7.48 (2H, s), 7.56 (1H, dd, J= 8.4, 1.8 Hz), 7.82 (1H, s), 8.91 (1H, dd, J = 4.4, 1.8 Hz)
Anal. Calcd for $C_{32}H_{29}N_3O_2F_6$
Calculated (%) : C 63.89, H 4.86, N 6.89
Found (%): C 63.82, H 4.70, N 7.13

(Reference Example 181)

(±)-5-(3,4-Dichlorophenyl)-7-(3,5-dimethoxybenzyl)-9-ethyl-6,7,8,9,10,11-hexahydro-6,13-dioxo-13H-[1,4]diazocino [2,1-g] [1,7]naphthylidine

Using the compound obtained in the Reference Example 162, the reaction and treatment were performed as in the case of the Reference Example 69. Then, the title compound was obtained as colorless crystals.
Melting point; 260-262°C (re-crystallization from ethyl acetate - isopropyl ether)
NMR (200MHz, $CDCl_3$) ppm : 0.85-1.00 (3H, m), 1.0-1.4 (2H, m), 1.5-1.9 (2H, m), 2.23 (1H, m), 3.19 (2H, m), 3.47-3.80 (2H, m), 3.75 (3H, s), 3.77 (3H, s), 5.09 (1H, dd, J = 14, 6.2 Hz), 5.28 (1H, dd, J = 14, 7.6 Hz), 5.99 (1H, d, J = 2.2 Hz), 6.13 (1H, d, J = 2.2 Hz), 6.36 (1H×1/2, t, J = 2.2 Hz), 6.40 (1H×1/2, t, J = 2.2 Hz), 6.76 (1H×1/2, dd, J = 8.2, 2.2 Hz), 7.09 (1H ×1/2, d, J = 2.2 Hz), 7.25-7.62 (4H, m), 8.91 (1H, m)
Anal. Calcd for $C_{30}H_{29}N_3O_4Cl_2$
Calculated (%): C 63.61, H 5.16, N 7.42
Found (%): C 63.20, H 5.15, N 7.58

(Reference Example 182)

(±)-5-(3,4-Dichlorophenyl)-7-(3,5-dimethoxybenzyl)-6,7,8,9,10,11-hexahydro-9-(1-methylethyl)-6,13-dioxo-13H-[1,4] diazocino[2,1-g][1,7]naphthylidine

Using the compound obtained in the Reference Example 163, the reaction and treatment were performed as in the case of the Reference Example 69. Then, the title compound was obtained as colorless crystals.
Melting point; 202-205°C (re-crystallization from ethyl acetate - isopropyl ether)
NMR (200MHz, $CDCl_3$) ppm : 0.85 (6H, m), 1.4-2.0 (3H, m), 2.15 (1H, m), 3.18 (2H, m), 3.4-3.8 (2H, m), 3.75 (3H, s), 3.77 (3H, s), 5.11 (1H, dd, J = 14, 6.4 Hz), 5.28 (1H, dd, J = 14, 6.2 Hz), 5.9-6.8 (3.5H, m), 7.0-7.6 (4.5H, m), 8.91 (1H, m)

(Reference Example 183)

| | | |
|---|---|---|
| (1) | Compound in the Reference Example 72 | 10.0 mg |
| (2) | Lactose | 60.0 mg |
| (3) | Corn starch | 35.0 mg |
| (4) | Hydroxypropyl methyl cellulose | 3.0 mg |
| (5) | Magnesium stearate | 2.0 mg |

A mixture of the compound (10 mg) obtained in the Reference Example 72, lactose (60 mg) and corn starch (35 mg) was granulated using 0.03 ml of an aqueous solution of 10 % w/w hydroxypropyl methyl cellulose (3.0 mg as hydroxypropyl methyl cellulose), and then was dried at 40°C and passed through meshes. The obtained granules were mixed with 2.0 mg of magnesium stearate and pressed. The obtained uncoated tablets were coated with sugarcoating of an aqueous suspension of sucrose, titanium dioxide, talc and gum arabic. The coating tablets were obtained by glazing the coated tablets with bee wax.

(Reference Example 184)

| | | |
|---|---|---|
| 1. | Compound of Reference Example 72 | 10.0 mg |
| 2. | Lactose | 70.0 mg |
| 3. | Cornstarch | 50.0 mg |
| 4. | Soluble starch | 7.0 mg |
| 5. | Magnesium stearate | 3.0 mg |

10.0 mg of the compound obtained in the Reference Example 72 and 3.0 mg of magnesium stearate were prepared into granule, using 0.07 ml of an aqueous solution of soluble starch (7.0 mg on a soluble starch basis). The

resulting granule was dried and mixed with 70.0 mg of lactose and 50.0 mg of cornstarch. The resulting mixture was compressed to give a tablet.

(Reference Example 185)

| | | |
|---|---|---|
| 1. | Compound of Reference Example 176 | 10.0 mg |
| 2. | Lactose | 60.0 mg |
| 3. | Cornstarch | 35.0 mg |
| 4. | Hydroxypropylmethyl cellulose | 3.0 mg |
| 5. | Magnesium stearate | 2.0 mg |

A mixture of 10.0 mg of the compound obtained in the Reference Example 176, 60.0 mg of lactose and 35.0 mg of cornstarch was prepared into granule, using 0.03 ml of an aqueous 10 wt % solution of hydroxypropylmethyl cellulose (3.0 mg on a hydroxypropylmethyl cellulose basis). The resulting granule was dried at 40 °C and filtered through a sieve. The resulting granule was mixed with 2.0 mg of magnesium stearate, and the mixture was compressed. The resulting raw tablet was coated with a sugar coating of an aqueous suspension of sucrose, titanium dioxide, talc and gum arabic. The coated tablet was glossed with bee wax to give a coated tablet.

(Reference Example 186)

| | | |
|---|---|---|
| 1. | Compound of Reference Example 176 | 10.0 mg |
| 2. | Lactose | 70.0 mg |
| 3. | Cornstarch | 50.0 mg |
| 4. | Soluble starch | 7.0 mg |
| 5. | Magnesium stearate | 3.0 mg |

10.0 mg of the compound recovered in the Reference Example 176 and 3.0 mg of magnesium stearate were prepared into granule, using 0.07 ml of an aqueous solution of soluble starch (7.0 mg on a soluble starch basis). The resulting granule was dried and mixed with 70.0 mg of lactose and 50.0 mg of cornstarch. The resulting mixture was compressed to give a tablet.

(Reference Example 187)

| | | |
|---|---|---|
| 1. | Compound No. 2 | 10.0 mg |
| 2. | Lactose | 60.0 mg |
| 3. | Cornstarch | 35.0 mg |
| 4. | Hydroxypropylmethyl cellulose | 3.0 mg |
| 5. | Magnesium stearate | 2.0 mg |

A mixture of 10.0 mg of the compound No. 2, 60.0 mg of lactose and 35.0 mg of cornstarch was prepared into granule, using 0.03 ml of an aqueous 10 wt % solution of hydroxypropylmethyl cellulose (3.0 mg on a hydroxypropylmethyl cellulose basis). The resulting granule was dried at 40°C and filtered through a sieve. The resulting granule was mixed with 2.0 mg of magnesium stearate, and the mixture was compressed. The resulting raw tablet was coated with a sugar coating of an aqueous suspension of sucrose, titanium dioxide, talc and gum arabic. The coated tablet was glossed with bee wax to give a coated tablet.

(Reference Example 188)

| | | |
|---|---|---|
| 1. | Compound No. 1 | 10.0 mg |
| 2. | Lactose | 70.0 mg |
| 3. | Cornstarch | 50.0 mg |
| 4. | Soluble starch | 7.0 mg |
| 5. | Magnesium stearate | 3.0 mg |

10.0 mg of the compound No. 1 and 3.0 mg of magnesium stearate were prepared into granule, using 0.07 ml of an aqueous solution of soluble starch (7.0 mg on a soluble starch basis). The resulting granule was dried and mixed with 70.0 mg of lactose. and 50.0 mg of cornstarch. The resulting mixture was compressed to give a tablet.

(Reference Example 189)

| | |
|---|---|
| Morphine hydrochloride | 200 mg |
| Sodium benzoate | 200 mg |
| Distilled water for injections | 2 ml in final total volume |

Morphine hydrochloride and sodium benzoate were dissolved in distilled water for injections, to prepare an aseptic injection.

(Reference Example 190)

| | |
|---|---|
| Morphine hydrochloride | 200 mg |
| Sodium benzoate | 500 mg |
| Distilled water for injections | 2 ml in final total volume |

Morphine hydrochloride and sodium benzoate were dissolved in distilled water for injections, to prepare an aseptic injection.

(Reference Example 191)

| | |
|---|---|
| Morphine hydrochloride | 300 mg |
| Sodium benzoate | 700 mg |
| Hydrochloric acid | q.s. |
| Distilled water for injections | 2 ml in final total volume |

Morphine hydrochloride and sodium benzoate were dissolved in distilled water for injections. The resulting solution was adjusted to pH 6, using hydrochloric acid, followed by aseptic preparation and subsequent sterilization under heating at high pressure.

(Reference Example 192)

| | |
|---|---|
| Morphine hydrochloride | 400 mg |
| Sodium benzoate | 500 mg |

(continued)

| | |
|---|---|
| Glucose | 400 mg |
| Distilled water for injections | 2 ml in final total volume |

Morphine hydrochloride, sodium benzoate and glucose were dissolved in distilled water for injections, to prepare an aseptic injection, followed by sterilization under heating at high pressure.

(Reference Example 193)

| | |
|---|---|
| Morphine hydrochloride | 400 mg |
| Sodium benzoate | 400 mg |
| Benzyl alcohol | 20 mg |
| Distilled water for injections | 2 ml in final total volume |

Morphine hydrochloride, sodium salicylate and benzyl alcohol were dissolved in distilled water for injections, to prepare an aseptic injection, followed by sterilization under heating at high pressure.

(Reference Example 194)

| | |
|---|---|
| Morphine hydrochloride | 500 mg |
| Sodium benzoate | 300 mg |
| Sodium salicylate | 300 mg |
| Distilled water for injections | 2 ml in final total volume |

Morphine hydrochloride, sodium benzoate and sodium salicylate were dissolved in distilled water for injections, to prepare an aseptic injection, followed by sterilization under heating at high pressure.

(Reference Example 195)

Preparation of morphine granule of rapid release type

Formulation examples of the rapid release type are shown below.

| Ingredient | Formulation 1 | Formulation 2 | Formulation 3 | Formulation 4 | Formulation 5 |
|---|---|---|---|---|---|
| Morphine hydrochloride | 24 mg | 48 mg | 48 mg | 24 mg | 48 mg |
| D-Mannitol | 14 | 20 | 20 | - | - |
| Crystal cellulose | - | - | - | 14.2 | 20 |
| Cornstarch | 6.7 | 6.5 | 6.5 | 4.5 | 4.5 |
| Low-substitution hydroxypropyl cellulose | 1.5 | 1.5 | 1.5 | 1.5 | 1.5 |
| Hydroxypropyl cellulose | 1.5 | 1.5 | 1.5 | 1.0 | 1.0 |
| Polyvinylpyrrol idone | - | - | - | 3.0 | 3.0 |
| Polyethylene glycol 6000 | - | - | - | 0.5 | 0.5 |
| Tartaric acid | 2.0 | 2.0 | - | 1.0 | 1.0 |
| Citric acid | - | - | 2.0 | - | - |
| Magnesium stearate | 0.3 | 0.5 | 0.5 | 0.3 | 0.5 |

(continued)

| Ingredient | Formulation 1 | Formulation 2 | Formulation 3 | Formulation 4 | Formulation 5 |
|---|---|---|---|---|---|
| Total | 50 mg | 80 mg | 80 mg | 50 mg | 80 mg |

| 1. | |
|---|---|
| Morphine hydrochloride | 48 g |
| D-Mannitol | 28 |
| Cornstarch | 13.4 |
| Low-substitution hydroxypropyl cellulose | 3 |

The above ingredients were mixed together, followed by addition of an aqueous solution (30 g) containing 3 g of hydroxypropyl cellulose and 4 g of tartaric acid. The resulting mixture was kneaded together in a mortar. The resulting kneaded mixture was dried under reduced pressure (40 °C, 16 hours) and was then subjected to size reduction through a sieve of 16 meshes. To 89.5 g of the resulting size-reduced powder was added 0.54 g of magnesium stearate, to prepare granule 1 of rapid release type.

| 2. | |
|---|---|
| Morphine hydrochloride | 48 g |
| D-Mannitol | 20 |
| Cornstarch | 6.5 |
| Low-substitution hydroxypropyl cellulose | 1.5 |

The above ingredients were mixed together, followed by addition of an aqueous solution (15 g) containing 1.5 g of hydroxypropyl cellulose and 2 g of tartaric acid. The resulting mixture was kneaded together in a mortar. The resulting kneaded mixture was dried under reduced pressure (40 °C, 16 hours) and was then subjected to size reduction through a sieve of 16 meshes. To 71.5 g of the resulting size-reduced powder was added 0.45 g of magnesium stearate, to prepare granule 2 of rapid release type.

| 3. | |
|---|---|
| Morphine hydrochloride | 48 g |
| Crystal cellulose | 28.4 |
| Cornstarch | 9 |
| Low-substitution hydroxypropyl cellulose 3 | |
| Polyethylene glycol 6000 | 1 |

The above ingredients were mixed together, followed by addition of an aqueous solution (25 g) containing 2 g of hydroxypropyl cellulose, 6 g of polyvinylpyrrolidone and 2 g of tartaric acid. The resulting mixture was kneaded together in a mortar. The resulting kneaded mixture was dried under reduced pressure (40 °C, 16 hours) and was then subjected to size reduction through a sieve of 16 meshes. To 89.5 g of the resulting size-reduced powder was added 0.54 g of magnesium stearate, to prepare granule 3 of rapid release type.

| 4. | |
|---|---|
| Morphine hydrochloride | 48 g |
| Crystal cellulose | 20 |
| Cornstarch | 4.5 |
| Low-substitution hydroxypropyl cellulose 1.5 | |
| Polyethylene glycol 6000 | 0.5 |

The above ingredients were mixed together, followed by addition of an aqueous solution (14 g) containing 1 g of hydroxypropyl cellulose, 3 g of polyvinylpyrrolidone and 1 g of tartaric acid. The resulting mixture was kneaded

together in a mortar. The resulting kneaded mixture was dried under reduced pressure (40 °C, 16 hours) and was then subjected to size reduction through a sieve of 16 meshes. To 71.5 g of the resulting size-reduced powder was added 0.45 g of magnesium stearate, to prepare granule 4 of rapid release type.

(Reference Example 196)

Preparation of morphine granule of sustained release type (release-controlled type)

1. Preparation of element (nucleus) granule

| | |
|---|---|
| Morphine hydrochloride<br>Crystal cellulose<br>Cornstarch<br>Low-substitution hydroxypropyl cellulose 36<br>Hydroxypropyl cellulose<br>Polyvinylpyrrolidone 72 | 780 g<br>144<br>108<br><br>24 |

The above ingredients were mixed together, followed by addition of an aqueous solution (290 g) containing 12 g of polyethylene glycol 6000 and 24 g of tartaric acid, followed by kneading with a vertical granulator (Type FM-VG-10, Powlec). The resulting mixture was extruded with a dome gran (Type DG-L 1, Fuji Powder) and particulated with a mulmerizer (QJ-230). The resulting particle was dried under reduced pressure (40 °C, 16 hours) and was then filtered through a sieve and size-reduced, to give an element (nucleus) granule of a particle size of 500 to 1250 μm at about 1050 g.

2. Production of undercoated granule

22.5 g of hydroxypropylmethyl cellulose and 0.45 g of tartaric acid were dissolved in hydrous ethanol of 340 ml (80 % ethanol (W/W)), to prepare an undercoating solution of 4.05 g, in which talc was dispersed. 450 g of the element (nucleus) granule obtained above in 1 was placed in a fluid granulator of Wooster type (Type FD-3, Powlec), followed by spraying of the undercoating solution, to recover an undercoated granule of about 470 g.

3. Formulation examples of release-controlled film solution (coating film solution)

| Ingredient | Formulation 1 | Formulation 2 | Formulation 3 |
|---|---|---|---|
| Ethyl cellulose (45 cps) | 11.6 mg | 12.2 mg | 12.6 mg |
| Crosslinking type polyacrylate polymer (Hi-bis Wako 103) | 1.6 | 1.6 | 1.6 |
| Hydroxypropyl cellulose | 4 | 4 | 4 |
| Polyethylene glycol 6000 | 2.4 | 1.8 | 1.4 |
| Tartaric acid | 0.4 | 0.4 | 0.4 |
| Cetyl alcohol | 0.7 | 0.7 | 0.7 |
| Talc | 6 | 6 | 6 |
| Ethanol/water (90/10 w/w) | 480 | 480 | 480 |
| Total | 506.7 mg | 506.7 mg | 506.7 mg |

i. Production Example 1 of morphine granule of sustained release type

200 g of the undercoated granule obtained above in 2 was placed in a fluid granulator of Wooster type (Type FD-3, Powlec), followed by spraying of the formulation 1 of the release-controlled film solution, to give a sustained

release granule. Herein, the coating was at 25 % by weight as a solid basis to the undercoated granule. After filtration through a sieve and size reduction, a sustained release granule of a particle size of 600 to 1600 µm was recovered at about 250 g. 0.25 g of talc was mixed with 250 g of the resulting granule, to give sustained release granule 1.

ii. Production Example 2 of morphine granule of sustained release type

200 g of the undercoated granule recovered above in 2 was placed in a fluid granulator of Wooster type (Type FD-3, Powlec), to give a sustained release granule of about 250 g, using the formulation 2 of the release-controlled film solution, by the same method as in i.

(Reference Example 197)

Formulation Example of morphine capsule

The granule 1 of rapid release type (50 mg) as obtained in the Reference Example 195, 1 and the sustained release granule 2 of 184 mg as obtained in the Reference Example 196, 3, ii. were charged in capsule No. 2, to give a capsule containing 120 mg of morphine hydrochloride.

(Reference Example 198)

Formulation Example of morphine capsule

The granule 4 of rapid release type (40 mg) as obtained in the Reference Example 195, 4 and the sustained release granule 1 of 69 mg as recovered in the Reference Example 196, 3, i. were charged in capsule No. 3, to give a capsule containing 60 mg of morphine hydrochloride.

(Reference Example 199)

Formulation Example of morphine capsule

The granule 4 of rapid release type (20 mg) as obtained in the Reference Example 195,4 and the sustained release granule 2 of 35 mg as obtained in the Reference Example 196, 3, ii. were charged in capsule No. 4, to give a capsule containing 30 mg of morphine hydrochloride.

(Reference Example 200)

Formulation examples of rapid release type are shown below.

| Ingredient | Formulation 1 | Formulation 2 | Formulation 3 |
|---|---|---|---|
| Morphine hydrochloride | 5 mg | 10 mg | 10 mg |
| D-Mannitol | 33 | 28 | 38 |
| Cornstarch | 6.7 | 6.7 | 6.65 |
| Low-substitution hydroxypropyl cellulose | 1.5 | 1.5 | 1.5 |
| Hydroxypropyl cellulose | 1.5 | 1.5 | 1.5 |
| Tartaric acid | 2.0 | --- | 2.0 |
| Citric acid | --- | 2.0 | --- |
| Magnesium stearate | 0.3 | 0.3 | 0.35 |
| Total | 50 mg | 50 mg | 60 mg |

| 1. | |
|---|---|
| Morphine hydrochloride | 50 g |

(continued)

| 1. | |
|---|---|
| D-Mannitol | 330 |
| Cornstarch | 67 |
| Low-substitution hydroxypropyl cellulose 15 | |

The above ingredients were mixed together, followed by addition of an aqueous solution (150 g) containing 15 g of hydroxypropyl cellulose and 20 g of tartaric acid and subsequent kneading with a vertical granulator (Type FM-VG-10, Powlec). The resulting kneaded mixture was dried under reduced pressure (40 °C for 16 hours) and filtered through a punching screen of 1.0 mm, using a power mill (Type P-3, Showa Science Machine Industry), to prepare a size-reduced powder. To 447 g of the size-reduced powder was added 2.7 g of magnesium stearate, to prepare a granule for charging.
50 mg of the granule for charging was charged in capsule No. 5, using a capsule charger (Type 6F, Zanacy), to prepare about 3000 capsules, each capsule containing 5 mg of morphine hydrochloride.

| 2. | |
|---|---|
| Morphine hydrochloride | 100 g |
| D-Mannitol | 380 |
| Cornstarch | 66.5 |
| Low-substitution hydroxypropyl cellulose 15 | |

The above ingredients were mixed together, followed by addition of an aqueous solution (150 g) containing 15 g of hydroxypropyl cellulose and 20 g of tartaric acid and subsequent kneading with a vertical granulator (Type FM-VG-10, Powlec). The resulting mixture was dried under reduced pressure (40 °C for 16 hours) and filtered through a punching screen of 1.0 mm, using a power mill (Type P-3, Showa Science Machine Industry), to prepare a size-reduced powder. To 536 g of the size-reduced powder was added 3.2 g of magnesium stearate, to prepare a granule for charging.
60 mg of the granule for charging was charged in capsule No. 5, using a capsule charger (Type 6F, Zanacy), to prepare about 1500 capsules, each capsule containing 10 mg of morphine hydrochloride.
Additionally, 30 mg of the granule for charging was similarly charged in capsule No. 5, to prepare about 2000 capsules, each capsule containing 5 mg of morphine hydrochloride.

(Reference Example 201)

Formulation examples of apomorphine sublingual tablet are shown below.

| 1. | |
|---|---|
| | % by weight |
| Apomorphine hydrochloride | 2.00 |
| Mannitol | 66.67 |
| Ascorbic acid | 3.33 |
| Citric acid | 2.00 |
| Avicel PH102 (crystal cellulose) | 15.00 |
| Methocel E4M (hydroxypropylmethyl cellulose) | 10.00 |
| Aspartame | 0.67 |
| Magnesium stearate | 0.33 |

| 2. | |
|---|---|
| | % by weight |
| Apomorphine hydrochloride | 2.66 |
| Mannitol | 66.00 |

(continued)

| 2. | |
|---|---|
| | % by weight |
| Ascorbic acid | 3.33 |
| Citric acid | 2.00 |
| Avicel PH102 (crystal cellulose) | 15.00 |
| Methocel E4M (hydroxypropylmethyl cellulose) | 10.00 |
| Aspartame | 0.67 |
| Magnesium stearate | 0.33 |

| 3. | |
|---|---|
| | % by weight |
| Apomorphine hydrochloride | 3.33 |
| Mannitol | 65.34 |
| Ascorbic acid | 3.33 |
| Citric acid | 2.00 |
| Avicel PH102 (crystal cellulose) | 15.00 |
| Methocel E4M (hydroxypropylmethyl cellulose) | 10.00 |
| Aspartame | 0.67 |
| Magnesium stearate | 0.33 |

(Reference Example 202)

Production of (3R, 4S, 5S, 6R)-5-methoxy-4-[(2R, 3R)-2-methyl-3-(3-methyl-2-butenyl)oxylanyl]-1-oxaspiro[2.5]oct-6-yl(chloroacetyl)carbamate

1. Production of fumagirol from fumagirin dicyclohexylammonium

Fumagirin dicyclohexylammonium (640 g) was added to 1.5N sodium hydroxide solution (2133 ml), for stirring at about 25 °C for 24 hours. To the resulting mixture solution were added ethyl acetate (1600 ml) and sodium chloride (288 g), for stirring for about 10 minutes. The resulting deposited crystals were filtered and rinsed in ethyl acetate. The filtrate was isolated to give an organic layer. Then, the organic layer was washed with water and subsequently washed with an aqueous solution of ammonium sulfate dissolved in 0.4N sulfuric acid and in aqueous 5 % ammonium sulfate solution. Magnesium sulfate (anhydrous) was added to the organic layer for dehydration. After filtration of magnesium sulfate, the organic layer was concentrated under reduced pressure to give a yellow dark brown oil. Subsequently, the oil was dissolved in a mixture solution of ethylene chloride and ethyl acetate (3:1 (v/v)), for adsorption on a silica gel chromatography column, followed by elution, using a mixture solution of methylene chloride and ethyl acetate, to give an effective fraction. The effective fraction was concentrated under reduced pressure to recover a pale yellow oil. n-Hexane was added to the oil for dissolution at about 40 °C. Then, the resulting solution was gradually cooled to about 3 °C and stirred for about 4 hours, to give crystals. After the crystals were filtered, washed with cooled n-hexane and dried in vacuum at about 30 °C for about 12 hours, fumagirol crystal was obtained at 198 g (yield: 70 %).

2. Production of (3R, 4S, 5S, 6R)-5-methoxy-4-[(2R, 3R)-2-methyl-3-(3-methyl-2-butenyl)oxylanyl]-1-oxaspiro[2.5]oct-6-yl(chloroacetyl)carbamate from fumagirol

198 g of fumagirol obtained above in 1 was added to methylene chloride (3260 ml) for dissolution. After the solution was cooled to about -3 °C in nitrogen atmosphere, α-chloroacetylisocyanate (92 g) was added dropwise to the resulting solution over about 10 minutes, while α-chloroacetyl isocyanate was retained at an inner temperature of 4 °C. Subsequently, the solution was stirred at -5 to 4 °C for about 20 minutes. To the reaction solution was added water (1160 ml) cooled below 5 °C. After the resulting solution was left to stand for about 15 minutes, the organic layer and the aqueous layer as obtained after separation were extracted twice in methylene chloride. The obtained organic layers were combined together. The resulting organic layer was washed with aqueous saline (20 % (w/v)). To the

organic layer was added magnesium sulfate (anhydrous) for dehydration. After magnesium sulfate was filtered, the organic layer was concentrated under reduced pressure, to give an oil. The oil was dissolved in a mixture solution of n-hexane and ethyl acetate (3:1 (v/v)), for adsorption on a silica gel chromatography column. The adsorbed fraction was eluted, using a mixture solution of n-hexane and ethyl acetate, to give and concentrate an effective fraction under reduced pressure, to give a dry solid of crude (3R, 4S, 5S, 6R)-5-methoxy-4-[(2R, 3R)-2-methyl-3-(3-methyl-2-butenyl) oxylanyl]-1-oxaspiro[2.5]oct-6-yl(chloroacetyl)carbamate in pale yellow. Subsequently, isopropyl ether was added to the dry solid of crude (3R, 4S, 5S, 6R)-5-methoxy-4-[(2R, 3R)-2-methyl-3-(3-methyl-2-butenyl)oxylanyl]-1-oxaspiro [2.5]oct-6-yl(chloroacetyl)carbamate to dissolve the carbamate at about 40 °C, followed by addition of active charcoal. After stirring for about 10 minutes, active charcoal was filtered. The resulting filtrate was cooled to about 15 °C, followed by addition of seed crystals for crystallization.
After the resulting crystals were filtered, rinsed in cooled isopropyl ether and dried in vacuum at about 30 °C, a dry crystal was recovered. The dry crystal was added to n-hexane cooled to about 10 °C with stirring, followed by dropwise addition of a small volume of distilled water with stirring. The mixture was stirred for about 4 hours and after that the crystals were filtered. The crystals were dried in vacuum at about 30 °C for about 15 hours, to give (3R, 4S, 5S, 6R)-5-methoxy-4-[(2R, 3R)-2-methyl-3-(3-methyl-2-butenyl)oxylanyl]-1-oxaspiro[2.5]oct-6-yl(chloroacetyl)carbamate in white crystals of 192 g (yield: 68 %).

(Reference Example 203)

| | | |
|---|---|---|
| 1. | Ondansetron | 10.0 mg |
| 2. | Lactose | 70.0 mg |
| 3. | Cornstarch | 50.0 mg |
| 4. | Soluble starch | 7.0 mg |
| 5. | Magnesium stearate | 3.0 mg |

Ondansetron (10.0 mg) and magnesium stearate (3.0 mg) were granulated with soluble starch in aqueous solution (0.07 ml) (7.0 mg on a soluble starch basis) . The resulting granule was dried and mixed with lactose (70.0 mg) and cornstarch (50.0 mg). The mixture is compressed to give a tablet.

(Reference Example 204)

| | | |
|---|---|---|
| 1. | Dexamethazone | 10.0 mg |
| 2. | Lactose | 70.0 mg |
| 3. | Cornstarch | 50.0 mg |
| 4. | Soluble starch | 7.0 mg |
| 5. | Magnesium stearate | 3.0 mg |

Dexamethazone (10.0 mg) and magnesium stearate (3.0 mg) were granulated with soluble starch in aqueous solution (0.07 ml) (7.0 mg on a soluble starch basis). The resulting granule was dried and mixed with lactose (70.0 mg) and cornstarch (50.0 mg). The mixture is compressed, to give a tablet.

(Reference Example 205)

Production of apomorphine of rapid oral disintegration type (rapid dispersion dosage formulation)

a. Production of apomorphine hydrochloride salt in 20 % dispersion

Gelatin (792 g) and mannitol (594 g) were thoroughly mixed together in the bowl of a vacuum mixer and dispersed in a part of distilled water (16 kg). Subsequently, the resulting mixture was heated to 40 °C ± 2 °C, for homogenization for 10 minutes. Subsequently, the mixture was cooled to ambient temperature (20 - 24 °C). At the cooled stage, apomorphine hydrochloride salt (360 g) was added. The mixture was homogenized to ensure the solubilization of the drug. While gradually adding citric acid (166.32 g) with stirring, the solution was adjusted to pH 3.0.

The remaining water (87.68 g) was added into the mixer to homogenize the bulk mixture and ensure the solubilization, to give the dispersion.

b. Production of apomorphine hydrochloride unit (10 mg)

500 mg of the apomorphine hydrochloride salt in 2.0 % dispersion as generated above in a. was divided in individual blister pockets of 16-mm pocket inner diameter in series. The blister laminate was composed of PVC (200 μm) coated with PVdC at a ratio of 40 g per 1 $m^2$. The product was immediately frozen in a liquid nitrogen freeze tunnel. The frozen product was then retained at -20 °C for 12 hours at minimum, prior to freeze-drying in a freeze dryer using a drying temperature of + 10 °C and a tower pressure of 0.5 hPa. Subsequently, the freeze-dried unit was examined so as to check the presence or absence of lethal defects. The remaining batch was sealed in a lid foil comprising a paper/foil laminate (20-μm aluminum). Subsequently, each blister was coded with batch No. and was then placed in a small package. By completely sealing the opening end of the small package, the blister was packaged in the small package. Each of the small packages was subsequently labeled with the product name, batch No., production date and supplier name.

| Ingredient | Weight (mg) | % by weight to composition |
|---|---|---|
| Distilled water USP/EP* | 446.880 | 89.4 |
| Apomorphine HCl EP/EP | 10.000 | 2.0 |
| Gelatin EP/USNF | 22.000 | 4.4 |
| Mannitol EP/USP | 16.500 | 3.3 |
| Citric acid EP/USP | 4.620 | 0.9 |
| Total | 500.000 | 100.0 |

*: meaning the removal of water during the freeze-drying process.

The following Reference Examples are additional lists of formulation examples possibly produced by the method.

| Ingredient | Weight (mg) | % by weight to composition |
|---|---|---|
| Distilled water EP/USP* | 438.500 | 87.70 |
| Apomorphine HCl BP/EP | 10.000 | 2.0 |
| Gelatin EP/USNF | 25.000 | 5.00 |
| Mannitol EP/USP | 20.000 | 4.00 |
| Citric acid EP/USP | 1.500 | 0.30 |
| Aspartame EP/USNF | 2.500 | 0.50 |
| Peppermint flavor | 2.500 | 0.50 |
| Total | 500.000 | 100.0 |

*: meaning the removal of water during the freeze-drying process.

| Ingredient | Weight (mg) | % by weight to composition |
|---|---|---|
| Distilled water EP/USP* | 215.000 | 86.00 |
| Apomorphine HCl BP/EP | 10.000 | 4.00 |
| Gelatin EP/USNF | 11.500 | 4.60 |
| Mannitol EP/USP | 10.000 | 4.00 |
| Citric acid EP/USP | 1.500 | 0.60 |

*: meaning the removal of water during the freeze-drying process.

(continued)

| Ingredient | Weight (mg) | % by weight to composition |
|---|---|---|
| Aspartame EP/USNF | 2.000 | 0.80 |
| Total | 250.000 | 100.0 |

| Ingredient | Weight (mg) | % by weight to composition |
|---|---|---|
| Distilled water EP/USP* | 441.000 | 88.20 |
| Apomorphine HCl BP/EP | 10.000 | 2.0 |
| Gelatin EP/USNF | 25.000 | 5.00 |
| Mannitol EP/USP | 20.000 | 4.00 |
| Citric acid EP/USP | 1.500 | 0.30 |
| Aspartame EP/USNF | 2.500 | 0.50 |
| Total | 500.000 | 100.0 |

*: meaning the removal of water during the freeze-drying process.

## Examples

### Example 1

Action of (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthylidine (compound No. 3) orally given to acute vomiting induced by an anticancer agent cisplatin

1. Action of the compound No. 3 orally given to acute vomiting induced by cisplatin in ferrets

[Procedures]

5 to 6 male ferrets (1.2 to 2.3 kg) per one group were used. Cisplatin was intraperitoneally given at 10 mg/10 ml/kg. One hour prior to cisplatin administration, a small volume of a feed was given to the animals. 30 minutes prior to cisplatin administration, predetermined doses of the compound No. 3 were orally given. Similarly, 0.5 % methyl cellulose (MC) solution was orally given to a control animal group under no fasting. The frequency of vomiting was counted in number, up to 5 hours after cisplatin administration.

The compound No. 3 was suspended in 0.5 % methyl cellulose solution to predetermined concentrations, for oral dosing at a ratio of 1 ml per 1 kg body weight. Additionally, 0.5% methyl cellulose solution was orally given at a ratio of 1 ml per 1 kg body weight. The data relating to the frequency of vomiting, the time period (latency) until first vomiting and the time period from the occurrence of vomiting to the termination thereof were statistically analyzed by the Dunnett test, with reference to the difference from the control group.

[Results]

The acute vomiting pattern induced by cisplatin in the ferrets is shown in Fig. 1. In the control group, retching and vomiting started 1.5 ± 0.1 hours (mean ± standard deviation (SD)) after cisplatin administration. 1.5 hours to 2.5 hours after the administration, vomiting frequently occurred. Vomiting continuously occurred thereafter. The frequencies of retching and vomiting induced within the observation period of 5 hours were 126.7 ± 19.4 and 10.7 ± 1.2, respectively. The oral administration of the compound No. 3 at 3 mg/kg significantly suppressed vomiting, which was prominently suppressed at the 10 mg/kg dose. The results are shown in Fig.2 and Table 1.

Table 1

| Pre-treatment | Dose (mg/k g, p.o.) | Number of ferrets with retching /number of tested ferrets | Frequency of retching (mean ± SD) | Time period until first retching (in hour; ± mean SD) | Duration of retching (in hour; mean ± SD) |
|---|---|---|---|---|---|
| 0.5 % MC | - | 6/6 | 126.7±19.4 | 1.5 ± 0.1 | 2.4 ± 0.4 |
| Cpd. No.3 | 1 | 6/6 | 79.0 ± 15.1 | 1.5 ± 0.1 | 1.7 ± 0.4 |
| | 3 | 6/6 | 59.3 ± 12.0 b | 1.6 ± 0.1 | 1.7 ± 0.3 |
| | 10 | 5/6 | 14.8 ± 3.7 b | 2.1 ± 0.6 | 2.4 ± 0.5 |
| Pre-treatment | Dose (mg/k g, p.o.) | Number of ferrets with vomiting /number of tested ferrets | Frequency of vomiting (mean ± SD) | Time period until first vomiting (in hour;, mean ± SD) | Duration of vomiting (in hour;, mean ± SD) |
| 0.5 % MC | - | 6/6 | 10.7 ± 1.2 | 1.5 ± 0.1 | 2.1 ± 0.4 |
| Cpd. No.3 | 1 | 6/6 | 8.3 ± 2.2 | 1.6 ± 0.1 | 1.4 ± 0.4 |
| | 3 | 6/6 | 7.8 ± 1.3 | 1.6 ± 0.1 | 1.6 ± 0.4 |
| | 10 | 5/6 | 3.2 ± 1.0 b | 2.2 ± 0.6 | 1.0 ± 0.4 |

(b: $p < 0.01$)

The compound No. 3 orally administered apparently suppressed the cisplatin-induced acute vomiting in ferrets.

2. Action of the compound No. 3 orally given on cisplatin-induced acute vomiting in dogs

[Procedures]

3 or 4 beagle dogs (7.9 to 13.3 kg), male or female, were used per one group. Cisplatin was intravenously administered at 3 mg/3 ml/kg. One hour before cisplatin dosing, a small volume of a feed was given to the animals. 30 minutes before cisplatin administration, predetermined doses of the compound No. 3 were orally given. Similarly, 0.5 % methyl cellulose solution was orally given to the control animals under no fasting. The frequency of vomiting was counted in number, up to 5 hours after cisplatin dosing.

The compound No. 3 was suspended in 0.5 % methyl cellulose solution to predetermined concentrations, for oral dosing at a ratio of 1 ml per 1 kg body weight. Additionally, the 0.5 % methyl cellulose solution was orally given at a ratio of 1 ml per 1 kg body weight. The data relating to the frequency of vomiting, the time period (latency) until first vomiting and the time period from the occurrence of vomiting to the termination thereof were statistically analyzed by the Dunnett test, with reference to the difference from the control group.

[Results]

The vomiting pattern induced by cisplatin in the dogs is shown in Fig. 3. In the control group, vomiting started 1.3 ± 0.2 hours (mean ± SD) after cisplatin administration. 2 hours to 3 hours after the administration, vomiting frequently occurred. The frequency of vomiting gradually decreased thereafter. The frequency of vomiting induced within the observation period of 5 hours was 13.7 ± 0.3 in number. The oral administration of the compound No. 3 at 3 mg/kg or higher significantly suppressed cisplatin-induced vomiting, which was almost completely suppressed at the 10 mg/kg dose of the compound No. 3. The dose prolonged the latency. The results are shown in Fig.4 and Table 2.

Table 2

| Pre-treatment | Dose (mg/kg, p. o.) | Number of dogs with vomiting/ number of tested ferrets | Frequency of vomiting (mean ± SD) | Time period until first vomiting (in hour;, mean ± SD) | Duration of vomiting (in hour;, mean ± SD) |
|---|---|---|---|---|---|
| 0.5 % MC | - | 3/3 | 13.7 ± 0.3 | 1.3 ± 0.2 | 1.8 ± 0.2 |
| Cpd. No.3 | 1 | 4/4 | 11.3 ± 0.5 | 1.6 ± 0.2 | 1.5 ± 0.2 |
| | 3 | 4/4 | 6.3 ± 0.8 a | 1.9 ± 0.1 | 1.1 ± 0.2 |
| | 10 | 2/4 | 1.0 ± 0.7 a | 3.6 ± 0.8 a | 0.2 ± 0.2 a |

(a: $p < 0.01$)

The compound No. 3 orally administered apparently suppressed the cisplatin-induced acute vomiting in the dogs.

3. Suppression ratio of cisplatin-induced acute vomiting due to the compound No. 3 orally given

The acute retching suppression ratio and acute vomiting suppression ratio were calculated by the following formula. The results are shown in Tables 3 and 4.

[Frequency of retching or vomiting in control] - [Frequency of

retching or vomiting in animals dosed with the compound No. 3]

/ [Frequency of retching or vomiting in control] x 100 (%)

Table 3

| Effects in ferrets | | | |
|---|---|---|---|
| | Dose (mg/kg, p.o.) | Suppression ratio of acute retching | Suppression ratio of acute vomiting |
| Cont. (0.5 % MC) | 1 mg/kg | Frequency of retching: 126.7 | frequency of vomiting: 10.7 |
| Cpd. No.3 | 1 | 38 % NS | 22 % NS |
| | 3 | 53 % b | 27 % NS |
| | 10 | 88 % b | 70 % b |

(b: $p < 0.01$; NS: no statistical significance)

Table 4

| Effects in dogs | | |
|---|---|---|
| | Dose (mg/kg, p.o.) | Suppression ratio of acute vomiting |
| Cont. (0.5 % MC) | 1 mg/kg | frequency of vomiting: 13.7 |
| Cpd. No.3 | 1 | 18 % NS |
| | 3 | 54 % NS |
| | 10 | 93 % b |

(b: $p < 0.01$; NS: no statistical significance)

Example 2

Action of the compound No. 3 intraperitoneally or intravenously given on acute vomiting induced by an anticancer agent cisplatin

1. Action of the compound No. 3 intraperitoneally given on acute vomiting induced by cisplatin in ferrets

[Procedures]

6 to 9 male ferrets (1.2 to 1.7 kg) per one group were used. Cisplatin was intraperitoneally given at 10 mg/kg. 30 minutes prior to cisplatin administration, predetermined doses of the compound No. 3 were intraperitoneally given to the animals under fasting. Similarly, PEG 400 was intraperitoneally given to a control animal group under fasting. The frequency of vomiting was counted in number, up to 5 hours after cisplatin administration.

The compound No. 3 was dissolved in PEG 400, for intraperitoneal dosing at a ratio of 1 ml per 1 kg body weight. Additionally, PEG 400 was intraperitoneally given at a ratio of 1 ml per 1 kg body weight. The data relating to the frequency of vomiting, the latency and the time period from the occurrence of vomiting to the termination thereof were statistically analyzed by the t test, with reference to the difference from the control group.

[Results]

Vomiting started in the control group, 1.3 ± 0.2 hours (mean ± SD) after cisplatin administration in the control animals. In the control group, the frequencies of retching and vomiting incurred within the observation period were 123.3 ± 12.7 and 11.2 ± 1.6 in number, respectively. At 1 mg/kg, the compound No. 3 significantly suppressed cisplatin-induced retching and actual vomiting (Table 5).

Table 5

| Pre-treatment | Dose (mg/kg, i.p.) | Number of ferrets with retching/ number of tested ferrets | Frequency of retching (mean ± SD) | Time period until first retching (in minute, mean ± SD) | Duration of retching (in minute, mean ± SD) |
|---|---|---|---|---|---|
| PEG 400 | - | 6/6 | 123.3 ± 12.7 | 1.3 ± 0.2 | 2.8 ± 0.2 |
| Cpd. No.3 | 1 | 6/6 | 57.5 ± 6.2 b | 1.5 ± 0.1 | 2.6 ± 0.3 |
| Pre-treatment | Dose (mg/kg, i.p.) | Number of ferrets with vomiting /number of tested ferrets | Frequency of vomiting (mean ± SD) | Time period until first vomiting (in minute, mean ± SD) | Duration of vomiting (in minute, mean ± SD) |
| 0.5 % MC | - | 6/6 | 11.2 ± 1.6 | 1.4 ± 0.2 | 2.7 ± 0.2 |
| Cpd. No.3 | 1 | 6/6 | 5.7 ± 1.0 a | 1.8 ± 0.1 | 1.7 ± 0.3 b |

(a: $p < 0.05$; b: $p < 0.01$)

The compound No. 3 intraperitoneally administered apparently suppressed the cisplatin-induced acute vomiting in the ferrets.

2. Action of the compound No. 3 intravenously given on cisplatin-induced acute vomiting in dogs

[Procedures]

Male or female 3 beagle dogs (7.3 to 12.0 kg) per one group were used. Cisplatin was intravenously administered at 3 mg/3 ml/kg. 30 minutes before cisplatin administration, predetermined doses of the compound No. 3 were intravenously given to the animals under fasting. Similarly, a predetermined volume of PEG 400 was intravenously given to a control animal group under fasting.

The compound No. 3 was dissolved in PEG 400 to predetermined concentrations, for intravenous dosing at a ratio of 1 ml per 1 kg body weight. Additionally, PEG 400 was intravenously given at a ratio of 1 ml per 1 kg body

weight. The data relating to the frequency of vomiting, the latency and the time period from the occurrence of vomiting to the termination thereof were statistically analyzed by the Dunnett test, with reference to the difference from the control group.

[Results]

In the control group, vomiting started, 0.9 ± 0.1 hour (mean ± SD) after cisplatin administration. The frequency of vomiting induced within the observation period of 5 hours was 18.3 ± 1.9 in number. The compound No. 3 at 0.3 mg/kg or higher significantly suppressed cisplatin-induced vomiting, which was almost completely suppressed at the 3 mg/kg dose of the compound No. 3. The compound No. 3 prolonged the latency. The results are shown in Fig.5 and Table 6.

Table 6

| Pre-treatment | Dose (mg/kg, i.v.) | Number of dogs with vomiting /number of tested dogs | Frequency of vomiting (mean ± SD) | Time period until first vomiting (in hour; mean ± SD) | Duration of vomiting (in hour; mean ± SD) |
|---|---|---|---|---|---|
| PEG 400 | - | 3/3 | 18.3 ± 1.9 | 0.9 ± 0.1 | 2.2 ± 0.1 |
| Cpd. No.3 | 0.3 | 3/3 | 12.0 ± 1.5a | 1.6 ± 0.2 | 1.6 ± 0.2 |
| | 1 | 3/3 | 5.3 ± 1.2 a | 1.4 ± 0.3 | 1.7 ± 0.5 |
| | 3 | 1/3 | 0.7 ± 0.7 a | 3.9 ± 1.1 a | 0.1 ± 0.1 a |

(a: $p < 0.01$).

3. Suppression ratio of cisplatin-induced acute vomiting due to the compound No. 3 intraperitoneally or intravenously given

The acute retching suppression ratio and acute vomiting suppression ratio were determined in the same manner as in Example 1,3. The results are shown in Tables 7 and 8.

Table 7

| Effects of intraperitoneal dosing in ferrets | | | |
|---|---|---|---|
| | Dose (mg/kg, i.p.) | Suppression ratio of acute retching | Suppression ratio of acute vomiting |
| Cont. (PEG 400) | 1 mg/kg | frequency of retching: 123.3 | frequency of vomiting: 11.2 |
| Cpd. No.3 | 1 | 53 % b | 49 % a |

(a: $p < 0.05$; b: $p < 0.01$)

Table 8

| Effects of intravenous dosing in dogs | | |
|---|---|---|
| | Dose (mg/kg, i.v.) | Suppression ratio of acute vomiting |
| Cont. (PEG400) | 1 mg/kg | frequency of vomiting: 18.3 |
| Cpd. No.3 | 0.3 | 34 % b |
| | 1 | 71 % b |
| | 3 | 96 % b |

(b: $p < 0.01$)

Example 3

Action of the compound No. 3 orally given on delayed vomiting induced by an anticancer agent cisplatin

1. Action of the compound No. 3 orally given on acute and delayed vomiting induced by cisplatin in ferrets

[Procedures]

Male 4 to 6 ferrets (1.2 to 2.2 kg) per one group were used. Cisplatin was intraperitoneally given at 5 mg/5ml/kg. 30 minutes prior to cisplatin administration and 24 and 48 hours after cisplatin administration, predetermined doses of the compound No. 3 were orally given in case of dosing once daily. 30 minutes prior to cisplatin administration and 12, 24, 36, 48 and 60 hours after cisplatin administration, predetermined doses of the compound No. 3 were orally given in case of dosing twice daily. 30 minutes prior to the dosing of the compound No. 3, a small amount of a feed was given to the animals in any of the cases. Similarly, a predetermined volume of 0.5 % methyl cellulose solution was similarly given orally to a control animal group under no fasting. A predetermined dose of ondansetron was orally given to the animals under fasting. To a control animal group of ondansetron, a predetermined volume of physiological saline was orally given to the animals under fasting. The frequency of vomiting was counted in number, up to 72 hours after cisplatin administration.

The compound No. 3 was suspended in 0.5 % methyl cellulose solution, for oral dosing at predetermined doses at a ratio of 1 ml per 1 kg body weight. An injection of ondansetron at 0.2 w/v % concentration was orally given at a predetermined volume. Additionally, 0.5 % methyl cellulose solution was orally given at a ratio of 1 ml per 1 kg body weight, while physiological saline was orally given at a ratio of 0.5 to 1.5 ml per 1 kg body weight. The data relating to the frequency of vomiting, the time period (latency) until first vomiting and the time period from the occurrence of vomiting to the termination thereof were statistically analyzed by the Dunnett test, with reference to the difference from the control group.

[Results]

The mean of the frequency of retching and vomiting (acute vomiting) in number within 24 hours after the intraperitoneal dosing of 5 mg/kg cisplatin to ferrets was 109.8; the mean of the frequency of retching and vomiting (delayed vomiting) in number 24 hours to 72 hours after the intraperitoneal dosing of 5 mg/kg cisplatin to ferrets was 347.8. The mean of the total frequencies of vomiting was 457.5 within 3 days. The compound No. 3 dosed once daily at 3 mg/kg significantly suppressed such retching and vomiting (suppression ratio: 42 %). The compound No. 3 dosed once daily at 10 mg/kg significantly suppressed acute and delayed vomiting. The suppression ratios of acute vomiting and delayed vomiting were 83 % and 65 %, respectively (Figs. 8 and 9 and Table 11). Further, the compound No. 3 dosed twice daily at 1 mg/kg suppressed delayed vomiting by 70 %, and the mean of the frequencies of acute and delayed vomiting within 3 days was suppressed by 54 %. Still further, the compound No. 3 dosed twice daily at 3 mg/kg prominently suppressed acute and delayed vomiting, while the suppression ratios were 82 % for acute vomiting and 59 % for delayed vomiting. The results are shown in Figs. 6 and 7 and Tables 9 and 10.

Table 9

Compound No. 3 dosed twice daily

| Pre-treatment | Dose (mg/kg × 2, p.o.) | On day 0 (0-24 hours) | | On days 1-2 (24-48 hours) | |
|---|---|---|---|---|---|
| | | number of ferrets with vomiting/ number of tested ferrets | frequency of retching and vomiting (mean ± SD) | number of ferrets with vomiting/ number of tested ferrets | frequency of retching and vomiting (mean ± SD°) |
| 0.5 % MC | - | 4/4 | 33.0 ± 5.0 | 4/4 | 354.8 ± 18.6 |
| Cpd. No.3 | 0.03 | 3/4 | 24.8 ± 14.8 | 4/4 | 245.8 ± 34.3 |
| | 0.1 | 3/4 | 26.0 ± 11.2 | 4/4 | 222.0 ± 20.7 a |
| | 0.3 | 0/4 | 0 | 4/4 | 209.3 ± 14.0 b |
| | 1 | 4/4 | 71.8 ± 20.6 | 4/4 | 108.0 ± 9.6 b |
| | 3 | 2/4 | 6.0 ± 4.5 | 4/4 | 145.0 ± 50.5 b |

| Pre-treatment | Dose (mg/kg × 2, p.o.) | Total frequency of retching and vomiting (on days 0-2; 0-72 hours) | Latency (in hour; mean ± SD) | Duration (in hour; mean ± SD) |
|---|---|---|---|---|

| | | | | |
|---|---|---|---|---|
| 0.5 % MC | - | 387.8 ± 21.2 | 6.2 ± 0.5 | 63.3 ± 1.2 |
| Cpd. No.3 | 0.03 | 270.5 ± 43.7 | 13.4 ± 4.9 | 50.8 ± 7.3 |
| | 0.1 | 248.0 ± 10.7 a | 13.3 ± 6.3 | 54.8 ± 6.1 |
| | 0.3 | 209.3 ± 14.0 b | 34.5 ± 3.4 b | 31.1 ± 1.9 a |
| | 1 | 179.8 ± 21.0 b | 3.4 ± 1.1 | 64.8 ± 2.2 |
| | 3 | 150.8 ± 53.1 b | 25.8 ± 10.7 | 40.9 ± 13.9 |

(a: $p < 0.05$; b: $p < 0.01$)

Table 10

Ondansetron dosed twice daily

| Pre-treatment | Dose (mg/kg × 2, p.o.) | On day 0 (0-24 hours) | | On days 1-2(24-48 hours) | |
|---|---|---|---|---|---|
| | | number of ferrets with vomiting /number of tested ferrets | frequency of retching and vomiting (mean ± SD°) | number of ferrets with vomiting /number of tested ferrets | frequency of retching and vomiting (mean ± SD) |
| Physiological saline | - | 5/5 | 66.8 ± 20.2 | 5/5 | 323.8 ± 34.6 |
| Ondansetron | 1 | 5/5 | 70.2 ± 25.2 | 5/5 | 327.4 ± 88.8 |
| | 3 | 4/6 | 27.8 ± 13.1 | 6/6 | 221.7 ± 62.9 |

| Pre-treatment | Dose (mg/kg × 2, p.o.) | Total frequency of retching and vomiting (on days 0~2; 0-72 hours) | Latency (in hour; mean ± SD) | Duration (in hour; mean ± SD) |
|---|---|---|---|---|
| Physiological saline | - | 390.6 ± 30.2 | 7.3 ± 1.6 | 62.4 ± 2.4 |
| Ondansetron | 1 | 397.6 ± 72.0 | 8.7 ± 0.9 | 56.5 ± 2.6 |
| | 3 | 249.5 ± 74.2 | 15.7 ± 5.6 | 44.2 ± 8.4 |

(a: $p < 0.05$; b: $p < 0.01$)

Table 11

Compound No. 3 dosed once daily

| Pre-treatment | Dose (mg/kg, p.o.) | On day 0 (0-24 hours) | | On days 1-2(24-48 hours) | |
|---|---|---|---|---|---|
| | | number of ferrets with vomiting/ number of tested ferrets | frequency of retching and vomiting (mean ± SD) | number of ferrets with vomiting/ number of tested ferrets | frequency of retching and vomiting (mean ± SD) |
| 0.5 % MC | - | 4/4 | 109.8 ± 19.6 | 4/4 | 347.8 ± 74.6 |
| Cpd. No.3 | 3 | 4/4 | 56.8 ± 18.4 | 4/4 | 209.5 ± 23.6 |
| | 10 | 3/4 | 19.0 ± 11.0 b | 4/4 | 120.5 ± 5.0 a |

| Pre-treatment | Dose (mg/kg × 2, p.o.) | Total frequency of retching and actual vomiting (on days 0-2; 0-72 hours) | Latency (in hour; mean ± SD) | Duration (in hour; mean ± SD) |
|---|---|---|---|---|
| 0.5 % MC | - | 457.5 ± 73.6 | 4.1 ± 1.4 | 67.3 ± 1.7 |
| Cpd. No.3 | 3 | 266.3 ± 20.6 a | 7.8 ± 1.1 | 61.2 ± 1.2 |
| | 10 | 139.5 ± 8.3 b | 16.2 ± 5.6 | 51.4 ± 7.0 a |

(a: $p < 0.05$; b: $p < 0.01$)

The oral administration of the compound No. 3 twice daily at 0.1 mg/kg significantly suppressed delayed vomiting induced by cisplatin in the ferrets, which was prominently suppressed with the compound No. 3 orally given once daily at 10 mg/kg.

2. Suppression ratio of cisplatin-induced acute vomiting and delayed vomiting in ferrets due to the compound No. 3

The acute vomiting suppression ratio, delayed vomiting suppression ratio and total vomiting suppression ratio were calculated by the following formula. The results are shown in Tables 12 and 13.

[Frequency of retching or vomiting in control] - [Frequency of

retching or vomiting in animals dosed with compound No. 3] /

[Frequency of retching or vomiting in control] x 100 (%)

Table 12

| Effects of oral dosing twice daily | | | | |
|---|---|---|---|---|
| | Dose (mg/kg, p.o.) | Acute vomiting suppression ratio (0 to 24 hours after cisplatin dosing) | Delayed vomiting suppression ratio (24 to 72 hours after cisplatin dosing) | Total vomiting suppression ratio (0 to 72 hours after cisplatin dosing) |
| Cont. (0.5 % MC) | 1 mg/kg | frequency of retching: 33.0 | frequency of vomiting: 354.8 | frequency of vomiting: 387.8 |
| Cpd. No.3 | 0.03 | 25 % NS | 31 % NS | 30 % NS |
| | 0.1 | 21 % NS | 37 % a | 36 % a |
| | 0.3 | 100 % NS | 41 % b | 46 % b |
| | 1 | 0 % NS | 70 % a | 54 % b |
| | 3 | 82 % NS | 59 % b | 61 % b |
| Cont. (physiological saline) | 0.5 ml/kg | frequency of vomiting: 66.8 | frequency of vomiting: 323.8 | frequency of vomiting: 390.6 |
| Ondansetron | 1 | 0 % NS | 0 % NS | 0 % NS |
| | 3 | 58 % NS | 32 % NS | 36 % NS |

(a: $p < 0.05$; b: $p < 0.01$; NS: no statistic significance)

Table 13

| Effects of oral dosing once daily | | | | |
|---|---|---|---|---|
| | Dose (mg/kg, p.o.) | Acute vomiting suppression ratio (0 to 24 hours after cisplatin dosing) | Delayed vomiting suppression ratio (24 to 72 hours after cisplatin dosing) | Delayed vomiting suppression ratio (0 to 72 hours after cisplatin dosing) |
| Cont. (0.5 % MC) | 1 mg/kg | frequency of retching: 109.8 | frequency of vomiting: 347.8 | frequency of vomiting: 457.5 |
| Cpd. No.3 | 3 | 48 % NS | 40 % NS | 42 % a |
| | 10 | 83 % NS | 65 % a | 70 % b |

(a: $p < 0.05$; b: $p < 0.01$; NS: no statistic significance)

Example 4

Action of the compound No. 3 orally given on the delayed vomiting induced by an anticancer agent methotrexate (MTX)

1. Action of the compound No. 3 orally given on methotrexate-induced delayed vomiting in dogs

[Procedures]

3 or 4 beagle dogs (8.3 to 12.6 kg), male or female per one group were used. Methotrexate was intravenously administered at 2.5 mg/ml/kg. 8, 24 and 48 hours after methotrexate dosing, predetermined doses of the compound No. 3, ondansetron and 0.5% methyl cellulose solution were orally given. The compound No. 3 and 0.5 % methyl cellulose solution were dosed 30 minutes after a small amount of a feed was given to the animals, while ondansetron was dosed to the animals under fasting. The frequency of vomiting was counted in number, up to 72 hours after methotrexate dosing.
The compound No. 3 was suspended in 0.5 % methyl cellulose solution and was then orally dosed at a ratio of 0.25 to 1 ml per 1 kg body weight. A predetermined volume of an ondansetron injection at a concentration of 0.2 w/v % was orally dosed. Additionally, 0.5 % methyl cellulose solution was orally given at a ratio of 1 ml per 1 kg body weight. The data relating to the frequency of vomiting, the latency and the time period from the occurrence of vomiting to the termination thereof were statistically analyzed by the Dunnett test, with reference to the difference from the control group.

[Results]

The vomiting pattern of the animals administered with the solvent is shown in Fig. 10. In the control group, vomiting started 25.3 ± 1.5 hours (mean ± SD) after methotrexate administration. Up to about 70 hours after the administration, vomiting was observed continuously. The frequency of vomiting induced within the observation period of 72 hours was 12.3 ± 0.7 in number. Methotrexate-induced vomiting was significantly suppressed by the compound No. 3 at 0.01 mg/kg or higher. The time period from the occurrence of vomiting to the termination thereof was significantly shortened. Still further, such vomiting was almost completely suppressed at 10 mg/kg of the compound No. 3. The results are shown in Fig.11 and Table 14.

Table 14

| | | | Frequency of vomiting (mean ± SD) | | |
|---|---|---|---|---|---|
| Pre-treatment | Dose (mg/kg, p.o.) | Number of dogs with vomiting/ number of tested dogs | Day 0 (0-24 hours) | Days 1 + 2 (24 - 72 hours) | Total frequency |
| 0.5 % MC | - | 3/3 | 0.7 ± 0.3 | 11.7 ± 0.9 | 12.3 ± 0.7 |
| Cpd. No.3 | 0.01 | 3/3 | 0 b | 4.7 ± 0.7 b | 4.7 ± 0.7 b |
| | 0.1 | 3/3 | 0 b | 3.0 ± 1.0 b | 3.0 ± 1.0 b |
| | 1 | 3/3 | 0 b | 3.7 ± 0.7 b | 3.7 ± 0.7 b |
| | 3 | 3/3 | 0 b | 1.7 ± 0.3 b | 1.7 ± 0.3 b |
| | 10 | 1/4 | 0 b | 0.3 ± 0.3 b | 0.3 ± 0.3 b |
| Ondansetron | 10 | 3/3 | 0 b | 9.3 ± 2.3 | 9.3 ± 2.3 |

| | Latency (in hour; mean ± SD) | Duration (in hour; mean ± SD) |
|---|---|---|
| 0.5 % MC | 25.3 ± 1.5 | 44.0 ± 1.7 |
| Cpd. No.3 | 33.8 ± 4.8 | 21.8 ± 5.8 a |
| | 35.7 ± 7.1 | 15.0 ± 8.0 b |
| | 46.7 ± 8.7 | 14.4 ± 6.1 b |
| | 45.5 ± 3.3 | 6.4 ± 5.9 b |
| | 66.2 ± 5.8 b | 0 b |
| Ondansetron | 28.6 ± 3.9 | 27.8 ± 2.7 |

(a: $p < 0.05$; b: $p < 0.01$)

The compound No. 3 orally administered once daily apparently suppressed the cisplatin-induced delayed vomiting in the dogs.

2. Suppression ratio of methotrexate-induced delayed vomiting in dogs due to the compound No. 3

The delayed vomiting suppression ratio and the suppression ratio of the total frequency of vomiting were calculated by the following formula. The results are shown in Table 15.

Table 15

| Effects of oral dosing once daily | | | |
|---|---|---|---|
| | Dose (mg/kg, p.o.) | Delayed vomiting suppression ratio (24 to 72 hours after MIX dosing) | Suppression ratio of total frequency of vomiting (0 to 72 hours after MTX dosing) |
| Cont. (0.5 % MC) | 1 mg/kg | Frequency of vomiting: 11.7 | Total frequency of vomiting: 12.3 |
| Cpd. No. 3 | 0.01 | 60 % b | 62 % b |
| | 0.1 | 74 % b | 76 % b |
| | 1 | 68 % b | 70 % b |
| | 3 | 85 % b | 86 % b |
| | 10 | 97 % b | 98 % b |
| Ondansetron | 10 | 21 % NS | 24 % NS |

(b: $p < 0.01$; NS: no statistic significance)

Example 5

Action of the compound No. 3 orally given on vomiting due to an analgesic morphine

1. Action of the compound No. 3 orally given on vomiting induced by morphine in ferrets

[Procedures]

Male 6 or 7 ferrets (1.6 to 2.0 kg) per one group were used. Morphine was subcutaneously administered at0.3 mg/0.3 ml/kg. 2.5 hours before morphine dosing, a small amount of a feed was given to the animals. 30 minutes thereafter, predetermined doses of the compound No. 3 were orally given. To a control animal group under no fasting was orally given a predetermined volume of 0.5 % methyl cellulose solution. A predetermined volume of ondansetron was orally given to the animals under fasting. To a control animal group of ondansetron under fasting was orally given a predetermined volume of physiological saline. The frequency of vomiting was counted up to 30 minutes after morphine dosing.
The compound No. 3 was suspended in 0.5 % methyl cellulose solution and was then orally dosed at a ratio of 1 ml per 1 kg body weight. An injection of ondansetron at a concentration of 0.2 w/v % was orally dosed. Additionally, 0.5 % methyl cellulose solution was orally given at a ratio of 1 ml per 1 kg body weight, while physiological saline was orally given at a ratio of 1 ml per 1 kg body weight. The data relating to the frequency of vomiting, the latency and the time period from the occurrence of vomiting to the termination thereof were statistically analyzed by the Dunnett test or *t* test, with reference to the difference from the control group.

[Results]

In the control group, retching or vomiting started, 3.3 ± 3.6 minutes (mean) after morphine administration. Since then, vomiting continued for about 10 minutes under observation. The frequencies of retching and vomiting induced within the observation period of 30 minutes were 31.9 ± 5.2 and 3.6 ± 0.7 in number, respectively. Such retching and vomiting were likely to be suppressed via the oral administration of the compound No. 3 at 1 mg/kg and were significantly suppressed via the 3 mg/kg dose. Alternatively, oral dosing of ondansetron at 3 mg/kg did not suppress the vomiting. The measurement results are shown in Figs. 12 and 13 and Tables 16 and 17.

Table 16

| Effects of the compound No. 3 dosed | | | | | | |
|---|---|---|---|---|---|---|
| Pre-treatment | Dose (mg/kg , p.o.) | A | B | C | D |
| 0.5 % MC | - | 7/7 | 31.9 ± 5.2 | 3.3 ± 0.3 | 8.0 ± 1.5 |
| Cpd No. 3 | 1 | 4/6 | 18.3 ± 7.0 | 12.3 ± 5.6 | 5.5 ± 1.9 |
| | 3 | 5/6 | 6.8 ± 2.6 b | 10.5 ± 3.9 | 2.7 ± 1.7 |
| | | | | | |
| Pre-treatment | Dose (mg/kg , p.o.) | E | F | G | H |
| 0.5 % MC | - | 7/7 | 3.6 ± 0.7 | 3.7 ± 0.4 | 5.6 ± 1.5 |
| Cpd No. 3 | 1 | 4/6 | 2.5 ± 1.0 | 13.3 ± 5.4 | 3.8 ± 1.9 |
| | 3 | 1/6 | 0.5 ± 0.5 a | 25.8 ± 4.2 b | 1.5 ± 1.5 |

A: Number of ferrets with retching/number of tested ferrets
B: Frequency of retching (mean ± SD)
C: Time period until first retching (minute; mean ± SD)
D: Duration of retching (minute; mean ± SD)
E: Number of ferrets with vomiting/number of tested ferrets
F: Frequency of vomiting (mean ± SD)
G: Time period until first vomiting (minute; mean ± SD)
H: Duration of vomiting (minute; mean ± SD)
(a: $p < 0.05$; b: $p < 0.01$)

Table 17

| Effects of ondansetron administration | | | | | | |
|---|---|---|---|---|---|
| Pre-treatment | Dose (mg/kg, p.o.) | A | B | C | D |
| Physiological saline | - | 6/6 | 19.5 ± 8.5 | 5.5 ± 0.9 | 3.5 ± 2.4 |
| Ondansetron | 3 | 5/6 | 17.2 ± 6.4 | 8.8 ± 4.3 | 5.0 ± 2.3 |
| | | | | | |
| Pre-treatment | Dose (mg/kg, p.o.) | E | F | G | H |
| Physiological saline | - | 5/6 | 2.2 ± 0.9 | 8.7 ± 4.3 | 2.8 ± 2.5 |
| Ondansetron | 3 | 5/6 | 2.3 ± 1.2 | 9.2 ± 4.2 | 3.7 ± 2.6 |

A: Number of ferrets with retching/number of tested ferrets
B: Frequency of retching (mean ± SD)
C: Time period until first retching (minute; mean ± SD)
D: Duration of retching (minute; mean ± SD)
E: Number of ferrets with vomiting/number of tested ferrets
F: Frequency of vomiting (mean ± SD)
G: Time period until first vomiting (minute; mean ± SD)
H: Duration of vomiting (minute; mean ± SD)

The oral administration of the compound No. 3 apparently suppressed the morphine-induced vomiting in the ferrets.

2. Action of the compound No. 3 orally given on morphine-induced vomiting in dogs

[Procedures]

Male 6 or 7 beagle dogs (8.8 to 14.5 kg) per one group were used. Morphine was subcutaneously administered at 0.3 mg/0. 03 ml/kg. 2.5 hours before morphine dosing, a small amount of a feed was given to the animals. 30 minutes

thereafter, predetermined doses of the compound No. 3 were orally given. To a control animal group under no fasting was orally given a predetermined volume of 0.5 % methyl cellulose solution. A predetermined dose of ondansetron was orally given to the animals under fasting. To a control animal group of ondansetron under fasting was orally given a predetermined volume of physiological saline. The frequency of vomiting was counted up to 30 minutes after morphine dosing.

The compound No. 3 was suspended in 0.5 % methyl cellulose solution and was then orally administered at a ratio of 1 ml per 1 kg body weight. An injection of ondansetron at a concentration of 0.2 w/v % was orally dosed. Additionally, 0.5 % methyl cellulose solution was orally given at a ratio of 1 ml per 1 kg body weight, while physiological saline was orally given at a ratio of 0.5 ml per 1 kg body weight. The data relating to the frequency of vomiting, the time period (latency) until first vomiting and the time period from the occurrence of vomiting to the termination thereof were statistically analyzed by the Dunnett test or t test, with reference to the difference from the control group.

[Results]

In the control group, vomiting started, 5.6 ± 0.5 minutes (mean ± SD) after morphine administration. Since then, vomiting continued for about 2 minutes under observation. The frequency of vomiting induced within the observation period of 30 minutes was 2.2 ± 0.5 in number. The vomiting was significantly suppressed via the oral administration of the compound No. 3 at 0.3 mg/kg and was completely suppressed at the 1 mg/kg dose. Alternatively, oral dosing of ondansetron at 1 mg/kg did not suppress the vomiting. The results are shown in Tables 18 and 19.

Table 18

| Effects of the compound No. 3 dosed | | | | | |
|---|---|---|---|---|---|
| Pre-treatment | Dose (mg/Kg, p.o.) | A | B | C | D |
| 0.5 % MC | - | 5/5 | 2.2 ± 0.5 | 5.6 ± 0.5 | 1.8 ± 0.9 |
| Cpd. No. 3 | 0.1 | 3/3 | 1.0 ± 0 | 8.7 ± 1.2 | 0 |
| | 0.3 | 1/3 | 0.3 ± 0.3a | 22.0 ± 8.0 a | 0 |
| | 1 | 0/3 | 0 b | 30 b | 0 |

A: Number of ferrets with vomiting/number of tested ferrets
B: Frequency of vomiting (mean ± 3D)
C: Time period until first vomiting (minute; mean ± SD)
D: Duration of vomiting (minute; mean ± SD)
(a: $p < 0.05$; b: $p < 0.01$)

Table 19

| Effects of ondansetron dosed | | | | | |
|---|---|---|---|---|---|
| Pre-treatment | Dose (mg/Kg, p.o.) | A | B | C | D |
| Physiological saline | - | 4/4 | 3.3 ± 0.9 | 4.5 ± 1.2 | 1.8 ± 0.6 |
| Ondansetron | 1 | 3/3 | 1.7 ± 0.3 | 5.0 ± 1.0 | 1.0 ± 0.6 |

A: Number of ferrets with vomiting/number of tested ferrets
B: Frequency of vomiting (mean ± SD)
C: Time period until first vomiting (minute; mean ± SD)
D: Duration of vomiting (minute; mean ± SD)

The oral administration of the compound No. 3 apparently suppressed the morphine-induced vomiting in the dogs.

The suppression ratio of the morphine-induced vomiting due to the orally dosed compound No. 3 is shown below.

The retching suppression ratio and vomiting suppression ratio were calculated by the following formula. The results are shown in Tables 20 and 21.

[Frequency of retching or vomiting in control] - [Frequency of

retching or vomiting in animals dosed with compound No. 3] /

[Frequency of retching or vomiting in control] x 100 (%)

Table 20

| Effects in ferrets | | | |
|---|---|---|---|
| | Dose (mg/kg, p.o.) | Pseudo-vomiting suppression ratio | Acute vomiting suppression ratio |
| Cont. (0.5 % MC) | 1 mg/kg | frequency of retching: 31.9 | frequency of vomiting: 3.6 |
| Cpd. No.3 | 1 | 43 % NS | 31 % NS |
| | 3 | 79 % b | 86 % a |
| Cont. (physiological saline) | 1.5 ml/kg | frequency of retching: 19.5 | frequency of vomiting: 2.2 |
| Ondansetron | 3 | 12 % NS | 0 % NS |

(a: $p < 0.05$; b: $p < 0.01$; NS: no statistical significance)

Table 21

| Effects in dogs | | |
|---|---|---|
| | Dose (mg/kg, p.o.) | Acute vomiting suppression ratio |
| Cont. (0.5 % MC) | 1 mg/kg | frequency of vomiting: 2.2 |
| Cpd. No.3 | 0.1 | 55 % NS |
| | 0.3 | 86 % NS |
| | 1 | 100 % b |
| Cont. (physiological saline) | 0.5 ml/kg | frequency of vomiting: 3.3 |
| Ondansetron | 1 | 48 % NS |

(a: $p < 0.05$; b: $p < 0.01$; NS: no statistical significance)

Example 6

Action of the compound No. 3 orally given on acute vomiting induced by a central emetic agent loperamide in dogs

[Procedures]

3 male or female beagle dogs (7.7 to 12.7 kg) per one group were used. Loperamide was subcutaneously given at 1 mg/ml/kg. 2.5 hours prior to loperamide administration, a small amount of a feed was given to the animals. 30 minutes thereafter, predetermined doses of the compound No. 3 were orally given. Similarly, 0.5 % methyl cellulose solution was orally given to a control animal group under no fasting. Predetermined doses of naloxone and ondansetron were given intravenously and orally, respectively to the animals under fasting, 30 minutes prior to loperamide administration. The frequency of vomiting was counted, up to 3 hours after loperamide administration.
The compound No. 3 was dissolved in 0.5 % methyl cellulose solution, for oral dosing at a ratio of 0.25 to 1 ml per 1 kg body weight. Additionally, naloxone was intravenously given in a solution at a concentration of 0.05 % w/v. An injection of ondansetron at a concentration of 0.2 % w/v was orally given. Still additionally, 0.5 % methyl cellulose solution was orally given at a ratio of 1 ml per 1 kg body weight. The data relating to the frequency of vomiting, the latency and the time period from the occurrence of vomiting to the termination thereof were statistically analyzed by the Dunnett test, with reference to the difference from the control group.

[Results]

In the control group, vomiting started, 0.73 ± 0.04 hour (mean ± SD) after loperamide administration. Since

then, vomiting continued for about 1.5 hours under observation. The frequency of vomiting induced within the observation period of 3 hours was 10.0 ± 0.6 in number. Such vomiting was significantly suppressed via the oral administration of the compound No. 3 at 0.03 mg/kg or higher and was completely suppressed via the 10 mg/kg dose. Alternatively, intravenous dosing of naloxone at 0.5 mg/kg almost completely suppressed the vomiting.
Alternatively, oral dosing of ondansetron at 10 mg/kg did not suppress the vomiting. The measurement results are shown in Figs. 14 and 15 and Table 22.

Table 22

| Pre-treatment | Dose (mg/Kg, p.o.) | A | B | C | D |
|---|---|---|---|---|---|
| 0.5 % MC | - | 3/3 | 10.0 ± 0.6 | 0.73± 0.04 | 1.56 ± 0.32 |
| Cpd. No. 3 | 0.01 | 3/3 | 8.0 ± 2.5 | 0.74 ± 0.07 | 1.24 ± 0.21 |
| | 0.03 | 3/3 | 2.0 ± 0.6 a | 1.08 ± 0.11 | 0.44 ± 0.22 |
| | 0.1 | 2/3 | 2.7 ± 2.2 a | 1.98 ± 0.55 a | 0.59 ± 0.59 |
| | 1 | 3/3 | 3.3 ± 1.2 a | 1.25 ± 0.35 | 0.68 ± 0.34 |
| | 3 | 3/3 | 3.0 ± 0.6 a | 1.63 ± 0.34 | 0.38 ± 0.13 a |
| | 10 | 0/3 | 0 b | 3 b | 0 b |
| Ondansetron | 10 | 3/3 | 9.7 ± 2.7 | 0.50 ± 0.14 | 1.30 ± 0.11 |
| Naloxone | 0.5 mg/kg i.v. | 1/3 | 0.7 ± 0.7 b | 2.61± 0.39b | 0.01 ± 0.01 b |

A: Number of ferrets with vomiting/number of tested ferrets
B: Frequency of vomiting (mean ± SD)
C: Time period until first vomiting (minute; mean ± SD)
D: Duration of vomiting (minute; mean ± SD)
(a: $p < 0.05$; b: $p < 0.01$)

The oral administration of the compound No. 3 apparently suppressed the loperamide-induced vomiting in the dogs.

The suppression ratio of the loperamide-induced vomiting due to the compound No. 3 is shown below.

The vomiting suppression ratio was calculated by the following formula. The results are shown in Table 23.

[Frequency of retching or vomiting in control] - [Frequency of retching or vomiting in animals dosed with compound No. 3] / [frequency of retching or vomiting in control] x 100 (%)

Table 23

| | Dose (mg/kg, p.o.) | Vomiting suppression ratio |
|---|---|---|
| Cont. (0.5 % MC) | 1 mg/kg | frequency of vomiting: 10.0 |
| Cpd. No.3 | 0.01 | 20 % NS |
| | 0.03 | 80 % a |
| | 0.1 | 73 % a |
| | 1 | 67 % a |
| | 3 | 70 % a |
| | 10 | 100 % b |
| Ondansetron | 10 | 3 % NS |
| Naloxone | 0.5 mg/kg i.v. | 93 % b |

(a: $p < 0.05$; b: $p < 0.01$; NS: no statistical significance)

Example 7

Action of the compound No. 3 on vomiting induced by a central emetic agent apomorphine in dogs

[Procedures]

3 male or female beagle dogs (7.8 to 15.9 kg) per one group were used. Apomorphine was subcutaneously given at 0.1 mg/ml/kg. 2.5 hours prior to apomorphine administration, a small amount of a feed was given to the animals. 30 minutes thereafter, predetermined doses of the compound No. 3 were orally given. Similarly, 0.5 % methyl cellulose solution was orally given to a control animal group under no fasting. A predetermined dose of ondansetron was given orally to the animals under fasting. The frequency of vomiting was counted in number, up to one hour after apomorphine administration.
The compound No. 3 was dissolved in 0.5 % methyl cellulose solution, for oral dosing at a ratio of 1 ml per 1 kg • body weight. An injection of ondansetron at a concentration of 0.2% w/v was orally given. Additionally, 0.5 % methyl cellulose solution was orally given at a ratio of 1 ml per 1 kg • body weight. The data relating to the frequency of vomiting, the latency and the time period from the occurrence of vomiting to the termination thereof were statistically analyzed by the Dunnett test, with reference to the difference from the control group.

[Results]

In the control group, vomiting started, 4.2 ±0.0 minutes (mean ± SD) after apomorphine administration. Since then, vomiting continued for about 30 minutes under observation. The frequency of vomiting induced within the observation period of one hour was 12.0 ± 3.6 in number. Such vomiting was likely to be suppressed via the administration of the compound No. 3 at 3 mg/kg and was prominently suppressed via the 10 mg/kg dose. Still further, the compound No. 3 at 10 mg/kg greatly shortened the duration of vomiting from the start to the termination. Alternatively, oral dosing of ondansetron at 10 mg/kg did not suppress the vomiting. The measurement results are shown in Figs. 16 and 17 and Table 24.

Table 24

| Pre-treatment | Dose (mg/Kg p.o.) | A | B | C | D |
|---|---|---|---|---|---|
| 0.5 % MC | - | 3/3 | 12.0 ± 3.6 | 4.2 ± 0.0 | 28.2 ± 12.0 |
| Cpd. No. 3 | 3 | 3/3 | 6.3 ± 1.3 | 7.2 ± 0.6 | 12.0 ± 3.0 |
| | 10 | 2/3 | 1.3± 0.9b | 27.6 ± 13.8 | 1.2 ± 1.2 a |
| Ondansetron | 10 | 3/3 | 13.7 ± 2.4 | 2.4 ± 0.6 | 22.8 ± 3.6 |

A: Number of ferrets with vomiting/number of tested ferrets
B: Frequency of vomiting (mean ± SD)
C: Time period until first vomiting (minute; mean ± SD)
D: Duration of vomiting (minute; mean ± SD)
(a: $p < 0.05$; b: $p < 0.01$)

The oral administration of the compound No. 3 apparently suppressed the apomorphine-induced vomiting in the dogs.
The vomiting suppression ratio was calculated by the following formula. The results are shown in Table 25.

[Frequency of retching or vomiting in control] - [Frequency of

retching or vomiting in animals dosed with compound No. 3] /

[Frequency of retching or vomiting in control] x 100 (%)

Table 25

| | Dose (mg/kg, p.o.) | Vomiting suppression ratio |
|---|---|---|
| Cont. (0.5 % MC) | 1 mg/kg | frequency of vomiting: 12.0 |
| Cpd. No.3 | 3 | 48 % NS |
| | 10 | 89 % a |
| Ondansetron | 10 | 0 % NS |

(b: $p < 0.01$; NS: no statistical significance)

Example 8

Action of the compound No. 3 and ondansetron in combination on vomiting induced by an anticancer agent cisplatin

[Procedures]

Male ferrets (0.8 to 1.6 kg) per one group were used. Cisplatin was intraperitoneally given at 10 mg/10 ml/kg. 30 minutes prior to cisplatin administration, the compound No. 3 (3 mg/kg) and ondansetron (0.03 mg/kg) were orally given to the animals. Similarly, 0.5 % w/v methyl cellulose (MC) solution was orally given to a control animal group. The frequency of retching and vomiting was counted, up to 180 minutes after cisplatin administration. The animal behavior was recorded with a closed-circuit video recording system. After the termination of the experiment, the animal behavior was analyzed. The data relating to the frequency of retching and vomiting were statistically analyzed by the Dunnett test, with reference to the difference from the control group.

[Results]

In the control group, the frequency of retching and vomiting induced within the observation period of 180 minutes after cisplatin administration was $116.5 \pm 22.5$ in number. The combination of the compound No. 3 (3 mg/kg) and ondansetron (0.03 mg/kg) significantly suppressed the frequency of retching and vomiting (Fig. 18).
The results apparently indicate that the combination of the compound No.3 and ondansetron significantly suppressed the cisplatin-induced acute vomiting in the ferrets.

Example 9

Effects of the compound No. 3 and dexamethazone in combination on delayed vomiting induced by an anticancer agent cisplatin

[Procedures]

Male ferrets (1.2 to 1.9 kg) were used. Cisplatin was intraperitoneally given at 5 mg/5 ml/kg. 30 minutes after cisplatin administration, the compound No. 3 (3 mg/kg) and dexamethazone (1 mg/kg) were orally given to the animals. Similarly, 0.5 % w/v methyl cellulose (MC) solution was orally given to a control animal group. The frequency of retching and vomiting was counted within 13 hours from 31 to 44 hours after cisplatin administration. When the ferrets vomited within one hour after the oral dosing, the data of the ferrets were not used. The animal behavior was recorded with a closed-circuit video recording system. After the termination of the experiment, the animal behavior was analyzed. The data relating to the frequency of retching and vomiting were statistically analyzed by the Dunnett test, with reference to the difference from the control group.

[Results]

In the control group, the frequency of retching and vomiting induced 31 to 44 hours after cisplatin administration was $74.0 \pm 20.7$ in number. The frequency of retching and vomiting was significantly suppressed by the combination of the compound No. 3 (3 mg/kg) and dexamethazone (1 mg/kg) (Fig. 19).
The results apparently indicate that the combination of the compound No.3 and dexamethazone significantly suppressed the cisplatin-induced delayed vomiting in the ferrets.

Example 10

Effects of the compound No. 3, ondansetron and dexamethazone in combination on acute vomiting induced by an anticancer agent cisplatin

[Procedures]

Male ferrets (1.7 to 2.0 kg) per one group were used. Cisplatin was intraperitoneally given at 10 mg/10 ml/kg. 30 minutes prior to cisplatin administration, the compound No. 3 (3 mg/kg), ondansetron (0.03 mg/kg) and dexamethazone (1 mg/kg) were orally given to the animals, 30 minutes prior to cisplatin administration. Similarly, 0.5 % w/v methyl cellulose (MC) solution was orally given to a control animal group. The frequency of retching and vomiting was counted within 180 minutes after cisplatin administration. The animal behavior was recorded with a closed-circuit video recording system. After the termination of the experiment, the animal behavior was analyzed. The data relating to the frequency of retching and vomiting were statistically analyzed by the Student's t test, with reference to the difference from the control group.

[Results]

In the control group, the frequency of retching and vomiting was 227 ± 52 in number. The frequency of retching and vomiting was significantly suppressed by the combination of the compound No. 3, ondansetron and dexamethazone. The results are shown in Fig. 20 and Table 26.

Table 26

| Pre-treatment | Number of ferrets with retching/number of tested ferrets | Frequency of retching (mean ± SD) |
|---|---|---|
| 0.5 % MC | 4/4 | 207 ± 51 |
| 3 drugs | 4/4 | 44 ± 18 * |
| Pre-treatment | Number of ferrets with vomiting/number of tested ferrets | Frequency of vomiting (mean ± SD) |
| 0.5 % MC | 4/4 | 20.3 ± 1.7 |
| 3 drugs | 4/4 | 5.0 ± 1.4 ** |

The term 3 "drugs" represents the combination of the compound No. 3, ondansetron and dexamethazone.

The results apparently indicate that the combination of the compound No.3, ondansetron and dexamethazone significantly suppressed the cisplatin-induced acute vomiting in the ferrets.

Example 11

Effects of the compound No. 3, ondansetron and dexamethazone in combination on delayed vomiting induced by an anticancer agent cisplatin

[Procedures]

Male ferrets (1.7 to 1.9 kg) per one group were used. Cisplatin was intraperitoneally given at 5 mg/5 ml/kg. 30 minutes prior to cisplatin administration, the compound No. 3 (3 mg/kg), ondansetron (0.03 mg/kg) and dexamethazone (1 mg/kg) were orally given to the animals, 30 minutes prior to cisplatin administration. Similarly, 0.5 % w/v methyl cellulose (MC) solution was given orally to a control animal group. The frequency of retching and vomiting was counted within 13 hours from 31 to 44 hours after cisplatin administration. The animal behavior was recorded with a closed-circuit video recording system. After the termination of the experiment, the animal behavior was analyzed. The data relating to the frequency of retching and vomiting were statistically analyzed by the Student's t test, with reference to the difference from the control group.

[Results]

In the control group, the frequency of retching and vomiting was 63.0 ± 9.1 in number. The frequency of retching and vomiting was significantly suppressed by the combination of the compound No. 3, ondansetron and dexamethazone (Fig. 21).

The results apparently indicate that the combination of the compound No.3, ondansetron and dexamethazone significantly suppressed the cisplatin-induced delayed vomiting in the ferrets.

Industrial applicability

The compound (I) or the salt thereof at a low dose can rapidly suppress vomiting induced by a drug with an emetic action in a safe manner and can enlarge the applicable range of such drug with an emetic action as a side effect and can additionally improve the QOL of patients.

## Claims

**1.** A medicine which comprises a compound (I) of the formula:

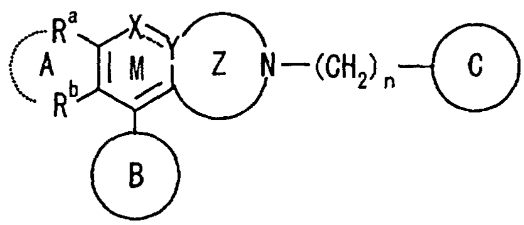

[wherein the ring M is a heterocylic ring having -N=C<, -CO-N< or -CS-N< as a partial structure —X=Y<;
$R^a$ and $R^b$ are bound to each other to form the ring A, or they are the same or different each representing a hydrogen atom or a substituent on the ring M;
the rings A and B each is an optionally substituted homocycle or heterocycle, and at least one of them is an optionally substituted heterocycle;
the ring C is an optionally substituted homocycle or heterocycle;
the ring Z is an optionally substituted nitrogen-containing heterocycle; and
n is an integer of 1 to 6]

or a salt thereof in combination with a drug having an emetic action.

**2.** A medicine according to Claim 1, wherein $R^a$ and $R^b$ each is a hydrogen atom or substituent selected from

(1) halogen atom;
(2) $C_{1-6}$ alkyl group which may have 1 - 5 substituents selected from (i) hydroxyl group, (ii) $C_{1-6}$ alkoxy group, (iii) $C_{1-6}$ alkylthio group, (iv) amino group, (v) $C_{1-7}$ acylamino group, (vi) carboxyl group, (vii) nitro group, (viii) mono- or di-$C_{1-6}$ alkylamino group, (ix) mono- or di-$C_{3-8}$ cycloalkylamino group, (x) $C_{6-10}$ arylamino, (xi) 5- to 9-membered cyclic amino group which may be substituted by $C_{1-6}$ alkyl and may contain 1 - 3 heteroatoms selected from oxygen atom and sulfur atom in addition to the nitrogen atom, (xii) 5- or 6-membered aromatic heterocyclic group which contains 1 - 3 heteroatoms selected from nitrogen atom, oxygen atom and sulfur atom in addition to the carbon atom, (xiii) 5- to 9-membered non-aromatic heterocyclic group which contains 1 - 3 heteroatoms selected from nitrogen atom, oxygen atom and sulfur atom in addition to the carbon atom, (xiv) $C_{1-4}$ alkylsulfonylamino group, (xv) $C_{1-6}$ alkyl-carbonyloxy group, and (xvi) halogen atom;
(3) optionally halogenated $C_{1-6}$ alkoxy group;
(4) optionally halogenated $C_{1-6}$ alkylthio group;
(5) $C_{3-10}$ cycloalkyl group;
(6) $C_{6-10}$ aryl group;
(7) $C_{1-7}$ acylamino group;
(8) $C_{1-3}$ acyloxy group;
(9) hydroxyl group;
(10) nitro group;
(11) cyano group;
(12) amino group;
(13) mono- or di-$C_{1-6}$ alkylamino group;
(14) 5- to 9-membered cyclic amino group which may be substituted by $C_{1-6}$ alkyl and may contain 1 - 3

heteroatoms selected from oxygen atom and sulfur atom in addition to the nitrogen atom;
(15) $C_{1-6}$ alkyl-carbonylamino group;
(16) $C_{1-6}$ alkyl-sulfonylamino group;
(17) $C_{1-6}$ alkoxy-carbonyl group;
(18) carboxyl group;
(19) $C_{1-6}$ alkyl-carbonyl group;
(20) carbamoyl group;
(21) mono- or di-$C_{1-6}$ alkylcarbamoyl group; and
(22) $C_{1-6}$ alkylsulfonyl group;

or $R^a$ and $R^b$ are bound to each other to form the ring A, wherein the ring A represents (i) 5- to 9-membered aromatic heterocycle which contains 1 to 3 of 1 or 2 kinds of heteroatoms selected from nitrogen atom, sulfur atom and oxygen atom in addition to the carbon atom; (ii) 5- to 9-membered non-aromatic heterocycle which contains 1 to 3 of 1 or 2 kinds of heteroatoms selected from nitrogen atom, sulfur atom and oxygen atom in addition to the carbon atom; or (iii) 3- to 10-membered cyclic hydrocarbon, which may be substituted by 1 to 4 substituents selected from:

(1) halogen atom;
(2) $C_{1-6}$ alkyl group which may have 1 - 5 substituents selected from (i) hydroxyl group, (ii) amino group, (iii) carboxyl group, (iv) nitro group, (v) mono- or di-$C_{1-6}$ alkylamino group, (vi) $C_{1-6}$ alkyl-carbonyloxy group, and (vii) halogen atom;
(3) optionally halogenated $C_{1-6}$ alkoxy group;
(4) optionally halogenated $C_{1-6}$ alkylthio group;
(5) $C_{6-10}$ aryl group;
(6) $C_{1-7}$ acylamino group;
(7) $C_{1-3}$ acyloxy group;
(8) hydroxyl group;
(9) nitro group;
(10) cyano group;
(11) amino group;
(12) mono- or di-$C_{1-6}$ alkylamino group;
(13) 5- to 9-membered cyclic amino group which may contain 1 - 3 heteroatoms selected from oxygen atom and sulfur atom in addition to the nitrogen atom;
(14) $C_{1-6}$ alkyl-carbonylamino group;
(15) $C_{1-6}$ alkyl-sulfonylamino group;
(16) $C_{1-6}$ alkoxy-carbonyl group;
(17) carboxyl group;
(18) $C_{1-6}$ alkylcarbonyl group;
(19) carbamoyl group;
(20) mono- or di-$C_{1-6}$ alkylcarbamoyl group;
(21) $C_{1-6}$ alkylsulfonyl group; and
(22) oxo group;

the ring B represents (i) 5- to 9-membered aromatic heterocycle which contains 1 to 3 of 1 or 2 kinds of heteroatoms selected from nitrogen atom, sulfur atom and oxygen atom in addition to the carbon atom; (ii) 5- to 9-membered non-aromatic heterocycle which contains 1 to 3 of 1 or 2 kinds of heteroatoms selected from nitrogen atom, sulfur atom and oxygen atom in addition to the carbon atom; or (iii) 3- to 10-membered cyclic hydrocarbon, which may be substituted by 1 to 4 substituents selected from:

(1) halogen atom;
(2) $C_{1-6}$ alkyl group which may have 1 - 5 substituents selected from (i) hydroxyl group, (ii) amino group, (iii) carboxyl group, (iv) nitro group, (v) mono- or di-$C_{1-6}$ alkylamino group, (vi) $C_{1-6}$ alkyl-carbonyloxy group, and (vii) halogen atom;
(3) optionally halogenated $C_{1-6}$ alkoxy group;
(4) optionally halogenated $C_{1-6}$ alkylthio group;
(5) $C_{6-10}$ aryl group;
(6) $C_{1-7}$ acylamino group;
(7) $C_{1-3}$ acyloxy group;

(8) hydroxyl group;
(9) nitro group;
(10) cyano group;
(11) amino group;
(12) mono- or di-$C_{1-6}$ alkylamino group;
(13) 5- to 9-membered cyclic amino group which may contain 1 - 3 heteroatoms selected from oxygen atom and sulfur atom in addition to the nitrogen atom;
(14) $C_{1-6}$ alkyl-carbonylamino group;
(15) $C_{1-6}$ alkyl-sulfonylamino group;
(16) $C_{1-6}$ alkoxy-carbonyl group;
(17) carboxyl group;
(18) $C_{1-6}$ alkylcarbonyl group;
(19) carbamoyl group;
(20) mono- or di-$C_{1-6}$ alkylcarbamoyl group;
(21) $C_{1-6}$ alkylsulfonyl group; and
(22) oxo group;

the ring C represents a 5- to 9-membered heterocycle which contains 1 to 3 of 1 or 2 kinds of heteroatoms selected from nitrogen atom, sulfur atom and oxygen atom in addition to the carbon atom, and which may be substituted by 1 to 5 substituents selected from:

(1) halogen atom;
(2) optionally halogenated $C_{1-10}$ alkyl group;
(3) $C_{1-4}$ alkyl group substituted by amino group;
(4) $C_{1-4}$ alkyl group substituted by mono- or di-$C_{1-4}$ alkylamino group;
(5) $C_{1-4}$ alkyl group substituted by carboxyl group;
(6) $C_{1-4}$ alkyl group substituted by $C_{1-4}$ alkoxy-carbonyl group;
(7) $C_{1-4}$ alkyl group substituted by hydroxyl group;
(8) $C_{3-10}$ cycloalkyl group;
(9) nitro group;
(10) cyano group;
(11) hydroxyl gorup;
(12) optionally halogenated $C_{1-10}$ alkoxy group;
(13) optionally halogenated $C_{1-4}$ alkylthio group;
(14) amino group;
(15) mono- or di-$C_{1-4}$ alkylamino group;
(16) 5- to 9-membered cyclic amino group which may contain 1 - 3 heteroatoms selected from oxygen atom and sulfur atom in addition to the nitrogen atom;
(17) $C_{1-4}$ alkyl-carbonylamino group;
(18) aminocarbonyloxy group;
(19) mono- or di-$C_{1-4}$ alkylaminocarbonyloxy group;
(20) $C_{1-4}$ alkylsulfonylamino group;
(21) $C_{1-4}$ alkoxy-carbonyl group;
(22) aralkyloxycarbonyl group;
(23) carboxyl group;
(24) $C_{1-6}$ alkyl-carbonyl group;
(25) $C_{3-6}$ cycloalkyl-carbonyl group;
(26) carbamoyl group;
(27) mono- or di-$C_{1-4}$ alkylcarbamoyl group;
(28) $C_{1-6}$ alkylsulfonyl group; and
(29) 5- or 6-membered aromatic monocyclic heterocycle which contains 1 to 4 of 1 or 2 kinds of heteroatoms selected from nitrogen atom, sulfur atom and oxygen atom in addition to the carbon atom and which may be substituted by 1 to 3 of optionally halogenated $C_{1-4}$ alkyl groups;

or a 3- to 10-membered homocycle which may be substituted by 1 to 5 substituents selected from:

(1) halogen atom;
(2) optionally halogenated $C_{1-10}$ alkyl group;

(3) $C_{1-4}$ alkyl group substituted by amino group;
(4) $C_{1-4}$ alkyl group substituted by mono- or di-$C_{1-4}$ alkylamino group;
(5) $C_{1-4}$ alkyl group substituted by carboxyl group;
(6) $C_{1-4}$ alkyl group substituted by $C_{1-4}$ alkoxy-carbonyl group;
(7) $C_{1-4}$ alkyl group substituted by hydroxyl group;
(8) $C_{3-10}$ cycloalkyl group;
(9) nitro group;
(10) cyano group;
(11) hydroxyl gorup;
(12) optionally halogenated $C_{1-10}$ alkoxy group;
(13) optionally halogenated $C_{1-4}$ alkylthio group;
(14) amino group;
(15) mono- or di-$C_{1-4}$ alkylamino group;
(16) 5- to 9-membered cyclic amino group which may contain 1 - 3 heteroatoms selected from oxygen atom and sulfur atom in addition to the nitrogen atom;
(17) $C_{1-4}$ alkyl-carbonylamino group;
(18) aminocarbonyloxy group;
(19) mono- or di-$C_{1-4}$ alkylaminocarbonyloxy group;
(20) $C_{1-4}$ alkylsulfonylamino group;
(21) $C_{1-4}$ alkoxy-carbonyl group;
(22) aralkyloxycarbonyl group;
(23) carboxyl group;
(24) $C_{1-6}$ alkyl-carbonyl group;
(25) $C_{3-6}$ cycloalkyl-carbonyl group;
(26) carbamoyl gorup;
(27) mono- or di-$C_{1-4}$ alkylcarbamoyl group;
(28) $C_{1-6}$ alkylsulfonyl group; and
(29) 5- or 6-membered aromatic monocyclic heterocycle which contains 1 to 4 of 1 or 2 kinds of heteroatoms selected from nitrogen atom, sulfur atom and oxygen atom in addition to the carbon atom and which may be substituted by 1 to 3 of optionally halogenated $C_{1-4}$ alkyl groups; and

the ring Z represents a 5- to 12-membered heterocycle which may contain at least one heteroatom selected from nitrogen atom, oxygen atom and sulfur atom in addition to Y and the carbon atom and the nitrogen atom and which may be substituted by 1 to 5 substituents selected from:

(1) $C_{1-6}$ alkyl group;
(2) $C_{2-6}$ alkenyl group;
(3) $C_{2-6}$ alkynyl group;
(4) $C_{3-8}$ cycloalkyl group;
(5) $C_{3-8}$ cycloalkyl-$C_{1-4}$ alkyl group;
(6) $C_{6-14}$ aryl group;
(7) nitro group;
(8) cyano group;
(9) hydroxyl group;
(10) $C_{1-4}$ alkoxy group;
(11) $C_{1-4}$ alkylthio group;
(12) amino group;
(13) mono- or di-$C_{1-4}$ alkylamino group;
(14) 5- to 9-membered cyclic amino group which may contain 1 - 3 heteroatoms selected from oxygen atom and sulfur atom in addition to the nitrogen atom;
(15) $C_{1-4}$ alkyl-carbonylamino group;
(16) $C_{1-4}$ alkylsulfonylamino group;
(17) $C_{1-4}$ alkoxy-carbonyl group;
(18) carboxyl group;
(19) $C_{1-6}$ alkyl-carbonyl group;
(20) carbamoyl group;
(21) mono- or di-$C_{1-4}$ alkylcarbamoyl group;
(22) $C_{1-6}$ alkylsulfonyl group;

(23) oxo group; and
(24) thioxo group.

**3.** A medicine according to Claim 1, wherein $R^a$ and $R^b$ are bound to each other to form the ring A, the ring C is an optionally substituted benzene ring or optionally substituted heterocycle, the ring Z is an optionally oxo-substituted nitrogen-containing heterocycle, and n is 1 or 2.

**4.** A medicine according to Claim 1, wherein the ring Z is an optionally oxo-substituted nitrogen-containing heterocycle.

**5.** A medicine according to Claim 1, wherein one of the rings A and B is an optionally substituted aromatic ring, and the other is an optionally substituted aromatic heterocycle.

**6.** A medicine according to Claim 1, wherein the ring A is an optionally substituted aromatic heterocycle, and the ring B is an optionally substituted benzene ring.

**7.** A medicine according to Claim 5, wherein the aromatic heterocycle is a 5- or 6-membered aromatic heterocycle which contains 1 or 2 kinds of heteroatoms selected from nitrogen atom, sulfur atom and oxygen atom in addition to the carbon atom.

**8.** A medicine according to Claim 1, wherein the ring C is an optionally substituted benzene ring.

**9.** A medicine according to Claim 1, wherein the ring C is a benzene ring which contains 1 to 3 substituents selected from halogen atom, optionally halogenated $C_{1-6}$ alkyl group and optionally halogenated $C_{1-6}$ alkoxy group.

**10.** A medicine according to Claim 1, wherein the ring Z is a 5- to 10-membered ring which may be substituted by 1 or 2 oxo groups.

**11.** A medicine according to Claim 1, wherein —X=Y< is -CO-N< or -N=C<.

**12.** A medicine according to Claim 1, wherein n is 1.

**13.** A medicine according to Claim 1, wherein the ring A is an optionally substituted pyridine ring, the ring B is an optionally substituted benzene ring, the ring C is an optionally substituted benzene ring, the ring Z is an optionally oxo-substituted 5- to 10-membered ring, —X=Y< is -CO-N< or -N=C<, and n is 1.

**14.** A medicine according to Claim 1, wherein $R^a$ and $R^b$ are the same or different each representing hydrogen atom, halogen atom, optionally substituted alkyl group, optionally halogenated alkoxy group, optionally halogenated alkylthio group, cycloalkyl group, aryl group, acylamino group, acyloxy group, hydroxyl group, nitro group, cyano group, amino group, mono- or di-alkylamino group, cyclic amino group, alkylcarbonylamino group, alkylsulfonylamino group, alkoxycarbonyl group, carboxyl group, alkylcarbonyl group, carbamoyl group, mono- or di-alkylcarbamoyl group, alkylsulfonyl group or oxo group.

**15.** A medicine according to Claim 1, wherein $R^a$ and $R^b$ are the same or different each representing hydrogen atom or $C_{1-6}$ alkyl group which may be substituted by (i) $C_{1-6}$ alkoxy group, (ii) $C_{1-6}$ alkylthio group, (iii) amino group, (iv) $C_{1-7}$ acylamino group, (v) mono- or di-$C_{1-6}$ alkylamino group, (vi) $C_{3-10}$ cyclic amino group, (vii) 5- or 6-membered cyclic amino group which may be substituted by $C_{1-6}$ alkyl group, (viii) $C_{1-6}$ alkylsulfonylamino group or (ix) $C_{1-6}$ alkyl group which may be substituted by $C_{1-6}$ alkylcarbonyloxy group;

or $R^a$ and $R^b$ are bound to each other to form a pyridine ring which may have 1 to 3 substituents selected from halogen atom and $C_{1-4}$ alkyl group;
the ring B is a benzene ring which may have 1 to 3 substituents selected from (i) halogen atom, (ii) optionally halogenated $C_{1-4}$ alkyl group, and (iii) optionally halogenated $C_{1-4}$ alkoxy group; the ring C is a benzene ring which may have 1 to 3 substituents selected from (i) halogen atom, (ii) optionally halogenated $C_{1-4}$ alkyl group, (iii) optionally halogenated $C_{1-4}$ alkoxy group, (iv) amino group which may be substituted by $C_{1-4}$ alkyl group, (v) $C_{1-3}$ acyloxy group, and (vi) hydroxyl group;
the ring Z is a 5- to 10-membered nitrogen-containing heterocycle which may be substituted by $C_{1-4}$ alkyl group or hydroxyl group and may be oxo-substituted;

—X=Y< is -CO-N< or -N=C<; and
n is 1.

**16.** A medicine according to Claim 15, wherein $R^a$ and $R^b$ are bound to each other to form a pyridine ring which may contain 1 to 3 substituents selected from halogen atom and $C_{1-4}$ alkyl group; and —X=Y< is -CO-N<

**17.** A medicine according to Claim 16, wherein the pyridine ring is an unsubstituted pyridine ring.

**18.** A medicine according to Claim 15, wherein the ring B is a benzene ring which may have 1 to 3 of optionally halogentated $C_{1-4}$ alkyl groups.

**19.** A medicine according to Claim 15, wherein the ring C is a benzene ring which may have 1 to 3 substituents selected from (i) halogen atom, (ii) optionally halogentated $C_{1-4}$ alkyl group, and (iii) optionally halogentated $C_{1-4}$ alkoxy group.

**20.** A medicine according to Claim 15, wherein the ring Z is represented by the formula:

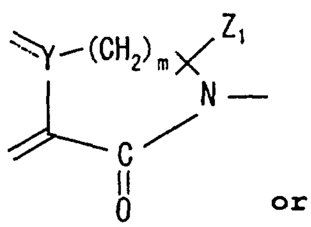

or

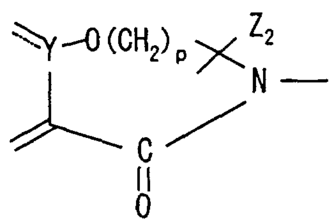

[wherein m and p are the same or different each indicating an integer of 1 to 5; $Z_1$ and $Z_2$ are the same or different each representing a hydrogen atom, $C_{1-4}$ alkyl group or hydroxyl group; and Y has the same meanings as described in Claim 15].

**21.** Amedicine according to Claim 1, wherein the compound (I) is (95)-7-[3,5-bis(trifluoromethyl)benzyl]-6,7,8,9,10,12-hexahydro-9-methyl-6,12-dioxo-5-phenyl[1,4]-diazepino[2,1-g][1,7]naphthyridine.

**22.** A medicine according to Claim 1, wherein the compound (I) is (9S)-7-[3,5-bis(trifluoromethyl)benzyl]-6,7,8,9,10,12-hexahydro-9-methyl-5-(4-methylphenyl)-6,12-dioxo-[1,4]diazepino[2,1-g][1,7]naphthyridine.

**23.** A medicine according to Claim 1, wherein the compound (I) is (9R)-7-[3,5-bis(trifluoromethyl)benzyl]-6,7,8,9,10,11-hexahydro-9-methyl-6,13-dioxo-5-phenyl-13H-[1,4]diazocino[2,1-g][1,7]naphthyridine.

**24.** Amedicine according to Claim 1, wherein the compound (I) is (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine.

**25.** A medicine according to Claim 1, wherein the compound (I) is (9R)-7-(3,5-dimethoxybenzyl)-5-(4-fluorophenyl)-6,7,8,9,10,11-hexahydro-9-methyl-6,13-dioxo-13H-[1,4]diazocino[2,1-g][1,7]naphthyridine.

**26.** A medicine according to Claim 1, wherein the compound (I) is (9R)-7-(3,5-dimethoxybenzyl)-6,7,8,9,10,11-hexahydro-9-methyl-5-(4-methylphenyl)-6,13-dioxo-13H-[1,4]diazocino[2,1-g][1,7]naphthyridine.

**27.** A medicine according to Claim 1, wherein the drug having an emetic action is an anti-cancer drug.

**28.** A medicine according to Claim 1, wherein the drug having an emetic action is morphine or a derivative thereof or a salt thereof.

**29.** A medicine according to Claim 1, wherein the drug having an emetic action is apomorphine or a salt thereof.

**30.** A medicine according to Claim 28, of which an orally applicable preparation comprises the compound (I) or a salt thereof.

**31.** A medicine according to Claim 28, of which a sustained release preparation comprises morphine or a derivative thereof or a salt thereof.

**32.** A medicine according to Claim 28, of which an orally applicable preparation comprises morphine or a derivative thereof or a salt thereof.

**33.** A medicine according to Claim 32, of which the orally applicable preparation is sublingual tablets or buccals.

**34.** A medicine according to Claim 32, of which the orally applicable preparation is orally rapidly disintegrating preparation.

**35.** A medicine according to Claim 28, which comprises an orally applicable preparation comprising the compound (I) or a salt thereof, in combination with an injection or orally applicable preparation comprising morphine or a derivative thereof or a salt thereof.

**36.** A medicine according to Claim 1, which comprises further combining with a serotonin antagonist and/or glucocorticoid.

**37.** A medicine which comprises the compound (I) represented by the formula:

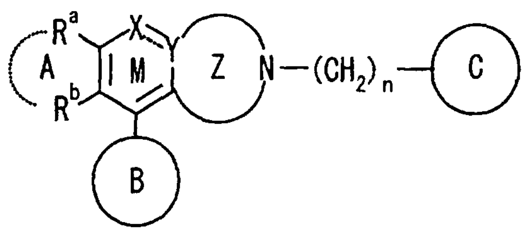

[wherein the ring M is a heterocylic ring having -N=C<, -CO-N< or -CS-N< as a partial structure —X=Y<;
$R^a$ and $R^b$ are bound to each other to form the ring A, or they are the same or different each representing a hydrogen atom or substituent on the ring M;
the rings A and B each is an optionally substituted homocycle or heterocycle, and at least one of them is an optionally substituted heterocycle;
the ring C is an optionally substituted homocycle or heterocycle;
the ring Z is an optionally substituted nitrogen-containing heterocycle; and
n is an integer of 1 to 6]

or a salt thereof and a drug having an emetic action.

**38.** Use of the compound (I) represented by the formula:

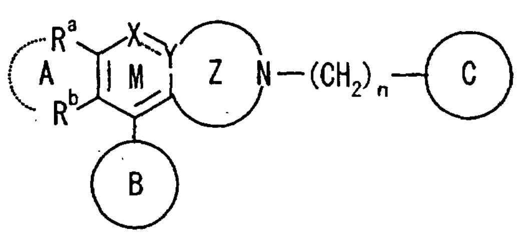

[wherein the ring M is a heterocylic ring having -N=C<, -CO-N< or -CS-N< as a partial structure —X=Y<;
$R^a$ and $R^b$ are bound to each other to form the ring A, or they are the same or different each representing a hydrogen atom or substituent on the ring M;
the rings A and B each is an optionally substituted homocycle or heterocycle, and at least one of them is an optionally substituted heterocycle;
the ring C is an optionally substituted homocycle or heterocycle;
the ring Z is an optionally substituted nitrogen-containing heterocycle; and

n is an integer of 1 to 6]

or a salt thereof for preparing a drug for inhibiting vomiting caused by a drug having an emetic action.

**39.** A method for inhibiting vomiting caused by a drug having an emetic action, which comprises administering the compound (I) represented by the formula:

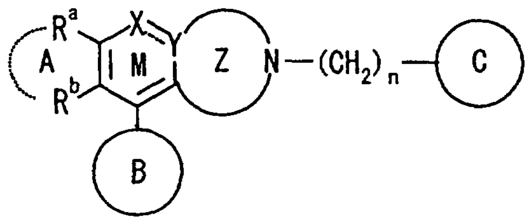

[wherein the ring M is a heterocylic ring having -N=C<, -CO-N< or -CS-N< as a partial structure -X=Y<;
$R^a$ and $R^b$ are bound to each other to form the ring A, or they are the same or different each representing a hydrogen atom or substituent on the ring M;
the rings A and B each is an optionally substituted homocycle or heterocycle, and at least one of them is an optionally substituted heterocycle;
the ring C is an optionally substituted homocycle or heterocycle;
the ring Z is an optionally substituted nitrogen-containing heterocycle; and
n is an integer of 1 to 6]

or a salt thereof to a mammal to whom said drug having an emetic action will be administered or has been administered.

Fig. 1

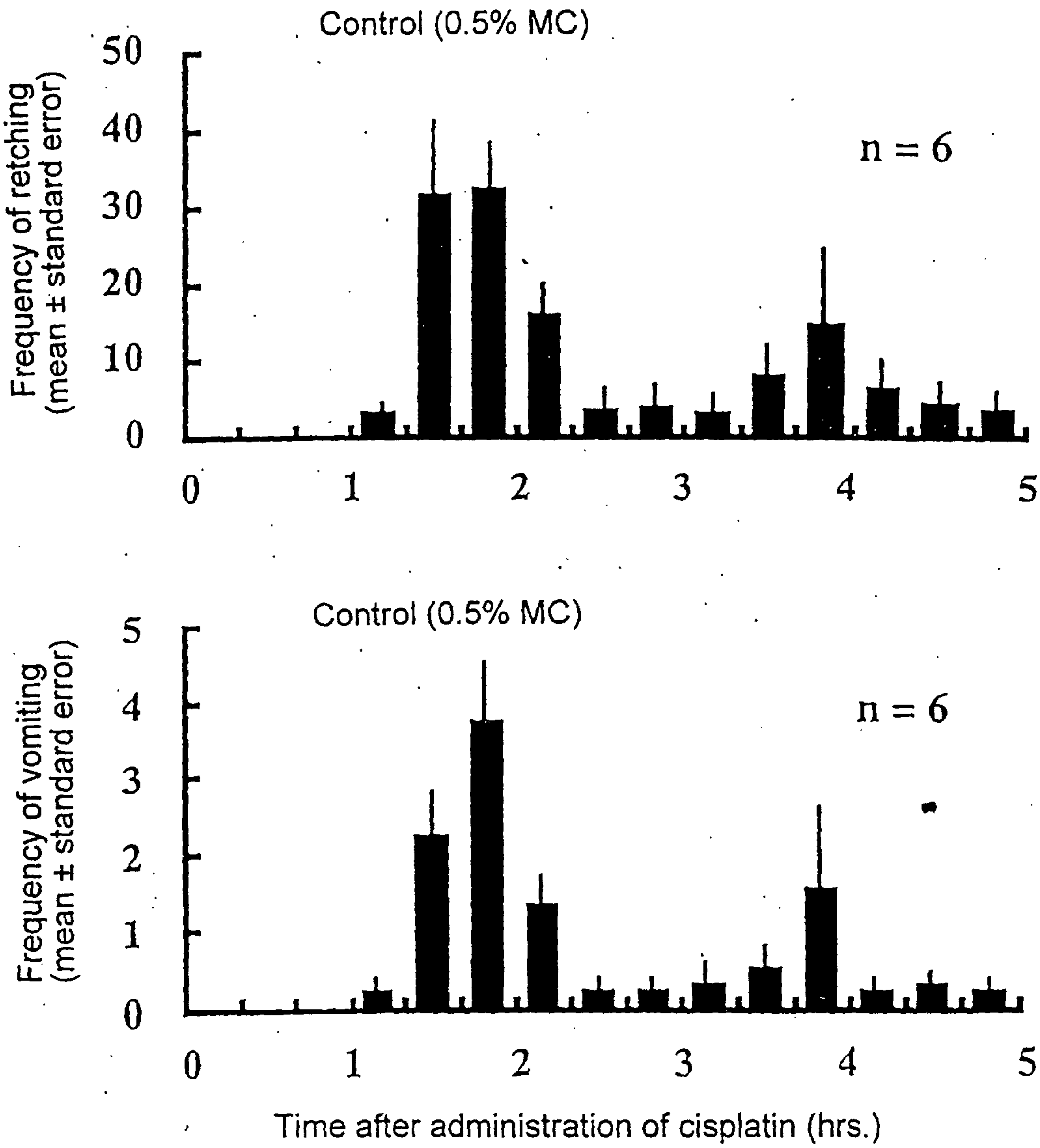
Control (0.5% MC)
50
40
30
20
10
0
Frequency of retching
(mean ± standard error)
n = 6
0
1
2
3
4
5
Control (0.5% MC)
5
4
3
2
1
0
Frequency of vomiting
(mean ± standard error)
n = 6
0
1
2
3
4
5
Time after administration of cisplatin (hrs.)

Fig. 2

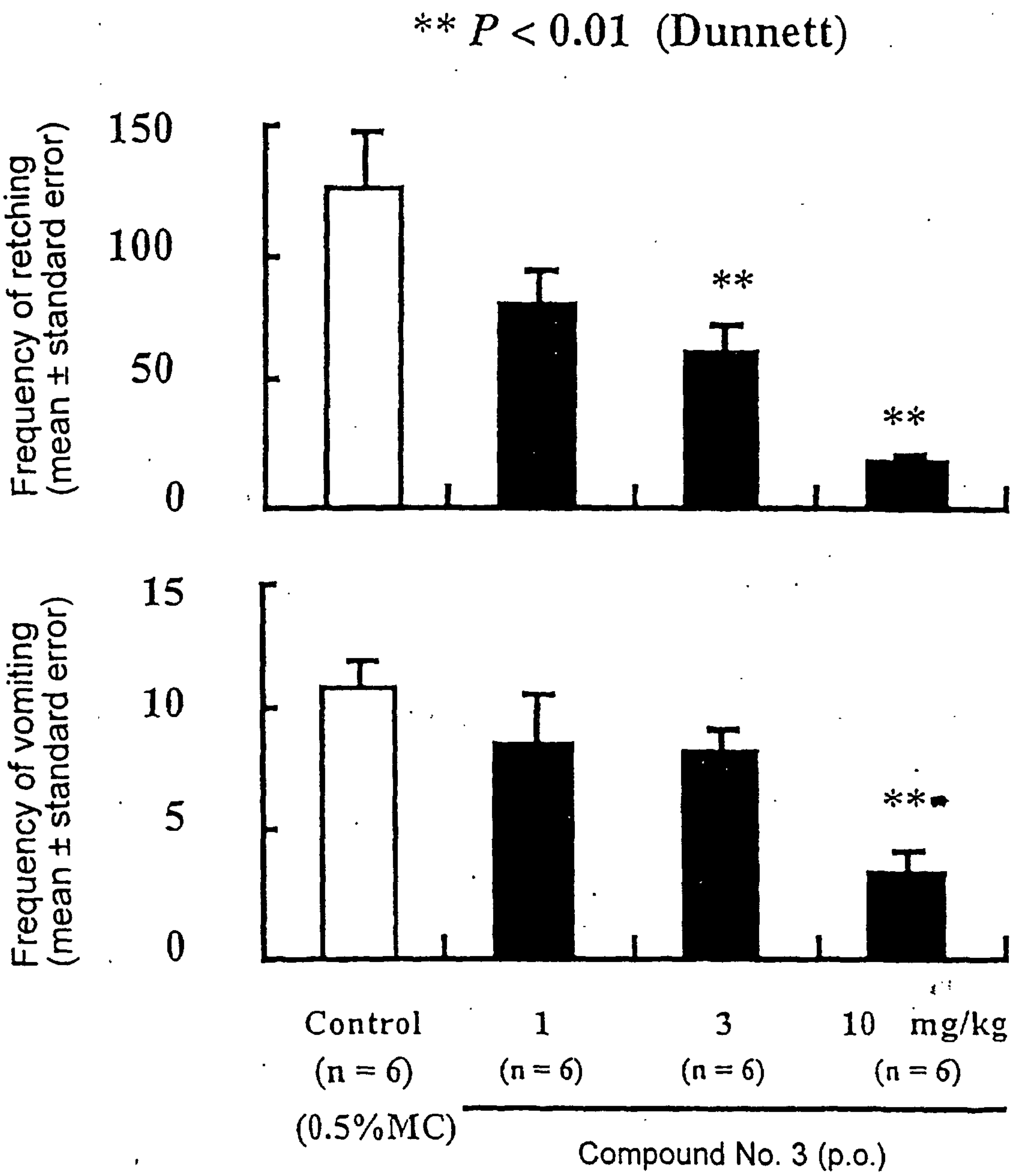
** P < 0.01 (Dunnett)
Frequency of retching (mean ± standard error)
150
100
50
0
**
**
Frequency of vomiting (mean ± standard error)
15
10
5
0
**
Control
(n = 6)
(0.5%MC)
1
(n = 6)
3
(n = 6)
10 mg/kg
(n = 6)
Compound No. 3 (p.o.)

Fig. 3

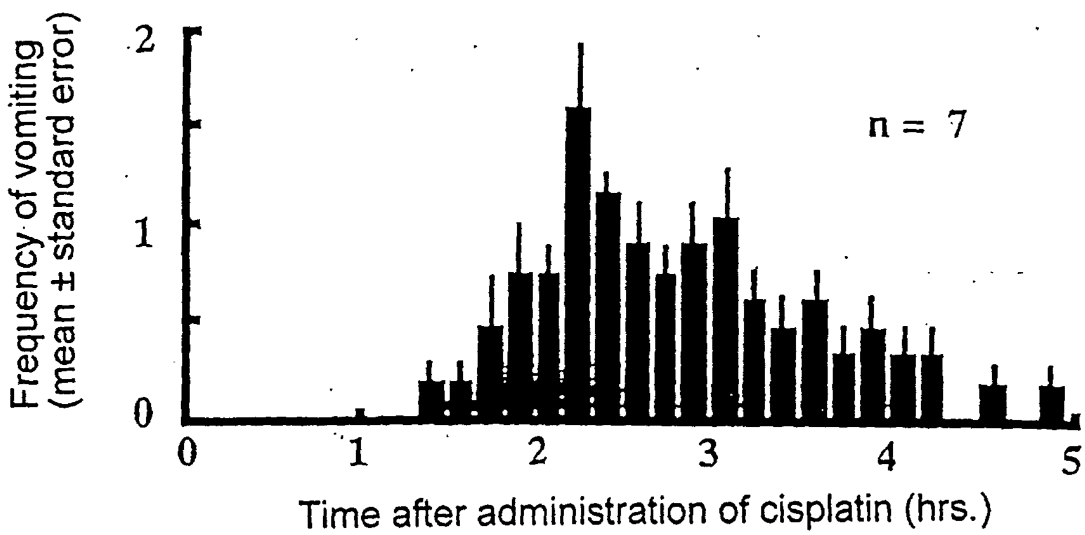


Fig. 4

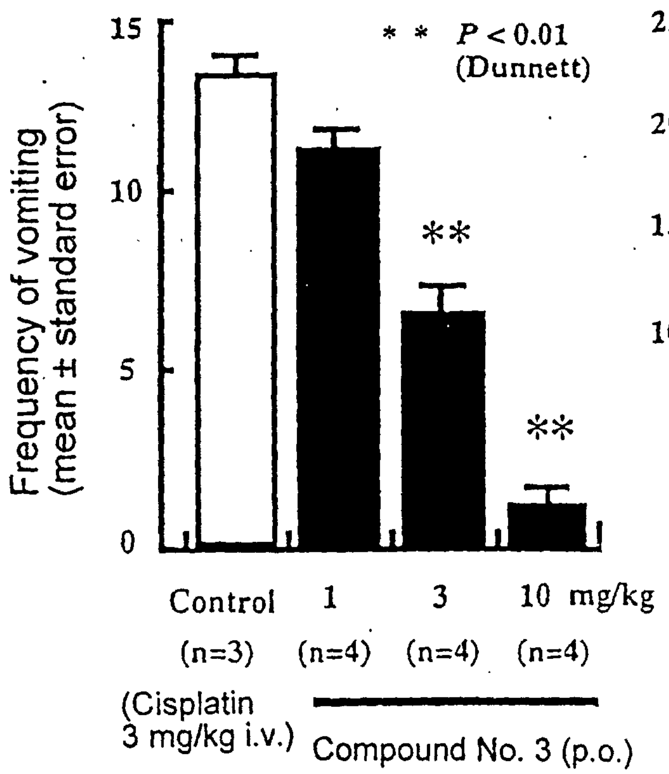


Fig. 5

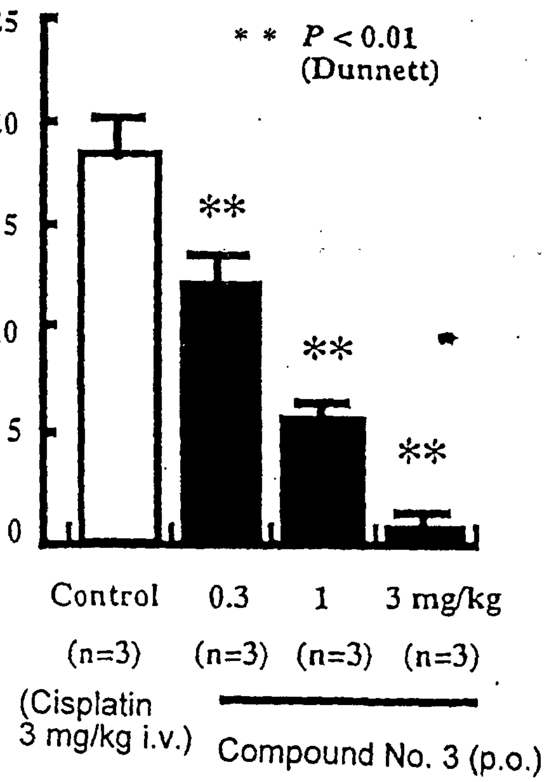

Fig. 6

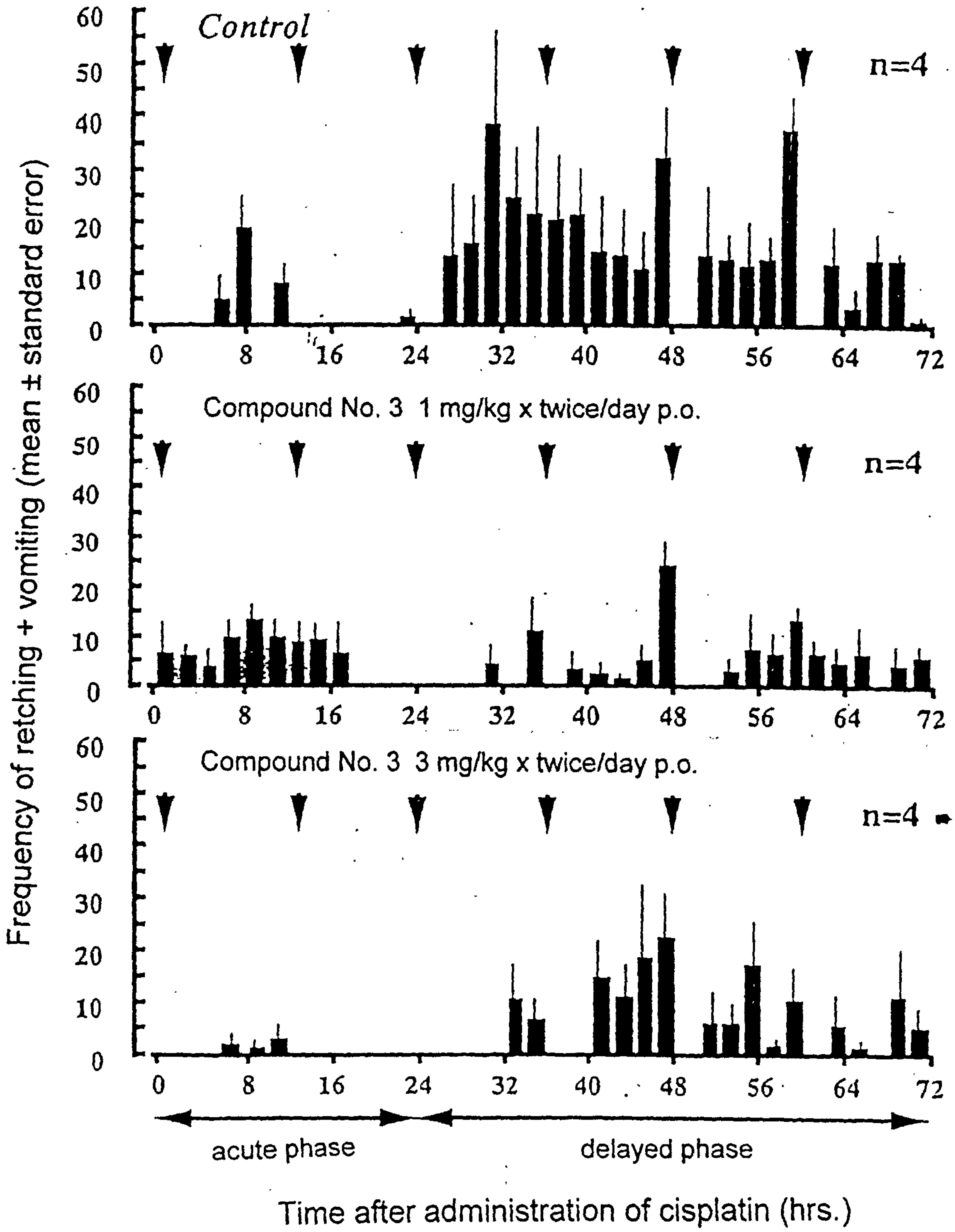
Frequency of retching + vomiting (mean ± standard error)
Control
n=4
Compound No. 3 1 mg/kg x twice/day p.o.
n=4
Compound No. 3 3 mg/kg x twice/day p.o.
n=4
0 8 16 24 32 40 48 56 64 72
acute phase
delayed phase
Time after administration of cisplatin (hrs.)

Fig. 7

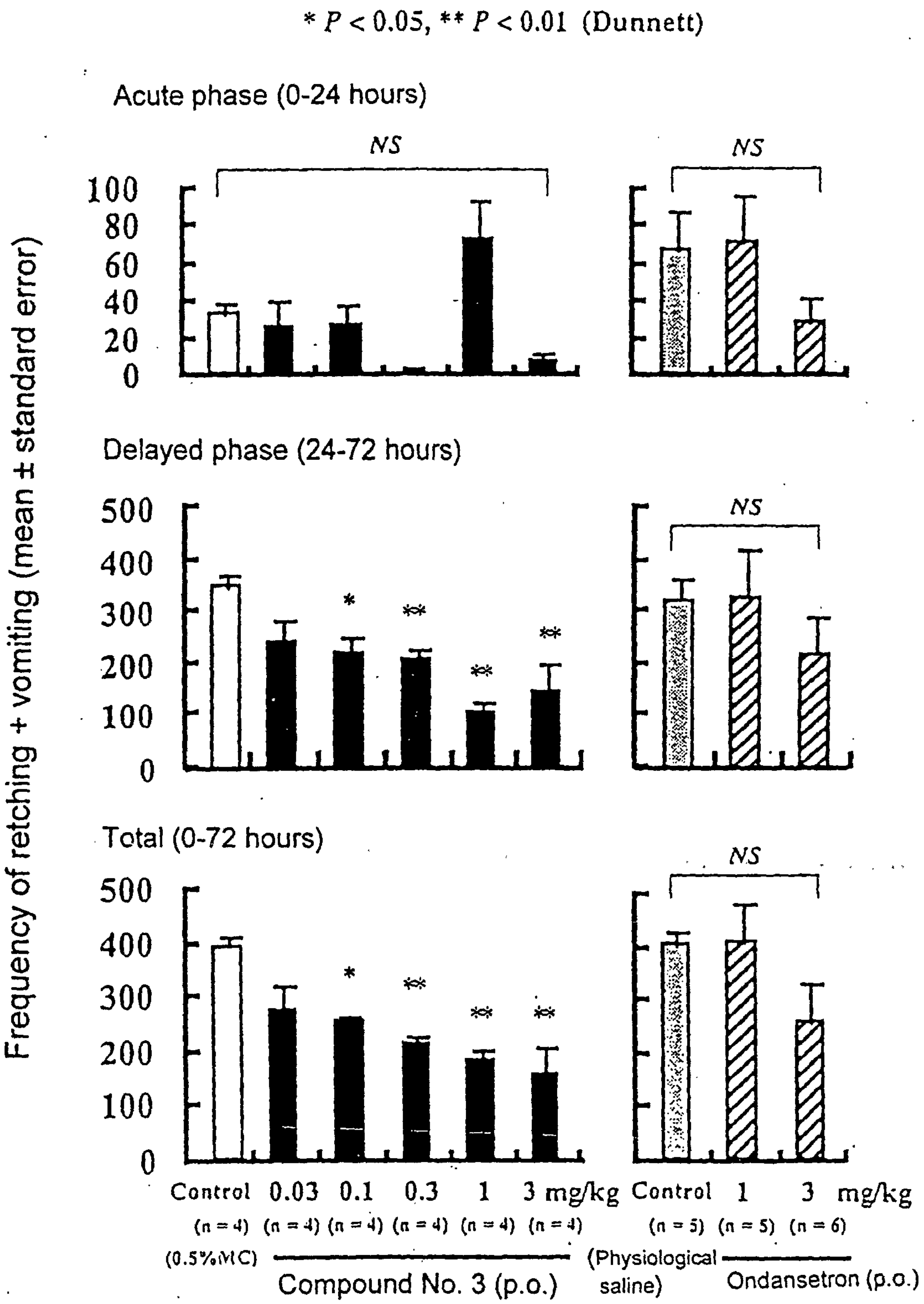
* P < 0.05, ** P < 0.01 (Dunnett)
Acute phase (0-24 hours)
NS
NS
100
80
60
40
20
0
Frequency of retching + vomiting (mean ± standard error)
Delayed phase (24-72 hours)
500
400
300
200
100
0
*
**
**
**
NS
Total (0-72 hours)
NS
500
400
300
200
100
0
*
**
**
**
Control 0.03 0.1 0.3 1 3 mg/kg Control 1 3 mg/kg
(n = 4) (n = 4) (n = 4) (n = 4) (n = 4) (n = 4) (n = 5) (n = 5) (n = 6)
(0.5%MC)
Compound No. 3 (p.o.)
(Physiological saline)
Ondansetron (p.o.)

Fig. 8

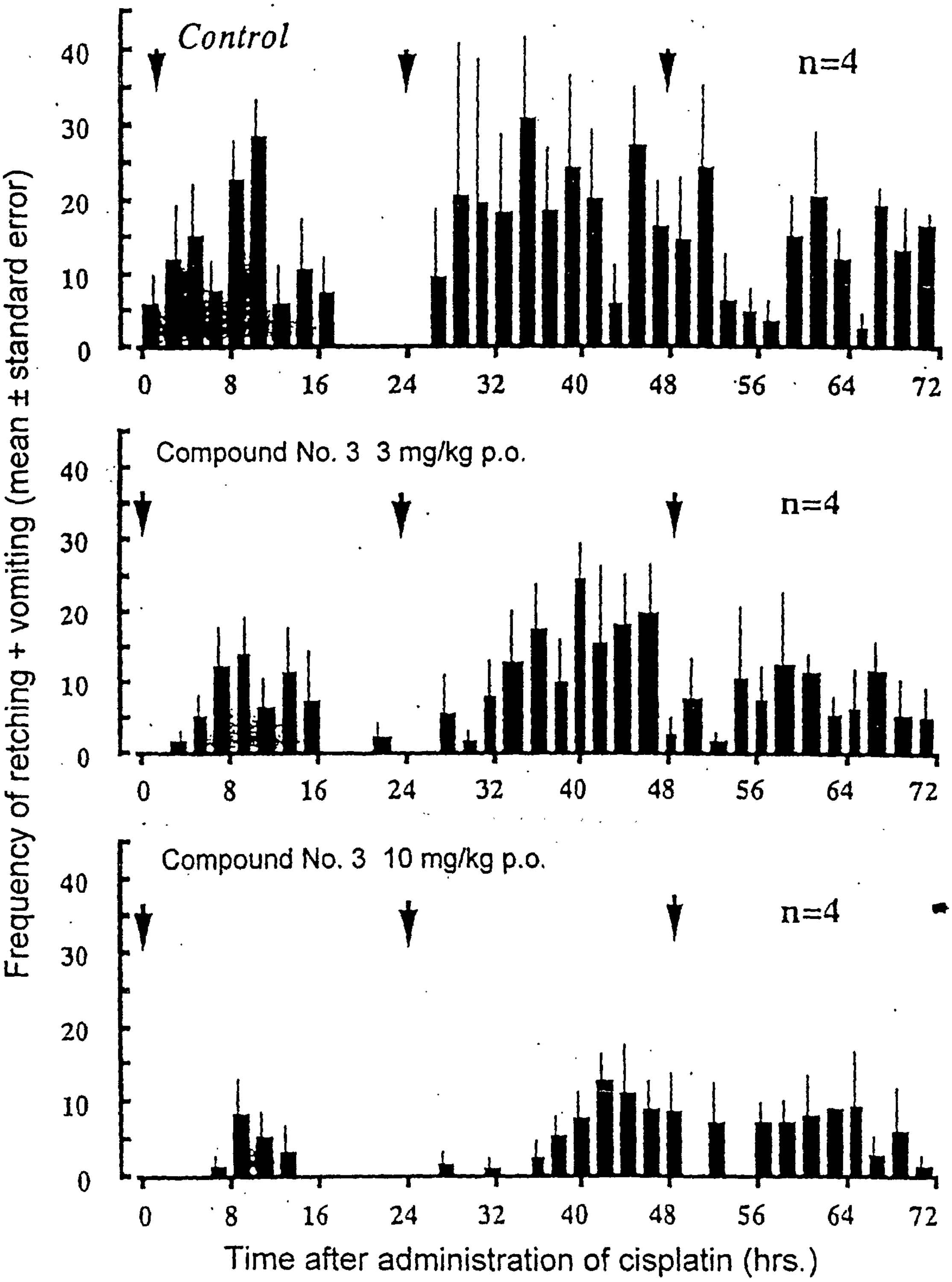
Control
n=4
Compound No. 3 3 mg/kg p.o.
n=4
Compound No. 3 10 mg/kg p.o.
n=4
Frequency of retching + vomiting (mean ± standard error)
Time after administration of cisplatin (hrs.)
0
10
20
30
40
0
8
16
24
32
40
48
56
64
72

Fig. 9

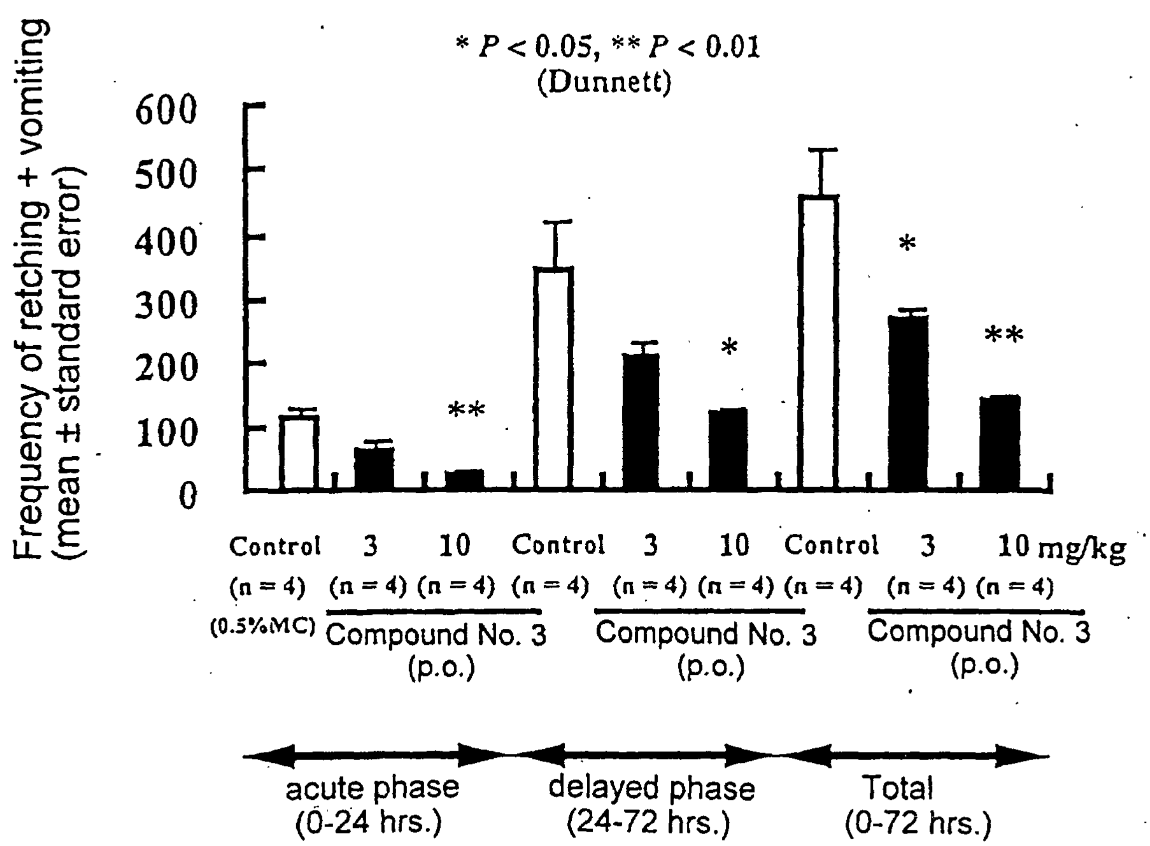
* P < 0.05, ** P < 0.01
(Dunnett)
Frequency of retching + vomiting
(mean ± standard error)
600
500
400
300
200
100
0
**
*
*
**
Control 3 10 Control 3 10 Control 3 10 mg/kg
(n = 4) (n = 4) (n = 4) (n = 4) (n = 4) (n = 4) (n = 4) (n = 4) (n = 4)
(0.5%MC)
Compound No. 3
(p.o.)
Compound No. 3
(p.o.)
Compound No. 3
(p.o.)
acute phase
(0-24 hrs.)
delayed phase
(24-72 hrs.)
Total
(0-72 hrs.)

Fig. 10

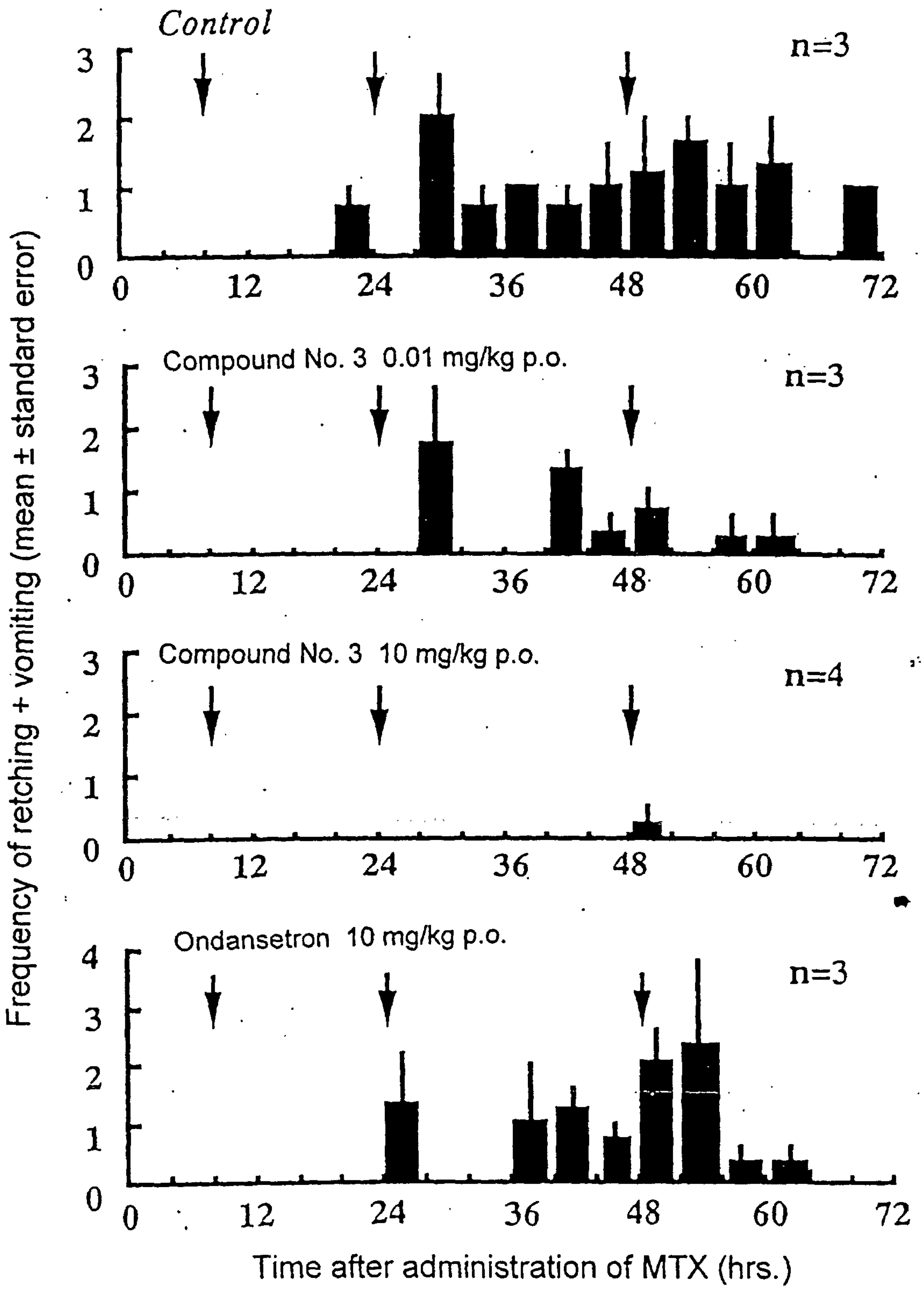
Control
n=3
Compound No. 3 0.01 mg/kg p.o.
n=3
Compound No. 3 10 mg/kg p.o.
n=4
Ondansetron 10 mg/kg p.o.
n=3
Frequency of retching + vomiting (mean ± standard error)
0
1
2
3
4
0
12
24
36
48
60
72
Time after administration of MTX (hrs.)

Fig. 11

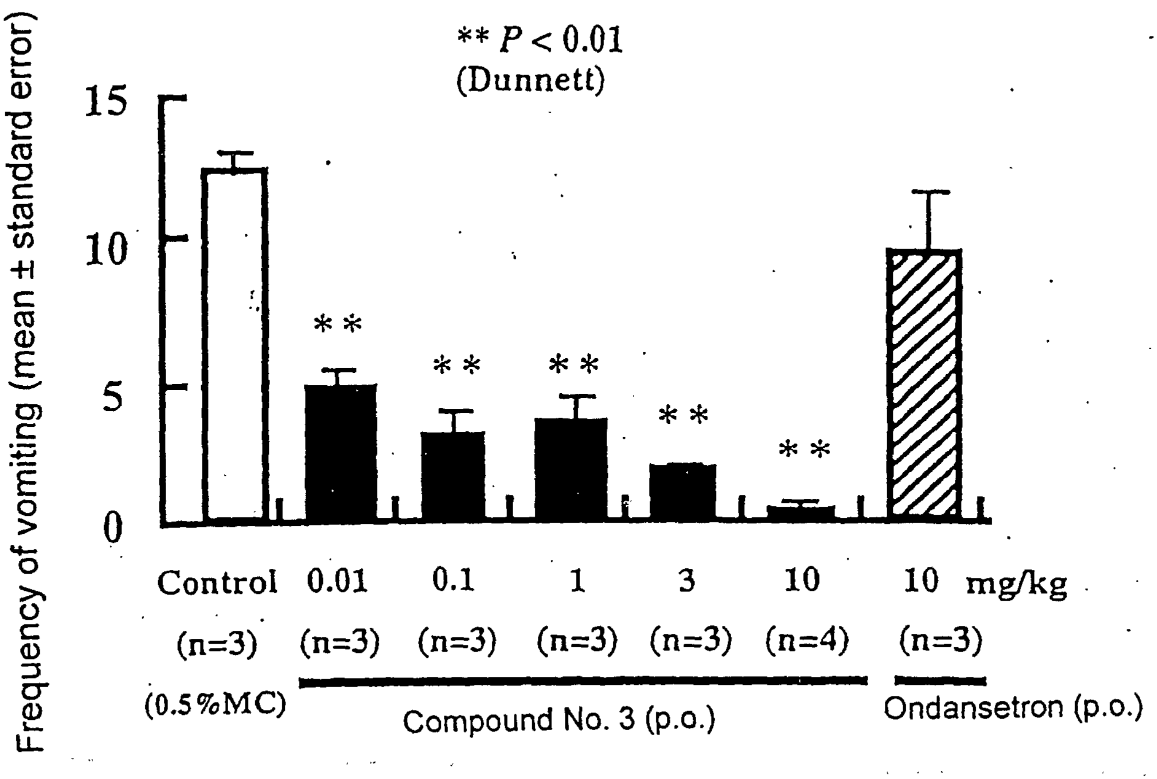
** P < 0.01
(Dunnett)
Frequency of vomiting (mean ± standard error)
15
10
5
0
**
**
**
**
**
Control
(n=3)
(0.5%MC)
0.01
(n=3)
0.1
(n=3)
1
(n=3)
3
(n=3)
10
(n=4)
Compound No. 3 (p.o.)
10 mg/kg
(n=3)
Ondansetron (p.o.)

Fig. 12

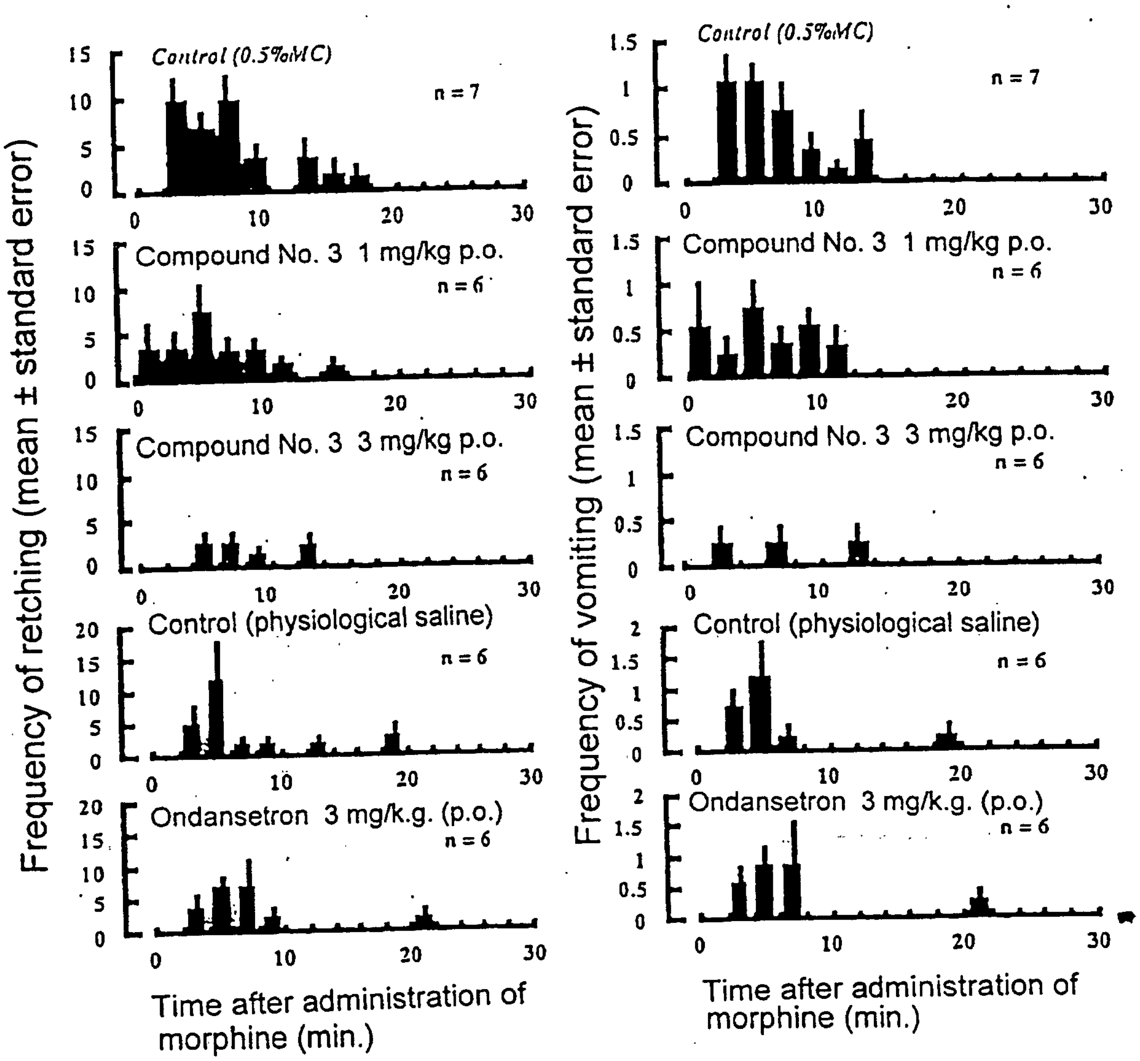

Frequency of retching (mean ± standard error)
Control (0.5%MC)
n = 7
Compound No. 3 1 mg/kg p.o.
n = 6
Compound No. 3 3 mg/kg p.o.
n = 6
Control (physiological saline)
n = 6
Ondansetron 3 mg/k.g. (p.o.)
n = 6
Time after administration of morphine (min.)
Frequency of vomiting (mean ± standard error)
Control (0.5%MC)
n = 7
Compound No. 3 1 mg/kg p.o.
n = 6
Compound No. 3 3 mg/kg p.o.
n = 6
Control (physiological saline)
n = 6
Ondansetron 3 mg/k.g. (p.o.)
n = 6
Time after administration of morphine (min.)

Fig. 13

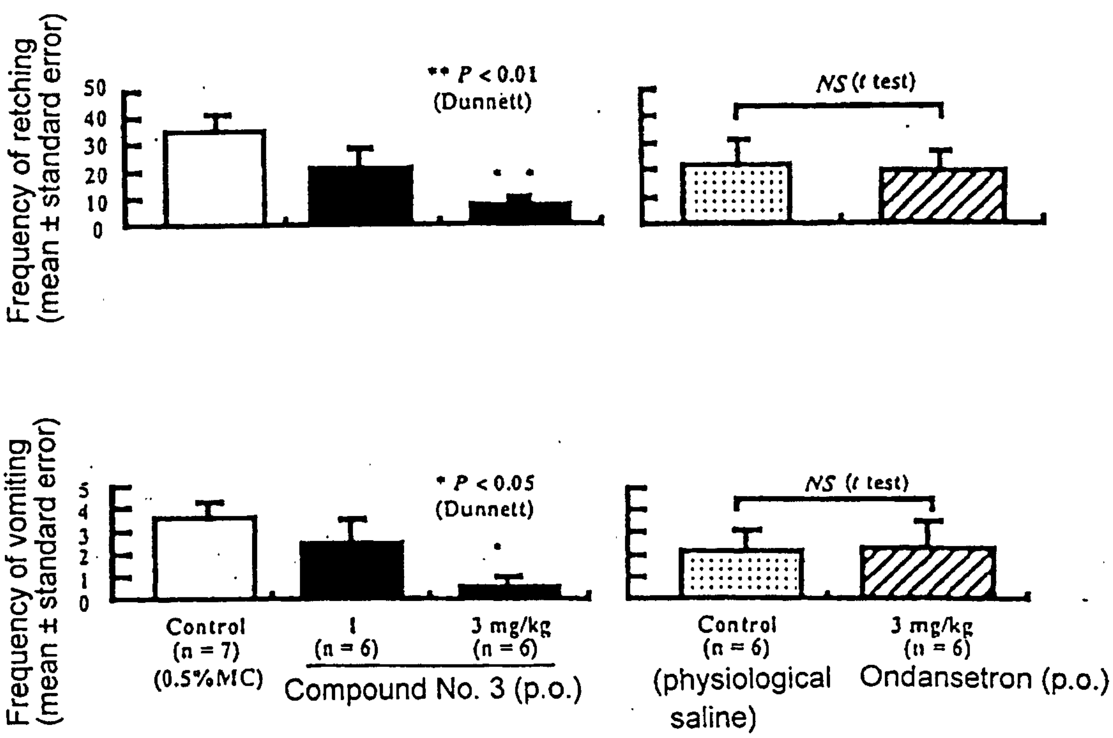
Frequency of retching
(mean ± standard error)
50
40
30
20
10
0
** P < 0.01
(Dunnett)
NS (t test)
Frequency of vomiting
(mean ± standard error)
5
4
3
2
1
0
* P < 0.05
(Dunnett)
NS (t test)
Control
(n = 7)
(0.5%MC)
1
(n = 6)
3 mg/kg
(n = 6)
Compound No. 3 (p.o.)
Control
(n = 6)
(physiological saline)
3 mg/kg
(n = 6)
Ondansetron (p.o.)

Fig. 14

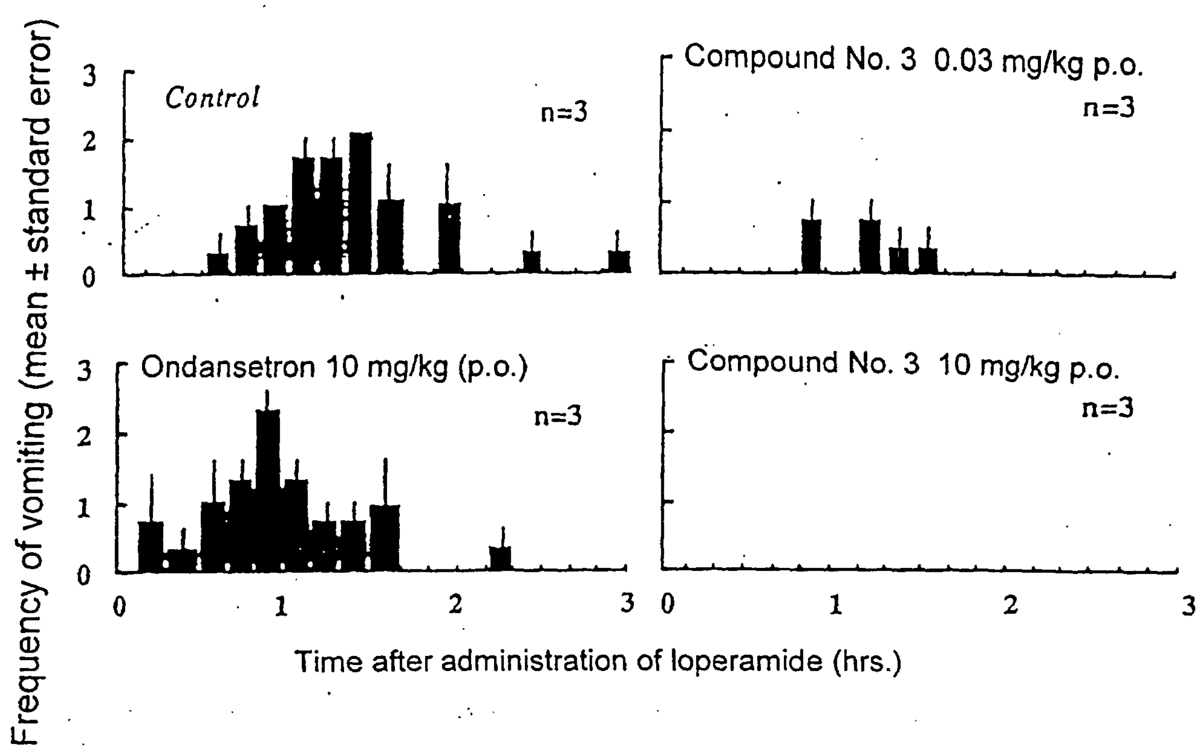
Frequency of vomiting (mean ± standard error)
Control
n=3
Compound No. 3 0.03 mg/kg p.o.
n=3
Ondansetron 10 mg/kg (p.o.)
n=3
Compound No. 3 10 mg/kg p.o.
n=3
0
1
2
3
Time after administration of loperamide (hrs.)

Fig. 15

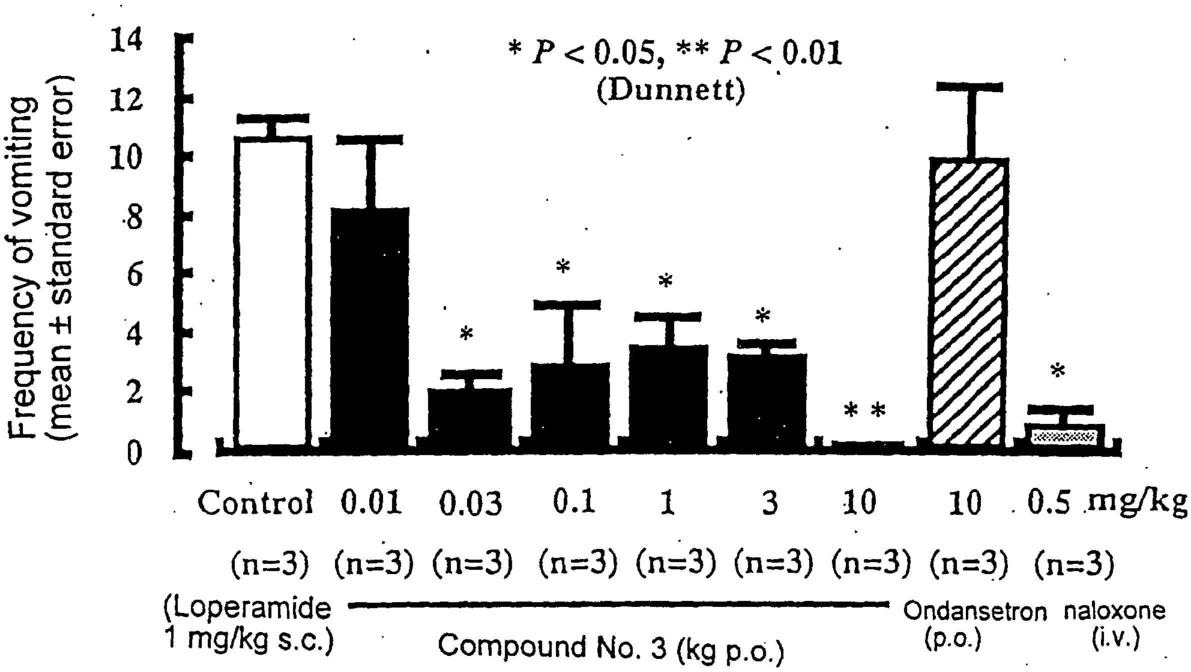
Frequency of vomiting
(mean ± standard error)
14
12
10
8
6
4
2
0
* P < 0.05, ** P < 0.01
(Dunnett)
*
*
*
*
*
* *
*
Control
0.01
0.03
0.1
1
3
10
10
0.5
mg/kg
(n=3)
(n=3)
(n=3)
(n=3)
(n=3)
(n=3)
(n=3)
(n=3)
(n=3)
(Loperamide
1 mg/kg s.c.)
Compound No. 3 (kg p.o.)
Ondansetron
(p.o.)
naloxone
(i.v.)

Fig. 16

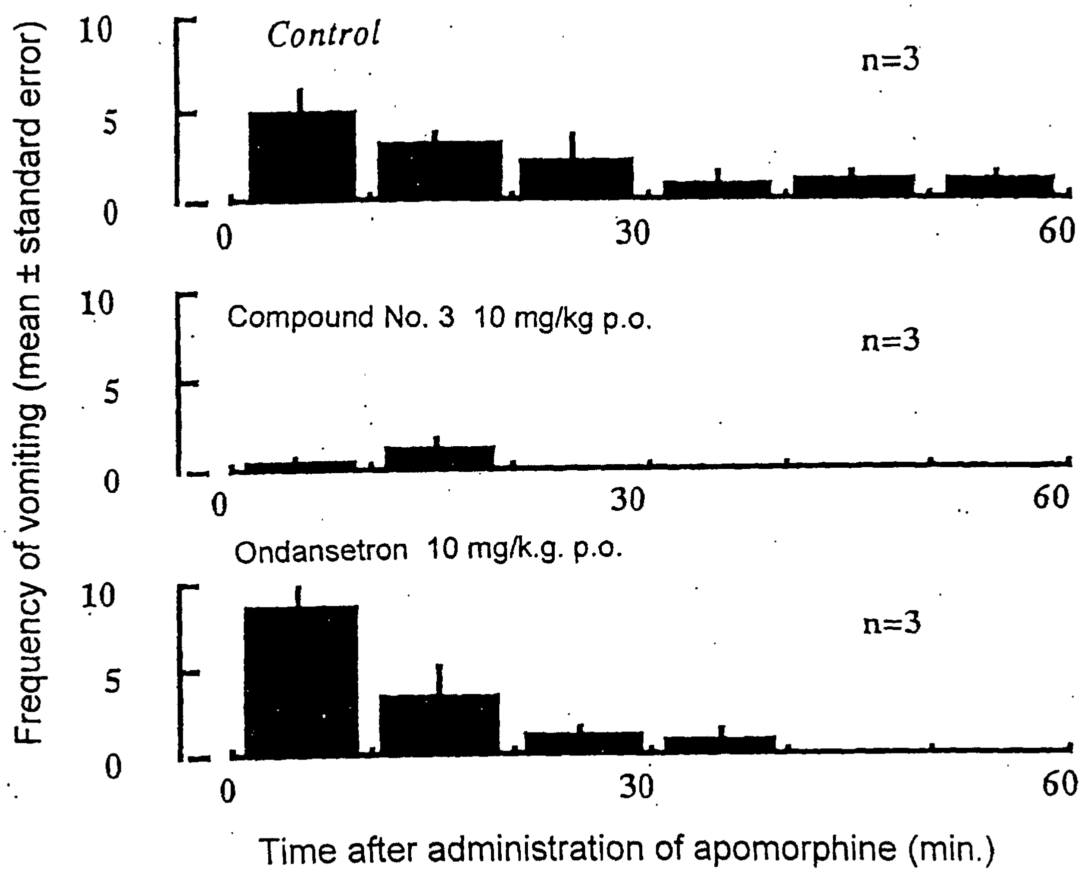
Frequency of vomiting (mean ± standard error)
10
5
0
Control
n=3
0
30
60
10
5
0
Compound No. 3 10 mg/kg p.o.
n=3
0
30
60
Ondansetron 10 mg/k.g. p.o.
10
5
0
n=3
0
30
60
Time after administration of apomorphine (min.)

Fig. 17

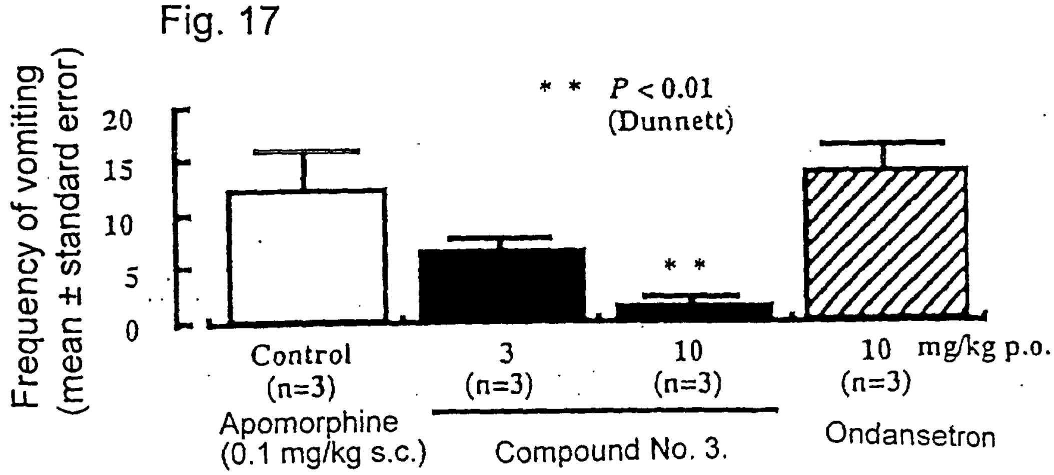
Frequency of vomiting (mean ± standard error)
20
15
10
5
0
* * P < 0.01
(Dunnett)
* *
Control
(n=3)
3
(n=3)
10
(n=3)
10 mg/kg p.o.
(n=3)
Apomorphine
(0.1 mg/kg s.c.)
Compound No. 3.
Ondansetron

Fig. 18

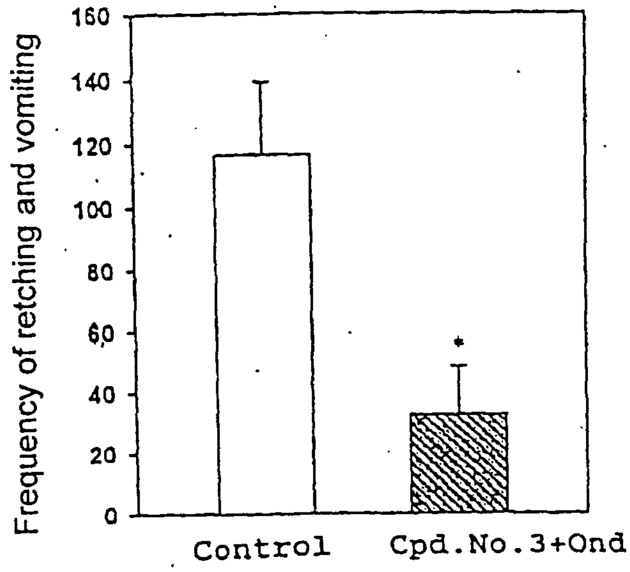
Frequency of retching and vomiting
160
140
120
100
80
60
40
20
0
*
Control
Cpd.No.3+Ond

Fig. 19

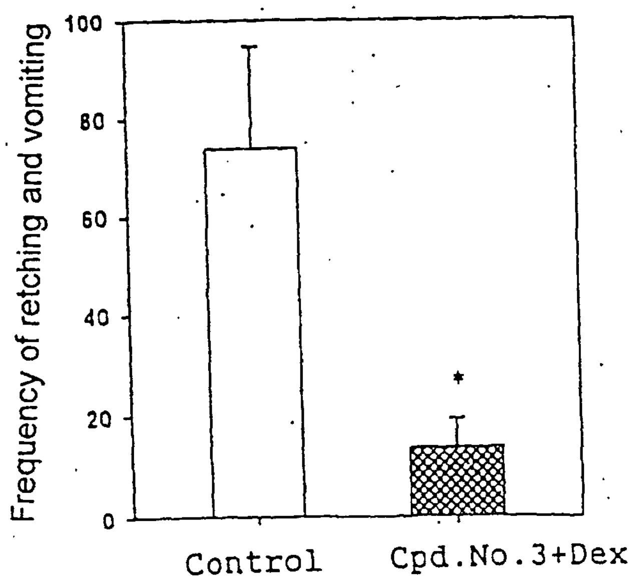
Frequency of retching and vomiting
100
80
60
40
20
0
*
Control
Cpd.No.3+Dex

Fig. 20

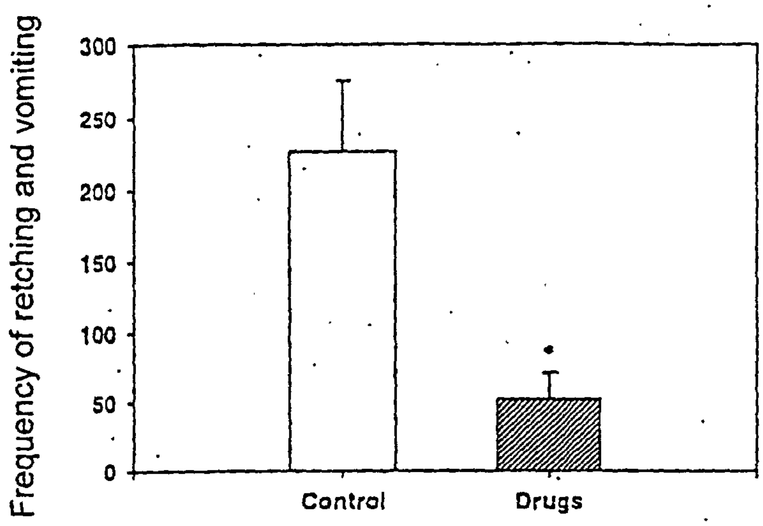
Frequency of retching and vomiting
300
250
200
150
100
50
0
Control
Drugs
*

Fig. 21

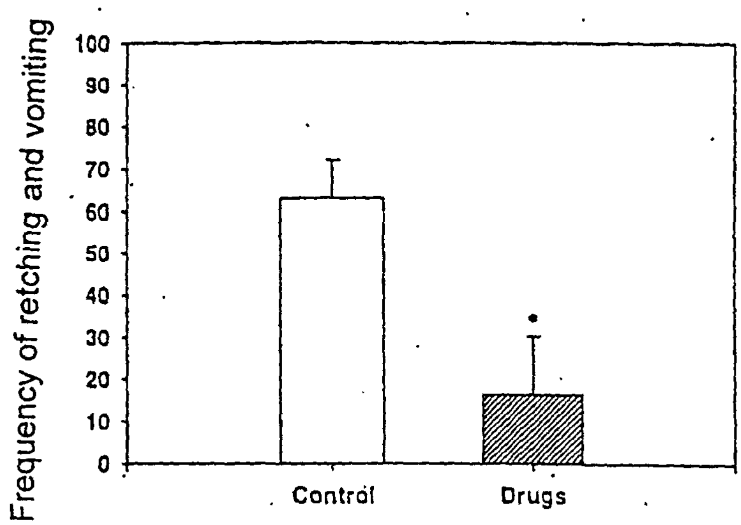
Frequency of retching and vomiting
100
90
80
70
60
50
40
30
20
10
0
Control
Drugs
*

## INTERNATIONAL SEARCH REPORT

International application No.
PCT/JP99/06569

A. CLASSIFICATION OF SUBJECT MATTER
Int.Cl[7] A61K31/4375, A61K31/4985, A61K45/00, A61P1/08

According to International Patent Classification (IPC) or to both national classification and IPC

B. FIELDS SEARCHED

Minimum documentation searched (classification system followed by classification symbols)
Int.Cl[7] A61K31/4375, A61K31/4985, A61K45/00, A61P1/08

Documentation searched other than minimum documentation to the extent that such documents are included in the fields searched

Electronic data base consulted during the international search (name of data base and, where practicable, search terms used)
CAS(STN),MEDLINE

C. DOCUMENTS CONSIDERED TO BE RELEVANT

| Category* | Citation of document, with indication, where appropriate, of the relevant passages | Relevant to claim No. |
|---|---|---|
| Y | EP, 733632, A1 (TAKEDA CHEM. IND. LTD.), 25 September, 1996 (25.09.96), Claims & JP, 9-263585, A | 1-38 |
| Y | EP, 533280, A1 (GLAXO GROUP LTD.), 24 March, 1993 (24.03.93), Claims & JP, 6-107563, A & US, 5360820, A | 1-38 |
| Y | EP, 627221, A1 (PFIZER INC.), 07 December, 1994 (07.12.94), Claims & JP, 7-53362, A & US, 5393762, A | 1-38 |
| Y | EP, 615751, A1 (GLAXO GROUP LTD.), 21 September, 1994 (21.09.94), Claims & JP, 7-2658, A & US, 5547964, A | 1-38 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 March, 2000 (06.03.00) | 14 March, 2000 (14.03.00) |
| Name and mailing address of the ISA/ Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP99/06569

**Box I Observations where certain claims were found unsearchable (Continuation of item 1 of first sheet)**

This international search report has not been established in respect of certain claims under Article 17(2)(a) for the following reasons:

1. ☒ Claims Nos.: 39
because they relate to subject matter not required to be searched by this Authority, namely:

The subject matter of claim 39 includes a method for treatment of the human body by therapy or operation, which does not require an international search report by this International Search Authority in accordance with PCT Article 17(2) (a)(i) and Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)